# EUROPEAN PATENT APPLICATION

(11) **EP 4 834 905 A1**
(43) Date of publication of application: **04.11.2026**
(21) Application number: 24911552.8
(22) Date of filing: 27.12.2024
(51) Int. Cl.: C07D 487/04

(54) **CRYSTAL FORM OF ACID SALT OF SULFOXIMINE COMPOUND, PREPARATION METHOD THEREFOR, AND USE THEREOF**

(30) Priority: 29.12.2023 CN 202311870772
(71) Applicant: Kinoteck Therapeutics Co., Ltd, Guangzhou, Guangdong 510630 (CN)
(72) Inventor: CHANG, Shaohua, Guangzhou, Guangdong 510630 (CN); LEI, Sijun, Guangzhou, Guangdong 510630 (CN); CHEN, Xiaofei, Guangzhou, Guangdong 510630 (CN); LI, Xueqiang, Guangzhou, Guangdong 510630 (CN); HU, Jian, Guangzhou, Guangdong 510630 (CN); DING, Ke, Guangzhou, Guangdong 510630 (CN); MA, Dawei, Guangzhou, Guangdong 510630 (CN); REN, Xiaomei, Guangzhou, Guangdong 510630 (CN)
(74) Representative: dompatent
(86) International application number: PCT/CN2024/143407
(87) International publication number: WO 2025/140636

(57) **Abstract**

The present invention relates to a crystal form of a sulfoximine compound, a preparation method therefor, and a use thereof. Specifically, disclosed are a crystal form of a compound as shown in a formula (I), a preparation method therefor, and a use thereof. The crystal form has excellent stability, and can be widely applied to the preparation of drugs for preventing and/or treating diseases related to increased FGFR2 activity and expression level. The crystal form is preferably a hydrochloride crystal form I of the compound represented by formula I, and the X-ray powder diffraction pattern of the hydrochloride crystal form I has a 2θ angle selected from the following group: 6.68 +/-0.2 degrees, 13.34 +/-0.2 degrees, and 21.42 +/-0.2 degrees.

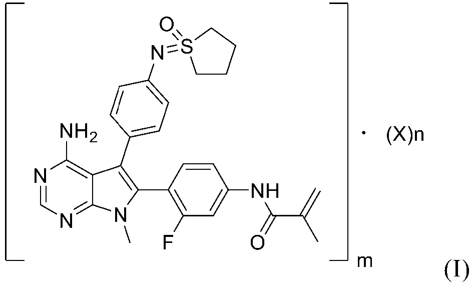

## Description

### Technical Field

The present invention relates to the field of medicinal development, specifically to a crystal form of an acid salt of a sulfoximine compound, preparation method therefor, and a use thereof.

### Background

Fibroblast growth factor receptors (FGFRs) are receptors for fibroblast growth factor (FGF) signaling, and its family consists of four members (FGFR1, FGFR2, FGFR3, and FGFR4). Each FGFR is a glycoprotein composed of an extracellular immunoglobulin (Ig)-like domain, a hydrophobic transmembrane region, and an intracellular domain that includes a tyrosine kinase domain. Binding of FGF ligands induces receptor dimerization and conformational changes in the intracellular domain, leading to intermolecular phosphorylation in the kinase domain and the intracellular tail. Phosphorylated residues serve as docking sites for adaptor proteins, which facilitate downstream signaling cascades, resulting in cellular behaviors including proliferation, survival, differentiation, migration, and angiogenesis. Abnormal FGFR signaling is implicated in various cancer types, including hepatocellular carcinoma, intrahepatic cholangiocarcinoma, bladder cancer, endometrial cancer, breast cancer, and lung cancer, and contributes to disease progression through overexpression, point mutations, and/or chromosomal translocations.

As research progresses, pan-FGFR1-3 inhibitors have demonstrated clinical responses in various FGFR-altered cancers, however, they also exhibit target-limited toxicities, leading to adverse side effects such as hyperphosphatemia and tissue mineralization, as the regulation of phosphate reabsorption is mediated by FGFR1 and FGFR3. Some studies have reported FGFR2 translocations in 14% of intrahepatic cholangiocarcinomas; FGFR2 mutations occur in 12-14% of endometrial cancers and 5% of squamous non-small cell lung cancers; FGFR2 is amplified in 12-14% of gastric cancers and 4% of breast cancers. FGFR2 contributes to the development of acquired resistance to human epidermal growth factor receptor 2 (HER2)-targeted therapy by indirectly overactivating FGFR2 in tumor-associated fibroblasts.

Therefore, given the unmet clinical needs, the development of FGFR2-selective inhibitors for treatment holds significant value and promising prospects.

### Summary of the Invention

The objective of the present invention is to develop pharmaceutically acceptable salt forms and crystal forms of a sulfoximine FGFR inhibitor compound, and a preparation method therefor.

In one aspect of the present invention, provided is a compound of formula (I), 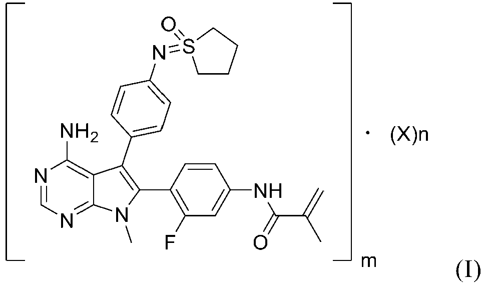 in an amorphous form or a crystal form or a solvate thereof;
wherein m is 1, 2, 3, 4, 5, 6, 7, 8, or 9;
n is 0, 0.5, 1, 1.5, 2, 2.5, or 3; and X is selected from the group consisting of: hydrochloric acid, hydrobromic acid, hydrofluoric acid, sulfuric acid, nitric acid or phosphoric acid, formic acid, acetic acid, trifluoroacetic acid, propionic acid, oxalic acid, malonic acid, succinic acid, fumaric acid, maleic acid, lactic acid, malic acid, tartaric acid, citric acid, picric acid, benzoic acid, methanesulfonic acid, ethanesulfonic acid, p-toluenesulfonic acid, benzenesulfonic acid, naphthalenesulfonic acid, hippuric acid, glycolic acid, or glutaric acid.

In another preferred embodiment, the structure of the compound of Formula I-1 is as follows: 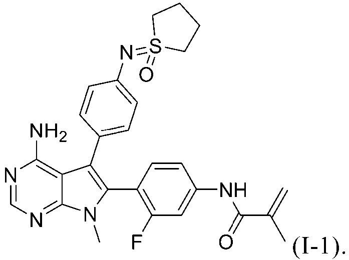 the present invention provides a crystal form A of the compound of Formula I-1, the X-ray powder diffraction pattern has characteristic diffraction peaks at the following 2θ angles: 11.59±0.2^{°}, 12.00±0.2°, 14.16±0.2°, 15.50±0.2°, 16.74±0.2°, 18.38±0.2°, 18.81±0.2°, 21.32 ± 0.2^{°}, 21.80 ± 0.2 °, 23.01 ± 0.2^{°}, 24.37 ± 0.2^{°}, 26.50 ± 0.2^{°}, 28.90 ± 0.2^{°}.

In some embodiments of the present invention, the X-ray powder diffraction pattern of the crystal form A of the compound of Formula I-1 has characteristic diffraction peaks at the following 2θ angles: 8.44±0.2°, 11.59±0.2^{°}, 12.00±0.2°, 12.34±0.2°, 14.16 ± 0.2^{°}, 15.50 ± 0.2^{°}, 16.50 ± 0.2^{°}, 16.74 ± 0.2^{°}, 18.38 ± 0.2^{°}, 18.81 ± 0.2^{°}, 19.79 ± 0.2^{°}, 20.44±0.2°, 21.32±0.2°, 21.53±0.2°, 21.80±0.2°, 22.24±0.2°, 23.01±0.2°, 24.37±0.2°, 25.43±0.2°, 26.50±0.2°, 27.13±0.2°, 27.74±0.2°, 28.90±0.2°, 29.72±0.2°, 29.93±0.2°, 30.83±0.2°, 31.56±0.2°, 32.47±0.2°, 33.64±0.2°, 34.12±0.2°, 35.03±0.2°, 36.17±0.2°.

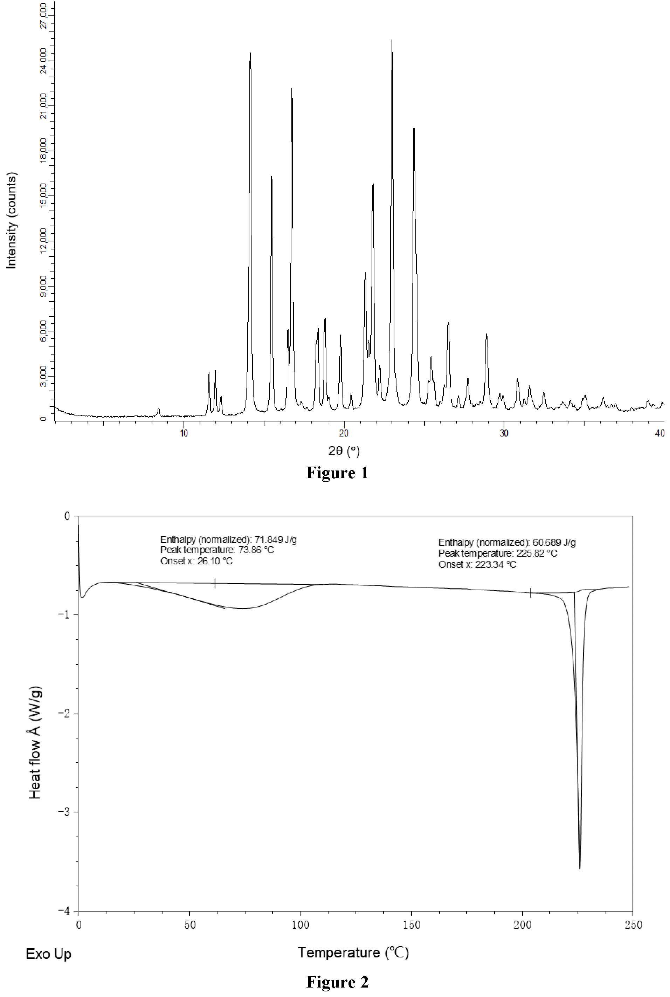

In some embodiments of the present invention, the XRPD pattern of the crystal form A of the compound of Formula I-1 is shown in Figure 1.

**Table 1 XRPD analysis data for the crystal form A of the compound of Formula I-1**

| Name | Angle | d Value | Net Intensity | Gross Intensity | Rel. Intensity | FWHM |
|---|---|---|---|---|---|---|
| Peak#1 | 8.443401 | 10.46377 | 465.3955 | 776.6761 | 0.02581691 | 0.100 |
| Peak#2 | 11.59481 | 7.625884 | 1981.868 | 2352.141 | 0.1099402 | 0.100 |
| Peak#3 | 12.00092 | 7.368722 | 2054.35 | 2439.293 | 0.113961 | 0.100 |
| Peak#4 | 12.33819 | 7.168041 | 863.7459 | 1256.884 | 0.04791462 | 0.100 |
| Peak#5 | 14.16416 | 6.247813 | 16,581.67 | 17,117.91 | 0.9198356 | 0.100 |
| Peak#6 | 15.49514 | 5.714016 | 11,020.04 | 11,669.23 | 0.6113151 | 0.100 |
| Peak#7 | 16.50191 | 5.367605 | 3768.419 | 4467.886 | 0.2090457 | 0.121 |
| Peak#8 | 16.74475 | 5.290301 | 15,225.11 | 15,931.87 | 0.8445831 | 0.100 |
| Peak#9 | 18.3777 | 4.823748 | 3973.455 | 4712.12 | 0.2204197 | 0.133 |
| Peak#10 | 18.81417 | 4.712814 | 4317.971 | 5066.824 | 0.239531 | 0.100 |
| Peak#11 | 19.79016 | 4.482541 | 3621.8 | 4405.136 | 0.2009123 | 0.100 |
| Peak#12 | 20.44199 | 4.341054 | 784.3597 | 1607.157 | 0.04351082 | 0.100 |
| Peak#13 | 21.32422 | 4.163405 | 6370.541 | 7270.211 | 0.3533933 | 0.102 |
| Peak#14 | 21.52857 | 4.124347 | 4452.761 | 5370.366 | 0.2470082 | 0.102 |
| Peak#15 | 21.79978 | 4.073646 | 10,626.56 | 11,565.94 | 0.5894879 | 0.100 |
| Peak#16 | 22.24138 | 3.99375 | 2008.293 | 2978.074 | 0.1114061 | 0.100 |
| Peak#17 | 23.00732 | 3.862497 | 18,026.77 | 19,034.55 | 1 | 0.100 |
| Peak#18 | 24.37473 | 3.648822 | 13,513.07 | 14,542.2 | 0.749611 | 0.132 |
| Peak#19 | 25.43489 | 3.499086 | 2514.506 | 3519.17 | 0.1394873 | 0.151 |
| Peak#20 | 26.50102 | 3.360687 | 3923.504 | 4867.41 | 0.2176488 | 0.104 |
| Peak#21 | 27.13202 | 3.283944 | 634.6851 | 1535.413 | 0.03520792 | 0.100 |
| Peak#22 | 27.73726 | 3.213646 | 1539.967 | 2427.688 | 0.08542667 | 0.100 |
| Peak#23 | 28.89928 | 3.087016 | 3747.38 | 4612.75 | 0.2078786 | 0.110 |
| Peak#24 | 29.72192 | 3.00342 | 798.2333 | 1621.757 | 0.04428044 | 0.228 |
| Peak#25 | 29.92524 | 2.983475 | 708.6496 | 1518.506 | 0.03931096 | 0.228 |
| Peak#26 | 30.83398 | 2.897586 | 1476.005 | 2267.967 | 0.08187852 | 0.106 |
| Peak#27 | 31.55616 | 2.832901 | 1184.099 | 1972.253 | 0.06568556 | 0.127 |
| Peak#28 | 32.46742 | 2.75544 | 834.8455 | 1614.822 | 0.04631143 | 0.123 |
| Peak#29 | 33.63531 | 2.662387 | 408.7598 | 1182.296 | 0.02267515 | 0.176 |
| Peak#30 | 34.12203 | 2.625516 | 495.4477 | 1258.51 | 0.02748399 | 0.109 |
| Peak#31 | 35.03429 | 2.559211 | 678.4774 | 1427.322 | 0.03763721 | 0.196 |
| Peak#32 | 36.17301 | 2.481217 | 667.2708 | 1389.91 | 0.03701555 | 0.124 |

In some embodiments of the present invention, the differential scanning calorimetry (DSC) curve of the crystal form A of the compound of Formula I-1 has the onsets of the endothermic peaks at 26.10^{°}C ± 2^{°}C and 223.34^{°}C ± 2^{°}C.

In some embodiments of the present invention, the crystal form A of the compound of Formula I-1 has a DSC pattern as shown in Figure 2.

In some embodiments of the present invention, the thermogravimetric analysis (TGA) curve of crystal form A of the compound of Formula I-1 has three small weight loss steps at 24.00^{°}C ± 2^{°}C, 110.00^{°}C ± 2^{°}C, and 200.00^{°}C ± 2^{°}C, and decomposition begins above 250.00^{°}C ± 2^{°}C.

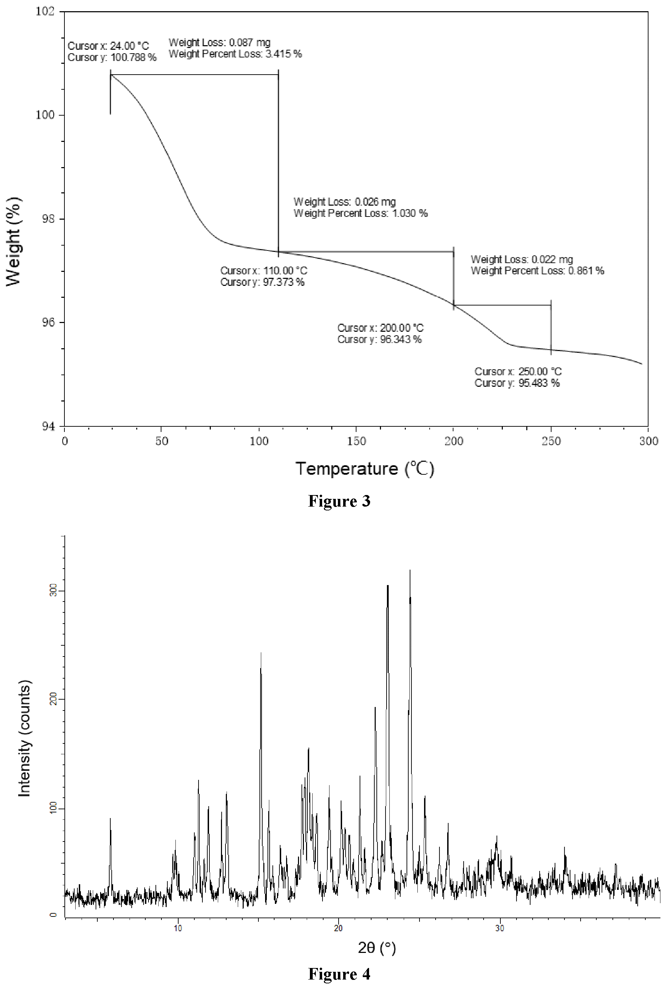

In some embodiments of the present invention, the TGA pattern of the crystal form A of the compound of Formula I-1 is as shown in Figure 3.

The present invention also provides a method for preparing the crystal form A of the compound of Formula I-1, which includes adding the compound of Formula I-1 to acetonitrile, an alcoholic solvent, an ester solvent, an ether solvent, or a mixed solvent of an alcoholic solvent and water, and then performing recrystallization or slurrying to obtain it, wherein the alcoholic solvent is selected from methanol, ethanol, isopropanol, etc., the ester solvent is selected from ethyl acetate, isopropyl acetate, methyl formate, ethyl formate, isopropyl formate, etc., the ether solvent is selected from methyl tert-butyl ether, tetrahydrofuran, ethylene glycol dimethyl ether, etc, and the mixed solvent of an alcoholic solvent and water is selected from a mixture of methanol and water, a mixture of ethanol and water, or a mixture of isopropanol and water, and in the mixed solvent of an alcoholic solvent and water, the volume ratio of the alcoholic solvent to water is selected from 1:0.1 to 1.5.

The present invention also provides the maleate crystal form A of the compound of Formula I-1, and its X-ray powder diffraction pattern has characteristic diffraction peaks at the following 2θ angles: 5.82±0.2°, 9.86±0.2°, 11.29±0.2°, 12.72 ± 0.2°, 13.04 ± 0.2°, 15.17 ± 0.2°, 17.88 + 0.2°, 18.11 ± 0.2 °, 22.28 + 0.2°, 23.02 ± 0.2°, 24.42 ± 0.2°, 25.34 ± 0.2°, 26.77 ± 0.2°.

In some embodiments of the present invention, the maleate crystal form A of the compound of Formula I-1 has characteristic diffraction peaks at the following 2θ angles in its X-ray powder diffraction pattern: 8.44±0.2°, 11.59±0.2°, 12.00±0.2°, 12.34±0.2°, 14.16 + 0.2°, 15.50 + 0.2°, 16.50 + 0.2°, 16.74 ± 0.2°, 18.38 ± 0.2°, 18.81 ± 0.2°, 19.79 ± 0.2°, 20.44±0.2°, 21.32±0.2°, 21.53±0.2°, 21.80±0.2°, 22.24±0.2°, 23.01±0.2°, 24.37±0.2°, 25.43±0.2°, 26.50±0.2°, 27.13±0.2°, 27.74±0.2°, 28.90±0.2°, 29.72±0.2°, 29.93±0.2°, 30.83±0.2°, 31.56±0.2°, 32.47±0.2°, 33.64±0.2°, 34.12 ±0.2°, 35.03±0.2° , 36.17±0.2°.

In some embodiments of the present invention, the XRPD pattern of the maleate crystal form A of the compound of Formula I-1 is shown in Figure 4.

**Table 2 XRPD analysis data for the maleate crystal form A of the compound of Formula I-1**

| Name | Angle | d Value | Net Intensity | Gross Intensity | Rel. Intensity | FWHM |
|---|---|---|---|---|---|---|
| Peak#1 | 5.816485 | 15.18233 | 49.66369 | 65.44668 | 0.2465863 | 0.100 |
| Peak#2 | 9.859919 | 8.96345 | 73.92227 | 91.99165 | 0.3670331 | 0.101 |
| Peak#3 | 11.06471 | 7.990012 | 33.00455 | 54.12584 | 0.1638716 | 0.142 |
| Peak#4 | 11.29159 | 7.829976 | 71.99976 | 93.48729 | 0.3574875 | 0.129 |
| Peak#5 | 11.64355 | 7.594069 | 28.69745 | 50.38568 | 0.1424863 | 0.133 |
| Peak#6 | 11.90079 | 7.430491 | 53.98288 | 75.53851 | 0.2680315 | 0.100 |
| Peak#7 | 12.72461 | 6.951217 | 49.88066 | 70.707 | 0.2476635 | 0.148 |
| Peak#8 | 13.0453 | 6.781051 | 55.81887 | 75.97722 | 0.2771474 | 0.168 |
| Peak#9 | 15.16811 | 5.836466 | 162.1551 | 178.0625 | 0.8051198 | 0.100 |
| Peak#10 | 15.66005 | 5.65421 | 60.71101 | 77.37029 | 0.3014376 | 0.100 |
| Peak#11 | 16.36081 | 5.413575 | 29.68445 | 47.71005 | 0.1473869 | 0.132 |
| Peak#12 | 17.70085 | 5.006646 | 96.75628 | 119.5969 | 0.4804066 | 0.122 |
| Peak#13 | 17.87988 | 4.956915 | 66.58391 | 89.8793 | 0.3305972 | 0.217 |
| Peak#14 | 18.1084 | 4.894873 | 88.23072 | 111.9404 | 0.4380763 | 0.215 |
| Peak#15 | 18.35028 | 4.830894 | 79.95369 | 103.8986 | 0.3969797 | 0.220 |
| Peak#16 | 18.61376 | 4.763102 | 50.00529 | 73.96861 | 0.2482823 | 0.100 |
| Peak#17 | 19.40942 | 4.569603 | 61.80075 | 86.36585 | 0.3068482 | 0.100 |
| Peak#18 | 20.15778 | 4.401615 | 61.40547 | 87.72269 | 0.3048856 | 0.134 |
| Peak#19 | 20.38478 | 4.353108 | 57.58932 | 84.15148 | 0.285938 | 0.162 |
| Peak#20 | 20.68509 | 4.290583 | 29.13599 | 55.74016 | 0.1446637 | 0.222 |
| Peak#21 | 21.32064 | 4.164098 | 69.45016 | 95.71144 | 0.3448285 | 0.100 |
| Peak#22 | 21.6195 | 4.107205 | 29.40524 | 56.03273 | 0.1460006 | 0.150 |
| Peak#23 | 22.28023 | 3.986875 | 117.0337 | 144.4462 | 0.5810865 | 0.100 |
| Peak#24 | 22.70711 | 3.912881 | 56.87783 | 84.4616 | 0.2824053 | 0.102 |
| Peak#25 | 23.02009 | 3.860384 | 201.405 | 228.6998 | 1 | 0.100 |
| Peak#26 | 24.42011 | 3.642144 | 192.9104 | 220.1278 | 0.9578236 | 0.110 |
| Peak#27 | 24.99754 | 3.559304 | 38.4386 | 65.54317 | 0.1908523 | 0.100 |
| Peak#28 | 25.33689 | 3.512397 | 60.09032 | 86.57373 | 0.2983557 | 0.100 |
| Peak#29 | 26.24092 | 3.393405 | 39.44109 | 63.79497 | 0.1958298 | 0.164 |
| Peak#30 | 26.77359 | 3.327089 | 56.9196 | 80.2638 | 0.2826127 | 0.100 |
| Peak#31 | 29.85257 | 2.990573 | 36.64283 | 61.98938 | 0.1819361 | 0.268 |
| Peak#32 | 34.08594 | 2.628213 | 27.72369 | 53.10707 | 0.1376515 | 0.100 |

The present invention also provides a method for preparing crystal form A of the compound of formula I-1, comprising adding the compound of formula I-1 and maleic acid to acetonitrile and dichloromethane, and then performing recrystallization or slurrying to obtain it, wherein the ratio of the compound of Formula I-1 to maleic acid is selected from 0.8 to 1.2.

The present invention also provides monomaleate crystal form B of the compound of Formula I-1, and its X-ray powder diffraction pattern has characteristic diffraction peaks at the following 2θ angles: 7.98±0.2°, 8.29 ± 0.2_{°,} 11.61 ± 0.2^{°}, 13.21 ± 0.2^{°} , 13.99 ± 0.2 °, 15.25 ± 0.2 °, 15.95 ± 0.2^{°}, 16.57 ± 0.2^{°}, 17.38 ± 0.2^{°}, 18.06 ± 0.2^{°}, 20.39 ± 0.2^{°} , 21.25 ± 0.2^{°}, 21.61 ± 0.2^{°}, 22.17 ± 0.2^{°}, 23.54±0.2°, 24.70±0.2°, 25.91±0.2°, 27.24±0.2°, 29.47±0.2°, 29.71±0.2°, 32.64±0.2°.

In some embodiments of the present invention, the X-ray powder diffraction pattern of the monomaleate crystal form B of the compound of Formula I-1 has characteristic diffraction peaks at the following 2θ angles: 7.98±0.2°, 8.29±0.2°, 10.27±0.2°, 11.61±0.2°, 13.21± 0.2°, 13.99±0.2°, 14.52±0.2°, 15.25±0.2°, 15.95±0.2°, 16.57±0.2°, 16.89±0.2°, 17.38± 0.2°, 18.06±0.2°, 20.39±0.2°, 20.61±0.2°, 21.25±0.2°, 21.61±0.2°, 22.17±0.2°, 23.31± 0.2^{°}, 23.54 ± 0.2^{°}, 23.98 ± 0.2^{°}, 24.70 ± 0.2^{°}, 24.93 ± 0.2^{°}, 25.32 ± 0.2^{°}, 25.91 ± 0.2^{°}, 26.55±0.2°, 27.24±0.2°, 28.23±0.2°, 28.54±0.2°, 29.00±0.2°, 29.47±0.2°, 29.71±0.2°, 32.64±0.2°, 35.12±0.2°.

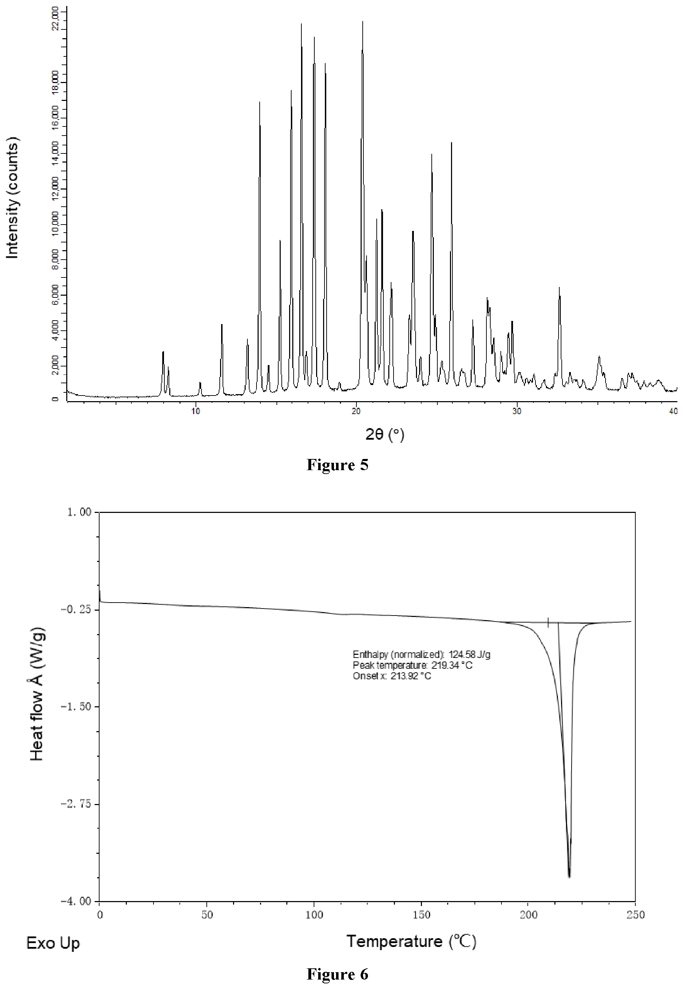

In some embodiments of the present invention, the XRPD pattern of the monomaleate crystal form B of the compound of Formula I-1 is shown in Figure 5.

**Table 3 X-ray diffraction data for the monomaleate crystal form B of the compound of Formula I-1**

| Name | Angle | d Value | Net Intensity | Gross Intensity | Rel. Intensity | FWHM |
|---|---|---|---|---|---|---|
| Peak#1 | 7.981822 | 11.06781 | 1723.857 | 1994.387 | 0.1169017 | 0.149 |
| Peak#2 | 8.291428 | 10.65522 | 1137.191 | 1412.092 | 0.07711747 | 0.164 |
| Peak#3 | 10.26916 | 8.607141 | 529.8829 | 820.8134 | 0.03593348 | 0.100 |
| Peak#4 | 11.61435 | 7.613101 | 2756.817 | 3085.549 | 0.1869509 | 0.100 |
| Peak#5 | 13.21075 | 6.696494 | 2119.543 | 2516.378 | 0.1437347 | 0.100 |
| Peak#6 | 13.98848 | 6.325882 | 11811.49 | 12269.85 | 0.8009846 | 0.100 |
| Peak#7 | 14.5199 | 6.095533 | 1061.446 | 1551.665 | 0.0719809 | 0.100 |
| Peak#8 | 15.25275 | 5.804268 | 6038.434 | 6581.751 | 0.4094904 | 0.100 |
| Peak#9 | 15.94566 | 5.553574 | 11989.22 | 12576.86 | 0.8130369 | 0.100 |
| Peak#10 | 16.57134 | 5.34527 | 14746.21 | 15361.82 | 1 | 0.100 |
| Peak#11 | 16.89114 | 5.244782 | 1575.195 | 2200.658 | 0.1068203 | 0.100 |
| Peak#12 | 17.37723 | 5.099149 | 13942.37 | 14577.09 | 0.9454884 | 0.100 |
| Peak#13 | 18.05592 | 4.908983 | 13096.95 | 13733.03 | 0.888157 | 0.100 |
| Peak#14 | 20.39062 | 4.351873 | 14023.45 | 14713.4 | 0.9509865 | 0.100 |
| Peak#15 | 20.61231 | 4.305566 | 5244.903 | 5947.155 | 0.355678 | 0.100 |
| Peak#16 | 21.24602 | 4.178554 | 7214.775 | 7944.283 | 0.4892629 | 0.100 |
| Peak#17 | 21.60538 | 4.109857 | 7723.3 | 8463.026 | 0.523748 | 0.175 |
| Peak#18 | 22.17168 | 4.006149 | 3996.755 | 4744.93 | 0.271036 | 0.100 |
| Peak#19 | 23.30505 | 3.813822 | 2970.041 | 3761.162 | 0.2014104 | 0.178 |
| Peak#20 | 23.53755 | 3.776674 | 6206.884 | 7010.807 | 0.4209137 | 0.108 |
| Peak#21 | 23.98418 | 3.707347 | 1311.471 | 2135.541 | 0.08893615 | 0.100 |
| Peak#22 | 24.70086 | 3.601379 | 9470.771 | 10314.98 | 0.642251 | 0.100 |
| Peak#23 | 24.92502 | 3.569496 | 3107.844 | 3955.256 | 0.2107554 | 0.100 |
| Peak#24 | 25.31763 | 3.515025 | 1042.744 | 1892.22 | 0.07071263 | 0.167 |
| Peak#25 | 25.91234 | 3.435686 | 10856.43 | 11700.45 | 0.736218 | 0.100 |
| Peak#26 | 26.54931 | 3.354684 | 685.3824 | 1512.089 | 0.04647854 | 0.205 |
| Peak#27 | 27.2363 | 3.271608 | 3106.794 | 3914.549 | 0.2106842 | 0.100 |
| Peak#28 | 28.22876 | 3.1588 | 2131.622 | 2937.217 | 0.1445538 | 0.224 |
| Peak#29 | 28.53832 | 3.125235 | 2016.763 | 2825.172 | 0.1367648 | 0.240 |
| Peak#30 | 28.99892 | 3.076636 | 1532.671 | 2340.075 | 0.1039366 | 0.100 |
| Peak#31 | 29.46878 | 3.028641 | 2286.111 | 3086.088 | 0.1550303 | 0.168 |
| Peak#32 | 29.7058 | 3.005013 | 3049.359 | 3843.141 | 0.2067892 | 0.153 |
| Peak#33 | 32.63929 | 2.741322 | 4630.014 | 5344.848 | 0.3139799 | 0.100 |
| Peak#34 | 35.11711 | 2.553365 | 1318.593 | 1961.014 | 0.08941906 | 0.213 |

In some embodiments of the present invention, the monomaleate crystal form B of the compound of Formula I-1 has the onset of the endothermic peak at 213.92°C ± 2°C in its differential scanning calorimetry (DSC) curve.

In some embodiments of the present invention, the monomaleate crystal form B of the compound of Formula I-1 has a DSC pattern as shown in Figure 6.

In some embodiments of the present invention, the monomaleate crystal form B of the compound of Formula I-1 has two weight loss steps at 24.00°C ± 2°C and 180.00°C ± 2°C in its thermogravimetric analysis (TGA) curve, and decomposition begins above 250.00°C ± 2°C.

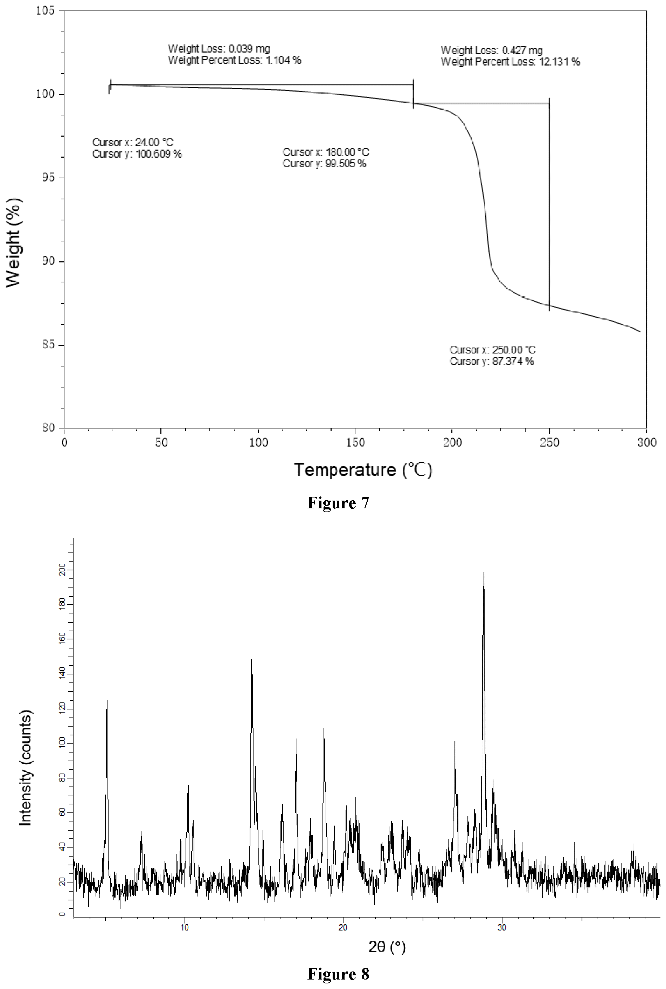

In some embodiments of the present invention, the TGA pattern of the monomaleate crystal form B of the compound of Formula I-1 is shown in Figure 7.

The present invention also provides a method for preparing the monomaleate crystal form B of the compound of Formula I-1, comprising adding the compound of Formula I-1 and maleic acid to tetrahydrofuran, and then performing recrystallization or slurrying to obtain it, wherein the ratio of the compound of Formula I-1 to maleic acid is selected from 0.8 to 1.2.

The present invention also provides the fumarate crystal form A of the compound of Formula I-1, and its X-ray powder diffraction pattern has characteristic diffraction peaks at the following 2θ angles: 5.11 ± 0.2°, 10.20±0.2°, 14.23±0.2°, 17.03±0.2°, 18.79 ± 0.2°, 28.85 ± 0.2 °, and 29.46 ± 0.2°.

In some embodiments of the present invention, the X-ray powder diffraction pattern of the fumarate crystal form A of the compound of Formula I-1 has characteristic diffraction peaks at the following 2θ angles: 5.11±0.2°, 7.26±0.2°, 10.20±0.2°, 10.51 ±0.2°, 14.23 ± 0.2°, 14.47 ± 0.2°, 14.93 ± 0.2°, 16.17 ± 0.2°, 17.03 ± 0.2°, 17.90 ± 0.2 °, 18.79 ± 0.2°, 19.44±0.2°, 20.19±0.2°, 20.80±0.2°, 22.48±0.2°, 23.07±0.2°, 23.74±0.2°, 24.07±0.2°, 27.06±0.2°, 27.85±0.2°, 28.30±0.2°, 28.85±0.2°, 29.46±0.2°, 38.24±0.2°.

In some embodiments of the present invention, the X-ray powder diffraction (XRPD) pattern of the fumarate crystal form A of the compound of Formula I-1 is shown in Figure 8.

**Table 4 XRPD analysis data for the fumarate crystal form A of the compound of Formula I-1**

| Name | Angle | d Value | Net Intensity | Gross Intensity | Rel. Intensity | FWHM |
|---|---|---|---|---|---|---|
| Peak#1 | 5.106943 | 17.29001 | 66.02386 | 80.74232 | 0.6328462 | 0.100 |
| Peak#2 | 7.257269 | 12.17109 | 15.48998 | 32.61639 | 0.1484732 | 0.123 |
| Peak#3 | 10.20005 | 8.665302 | 44.82843 | 61.36366 | 0.4296856 | 0.171 |
| Peak#4 | 10.50647 | 8.413257 | 25.31782 | 41.40717 | 0.2426742 | 0.100 |
| Peak#5 | 14.22988 | 6.219109 | 94.52111 | 109.7326 | 0.9059956 | 0.138 |
| Peak#6 | 14.46519 | 6.118463 | 44.95081 | 60.22918 | 0.4308586 | 0.153 |
| Peak#7 | 14.93302 | 5.927817 | 26.26917 | 41.2175 | 0.251793 | 0.100 |
| Peak#8 | 16.16592 | 5.478399 | 35.9365 | 51.60095 | 0.3444554 | 0.117 |
| Peak#9 | 17.02807 | 5.202913 | 44.72762 | 61.55038 | 0.4287193 | 0.100 |
| Peak#10 | 17.90158 | 4.950956 | 21.9525 | 40.30973 | 0.2104172 | 0.178 |
| Peak#11 | 18.78968 | 4.718902 | 49.85003 | 67.55918 | 0.4778182 | 0.107 |
| Peak#12 | 19.44071 | 4.562319 | 28.59071 | 45.95156 | 0.2740452 | 0.100 |
| Peak#13 | 20.19247 | 4.394129 | 33.90914 | 52.76979 | 0.325023 | 0.149 |
| Peak#14 | 20.79758 | 4.267629 | 33.02869 | 51.93834 | 0.3165837 | 0.201 |
| Peak#15 | 22.48454 | 3.951108 | 11.28123 | 28.946 | 0.1081319 | 0.169 |
| Peak#16 | 23.06632 | 3.852751 | 12.43236 | 31.23554 | 0.1191656 | 0.167 |
| Peak#17 | 23.73916 | 3.745054 | 18.25035 | 37.17823 | 0.1749317 | 0.152 |
| Peak#18 | 24.07149 | 3.694097 | 20.07611 | 38.59465 | 0.1924318 | 0.207 |
| Peak#19 | 27.06206 | 3.292275 | 40.86212 | 66.92372 | 0.3916681 | 0.119 |
| Peak#20 | 27.84687 | 3.201246 | 20.41484 | 49.1985 | 0.1956785 | 0.160 |
| Peak#21 | 28.29739 | 3.151295 | 22.67108 | 52.42942 | 0.2173048 | 0.199 |
| Peak#22 | 28.84687 | 3.092505 | 104.3285 | 134.4994 | 1 | 0.101 |
| Peak#23 | 29.46212 | 3.02931 | 23.97766 | 53.60007 | 0.2298286 | 0.138 |
| Peak#24 | 38.24442 | 2.351456 | 16.4544 | 35.48725 | 0.1577173 | 0.165 |

The present invention also provides a method for preparing the fumarate crystal form A of the compound of Formula I-1, comprising adding the compound of Formula I-1 and fumaric acid to acetonitrile and dichloromethane, and then performing recrystallization or slurrying to obtain it, wherein the ratio of the compound of Formula I-1 to fumaric acid is selected from 0.8 to 1.2.

The present invention also provides the hemifumarate crystal form B of the compound of Formula I-1, and its X-ray powder diffraction pattern has characteristic diffraction peaks at the following 2θ angles: 5.12±0.2°, 10.20±0.2°, 10.53±0.2°, 14.24±0.2°, 16.99 ± 0.2°, 18.81 ± 0.2^{°}, 19.45 ± 0.2^{°}, 20.22 ± 0.2^{°}, 20.43 ± 0.2^{°}, 20.84 ± 0.2^{°}, 27.04 ± 0.2^{°}, 27.904±0.2°, 28.254±0.2° , 28.754±0.2°.

In some embodiments of the present invention, the X-ray powder diffraction pattern of the hemifumarate crystal form B of the compound of formula I-1 has characteristic diffraction peaks at the following 2θ angles: 5.12±0.2°, 7.27±0.2°, 7.48±0.2°, 8.77 ± 0.2°, 9.18 ± 0.2°, 9.74 ± 0.2°, 10.20 ± 0.2°, 10.53 ± 0.2°, 14.24 ± 0.2°, 14.96 ± 0.2°, 16.09±0.2°, 16.99 ±0.2°, 17.61±0.2° , 17.95±0.2°, 18.81±0.2°, 19.45±0.2°, 20.22±0.2°, 20.43±0.2°, 20.84 ±0.2°, 22.51±0.2°, 23.11±0.2°, 23.76±0.2°, 24.19±0.2°, 25.24±0.2°, 27.04±0.2°, 27.90 ± 0.2°, 28.25 ± 0.2°, 28.75 ± 0.2°, 29.73 ± 0.2°, 30.14 ± 0.2°, 30.77 ± 0.2°, 31.28 ± 0.2°, 33.95±0.2°, 34.52±0.2°, 37.07±0.2°, 38.26±0.2°.

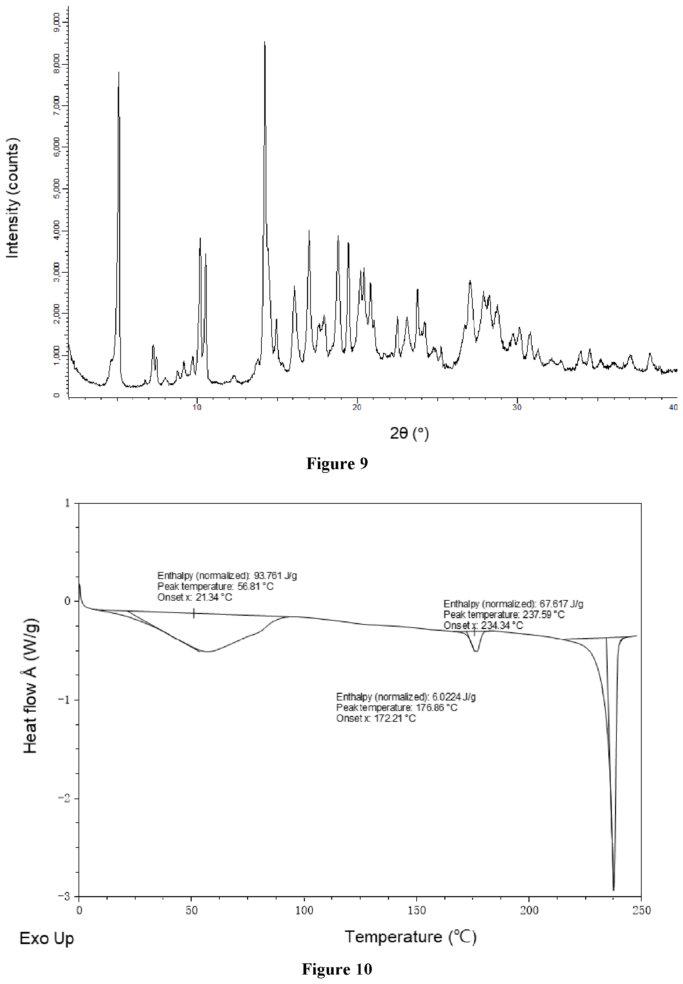

In some embodiments of the present invention, the XRPD pattern of the hemifumarate crystal form B of the compound of Formula I-1 is shown in Figure 9.

**Table 5 XRPD analysis data for the hemifumarate crystal form B of the compound of Formula I-1**

| Name | Angle | d Value | Net Intensity | Gross Intensity | Rel. Intensity | FWHM |
|---|---|---|---|---|---|---|
| Peak#1 | 5.12113 | 17.24215 | 4647.751 | 4910.723 | 0.8420983 | 0.100 |
| Peak#2 | 7.273411 | 12.14412 | 623.8793 | 910.8909 | 0.113037 | 0.142 |
| Peak#3 | 7.477666 | 11.81285 | 412.141 | 704.9915 | 0.07467337 | 0.142 |
| Peak#4 | 8.774699 | 10.06943 | 198.996 | 518.1956 | 0.03605491 | 0.129 |
| Peak#5 | 9.180456 | 9.62527 | 368.1948 | 696.2799 | 0.06671103 | 0.133 |
| Peak#6 | 9.736835 | 9.076479 | 448.8479 | 784.1718 | 0.08132408 | 0.128 |
| Peak#7 | 10.19865 | 8.666491 | 2325.105 | 2662.089 | 0.421272 | 0.188 |
| Peak#8 | 10.53449 | 8.390942 | 2150.306 | 2486.023 | 0.3896012 | 0.100 |
| Peak#9 | 14.24094 | 6.214301 | 5519.25 | 6023.346 | 1 | 0.100 |
| Peak#10 | 14.9559 | 5.918799 | 899.1369 | 1478.035 | 0.1629093 | 0.106 |
| Peak#11 | 16.09258 | 5.503201 | 1338.067 | 2023.104 | 0.2424364 | 0.154 |
| Peak#12 | 16.99405 | 5.213253 | 2242.453 | 3003.123 | 0.4062966 | 0.122 |
| Peak#13 | 17.60838 | 5.03273 | 547.075 | 1350.681 | 0.09912127 | 0.100 |
| Peak#14 | 17.95021 | 4.937653 | 762.8884 | 1587.368 | 0.1382232 | 0.100 |
| Peak#15 | 18.80551 | 4.714965 | 2077.941 | 2945.17 | 0.3764898 | 0.133 |
| Peak#16 | 19.45143 | 4.55983 | 1987.91 | 2878.464 | 0.3601776 | 0.100 |
| Peak#17 | 20.2158 | 4.389112 | 1462.113 | 2370.312 | 0.2649115 | 0.315 |
| Peak#18 | 20.42854 | 4.343882 | 1577.348 | 2488.53 | 0.2857904 | 0.310 |
| Peak#19 | 20.84079 | 4.258877 | 1320.896 | 2235.486 | 0.2393253 | 0.176 |
| Peak#20 | 22.50678 | 3.947253 | 631.9739 | 1528.338 | 0.1145036 | 0.100 |
| Peak#21 | 23.11194 | 3.84525 | 710.6365 | 1587.676 | 0.128756 | 0.189 |
| Peak#22 | 23.75574 | 3.742477 | 1328.016 | 2177.066 | 0.2406154 | 0.100 |
| Peak#23 | 24.18724 | 3.676679 | 635.7908 | 1461.799 | 0.1151951 | 0.100 |
| Peak#24 | 25.23851 | 3.525865 | 378.4447 | 1133.886 | 0.06856815 | 0.100 |
| Peak#25 | 27.04291 | 3.294563 | 1310.952 | 2070.533 | 0.2375235 | 0.253 |
| Peak#26 | 27.90105 | 3.195153 | 1727.994 | 2515.479 | 0.313085 | 0.100 |
| Peak#27 | 28.25099 | 3.156364 | 1116.084 | 1911.047 | 0.2022166 | 0.100 |
| Peak#28 | 28.74762 | 3.102957 | 1000.29 | 1801.975 | 0.1812366 | 0.100 |
| Peak#29 | 29.73267 | 3.002359 | 710.9998 | 1512.519 | 0.1288218 | 0.100 |
| Peak#30 | 30.14065 | 2.962642 | 604.3896 | 1400.594 | 0.1095058 | 0.211 |
| Peak#31 | 30.76595 | 2.903837 | 492.9324 | 1275.009 | 0.08931148 | 0.161 |
| Peak#32 | 31.27625 | 2.857613 | 378.292 | 1143.485 | 0.06854048 | 0.140 |
| Peak#33 | 33.94976 | 2.638443 | 415.4766 | 1098.577 | 0.07527773 | 0.125 |
| Peak#34 | 34.52381 | 2.595875 | 324.9706 | 1017.928 | 0.05887949 | 0.115 |
| Peak#35 | 37.06812 | 2.423331 | 331.5999 | 994.89 | 0.06008062 | 0.178 |
| Peak#36 | 38.25865 | 2.350615 | 330.8221 | 950.937 | 0.05993968 | 0.142 |

In some embodiments of the present invention, the hemifumarate crystal form B of the compound of Formula I-1 has the onsets of the endothermic peaks at 21.34°C ± 2°C, 172.21°C ± 2^{°}C, and 234.34°C ± 2^{°}C in its differential scanning calorimetry (DSC) curve.

In some embodiments of the present invention, the hemifumarate crystal form B of the compound of Formula I-1 has a DSC pattern as shown in Figure 10.

In some embodiments of the present invention, the hemifumarate crystal form B of the compound of Formula I-1 has three weight loss steps at 31.90°C ± 2^{°}C, 90.00°C ± 2^{°}C, and 190.00^{°}C ± 2^{°}C in its thermogravimetric analysis (TGA) curve, and decomposition begins above 270.00°C ± 2^{°}C.

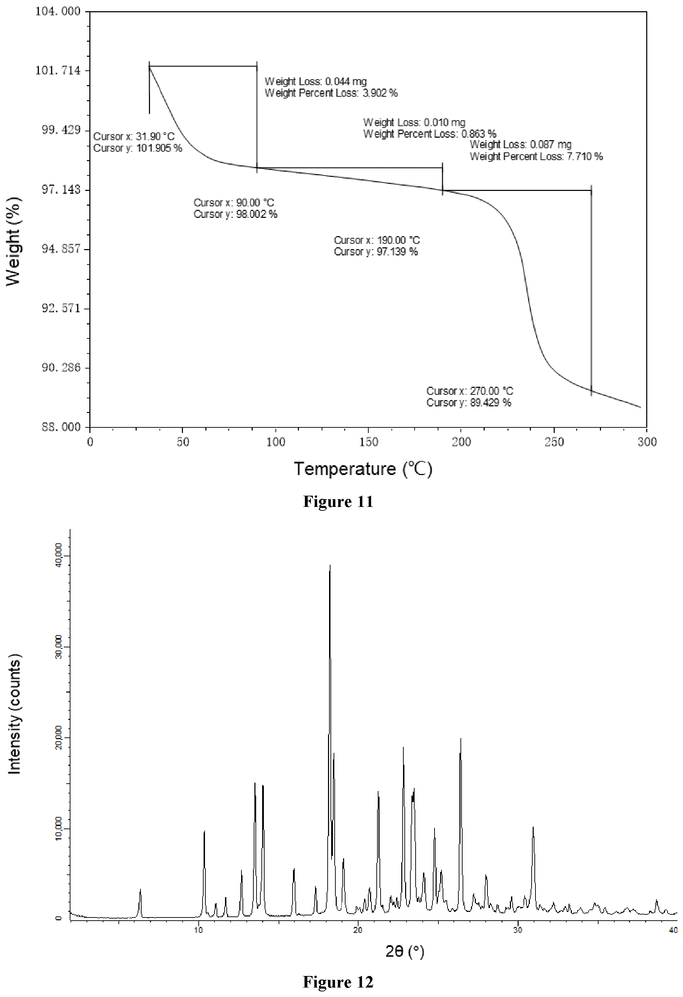

In some embodiments of the present invention, the TGA pattern of the hemifumarate crystal form B of the compound of Formula I-1 is shown in Figure 11.

The present invention also provides a method for preparing the hemifumarate crystal form B of the compound of Formula I-1, comprising adding the compound of Formula I-1 and fumaric acid to tetrahydrofuran, and then performing recrystallization or slurrying to obtain it, wherein the ratio of the compound of Formula I-1 to fumaric acid is selected from 1.6 to 2.4.

The present invention also provides the monoglycolate crystal form A of the compound of Formula I-1, and its X-ray powder diffraction pattern has characteristic diffraction peaks at the following 2θ angles: 6.37 ± 0.2^{°}, 10.37 ± 0.2^{°}, 12.69 ± 0.2^{°}, 13.55 ± 0.2^{°}, 14.06 ± 0.2^{°}, 18.22 ± 0.2^{°}, 18.47 ± 0.2^{°}, 21.25 ± 0.2^{°}, 22.83 ± 0.2^{°}, 23.49 ± 0.2^{°}, 26.41 ± 0.2^{°}, 30.96 ± 0.2^{°}.

In some embodiments of the present invention, the monoglycolate crystal form A of the compound of Formula I-1, and its X-ray powder diffraction pattern has characteristic diffraction peaks at the following 2θ angles: 6.37±0.2°, 10.37±0.2°, 11.09±0.2°, 11.70±0.2°, 12.69 ± 0.2°, 13.55 ± 0.2 °, 14.06 ± 0.2°, 15.97 ± 0.2°, 17.34 ± 0.2°, 18.22 ± 0.2°, 18.47±0.2°, 19.09±0.2°, 20.41±0·2°, 20.71±0.2°, 21.25±0.2°, 22.06±0.2°, 22.43±0.2°, 22.83±0.2°, 23.49±0.2°, 24.09±0.2°, 24.79±0.2°, 25.21±0.2°, 26.41±0.2°, 27.23±0.2°, 28.00±0.2°, 29.59±0.2°, 30.44±0.2°, 30.96±0.2°, 38.67±0.2°.

In some embodiments of the present invention, the XRPD pattern of the monoglycolate crystal form A of the compound of Formula I-1 is shown in Figure 12.

**Table 6 XRPD analysis data for the monoglycolate crystal form A of the compound of Formula I-1**

| Name | Angle | dValue | Net Intensity | Gross Intensity | Rel. Intensity | FWHM |
|---|---|---|---|---|---|---|
| Peak#1 | 6.367303 | 13.87013 | 2029.288 | 2272.519 | 0.07434029 | 0.100 |
| Peak#2 | 10.37207 | 8.521972 | 6470.729 | 6785.768 | 0.2370466 | 0.100 |
| Peak#3 | 11.09208 | 7.970361 | 1010.483 | 1340.449 | 0.0370177 | 0.100 |
| Peak#4 | 11.70461 | 7.55459 | 1500.982 | 1841.876 | 0.05498648 | 0.100 |
| Peak#5 | 12.69449 | 6.967646 | 3589.622 | 3976.064 | 0.131501 | 0.100 |
| Peak#6 | 13.55449 | 6.527435 | 10232.45 | 10650.02 | 0.3748523 | 0.100 |
| Peak#7 | 14.05714 | 6.29514 | 10135.51 | 10557.75 | 0.3713008 | 0.100 |
| Peak#8 | 15.97294 | 5.544148 | 3536.131 | 4003.394 | 0.1295415 | 0.100 |
| Peak#9 | 17.33713 | 5.110852 | 2288.859 | 2836.97 | 0.0838493 | 0.100 |
| Peak#10 | 18.21904 | 4.865397 | 27297.29 | 27912.26 | 1 | 0.100 |
| Peak#11 | 18.46709 | 4.800599 | 12375.91 | 13004.16 | 0.453375 | 0.138 |
| Peak#12 | 19.08811 | 4.645792 | 4405.293 | 5056.107 | 0.1613821 | 0.100 |
| Peak#13 | 20.40513 | 4.348813 | 1117.151 | 1859.68 | 0.04092536 | 0.179 |
| Peak#14 | 20.71391 | 4.284677 | 1886.923 | 2660.549 | 0.06912491 | 0.165 |
| Peak#15 | 21.2549 | 4.176828 | 9592.014 | 10411.04 | 0.3513907 | 0.100 |
| Peak#16 | 22.05512 | 4.027056 | 1278.383 | 2158.564 | 0.04683188 | 0.127 |
| Peak#17 | 22.43277 | 3.960109 | 1131.067 | 2042.437 | 0.04143513 | 0.131 |
| Peak#18 | 22.83447 | 3.891343 | 14043.22 | 14981.57 | 0.5144547 | 0.100 |
| Peak#19 | 23.48801 | 3.784526 | 9793.021 | 10761.67 | 0.3587543 | 0.157 |
| Peak#20 | 24.09406 | 3.690687 | 3140.149 | 4121.826 | 0.1150352 | 0.108 |
| Peak#21 | 24.78808 | 3.588905 | 6995.654 | 7974.427 | 0.2562765 | 0.100 |
| Peak#22 | 25.21228 | 3.529473 | 3274.032 | 4241.681 | 0.1199398 | 0.108 |
| Peak#23 | 26.41114 | 3.37192 | 15344.24 | 16294.99 | 0.5621157 | 0.100 |
| Peak#24 | 27.22701 | 3.272703 | 1465.29 | 2397.752 | 0.05367895 | 0.114 |
| Peak#25 | 27.99766 | 3.184346 | 3541.794 | 4432.819 | 0.1297489 | 0.100 |
| Peak#26 | 29.59053 | 3.016456 | 1490.567 | 2310.24 | 0.05460494 | 0.100 |
| Peak#27 | 30.43898 | 2.934282 | 1485.749 | 2333.618 | 0.05442845 | 0.100 |
| Peak#28 | 30.96021 | 2.886059 | 7547.225 | 8398.33 | 0.2764826 | 0.100 |
| Peak#29 | 38.66922 | 2.326597 | 1355.032 | 1979.99 | 0.04963982 | 0.100 |

In some embodiments of the present invention, the monoglycolate crystal form A of the compound of Formula I-1 has the onsets of the endothermic peaks at 109.66°C ± 2^{°}C and 198.83^{°}C ± 2^{°}C in its differential scanning calorimetry (DSC) curve.

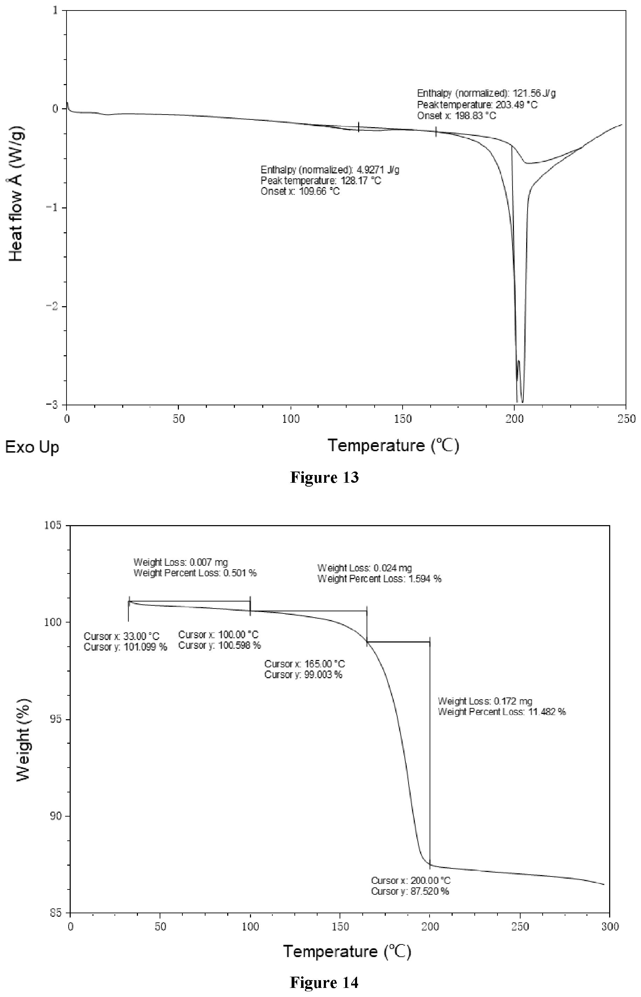

In some embodiments of the present invention, the monoglycolate crystal form A of the compound of Formula I-1 has a DSC pattern as shown in Figure 13.

In some embodiments of the present invention, the monoglycolate crystal form A of the compound of Formula I-1 has three weight loss steps at 33.00°C ± 2^{°}C, 100.00°C ± 2^{°}C, and 165.00^{°}C ± 2^{°}C in its thermogravimetric analysis (TGA) curve, and decomposition begins above 200.00°C ± 2^{°}C.

In some embodiments of the present invention, the TGA pattern of the monoglycolate crystal form A of the compound of Formula I-1 is shown in Figure 14.

The present invention also provides a method for preparing the monoglycolate crystal form A of the compound of Formula I-1, comprising adding the compound of Formula I-1 and glycolic acid to acetonitrile, and then performing recrystallization or slurrying to obtain it, wherein the ratio of the compound of Formula I-1 to glycolic acid is selected from 0.8 to 1.2.

The present invention also provides the glycolate crystal form B of the compound of Formula I-1, and its X-ray powder diffraction pattern has characteristic diffraction peaks at the following 2θ angles: 5.95±0.2°, 12.20 ± 0.2^{°}, 13.87 ± 0.2^{°}, 15.79 ± 0.2^{°}, 15.97 ± 0.2^{°}, 16.44 ± 0.2^{°}, 16.63 ± 0.2^{°}, 16.79 ± 0.2^{°}, 17.30 ± 0.2^{°}, 19.49 ± 0.2^{°}, 23.50 ± 0.2^{°}, 26.68 ± 0.2^{°}.

In some embodiments of the present invention, the X-ray powder diffraction pattern of the glycolate crystal form B of the compound of Formula I-1 has characteristic diffraction peaks at the following 2θ angles: 5.95±0.2°, 11.06±0.2°, 12.20±0.2°, 13.87±0.2°, 15.01 ±0.2°, 15.79±0.2°, 15.97±0.2°, 16.44±0.2°, 16.63±0.2°, 16.79±0.2°, 17.30±0.2°, 17.90 ±0.2°, 18.33±0.2°, 18.91±0.2°, 19.49±0.2°, 21.59±0.2°, 22.18±0.2°, 22.80±0.2°, 23.16 ±0.2°, 23.50±0.2°, 23.93±0.2°, 24.49±0.2° , 25.13±0.2°, 26.68±0.2°, 27.95±0.2°, 30.49 ±0.2°, 31.62±0.2°, 32.27±0.2°.

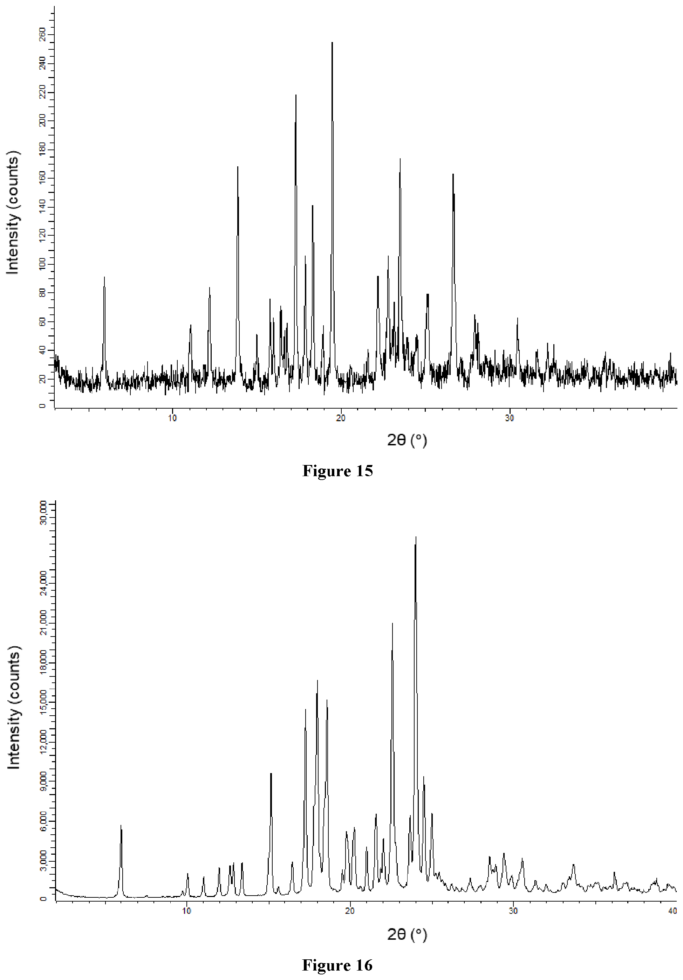

In some embodiments of the present invention, the XRPD pattern of the glycolate crystal form B of the compound of Formula I-1 is shown in Figure 15.

**Table 7 XRPD analysis data for the glycolate crystal form B of the compound of Formula I-1**

| Name | Angle | d Value | Net Intensity | Gross Intensity | Rel. Intensity | FWHM |
|---|---|---|---|---|---|---|
| Peak#1 | 5.94826 | 14.84628 | 47.21226 | 62.80721 | 0.2820042 | 0.100 |
| Peak#2 | 11.05959 | 7.993703 | 27.85228 | 46.52969 | 0.1663649 | 0.100 |
| Peak#3 | 12.19964 | 7.249135 | 42.98131 | 61.03759 | 0.2567323 | 0.100 |
| Peak#4 | 13.8704 | 6.379468 | 111.6527 | 128.6609 | 0.6669146 | 0.100 |
| Peak#5 | 15.01254 | 5.896596 | 26.83811 | 41.78902 | 0.1603071 | 0.100 |
| Peak#6 | 15.78903 | 5.60831 | 44.40633 | 61.01559 | 0.2652441 | 0.100 |
| Peak#7 | 15.97478 | 5.543516 | 34.20984 | 51.4254 | 0.2043393 | 0.100 |
| Peak#8 | 16.43714 | 5.388608 | 14.20148 | 32.83326 | 0.08482705 | 0.166 |
| Peak#9 | 16.6293 | 5.326771 | 22.48186 | 41.62296 | 0.1342867 | 0.166 |
| Peak#10 | 16.79174 | 5.275602 | 31.88576 | 51.36475 | 0.1904573 | 0.100 |
| Peak#11 | 17.30051 | 5.12159 | 142.7281 | 162.7214 | 0.8525316 | 0.100 |
| Peak#12 | 17.89638 | 4.952384 | 43.38684 | 63.59336 | 0.2591546 | 0.100 |
| Peak#13 | 18.32724 | 4.836915 | 84.93102 | 104.8279 | 0.5073028 | 0.100 |
| Peak#14 | 18.91279 | 4.688461 | 18.65536 | 37.72074 | 0.1114306 | 0.100 |
| Peak#15 | 19.48954 | 4.550999 | 167.4168 | 186.2042 | 1 | 0.100 |
| Peak#16 | 21.58975 | 4.112796 | 17.46467 | 35.08154 | 0.1043185 | 0.100 |
| Peak#17 | 22.18036 | 4.0046 | 52.74449 | 72.09505 | 0.3150489 | 0.100 |
| Peak#18 | 22.80481 | 3.896337 | 60.85134 | 82.53977 | 0.363472 | 0.173 |
| Peak#19 | 23.15596 | 3.83804 | 45.15902 | 67.61517 | 0.26974 | 0.125 |
| Peak#20 | 23.49665 | 3.783154 | 107.6953 | 130.5205 | 0.6432761 | 0.100 |
| Peak#21 | 23.92768 | 3.715973 | 15.04318 | 37.80346 | 0.08985461 | 0.191 |
| Peak#22 | 24.49098 | 3.631765 | 17.92286 | 39.70282 | 0.1070553 | 0.161 |
| Peak#23 | 25.12531 | 3.541494 | 38.13791 | 58.55093 | 0.2278021 | 0.119 |
| Peak#24 | 26.67703 | 3.338912 | 82.84576 | 102.2695 | 0.4948473 | 0.100 |
| Peak#25 | 27.94708 | 3.189994 | 33.99763 | 54.5564 | 0.2030717 | 0.107 |
| Peak#26 | 30.48514 | 2.929944 | 31.34254 | 53.22852 | 0.1872126 | 0.100 |
| Peak#27 | 31.62302 | 2.827063 | 15.2675 | 32.42916 | 0.09119453 | 0.100 |
| Peak#28 | 32.26844 | 2.771976 | 22.99334 | 38.4986 | 0.1373419 | 0.192 |

The present invention also provides a method for preparing the glycolate crystal form B of the compound of Fomular I-1, comprising adding the compound of Formula I-1 and glycolic acid to tetrahydrofuran and dichloromethane, and then performing recrystallization or slurrying to obtain it, wherein the ratio of the compound of Formula I-1 to glycolic acid is selected from 0.8 to 1.2.

The present invention also provides the mono-L-malate crystal form A of the compound of Formula I-1, and its X-ray powder diffraction pattern has characteristic diffraction peaks at the following 2θ angles: 6.01 ±0.2°, 15.15±0.2°, 17.27±0.2°, 17.99±0.2°, 18.58 ± 0.2°, 22.58 ± 0.2 °, 24.00 ± 0.2°, 24.50 ± 0.2°, 25.00 ± 0.2°, 29.40 ± 0.2°, 30.53 ± 0.2°.

In some embodiments of the present invention, the X-ray powder diffraction pattern of the mono-L-malate crystal form A of the compound of Formula I-1 has characteristic diffraction peaks at the following 2θ angles: 6.01±0.2°, 10.06±0.2°, 11.04±0.2°, 11.98± 0.2°, 12.66 ± 0.2° , 12.86 ± 0.2°, 13.39 ± 0.2° , 15.15 ± 0.2°, 15.62 ± 0.2°, 16.46 ± 0.2°, 17.27 ± 0.2°, 17.99±0.2°, 18.58±0.2°, 19.53±0.2°, 19.77±0.2°, 20.25±0.2°, 21.01±0.2°, 21.57±0.2°, 22.04±0.2°, 22.58±0.2°, 23.67±0.2°, 24.00±0.2°, 24.50±0.2°, 25.00±0.2°, 27.34±0.2°, 28.54±0.2°, 28.91±0.2°, 29.40±0.2°, 29.89±0.2°, 30.53±0.2°, 31.33±0.2°, 33.67±0.2°, 36.16±0.2°.

In some embodiments of the present invention, the XRPD pattern of the mono-L-malate crystal form A of the compound of Formula I-1 is shown in Figure 16.

**Table 8. XRPD analysis data for the mono-L-malate crystal form A of the compound of Formula I-1**

| Name | Angle | d Value | Net Intensity | Gross Intensity | Rel. Intensity | FWHM |
|---|---|---|---|---|---|---|
| Peak#1 | 6.005817 | 14.70413 | 3458.47 | 3698.186 | 0.1756963 | 0.100 |
| Peak#2 | 10.05991 | 8.785703 | 1235.219 | 1524.838 | 0.06275127 | 0.100 |
| Peak#3 | 11.04048 | 8.007493 | 1010.378 | 1324.343 | 0.05132898 | 0.100 |
| Peak#4 | 11.98007 | 7.381497 | 1546.43 | 1893.062 | 0.07856131 | 0.100 |
| Peak#5 | 12.65977 | 6.986674 | 1564.73 | 1932.612 | 0.07949098 | 0.141 |
| Peak#6 | 12.86478 | 6.875793 | 1744.169 | 2115.445 | 0.0886068 | 0.133 |
| Peak#7 | 13.39037 | 6.607066 | 1748.559 | 2122.191 | 0.08882982 | 0.100 |
| Peak#8 | 15.15275 | 5.842348 | 6447.348 | 6874.498 | 0.3275365 | 0.100 |
| Peak#9 | 15.61757 | 5.669495 | 415.1237 | 858.8148 | 0.021089 | 0.100 |
| Peak#10 | 16.46146 | 5.3807 | 1753.405 | 2260.688 | 0.08907603 | 0.100 |
| Peak#11 | 17.27465 | 5.129198 | 9650.521 | 10,238.98 | 0.4902632 | 0.100 |
| Peak#12 | 17.99119 | 4.926498 | 11,219.57 | 11,861.34 | 0.5699735 | 0.109 |
| Peak#13 | 18.57705 | 4.772433 | 10,433.66 | 11,106.38 | 0.5300483 | 0.108 |
| Peak#14 | 19.52605 | 4.542572 | 1184.517 | 1891.812 | 0.06017552 | 0.222 |
| Peak#15 | 19.77436 | 4.486085 | 3155.73 | 3872.169 | 0.1603165 | 0.130 |
| Peak#16 | 20.24735 | 4.382343 | 3376.988 | 4105.192 | 0.1715568 | 0.121 |
| Peak#17 | 21.0078 | 4.225396 | 2453.564 | 3206.388 | 0.1246453 | 0.100 |
| Peak#18 | 21.5734 | 4.115878 | 3826.304 | 4620.834 | 0.1943829 | 0.100 |
| Peak#19 | 22.04117 | 4.029573 | 2859.582 | 3680.584 | 0.1452717 | 0.100 |
| Peak#20 | 22.58438 | 3.933865 | 15,035.12 | 15,877.77 | 0.7638101 | 0.100 |
| Peak#21 | 23.67221 | 3.755494 | 3964.6 | 4821.112 | 0.2014086 | 0.100 |
| Peak#22 | 24.00331 | 3.704436 | 19,684.37 | 20,537.32 | 1 | 0.100 |
| Peak#23 | 24.50464 | 3.629771 | 6524.69 | 7365.307 | 0.3314656 | 0.100 |
| Peak#24 | 24.99671 | 3.559421 | 4225.784 | 5046.185 | 0.2146772 | 0.104 |
| Peak#25 | 27.33987 | 3.259448 | 1009.065 | 1702.707 | 0.05126226 | 0.100 |
| Peak#26 | 28.53714 | 3.125362 | 2079.627 | 2806.331 | 0.1056487 | 0.100 |
| Peak#27 | 28.90615 | 3.086298 | 1564.794 | 2296.025 | 0.07949425 | 0.100 |
| Peak#28 | 29.4012 | 3.035449 | 2180.341 | 2910.555 | 0.1107651 | 0.142 |
| Peak#29 | 29.89372 | 2.986549 | 849.0558 | 1570.18 | 0.04313351 | 0.153 |
| Peak#30 | 30.52674 | 2.926045 | 1898.222 | 2595.839 | 0.09643298 | 0.163 |
| Peak#31 | 31.33463 | 2.852422 | 933.9003 | 1606.439 | 0.04744376 | 0.120 |
| Peak#32 | 33.66905 | 2.659796 | 1560.925 | 2238.357 | 0.07929768 | 0.183 |
| Peak#33 | 36.16417 | 2.481803 | 1357.96 | 2054.411 | 0.06898671 | 0.100 |

In some embodiments of the present invention, the mono-L-malate crystal form A of the compound of Formula I-1 has the onset of endothermic peak at 204.74°C ± 2°C in its differential scanning calorimetry (DSC) curve.

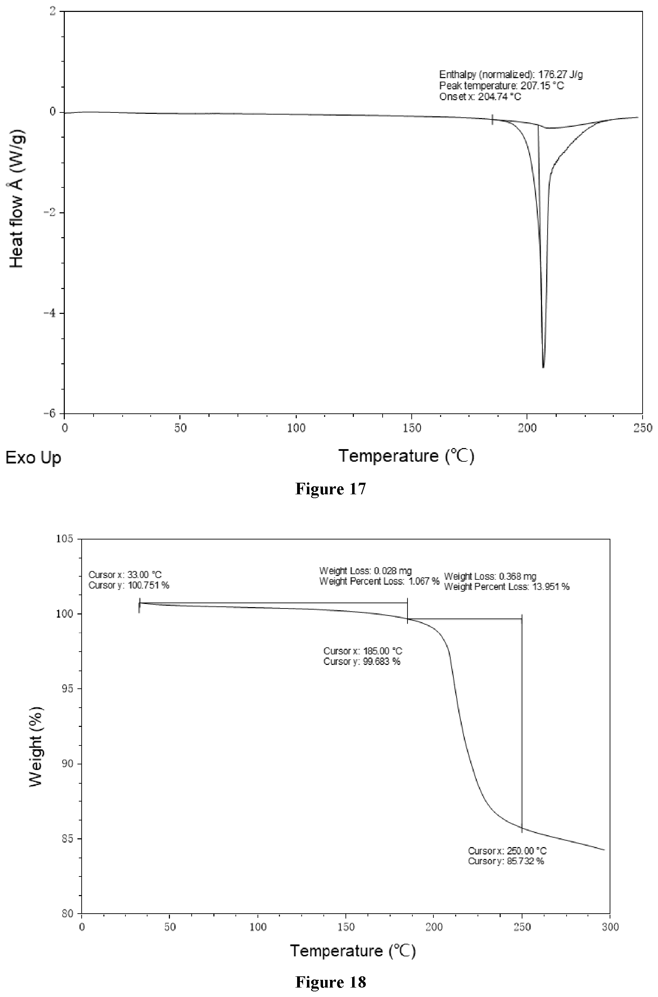

In some embodiments of the present invention, the DSC pattern of the mono-L-malate crystal form A of the compound of Formula I-1 is shown in Figure 17.

In some embodiments of the present invention, the mono-L-malate crystal form A of the compound of Formula I-1 has two weight loss steps at 33.00°C ± 2°C and 185.00°C ± 2°C in its thermogravimetric analysis (TGA) curve, and decomposition begins above 250.00^{°}C ± 2^{°}C.

In some embodiments of the present invention, the TGA pattern of mono-L-malate crystal form A of the compound of Formula I-1 is shown in Figure 18.

The present invention also provides a method for preparing the mono-L-malate crystal form A of the compound of Formula I-1, comprising adding the compound of Formula I-1 and L-malic acid to acetonitrile, tetrahydrofuran, and dichloromethane, and then performing recrystallization or slurrying to obtain it, wherein the ratio of the compound of Formula I-1 to L-malic acid is selected from 0.8 to 1.2.

The present invention also provides the monosuccinate crystal form A of the compound of Formula I-1, and its X-ray powder diffraction pattern has characteristic diffraction peaks at the following 2θ angles: 6.15±0.2^{°}, 12.26 ± 0.2^{°}, 17.59 ± 0.2^{°}, 18.40 ± 0.2^{°}, 18.89 ± 0.2°, 19.11 ± 0.2°, 19.73 ± 0.2°, 20.90 ± 0.2°, 23.12±0.2°, 23.83±0.2°, 24.09±0.2°, 24.35±0.2°, 24.62±0.2°, 24.93±0.2°, 25.17±0.2°.

In some embodiments of the present invention, the X-ray powder diffraction pattern of the monosuccinate crystal form A of the compound of formula I-1 has characteristic diffraction peaks at the following 2θ angles: 6.15±0.2°, 9.64±0.2°, 9.95±0.2°, 11.19±0.2°, 12.26 ± 0.2 °, 12.70 ± 0.2 °, 13.30 ± 0.2°, 14.95 ± 0.2°, 15.25 ± 0.2°, 16.18 ± 0.2°, 17.59 ± 0.2°, 18.40±0.2°, 18.89±0.2°, 19.11±0.2°, 19.73±0.2°, 20.28±0.2°, 20.90±0.2°, 21.27± 0.2°, 21.64±0.2°, 22.12±0.2°, 23.12±0.2°, 23.83±0.2°, 24.09±0.2°, 24.35±0.2°, 24.62± 0.2°, 24.93±0.2°, 25.17±0.2°, 26.05±0.2°, 27.00±0.2°, 28.27±0.2°, 28.72±0.2°, 30.12±0.2°, 31.20±0.2°, 33.57±0.2°, 34.13±0.2°, 34.58±0.2°, 35.60±0.2°, 38.23±0.2°.

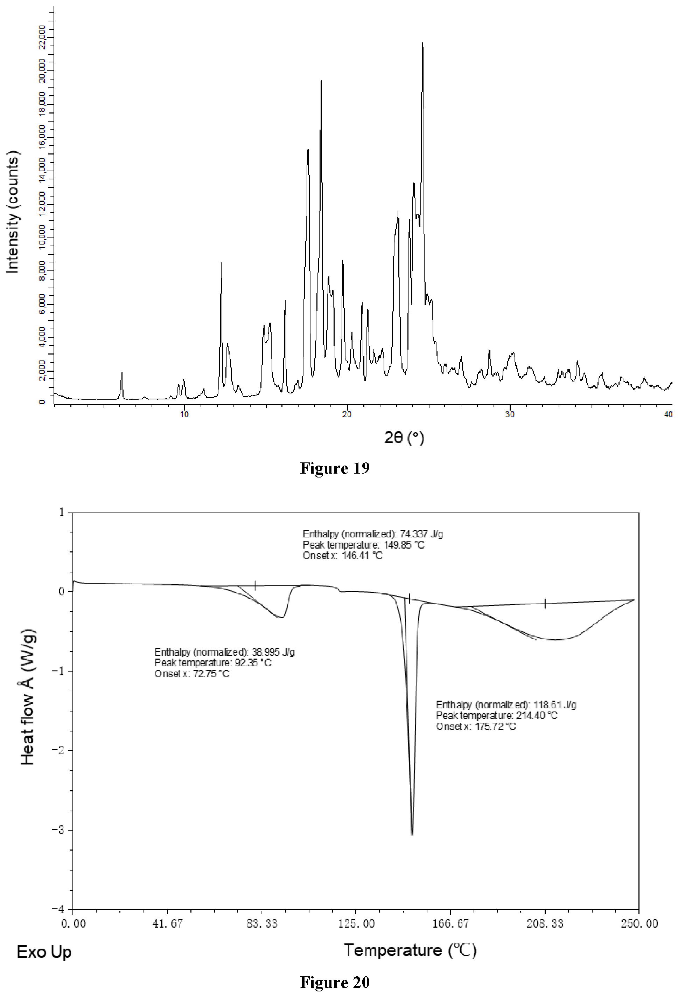

In some embodiments of the present invention, the XRPD pattern of the monosuccinate crystal form A of the compound of Formula I-1 is shown in Figure 19.

**Table 9: XRPD analysis data for the monosuccinate crystal form A of the compound of Formula I-1**

| Name | Angle | d Value | Net Intensity | Gross Intensity | Rel. Intensity | FWHM |
|---|---|---|---|---|---|---|
| Peak #1 | 6.150476 | 14.35861 | 1079.754 | 1326.613 | 0.07385032 | 0.100 |
| Peak #2 | 9.639017 | 9.168368 | 507.5315 | 837.8813 | 0.03471289 | 0.100 |
| Peak #3 | 9.947687 | 8.884562 | 756.1947 | 1093.582 | 0.05172034 | 0.193 |
| Peak #4 | 11.19269 | 7.898941 | 418.548 | 786.0864 | 0.02862681 | 0.104 |
| Peak #5 | 12.26293 | 7.211865 | 5643.144 | 6055.72 | 0.3859659 | 0.100 |
| Peak #6 | 12.69721 | 6.966156 | 1808.497 | 2238.503 | 0.1236932 | 0.183 |
| Peak #7 | 13.30472 | 6.649406 | 441.7258 | 886.793 | 0.03021207 | 0.170 |
| Peak #8 | 14.94649 | 5.922504 | 2066.371 | 2623.71 | 0.1413305 | 0.100 |
| Peak #9 | 15.25239 | 5.804404 | 2862.093 | 3448.346 | 0.1957544 | 0.100 |
| Peak #10 | 16.17555 | 5.475159 | 3974.573 | 4631.365 | 0.271843 | 0.100 |
| Peak #11 | 17.58521 | 5.039309 | 9714.239 | 10,581.19 | 0.6644106 | 0.178 |
| Peak #12 | 18.40139 | 4.817592 | 12,708.81 | 13,696.54 | 0.8692256 | 0.109 |
| Peak #13 | 18.8852 | 4.69525 | 3,885.743 | 4935.811 | 0.2657675 | 0.275 |
| Peak #14 | 19.10943 | 4.640657 | 3969.688 | 5046.307 | 0.271509 | 0.284 |
| Peak #15 | 19.73177 | 4.495673 | 5384.054 | 6526.596 | 0.3682452 | 0.100 |
| Peak #16 | 20.28191 | 4.374954 | 2270.541 | 3461.857 | 0.1552948 | 0.126 |
| Peak #17 | 20.90259 | 4.246425 | 3615.605 | 4851.244 | 0.2472912 | 0.100 |
| Peak #18 | 21.27065 | 4.173772 | 3046.671 | 4303.229 | 0.2083787 | 0.102 |
| Peak #19 | 21.63627 | 4.10406 | 1379.22 | 2652.606 | 0.0943325 | 0.100 |
| Peak #20 | 22.1207 | 4.015265 | 1392.721 | 2682.337 | 0.0952559 | 0.100 |
| Peak #21 | 23.11513 | 3.844727 | 6964.299 | 8265.567 | 0.476327 | 0.234 |
| Peak #22 | 23.83111 | 3.730811 | 7252.805 | 8544.42 | 0.4960595 | 0.101 |
| Peak #23 | 24.0942 | 3.690666 | 12,167.04 | 13,451.31 | 0.832171 | 0.100 |
| Peak #24 | 24.35121 | 3.652293 | 10,144.41 | 11,419.54 | 0.6938321 | 0.100 |
| Peak #25 | 24.61575 | 3.613639 | 14,620.84 | 15,884.53 | 1 | 0.130 |
| Peak #26 | 24.93286 | 3.568392 | 5294.415 | 6541.669 | 0.3621143 | 0.100 |
| Peak #27 | 25.16802 | 3.535581 | 5032.057 | 6265.21 | 0.3441702 | 0.100 |
| Peak #28 | 26.0534 | 3.417403 | 778.8174 | 1944.266 | 0.05326763 | 0.100 |
| Peak #29 | 27.00305 | 3.299335 | 1246.342 | 2313.507 | 0.08524425 | 0.216 |
| Peak #30 | 28.26834 | 3.154467 | 725.8612 | 1800.543 | 0.04964567 | 0.228 |
| Peak #31 | 28.7181 | 3.106079 | 1720.941 | 2828.181 | 0.1177047 | 0.111 |
| Peak #32 | 30.12463 | 2.964181 | 1318.532 | 2489.126 | 0.09018173 | 0.343 |
| Peak #33 | 31.20475 | 2.863997 | 646.1497 | 1825.823 | 0.04419375 | 0.353 |
| Peak #34 | 33.56967 | 2.667443 | 732.0571 | 1829.258 | 0.05006944 | 0.187 |
| Peak #35 | 34.12764 | 2.625098 | 1220.828 | 2279.93 | 0.08349918 | 0.114 |
| Peak #36 | 34.58483 | 2.591435 | 594.5156 | 1615.563 | 0.04066221 | 0.137 |
| Peak #37 | 35.60008 | 2.519824 | 551.2985 | 1542.296 | 0.03770636 | 0.154 |
| Peak #38 | 38.23304 | 2.352131 | 558.504 | 1479.673 | 0.03819918 | 0.162 |

In some embodiments of the present invention, the monosuccinate crystal form A of the compound of Formula I-1 has the onsets of the endothermic peaks at 72.75°C ± 2°C, 146.41°C ± 2°C, and 175.72°C ± 2°C in its differential scanning calorimetry (DSC) curve.

In some embodiments of the present invention, the DSC pattern of the monosuccinate crystal form A of the compound of Formula I-1 is shown in Figure 20.

In some embodiments of the present invention, the monosuccinate crystal form A of the compound of Formula I-1 has three weight loss steps at 33.00°C ± 2°C, 90.00°C ± 2°C, and 160.00°C ± 2°C in its thermogravimetric analysis (TGA) curve, and decomposition begins above 260.00°C ± 2°C.

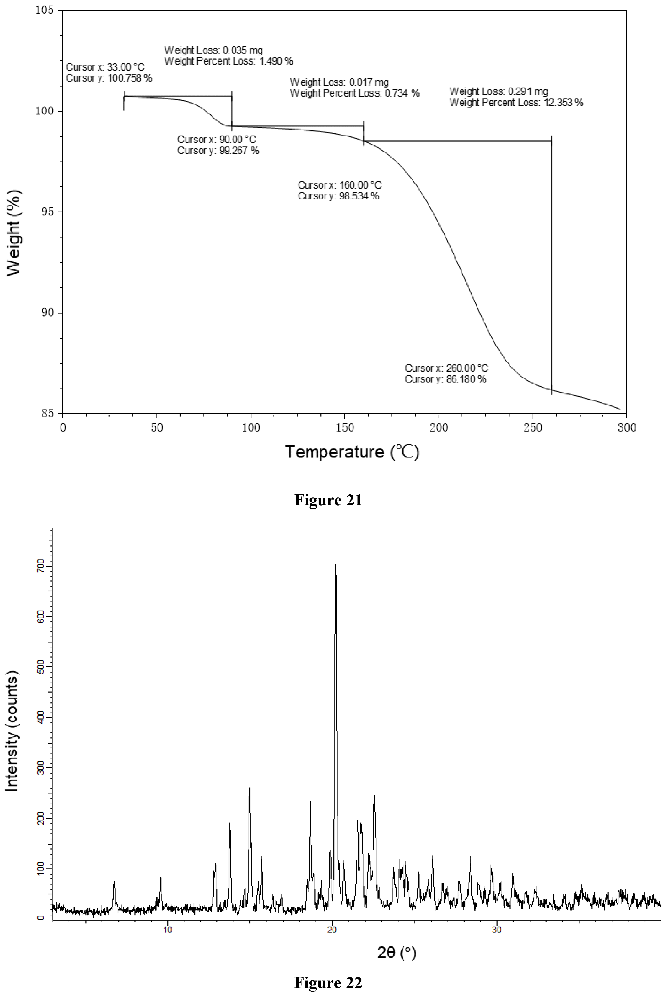

In some embodiments of the present invention, the TGA pattern of the monosuccinate crystal form A of the compound of Formula I-1 is shown in Figure 21.

The present invention also provides a method for preparing the monosuccinate crystal form A of the compound of Formula I-1, comprising adding the compound of Formula I-1 and succinic acid to acetonitrile, tetrahydrofuran, dichloromethane, and then performing recrystallization or slurrying to obtain it, wherein the ratio of the compound of Formula I-1 to succinic acid is selected from 0.8 to 1.2.

The present invention also provides the sulfate crystal form A of the compound of Formula I-1, and its X-ray powder diffraction pattern has characteristic diffraction peaks at the following 2θ angles: 6.74±0.2°, 9.56±0.2°, 12.89±0.2°, 13.78 ± 0.2°, 14.97 ± 0.2 °, 15.70 ± 0.2 °, 18.68 ± 0.2 °, 19.87 ± 0.2 °, 20.21 ± 0.2 °, 21.54 ± 0.2°, 21.76±0.2°, 22.58 ±0.2°, 23.75±0.2°, 24.11 ±0.2°, 24.50±0.2°, 25.25±0.2°, 25.86±0.2°, 27.73±0.2°, 28.41 ±0.2°, 29.70±0.2°, 30.21±0.2°, 30.99±0.2°.

In some embodiments of the present invention, the X-ray powder diffraction pattern of the sulfate crystal form A of the compound of formula I-1 has characteristic diffraction peaks at the following 2θ angles: 6.74±0.2°, 9.56±0.2°, 12.89 ± 0.2°, 13.78 ± 0.2 ° , 14.68 ± 0.2°, 14.97 ± 0.2°, 15.49 ± 0.2 °, 15.70 ± 0.2 °, 16.41 ± 0.2°, 16.90±0.2°, 18.47±0.2°, 18.68±0.2°, 18.86±0.2°, 19.33±0.2°, 19.87±0.2°, 20.21±0.2°, 20.70±0.2°, 21.54±0.2°, 21.76±0.2°, 22.25±0.2°, 22.58±0.2°, 23.75±0.2°, 24.11±0.2°, 24.50±0.2°, 25.25±0.2°, 25.86±0.2°, 26.10±0.2°, 26.71±0.2°, 26.96±0.2°, 27.73±0.2°, 28.41±0.2°, 28.90±0.2°, 29.704±0.2°, 30.21±0.2°, 30.99±0.2°.

In some embodiments of the present invention, the XRPD pattern of sulfate crystal form A of the compound of Formula I-1 is shown in Figure 22.

**Table 10 XRPD analysis data for the sulfate crystal form A of the compound of Formula I-1**

| Name | Angle | d Value | Net Intensity | Gross Intensity | Rel. Intensity | FWHM |
|---|---|---|---|---|---|---|
| Peak #1 | 6.744119 | 13.09598 | 35.65135 | 52.09074 | 0.07305558 | 0.100 |
| Peak #2 | 9.561178 | 9.242833 | 46.10621 | 65.47356 | 0.09447933 | 0.100 |
| Peak #3 | 12.8933 | 6.860647 | 63.02497 | 82.15709 | 0.1291487 | 0.100 |
| Peak #4 | 13.78205 | 6.420164 | 125.4007 | 141.7527 | 0.256967 | 0.100 |
| Peak #5 | 14.67751 | 6.03043 | 43.68357 | 60.03772 | 0.08951494 | 0.120 |
| Peak #6 | 14.9725 | 5.912276 | 162.9075 | 180.921 | 0.3338248 | 0.100 |
| Peak #7 | 15.48903 | 5.716255 | 33.8544 | 53.65991 | 0.06937333 | 0.137 |
| Peak #8 | 15.70464 | 5.638256 | 75.82063 | 95.95579 | 0.1553692 | 0.107 |
| Peak #9 | 16.40931 | 5.397684 | 27.52728 | 46.81891 | 0.056408 | 0.122 |
| Peak #10 | 16.89674 | 5.243056 | 26.01051 | 42.90306 | 0.05329987 | 0.137 |
| Peak #11 | 18.46692 | 4.800645 | 66.26648 | 87.1077 | 0.1357911 | 0.100 |
| Peak #12 | 18.68092 | 4.74613 | 150.3831 | 172.4377 | 0.3081601 | 0.100 |
| Peak #13 | 18.85655 | 4.702318 | 68.76389 | 91.63316 | 0.1409087 | 0.100 |
| Peak #14 | 19.32761 | 4.58876 | 43.94986 | 68.19607 | 0.09006061 | 0.100 |
| Peak #15 | 19.86924 | 4.464878 | 73.86705 | 99.83997 | 0.1513659 | 0.100 |
| Peak #16 | 20.2137 | 4.389563 | 488.0031 | 514.8398 | 1 | 0.100 |
| Peak #17 | 20.70499 | 4.286504 | 63.89549 | 90.87065 | 0.1309325 | 0.100 |
| Peak #18 | 21.54397 | 4.121434 | 97.47784 | 125.4785 | 0.1997484 | 0.159 |
| Peak #19 | 21.76018 | 4.080969 | 98.40488 | 127.1933 | 0.2016481 | 0.180 |
| Peak #20 | 22.24623 | 3.992892 | 67.09114 | 96.74156 | 0.137481 | 0.122 |
| Peak #21 | 22.57571 | 3.935357 | 161.0002 | 190.5227 | 0.3299163 | 0.100 |
| Peak #22 | 23.74843 | 3.743612 | 56.21038 | 83.58575 | 0.1151845 | 0.100 |
| Peak #23 | 24.11135 | 3.68808 | 61.4445 | 89.07555 | 0.1259101 | 0.193 |
| Peak #24 | 24.4972 | 3.630857 | 66.60504 | 93.7385 | 0.1364849 | 0.117 |
| Peak #25 | 25.25065 | 3.524197 | 48.23406 | 75.23211 | 0.09883966 | 0.100 |
| Peak #26 | 25.8563 | 3.443004 | 47.72845 | 74.68002 | 0.09780359 | 0.197 |
| Peak #27 | 26.09526 | 3.412015 | 76.15202 | 102.5496 | 0.1560482 | 0.133 |
| Peak #28 | 26.70874 | 3.33502 | 23.33187 | 50.96179 | 0.04781092 | 0.168 |
| Peak #29 | 26.96348 | 3.304087 | 15.27831 | 43.83334 | 0.03130781 | 0.168 |
| Peak #30 | 27.73352 | 3.214072 | 36.22639 | 65.48412 | 0.07423393 | 0.100 |
| Peak #31 | 28.40891 | 3.139177 | 73.84084 | 101.121 | 0.1513122 | 0.100 |
| Peak #32 | 28.90162 | 3.086772 | 32.87369 | 60.11243 | 0.06736368 | 0.171 |
| Peak #33 | 29.6984 | 3.005745 | 44.70893 | 73.78767 | 0.09161608 | 0.101 |
| Peak #34 | 30.20773 | 2.956216 | 44.79463 | 73.28409 | 0.09179169 | 0.100 |
| Peak #35 | 30.98633 | 2.883686 | 44.91674 | 71.77048 | 0.09204191 | 0.100 |

The present invention also provides a method for preparing the sulfate crystal form A of the compound of Formula I-1, comprising adding the compound of Formula I-1 and sulfuric acid to acetonitrile, and then performing recrystallization or slurrying to obtain it, wherein the ratio of the compound of Formula I-1 to sulfuric acid is selected from 0.8 to 1.2.

The present invention also provides the L-tartarate crystral form A of the compound of Formula I-1, and its X-ray powder diffraction pattern has characteristic diffraction peaks at the following 2θ angles: 5.53±0.2°, 8.90±0.2°, 9.35±0.2°, 11.05±0.2°, 12.54 ± 0.2 °, 12.90 ± 0.2 °, 14.35 ± 0.2 °, 14.64 ± 0.2 °, 16.95 ± 0.2°, 18.70±0.2°, 19.45±0.2°, 19.74 ±0.2°, 20.38±0.2°, 20.79±0.2°, 21.45±0.2°, 21.94±0.2°, 22.88±0.2°, 23.80±0.2°, 25.01 ±0.2°, 25.77±0.2°, 27.33±0.2°, 27.80±0.2°, 28.65±0.2°, 29.41±0.2°, 30.00±0.2°, 31.09 ±0.2^{°}.

In some embodiments of the present invention, the X-ray powder diffraction pattern of the L-tartarate crystal form A of the compound of Formula I-1 has characteristic diffraction peaks at the following 2θ angles: 5.53±0.2°, 8.90±0.2°, 9.35±0.2°, 11.05±0.2°, 12.54 ± 0.2^{°}, 12.90 ± 0.2^{°}, 14.35 ± 0.2^{°}, 14.64 ± 0.2^{°}, 16.95 ± 0.2^{°}, 18.70 ± 0.2^{°}, 19.45 ± 0.2^{°}, 19.74±0.2°, 20.38±0.2°, 20.79±0.2°, 21.45±0.2°, 21.94±0.2°, 22.88±0.2°, 23.80±0.2°, 25.01±0.2°, 25.77±0.2°, 27.33±0.2°, 27.80±0.2°, 28.65±0.2°, 29.41±0.2°, 30.00±0.2°, 31.09±0.2°.

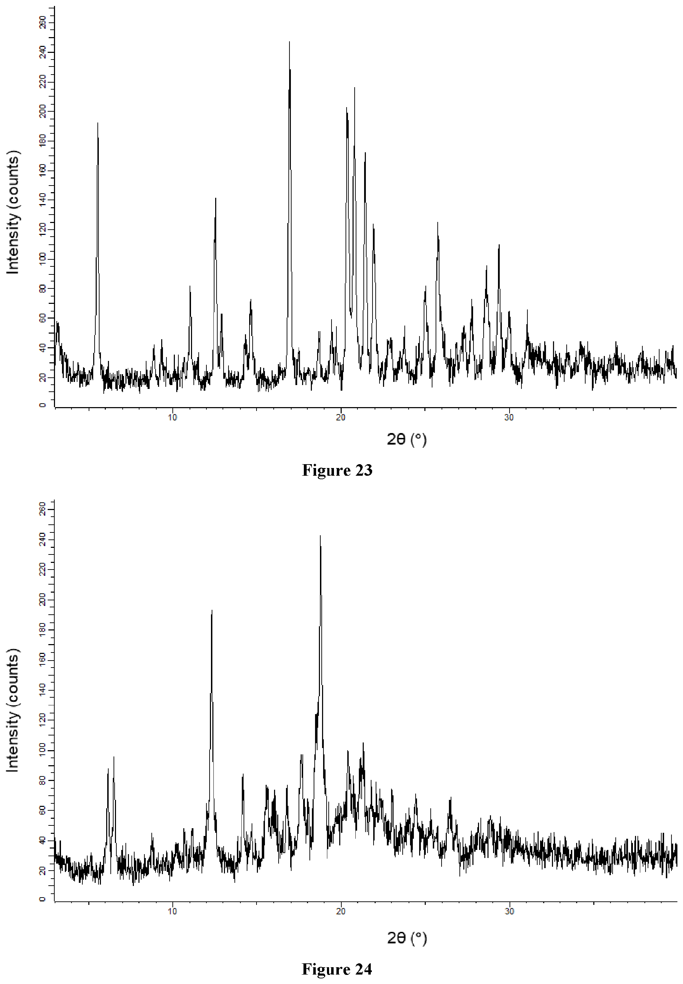

In some embodiments of the present invention, the XRPD pattern of the L-tartrate crystal form A of the compound of Formula I-1 is shown in Figure 23.

**Table 11 XRPD analysis data for the L-tartrate crystal form A of the compound of Formula I-1**

| Name | Angle | d Value | Net Intensity | Intensity Gross | Rel. Intensity | FWHM |
|---|---|---|---|---|---|---|
| Peak #1 | 5.532435 | 15.96118 | 109.1809 | 126.6481 | 0.7936769 | 0.100 |
| Peak #2 | 8.902002 | 9.925714 | 17.76301 | 34.91975 | 0.129126 | 0.100 |
| Peak #3 | 9.347501 | 9.453633 | 20.67122 | 38.23275 | 0.1502669 | 0.107 |
| Peak #4 | 11.05106 | 7.999851 | 40.37062 | 57.19987 | 0.2934692 | 0.100 |
| Peak #5 | 12.53919 | 7.053588 | 73.71306 | 91.06278 | 0.5358478 | 0.100 |
| Peak #6 | 12.8953 | 6.859589 | 31.62126 | 49.20035 | 0.2298668 | 0.106 |
| Peak #7 | 14.3487 | 6.167873 | 24.90215 | 41.21078 | 0.1810231 | 0.181 |
| Peak #8 | 14.63806 | 6.046594 | 38.96151 | 55.6525 | 0.2832258 | 0.169 |
| Peak #9 | 16.94772 | 5.227398 | 113.6682 | 132.2339 | 0.8262969 | 0.100 |
| Peak #10 | 18.69887 | 4.741615 | 16.89806 | 36.56548 | 0.1228383 | 0.100 |
| Peak #11 | 19.44714 | 4.560826 | 31.79594 | 53.59855 | 0.2311366 | 0.134 |
| Peak #12 | 19.74014 | 4.493786 | 40.01748 | 62.17728 | 0.290902 | 0.139 |
| Peak #13 | 20.37972 | 4.354178 | 102.8208 | 125.9592 | 0.7474431 | 0.112 |
| Peak #14 | 20.79081 | 4.269002 | 137.5634 | 161.0057 | 1 | 0.100 |
| Peak #15 | 21.45161 | 4.138969 | 104.2521 | 127.0696 | 0.7578474 | 0.100 |
| Peak #16 | 21.942 | 4.047562 | 66.40536 | 87.87263 | 0.4827255 | 0.118 |
| Peak #17 | 22.88227 | 3.883322 | 13.39118 | 34.40554 | 0.09734552 | 0.175 |
| Peak #18 | 23.79606 | 3.736227 | 26.73411 | 47.92166 | 0.1943403 | 0.100 |
| Peak #19 | 25.01189 | 3.557295 | 31.7189 | 53.23115 | 0.2305766 | 0.117 |
| Peak #20 | 25.77077 | 3.454237 | 53.50304 | 75.36505 | 0.3889337 | 0.121 |
| Peak #21 | 27.33488 | 3.260032 | 22.88951 | 45.80283 | 0.1663924 | 0.213 |
| Peak #22 | 27.80017 | 3.206517 | 29.94634 | 54.00067 | 0.2176912 | 0.141 |
| Peak #23 | 28.65149 | 3.113149 | 48.81897 | 74.25991 | 0.3548834 | 0.140 |
| Peak #24 | 29.40593 | 3.034971 | 65.22767 | 90.68871 | 0.4741644 | 0.100 |
| Peak #25 | 30.00085 | 2.976129 | 29.92109 | 54.13644 | 0.2175076 | 0.149 |
| Peak #26 | 31.09079 | 2.874235 | 31.22145 | 54.13402 | 0.2269605 | 0.100 |

The present invention also provides a method for preparing the L-tartarate crystal form A of the compound of Formula I-1, comprising adding the compound of Formula I-1 and L-tartaric acid to acetonitrile, and then performing recrystallization or slurrying to obtain it, wherein the ratio of the compound of Formula I-1 to L-tartaric acid is selected from 0.8 to 1.2.

The present invention also provides the hippurate crystal form A of the compound of Formula I-1, and its X-ray powder diffraction pattern has characteristic diffraction peaks at the following 2θ angles: 6.17±0.2°, 6.52±0.2°, 12.30+0.2°, 14.18+0.2°, 15.62±0.2°, 16.06 ± 0.2°, 16.78 ± 0.2°, 17.64 ± 0.2°, 18.78±0.2°, 20.42±0.2°, 21.15±0.2°, 21.29±0.2°, 21.79±0.2°, 24.42±0.2°, 26.45±0.2°.

In some embodiments of the present invention, the X-ray powder diffraction pattern of the hippurate crystal form A of the compound of Formula I-1 has characteristic diffraction peaks at the following 2θ angles: 6.17±0.2°, 6.52±0.2°, 8.77±0.2°, 10.74±0.2°, 11.16± 0.2°, 12.30±0.2°, 14.18±0.2°, 14.70±0.2°, 15.62±0.2°, 16.06±0.2°, 16.78±0.2°, 17.64±0.2°, 18.78±0.2°, 20.42±0.2°, 21.15±0.2°, 21.29±0.2°, 21.79±0.2°, 23.03±0.2°, 24.05±0.2°, 24.42±0.2°, 25.32±0.2°, 26.45±0.2°, 26.87±0.2°, 28.85±0.2°.

In some embodiments of the present invention, the XRPD pattern of the hippurate crystal form A of the compound of Formula I-1 is shown in Figure 24.

**Table 12 XRPD analysis data for the hippurate crystal form A of the compound of Formula I-1**

| Name | Angle | d Value | Net Intensity | Gross Intensity | Rel. Intensity | FWHM |
|---|---|---|---|---|---|---|
| Peak #1 | 6.165727 | 14.32313 | 46.84962 | 63.97629 | 0.3607576 | 0.100 |
| Peak #2 | 6.518609 | 13.54852 | 37.30031 | 54.89573 | 0.2872247 | 0.183 |
| Peak #3 | 8.770015 | 10.07479 | 10.25155 | 31.52564 | 0.07894034 | 0.100 |
| Peak #4 | 10.73528 | 8.23445 | 17.72863 | 43.06414 | 0.1365163 | 0.199 |
| Peak #5 | 11.15514 | 7.925448 | 11.28448 | 37.4897 | 0.08689421 | 0.131 |
| Peak #6 | 12.30332 | 7.188276 | 107.018 | 134.0704 | 0.8240739 | 0.100 |
| Peak #7 | 14.18347 | 6.239351 | 32.73286 | 58.3698 | 0.2520539 | 0.100 |
| Peak #8 | 14.69591 | 6.022922 | 13.75151 | 40.19582 | 0.1058912 | 0.226 |
| Peak #9 | 15.6192 | 5.668907 | 24.5425 | 53.66223 | 0.1889853 | 0.216 |
| Peak #10 | 16.064 | 5.512926 | 40.11842 | 71.12805 | 0.3089251 | 0.160 |
| Peak #11 | 16.78293 | 5.278353 | 30.71124 | 63.47039 | 0.2364867 | 0.100 |
| Peak #12 | 17.64042 | 5.02366 | 38.97564 | 76.05798 | 0.3001254 | 0.146 |
| Peak #13 | 18.78433 | 4.720233 | 129.8645 | 172.2274 | 1 | 0.106 |
| Peak #14 | 20.41602 | 4.346517 | 36.85044 | 83.07684 | 0.2837606 | 0.194 |
| Peak #15 | 21.14778 | 4.197742 | 32.82731 | 78.65607 | 0.2527812 | 0.181 |
| Peak #16 | 21.29136 | 4.169756 | 29.05938 | 74.61411 | 0.2237668 | 0.155 |
| Peak #17 | 21.79278 | 4.074936 | 27.59339 | 71.68633 | 0.2124783 | 0.186 |
| Peak #18 | 23.02744 | 3.859168 | 22.89831 | 60.05222 | 0.1763246 | 0.100 |
| Peak #19 | 24.05033 | 3.697299 | 16.04067 | 52.94743 | 0.1235185 | 0.171 |
| Peak #20 | 24.42373 | 3.641612 | 21.46288 | 58.33859 | 0.1652713 | 0.230 |
| Peak #21 | 25.32431 | 3.514113 | 21.20499 | 56.21917 | 0.1632854 | 0.182 |
| Peak #22 | 26.44801 | 3.367302 | 20.40374 | 50.96436 | 0.1571156 | 0.203 |
| Peak #23 | 26.8689 | 3.315503 | 12.99294 | 41.55479 | 0.1000499 | 0.117 |
| Peak #24 | 28.85276 | 3.091887 | 14.44897 | 46.59393 | 0.1112619 | 0.138 |

The present invention also provides a method for preparing the hippurate crystal form A of compound of Formula I-1, comprising adding the compound of Formula I-1 and hippuric acid to tetrahydrofuran, and then performing recrystallization or slurrying to obtain it, wherein the ratio of compound of Formula I-1 to hippuric acid is selected from 0.8 to 1.2.

The present invention also provides the glutarate crystal form A of the compound of Formula I-1, and its X-ray powder diffraction pattern has characteristic diffraction peaks at the following 2θ angles: 11.82±0.2°, 12.75±0.2°, 13.19±0.2°, 14.87±0.2°, 15.10±0.2°, 17.10±0.2°, 17.82±0.2°, 18.35±0.2°, 19.75±0.2°, 20.14±0.2°, 20.70±0.2°, 22.23±0.2°, 23.20±0.2°, 24.26±0.2°, 24.47±0.2°, 29.92±0.2°.

In some embodiments of the present invention, the X-ray powder diffraction pattern of the glutarate crystal form A of the compound of Formula I-1 has characteristic diffraction peaks at the following 2θ angles: 9.86±0.2°, 10.97±0.2°, 11.82±0.2°, 12.29±0.2°, 12.75 ± 0.2°, 13.19 ± 0.2°, 14.87 ± 0.2°, 15.10 ± 0.2°, 15.37 ± 0.2°, 16.33 ± 0.2°, 17.10 ± 0.2°, 17.82±0.2°, 18.35±0.2°, 19.17±0.2°, 19.75±0.2°, 20.14±0.2°, 20.70±0.2°, 21.14±0.2°, 22.23±0.2°, 23.20±0.2°, 24.26±0.2°, 24.47±0.2°, 24.86±0.2°, 25.28±0.2°, 29.92±0.2°.

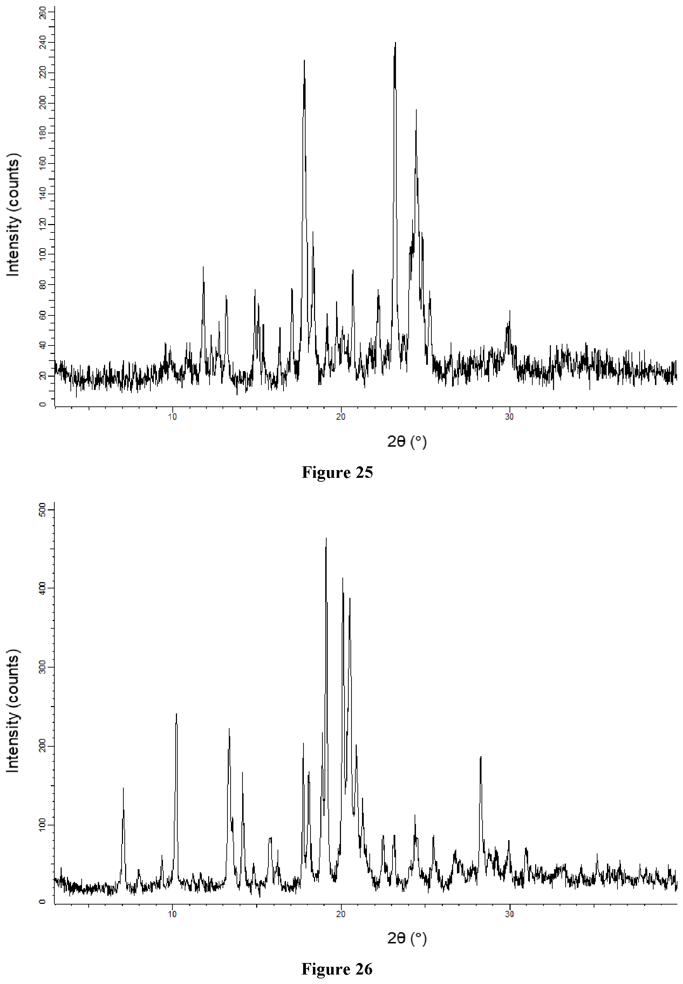

In some embodiments of the present invention, the XRPD pattern of the glutarate crystal form A of the compound of Formula I-1 is shown in Figure 25.

**Table 13 XRPD analysis data for the glutarate crystal form A of the compound of Formula I-1**

| Name | Angle | d Value | Net Intensity | Gross Intensity | Rel. Intensity | FWHM |
|---|---|---|---|---|---|---|
| Peak #1 | 9.863811 | 8.959923 | 12.85162 | 32.81104 | 0.08830153 | 0.172 |
| Peak #2 | 10.98613 | 8.046987 | 10.89521 | 32.04998 | 0.07485931 | 0.116 |
| Peak #3 | 11.82328 | 7.479033 | 49.71856 | 70.34775 | 0.3416087 | 0.100 |
| Peak #4 | 12.28926 | 7.196469 | 17.15315 | 37.49989 | 0.1178567 | 0.218 |
| Peak #5 | 12.74523 | 6.94002 | 19.68536 | 39.10459 | 0.1352551 | 0.100 |
| Peak #6 | 13.18652 | 6.708745 | 33.55265 | 51.90237 | 0.2305351 | 0.100 |
| Peak #7 | 14.8743 | 5.951086 | 39.69448 | 53.88385 | 0.2727347 | 0.129 |
| Peak #8 | 15.09693 | 5.863826 | 34.88612 | 49.31273 | 0.2396972 | 0.216 |
| Peak #9 | 15.37447 | 5.758588 | 31.72464 | 46.23314 | 0.2179752 | 0.115 |
| Peak #10 | 16.33454 | 5.422222 | 16.56744 | 32.81384 | 0.1138324 | 0.100 |
| Peak #11 | 17.103 | 5.180287 | 40.75071 | 60.68394 | 0.2799919 | 0.100 |
| Peak #12 | 17.81628 | 4.974467 | 117.4363 | 139.108 | 0.8068871 | 0.142 |
| Peak #13 | 18.34729 | 4.831675 | 68.64827 | 90.59112 | 0.4716718 | 0.100 |
| Peak #14 | 19.17292 | 4.625432 | 24.33756 | 46.22366 | 0.1672196 | 0.100 |
| Peak #15 | 19.7465 | 4.492352 | 38.56743 | 60.27261 | 0.2649909 | 0.156 |
| Peak #16 | 20.14022 | 4.405412 | 21.05011 | 42.03999 | 0.1446321 | 0.236 |
| Peak #17 | 20.69634 | 4.288275 | 50.58425 | 69.74656 | 0.3475567 | 0.100 |
| Peak #18 | 21.13538 | 4.200177 | 21.00321 | 38.04622 | 0.1443098 | 0.128 |
| Peak #19 | 22.23299 | 3.99524 | 31.02705 | 52.27459 | 0.2131821 | 0.116 |
| Peak #20 | 23.19805 | 3.83117 | 145.5424 | 170.8203 | 1 | 0.110 |
| Peak #21 | 24.25913 | 3.665946 | 52.19791 | 78.5784 | 0.3586439 | 0.282 |
| Peak #22 | 24.47135 | 3.634633 | 118.7159 | 144.8973 | 0.8156791 | 0.263 |
| Peak #23 | 24.86463 | 3.578029 | 53.22074 | 78.66595 | 0.3656716 | 0.103 |
| Peak #24 | 25.28382 | 3.519649 | 25.77899 | 49.91108 | 0.1771235 | 0.100 |
| Peak #25 | 29.92096 | 2.983892 | 13.18526 | 36.99868 | 0.09059394 | 0.107 |

The present invention also provides a method for preparing the glutarate crystal form A of the compound of Formula I-1, comprising adding the compound of Formula I-1 and glutaric acid to acetonitrile and dichloromethane, and then performing recrystallization or slurrying to obtain it, wherein the ratio of the compound of Formula I-1 to glutaric acid is selected from 0.8 to 1.2.

The present invention also provides the p-toluenesulfonate crystal form A of the compound of Formula I-1, and its X-ray powder diffraction pattern has characteristic diffraction peaks at the following 2θ angles: 7.08 ±0.2°, 10.23±0.2°, 13.36±0.2°, 14.16± 0.2°, 17.77 ± 0.2°, 18.10 ± 0.2°, 18.90 ± 0.2 °, 19.12 ± 0.2°, 20.11 ± 0.2°, 20.53 ± 0.2°, 20.91 ± 0.2°, 24.41±0.2°, 25.51±0.2°, 28.28±0.2°, 29.95±0.2°, 30.98±0.2°, 35.22±0.2°.

In some embodiments of the present invention, the X-ray powder diffraction pattern of the p-toluenesulfonate crystal form A of the compound of Formula I-1 has characteristic diffraction peaks at the following 2θ angles: 7.08 ±0.2°, 8.00±0.2°, 9.39±0.2°, 10.23 ±0.2°, 13.36 ± 0.2°, 14.16 ± 0.2°, 14.81 ± 0.2°, 15.77 ± 0.2°, 16.21 ± 0.2°, 17.77 ± 0.2°, 18.10 + 0.2°, 18.90±0.2°, 19.12±0.2°, 20.11±0.2°, 20.53±0.2°, 20.91±0.2°, 21.29±0.2°, 22.49 ±0.2°, 23.11±0.2°, 24.41±0.2°, 25.51±0.2°, 26.77±0.2°, 28.28±0.2°, 28.81±0.2°, 29.22 0.2°, 29.95±0.2°, 30.98±0.2°, 35.22±0.2°.

In some embodiments of the present invention, the X-ray powder diffraction (XRPD) pattern of the p-toluenesulfonate crystal form A of the compound of Formula I-1 is shown in Figure 26.

**Table 14 XRPD analysis data for the p-toluenesulfonate crystal form A of the compound of Formula I-1**

| Name | Angle | d Value | Net Intensity | Gross Intensity | Rel. Intensity | FWHM |
|---|---|---|---|---|---|---|
| Peak #1 | 7.079945 | 12.47553 | 85.42383 | 101.1101 | 0.2852689 | 0.100 |
| Peak #2 | 7.997392 | 11.0463 | 13.96909 | 31.70288 | 0.04664912 | 0.102 |
| Peak #3 | 9.390173 | 9.410768 | 23.25926 | 45.09923 | 0.07767321 | 0.100 |
| Peak #4 | 10.22862 | 8.641167 | 147.574 | 170.6116 | 0.4928164 | 0.100 |
| Peak #5 | 13.36138 | 6.621338 | 128.1409 | 150.1271 | 0.4279206 | 0.106 |
| Peak #6 | 14.15906 | 6.250054 | 101.5931 | 123.0315 | 0.3392653 | 0.100 |
| Peak #7 | 14.81465 | 5.974915 | 18.95654 | 40.14683 | 0.06330448 | 0.100 |
| Peak #8 | 15.77269 | 5.614086 | 40.74862 | 62.03128 | 0.1360781 | 0.148 |
| Peak #9 | 16.20564 | 5.46506 | 21.01715 | 41.812 | 0.07018578 | 0.100 |
| Peak #10 | 17.76859 | 4.98771 | 130.6191 | 157.9745 | 0.4361964 | 0.159 |
| Peak #11 | 18.10162 | 4.896693 | 89.64201 | 119.1474 | 0.2993553 | 0.175 |
| Peak #12 | 18.89753 | 4.692213 | 123.9464 | 156.6549 | 0.4139133 | 0.146 |
| Peak #13 | 19.11654 | 4.638949 | 299.4503 | 332.5616 | 1 | 0.100 |
| Peak #14 | 20.11447 | 4.410995 | 277.3101 | 311.3669 | 0.9260639 | 0.100 |
| Peak #15 | 20.52951 | 4.322745 | 236.2305 | 269.9984 | 0.7888807 | 0.164 |
| Peak #16 | 20.90733 | 4.245474 | 119.2478 | 152.1095 | 0.3982224 | 0.239 |
| Peak #17 | 21.2862 | 4.170756 | 69.93179 | 101.2655 | 0.2335339 | 0.129 |
| Peak #18 | 22.49223 | 3.949774 | 43.0219 | 68.77164 | 0.1436696 | 0.100 |
| Peak #19 | 23.11408 | 3.844899 | 30.92543 | 55.90915 | 0.103274 | 0.101 |
| Peak #20 | 24.4086 | 3.643835 | 70.81258 | 94.66067 | 0.2364753 | 0.125 |
| Peak #21 | 25.51375 | 3.488449 | 44.21862 | 66.18475 | 0.147666 | 0.100 |
| Peak #22 | 26.77312 | 3.327147 | 28.30337 | 54.19617 | 0.09451779 | 0.228 |
| Peak #23 | 28.28136 | 3.153044 | 122.5069 | 156.2957 | 0.409106 | 0.100 |
| Peak #24 | 28.80565 | 3.096838 | 18.56351 | 52.80252 | 0.06199198 | 0.183 |
| Peak #25 | 29.21899 | 3.053962 | 27.92734 | 61.68496 | 0.09326204 | 0.145 |
| Peak #26 | 29.94764 | 2.981295 | 30.47235 | 61.59036 | 0.101761 | 0.100 |
| Peak #27 | 30.98179 | 2.884098 | 24.56855 | 53.25895 | 0.0820455 | 0.117 |
| Peak #28 | 35.21519 | 2.546478 | 18.38519 | 46.0497 | 0.06139646 | 0.100 |

The present invention also provides a method for preparing the p-toluenesulfonate crystal form A of the compound of Formula I-1, comprising adding the compound of Formula I-1 and p-toluenesulfonic acid to acetonitrile, and then performing recrystallization or slurrying to obtain it, wherein the ratio of the compound of Formula I-1 to p-toluenesulfonic acid is selected from 0.8 to 1.2.

The present invention also provides the p-toluenesulfonate crystal form B of the compound of Formula I-1, and its X-ray powder diffraction pattern has characteristic diffraction peaks at the following 2θ angles: 8.27 ± 0.2^{°}, 13.69 ± 0.2^{°}, 13.95 ± 0.2^{°}, 16.15 ± 0.2^{°}, 17.72 ± 0.2^{°}, 19.52 ± 0.2^{°}, 21.05 ± 0.2^{°}, 22.45 ± 0.2^{°}, 23.91 ± 0.2°, 25.40 ± 0.2^{°}, 27.33 ± 0.2^{°}, 29.25 ± 0. °.

In some embodiments of the present invention, the X-ray powder diffraction pattern of the p-toluenesulfonate crystal form B of the compound of Formula I-1 has characteristic diffraction peaks at the following 2θ angles: 8.27±0.2°, 12.59±0.2°, 13.69±0.2°, 13.95± 0.2°, 14.63 ± 0.2 °, 16.15 ± 0.2°, 17.72 ± 0.2 °, 18.68 ± 0.2 °, 19.52 ± 0.2°, 20.75 ± 0.2°, 21.05 ± 0.2°, 21.92±0.2°, 22.45±0.2°, 23.23±0.2°, 23.91±0.2°, 25.40±0.2°, 27.33±0.2°, 29.25±0.2°.

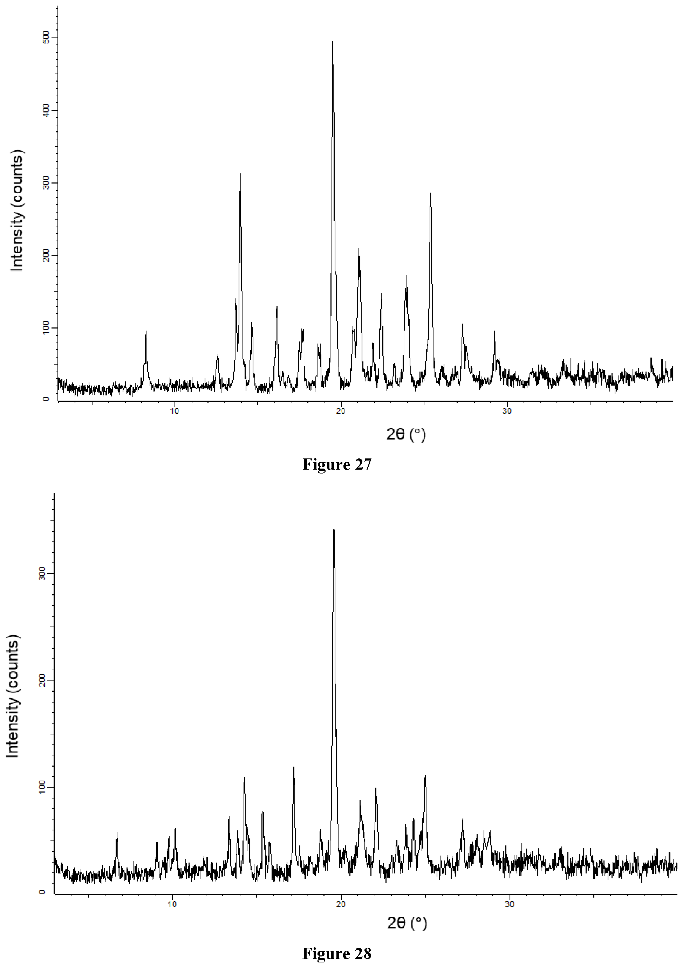

In some embodiments of the present invention, the XRPD pattern of the p-toluenesulfonate crystal form B of the compound of Formula I-1 is shown in Figure 27.

**Table 15 XRPD analysis data for the p-toluenesulfonate crystal form B of the compound of Formula I-1**

| Name | Angle | d Value | Net Intensity | Gross Intensity | Rel. Intensity | FWHM |
|---|---|---|---|---|---|---|
| Peak #1 | 8.269233 | 10.68376 | 66.65063 | 82.69354 | 0.205002 | 0.100 |
| Peak #2 | 12.58982 | 7.025336 | 25.67633 | 42.4886 | 0.07897448 | 0.113 |
| Peak #3 | 13.68799 | 6.464071 | 66.10264 | 84.78497 | 0.2033165 | 0.100 |
| Peak #4 | 13.95157 | 6.342537 | 205.2721 | 224.0923 | 0.6313697 | 0.100 |
| Peak #5 | 14.63412 | 6.048212 | 56.90355 | 74.64372 | 0.1750222 | 0.100 |
| Peak #6 | 16.14528 | 5.485354 | 75.07107 | 90.46928 | 0.2309013 | 0.100 |
| Peak #7 | 17.7228 | 5.000495 | 52.54952 | 70.41904 | 0.1616302 | 0.161 |
| Peak #8 | 18.68129 | 4.746037 | 24.92735 | 44.40676 | 0.0766708 | 0.106 |
| Peak #9 | 19.51727 | 4.544596 | 325.1219 | 346.1157 | 1 | 0.100 |
| Peak #10 | 20.74879 | 4.277555 | 39.79155 | 64.27892 | 0.1223896 | 0.131 |
| Peak #11 | 21.04592 | 4.217828 | 131.2454 | 156.5197 | 0.4036806 | 0.142 |
| Peak #12 | 21.91903 | 4.051752 | 33.07592 | 58.38848 | 0.1017339 | 0.100 |
| Peak #13 | 22.4462 | 3.95777 | 89.58733 | 113.2832 | 0.27555 | 0.104 |
| Peak #14 | 23.22516 | 3.82676 | 15.41252 | 37.83067 | 0.04740537 | 0.159 |
| Peak #15 | 23.9116 | 3.718435 | 104.2562 | 125.6726 | 0.3206681 | 0.176 |
| Peak #16 | 25.40249 | 3.503475 | 177.7236 | 200.8911 | 0.5466368 | 0.100 |
| Peak #17 | 27.3258 | 3.261095 | 60.00591 | 86.0919 | 0.1845644 | 0.141 |
| Peak #18 | 29.24715 | 3.051085 | 41.28314 | 68.31211 | 0.1269774 | 0.100 |

The present invention also provides a method for preparing the p-toluenesulfonate crystal form B of the compound of Formula I-1, comprising adding the compound of Formula I-1 and p-toluenesulfonic acid to tetrahydrofuran, and then performing recrystallization or slurrying to obtain it, wherein the ratio of the compound of Formula I-1 to p-toluenesulfonic acid is selected from the 0.8 to 1.2.

The present invention also provides the methanesulfonate crystal form A of the compound of Formula I-1, and its X-ray powder diffraction pattern has characteristic diffraction peaks at the following 2θ angles: 6.70 ± 0.2°, 9.10 ± 0.2°, 9.81 ± 0.2°, 10.16 ± 0.2°, 13.34 ± 0.2°, 13.87 ± 0.2°, 14.27 ± 0.2°, 15.37 ± 0.2°, 17.21 ± 0.2°, 19.57 ± 0.2°, 21.16 ± 0.2°, 22.09 ± 0.2°, 23.86 ± 0.2°, 24.30 ± 0.2°, 24.99 ± 0.2° , 27.18 ± 0.2°.

In some embodiments of the present invention, the X-ray powder diffraction pattern of the methanesulfonate crystal form A of the compound of Formula I-1 has characteristic diffraction peaks at the following 2θ angles: 6.70±0.2°, 9.10±0.2°, 9.81±0.2°, 10.16±0.2°, 13.34±0.2°, 13.87±0.2°, 14.27±0.2°, 15.37±0.2°, 15.79±0.2°, 17.21±0.2°, 18.75±0.2°, 19.57±0.2°, 21.16±0.2°, 22.09±0.2°, 23.31±0.2°, 23.86±0.2°, 24.30±0.2°, 24.99±0.2°, 27.18±0.2°, 28.04±0.2°, 28.83±0.2°.

In some embodiments of the present invention, the XRPD pattern of methanesulfonate crystal form A of the compound of Formula I-1 is shown in Figure 28.

**Table 16 XRPD analysis data for the methanesulfonate crystal form A of the compound of Formula I-1**

| Name | Angle | d Value | Net Intensity | Gross Intensity | Rel. Intensity | FWHM |
|---|---|---|---|---|---|---|
| Peak #1 | 6.697615 | 13.18681 | 29.2205 | 44.84843 | 0.1374156 | 0.100 |
| Peak #2 | 9.102916 | 9.707085 | 21.63046 | 38.52872 | 0.1017218 | 0.100 |
| Peak #3 | 9.807705 | 9.011051 | 21.38057 | 39.66219 | 0.1005466 | 0.143 |
| Peak #4 | 10.16039 | 8.699037 | 29.45585 | 47.7616 | 0.1385223 | 0.161 |
| Peak #5 | 13.34429 | 6.629781 | 27.07287 | 46.42973 | 0.1273159 | 0.100 |
| Peak #6 | 13.87172 | 6.378866 | 29.43882 | 48.18745 | 0.1384423 | 0.100 |
| Peak #7 | 14.27292 | 6.20045 | 66.19603 | 83.79364 | 0.3113008 | 0.100 |
| Peak #8 | 15.36627 | 5.761644 | 37.38631 | 53.25999 | 0.175817 | 0.100 |
| Peak #9 | 15.78599 | 5.609385 | 17.09895 | 33.88383 | 0.0804114 | 0.134 |
| Peak #10 | 17.21072 | 5.148107 | 48.49517 | 67.62392 | 0.2280588 | 0.100 |
| Peak #11 | 18.75375 | 4.727863 | 28.35833 | 51.94065 | 0.133361 | 0.100 |
| Peak #12 | 19.57472 | 4.531386 | 212.6433 | 239.0555 | 1 | 0.110 |
| Peak #13 | 21.16175 | 4.195003 | 37.02691 | 62.74215 | 0.1741268 | 0.160 |
| Peak #14 | 22.09032 | 4.020719 | 46.08796 | 67.99896 | 0.2167384 | 0.100 |
| Peak #15 | 23.31411 | 3.81236 | 19.21252 | 40.88506 | 0.09035092 | 0.173 |
| Peak #16 | 23.85573 | 3.727016 | 34.5036 | 58.00617 | 0.1622605 | 0.100 |
| Peak #17 | 24.30463 | 3.659186 | 33.63316 | 57.83051 | 0.158167 | 0.190 |
| Peak #18 | 24.98785 | 3.560663 | 65.20639 | 89.03184 | 0.3066468 | 0.106 |
| Peak #19 | 27.18207 | 3.278012 | 31.54118 | 56.58994 | 0.148329 | 0.104 |
| Peak #20 | 28.04009 | 3.179624 | 18.07375 | 44.88169 | 0.08499562 | 0.164 |
| Peak #21 | 28.8345 | 3.093804 | 24.47278 | 50.48177 | 0.1150884 | 0.249 |

The present invention also provides a method for preparing methanesulfonate crystal form A of the compound of Formula I-1, comprising adding the compound of Formula I-1 and methanesulfonic acid to tetrahydrofuran, and then performing recrystallization or slurrying to obtain it, wherein the ratio of the compound of Formula I-1 to methanesulfonic acid is selected from 0.8 to 1.2.

The present invention also provides the hydrochloride crystal form C1 of the compound of Formula I-1, and its X-ray powder diffraction pattern has characteristic diffraction peaks at the following 2θ angles: 7.47±0.2°, 7.73±0.2°, 12.25±0.2°, 13.18±0.2°, 13.51 ± 0.2 °, 14.96 ± 0.2°, 15.46 ± 0.2°, 17.31 ± 0.2° , 18.56 ± 0.2°, 19.34 ± 0.2°, 19.57 ± 0.2°, 19.78±0.2°, 20.01±0.2°, 20.41±0.2°, 20.76±0.2°, 24.36±0.2°, 25.63±0.2°, 26.47±0.2°, 27.51±0.2°.

In some embodiments of the present invention, the X-ray powder diffraction pattern of the hydrochloride crystal form C1 of the compound of Formula I-1 has characteristic diffraction peaks at the following 2θ angles: 7.47±0.2°, 7.73±0.2°, 9.63±0.2°, 11.46±0.2°, 12.25 ± 0.2^{°}, 13.18 ± 0.2^{°}, 13.51 ± 0.2^{°}, 14.96 ± 0.2^{°}, 15.46 ± 0.2^{°}, 16.79 ± 0.2^{°}, 17.04 ± 0.2^{°}, 17.31±0.2°, 17.72±0.2°, 18.56±0.2°, 19.34±0.2°, 19.57±0.2°, 19.78±0.2°, 20.01±0.2°, 20.41±0.2°, 20.76±0.2°, 22.60±0.2°, 23.09±0.2°, 23.80±0.2°, 24.36±0.2°, 25.63±0.2°, 26.47±0.2°, 27.51±0.2°, 29.09±0.2°, 30.76±0.2°, 31.99±0.2°, 36.24±0.2°.

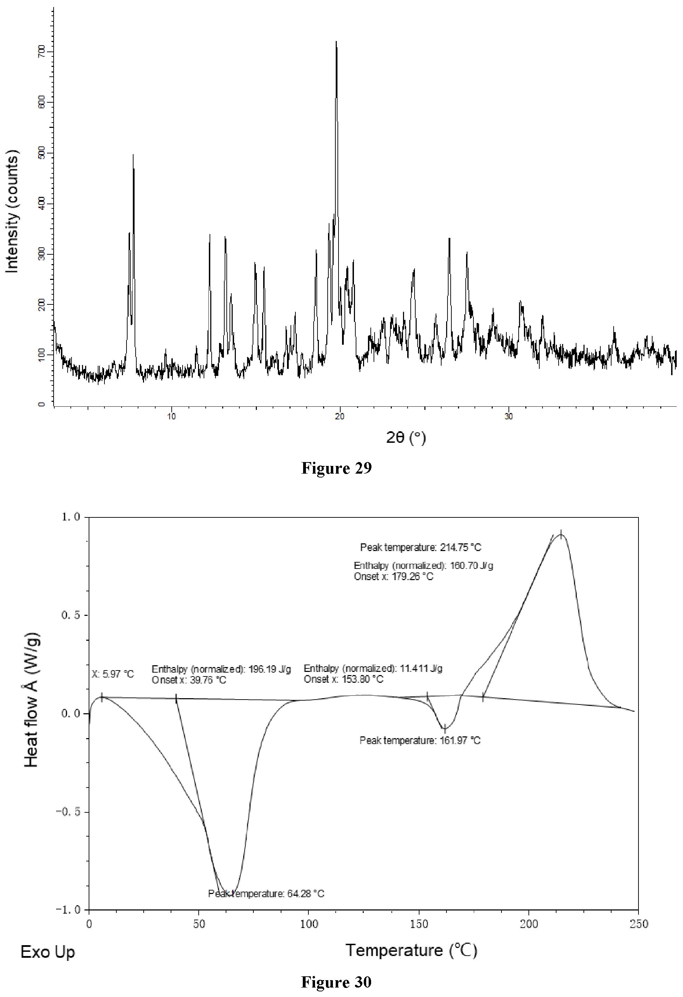

In some embodiments of the present invention, the X-ray diffraction (XRPD) pattern of the hydrochloride crystal form C1 of the compound of Formula I-1 is shown in Figure 29.

**Table 17 XRPD analysis data for the hydrochloride crystal form C1 of the compound of Formula I-1**

| Name | Angle | d Value | Net Intensity | Gross Intensity | Rel. Intensity | FWHM |
|---|---|---|---|---|---|---|
| Peak #1 | 7.469565 | 11.82564 | 170.3781 | 233.8982 | 0.3741632 | 0.165 |
| Peak # 2 | 7.727614 | 11.43132 | 288.2178 | 351.6654 | 0.6329481 | 0.135 |
| Peak # 3 | 9.625598 | 9.181119 | 30.23456 | 95.12409 | 0.06639738 | 0.154 |
| Peak # 4 | 11.46039 | 7.715022 | 35.40674 | 99.13931 | 0.07775588 | 0.100 |
| Peak # 5 | 12.25008 | 7.219398 | 187.329 | 254.1297 | 0.4113886 | 0.100 |
| Peak # 6 | 13.18162 | 6.711229 | 176.609 | 244.8173 | 0.3878468 | 0.165 |
| Peak # 7 | 13.50989 | 6.548882 | 101.0302 | 168.6256 | 0.22187 | 0.120 |
| Peak # 8 | 14.96371 | 5.915727 | 130.4177 | 199.7892 | 0.2864071 | 0.106 |
| Peak # 9 | 15.46443 | 5.725291 | 145.8036 | 215.9147 | 0.3201957 | 0.100 |
| Peak # 10 | 16.79406 | 5.27488 | 51.23669 | 120.018 | 0.1125197 | 0.134 |
| Peak # 11 | 17.03815 | 5.199857 | 68.14204 | 136.5628 | 0.1496451 | 0.220 |
| Peak # 12 | 17.31437 | 5.117519 | 68.7573 | 136.3839 | 0.1509962 | 0.143 |
| Peak # 13 | 17.72217 | 5.000671 | 20.89011 | 88.21156 | 0.04587625 | 0.100 |
| Peak # 14 | 18.56032 | 4.776697 | 158.9897 | 234.8893 | 0.3491534 | 0.100 |
| Peak # 15 | 19.33619 | 4.586743 | 193.7012 | 275.9999 | 0.4253825 | 0.106 |
| Peak # 16 | 19.57052 | 4.532349 | 243.5362 | 327.1351 | 0.5348239 | 0.151 |
| Peak # 17 | 19.78009 | 4.4848 | 455.3577 | 539.8668 | 1 | 0.100 |
| Peak # 18 | 20.01093 | 4.433581 | 150.447 | 235.6875 | 0.330393 | 0.100 |
| Peak # 19 | 20.41269 | 4.347219 | 117.9435 | 203.7759 | 0.2590128 | 0.250 |
| Peak # 20 | 20.7621 | 4.274841 | 143.6874 | 229.3326 | 0.3155484 | 0.146 |
| Peak # 21 | 22.59736 | 3.931636 | 57.59861 | 153.2617 | 0.1264909 | 0.179 |
| Peak # 22 | 23.0917 | 3.848574 | 47.79654 | 145.3939 | 0.1049648 | 0.222 |
| Peak # 23 | 23.80311 | 3.735136 | 47.23293 | 145.9101 | 0.1037271 | 0.250 |
| Peak # 24 | 24.35631 | 3.65154 | 118.0796 | 215.7209 | 0.2593118 | 0.170 |
| Peak # 25 | 25.63398 | 3.472358 | 54.12675 | 146.5311 | 0.1188664 | 0.100 |
| Peak # 26 | 26.4743 | 3.364018 | 165.1317 | 261.6809 | 0.3626417 | 0.106 |
| Peak # 27 | 27.51233 | 3.239408 | 144.2079 | 250.5756 | 0.3166913 | 0.122 |
| Peak # 28 | 29.09444 | 3.066751 | 56.66905 | 168.875 | 0.1244495 | 0.150 |
| Peak # 29 | 30.76101 | 2.904293 | 47.74388 | 156.956 | 0.1048492 | 0.218 |
| Peak # 30 | 31.99164 | 2.795325 | 54.65399 | 155.6174 | 0.1200243 | 0.100 |
| Peak # 31 | 36.23993 | 2.476788 | 45.47268 | 131.7606 | 0.09986144 | 0.111 |

In some embodiments of the present invention, the hydrochloride crystal form C1 of the compound of Formula I-1 has the onsets of the endothermic peaks at 39.76°C ± 2^{°}C and 153.80^{°}C ± 2^{°}C in its differential scanning calorimetry (DSC) curve, and decomposition begins above 214.75°C ± 2^{°}C.

In some embodiments of the present invention, the DSC pattern of the hydrochloride crystal form C1 of the compound of Formula I-1 is shown in Figure 30.

In some embodiments of the present invention, the hydrochloride crystal form C1 of the compound of Formula I-1 has three weight loss steps at 26.03°C ± 2^{°}C, 100.00°C ± 2^{°}C, and 150.00^{°}C ± 2^{°}C in its thermogravimetric analysis (TGA) curve.

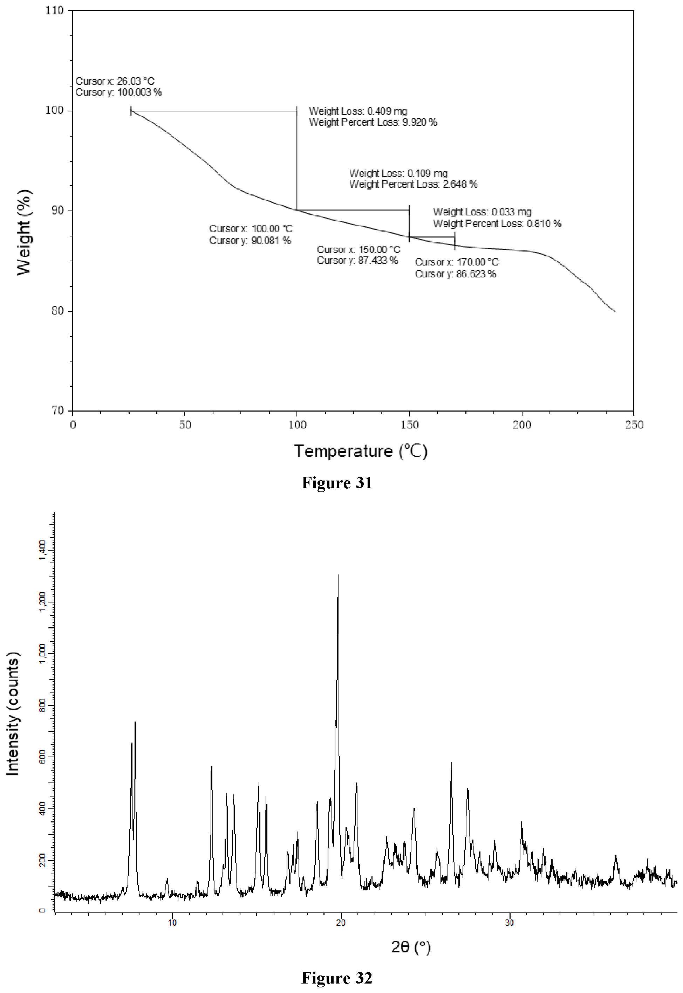

In some embodiments of the present invention, the TGA pattern of the hydrochloride crystal form C1 of the compound of Formula I-1 is shown in Figure 31.

The present invention also provides a method for preparing the hydrochloride crystal form C1 of the compound of Formula I-1, comprising adding the compound of Formula I-1 and hydrochloric acid to ethyl acetate, and then performing recrystallization or slurrying to obtain it, wherein the ratio of the compound of Formula I-1 to hydrochloric acid is selected from 0.8 to 1.2, the hydrochloride crystal form C1 of the compound of Formula I-1 is an ethyl acetate solvate and hydrate crystal form, with an ethyl acetate content of 0.62 equivalents and a water content of 3.0 equivalents.

The present invention also provides the hydrochloride crystal form C2 of the compound of Formula I-1, and its X-ray powder diffraction pattern has characteristic diffraction peaks at the following 2θ angles: 7.56±0.2°, 7.79±0.2^{°}, 12.32 ± 0.2^{°}, 13.21 ± 0.2^{°}, 13.63 ± 0.2^{°}, 15.10 ± 0.2°, 15.56 ± 0.2°, 18.58 ± 0.2°, 19.36 ± 0.2°, 19.66±0.2°, 19.82±0.2°, 20.91±0.2°, 24.35±0.2°, 26.56±0.2°, 30.74±0.2°.

In some embodiments of the present invention, the X-ray powder diffraction pattern of the hydrochloride crystal form C2 of the compound of Formula I-1 has characteristic diffraction peaks at the following 2θ angles: 7.56±0.2°, 7.79±0.2°, 9.65±0.2°, 11.48±0.2°, 12.32 ± 0.2°, 13.21 ± 0.2°, 13.63 ± 0.2°, 15.10 ± 0.2°, 15.56 ± 0.2°, 16.86±0.2°, 17.16±0.2°, 17.41±0.2°, 17.77±0.2°, 18.58±0.2°, 19.36±0.2°, 19.66±0.2°, 19.82±0.2°, 20.32±0.2°, 20.91±0.2°, 22.71±0.2°, 23.21±0.2°, 23.80±0.2°, 24.35±0.2°, 25.71±0.2°, 26.56±0.2°, 27.53±0.2°, 27.80±0.2°, 28.20±0.2°, 29.15±0.2°, 30.74±0.2°, 32.06±0.2°, 36.30±0.2°, 38.22±0.2°.

In some embodiments of the present invention, the X-ray diffraction (XRPD) pattern of the hydrochloride crystal form C2 of the compound of Formula I-1 is shown in Figure 32.

**Table 18 XRPD analysis data for the hydrochloride crystal form C2 of the compound of Formula I-1**

| Name | Angle | d Value | Net Intensity | Gross Intensity | Rel. Intensity | FWHM |
|---|---|---|---|---|---|---|
| Peak #1 | 7.561379 | 11.68225 | 594.0467 | 654.5096 | 0.4930706 | 0.153 |
| Peak # 2 | 7.785987 | 11.34575 | 665.0544 | 725.5987 | 0.5520085 | 0.143 |
| Peak # 3 | 9.653943 | 9.154226 | 64.07269 | 124.0171 | 0.05318161 | 0.100 |
| Peak # 4 | 11.47599 | 7.704572 | 59.76658 | 119.5923 | 0.04960746 | 0.100 |
| Peak # 5 | 12.32226 | 7.177275 | 486.3391 | 550.8688 | 0.4036712 | 0.100 |
| Peak # 6 | 13.20849 | 6.697635 | 382.8189 | 452.7995 | 0.3177474 | 0.100 |
| Peak # 7 | 13.63449 | 6.489313 | 383.4615 | 454.04 | 0.3182808 | 0.102 |
| Peak # 8 | 15.10227 | 5.861764 | 417.983 | 490.954 | 0.3469343 | 0.110 |
| Peak # 9 | 15.55625 | 5.691705 | 359.4082 | 431.791 | 0.298316 | 0.100 |
| Peak # 10 | 16.8609 | 5.254121 | 157.987 | 227.7439 | 0.1311324 | 0.100 |
| Peak # 11 | 17.16267 | 5.162411 | 180.6607 | 252.0321 | 0.149952 | 0.249 |
| Peak # 12 | 17.41028 | 5.089545 | 224.9247 | 297.1292 | 0.186692 | 0.132 |
| Peak # 13 | 17.77288 | 4.986517 | 60.75385 | 133.3784 | 0.0504269 | 0.100 |
| Peak # 14 | 18.58392 | 4.770684 | 327.1711 | 410.2261 | 0.2715586 | 0.102 |
| Peak # 15 | 19.35654 | 4.581967 | 330.6791 | 422.6897 | 0.2744703 | 0.152 |
| Peak # 16 | 19.66373 | 4.511075 | 642.4008 | 736.7739 | 0.5332054 | 0.141 |
| Peak # 17 | 19.82029 | 4.475794 | 1204.79 | 1300.107 | 1 | 0.141 |
| Peak # 18 | 20.31755 | 4.36736 | 218.6828 | 315.8102 | 0.1815111 | 0.229 |
| Peak # 19 | 20.91171 | 4.244595 | 405.4756 | 502.4253 | 0.3365528 | 0.110 |
| Peak # 20 | 22.71078 | 3.912256 | 178.8163 | 287.4753 | 0.1484211 | 0.150 |
| Peak # 21 | 23.21358 | 3.828644 | 150.452 | 262.2883 | 0.1248782 | 0.283 |
| Peak # 22 | 23.79538 | 3.736332 | 153.2322 | 266.4649 | 0.1271858 | 0.169 |
| Peak # 23 | 24.35296 | 3.652033 | 290.4572 | 402.7315 | 0.2410853 | 0.185 |
| Peak # 24 | 25.71424 | 3.461702 | 106.9341 | 225.8246 | 0.08875742 | 0.149 |
| Peak # 25 | 26.56012 | 3.353343 | 433.4701 | 562.3314 | 0.3597888 | 0.100 |
| Peak # 26 | 27.52862 | 3.237527 | 341.6432 | 479.7618 | 0.2835706 | 0.127 |
| Peak # 27 | 27.8036 | 3.206129 | 133.144 | 272.6549 | 0.1105122 | 0.236 |
| Peak # 28 | 28.20096 | 3.161851 | 78.81534 | 219.3735 | 0.0654183 | 0.108 |
| Peak #29 | 29.15277 | 3.060749 | 104.427 | 242.8467 | 0.08667652 | 0.174 |
| Peak # 30 | 30.74315 | 2.90594 | 210.473 | 344.75 | 0.1746968 | 0.189 |
| Peak # 31 | 32.06215 | 2.789339 | 87.68976 | 212.1315 | 0.07278425 | 0.163 |
| Peak # 32 | 36.29795 | 2.472962 | 112.9378 | 222.361 | 0.0937406 | 0.138 |
| Peak # 33 | 38.21926 | 2.352947 | 80.73588 | 192.5904 | 0.06701239 | 0.238 |

In some embodiments of the present invention, the hydrochloride crystal form C2 of the compound of Formula I-1 has the onsets of the endothermic peaks at 33.27°C ± 2^{°}C and 155.72^{°}C ± 2^{°}C in its differential scanning calorimetry (DSC) curve, and decomposition of the sample begins above 212.45°C ± 2^{°}C.

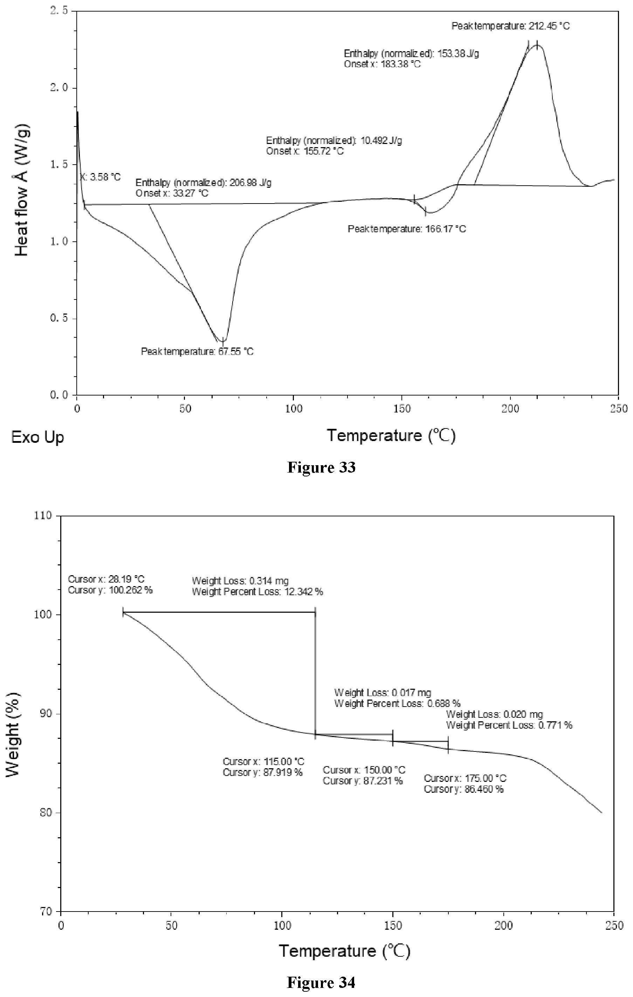

In some embodiments of the present invention, the DSC pattern of the hydrochloride crystal form C2 of the compound of Formula I-1 is shown in Figure 33.

In some embodiments of the present invention, the hydrochloride crystal form C2 of the compound of Formula I-1 has three weight loss steps at 28.19°C ± 2^{°}C, 115.00°C ± 2^{°}C, and 150.00^{°}C ± 2^{°}C in its thermogravimetric analysis (TGA) curve.

In some embodiments of the present invention, the TGA pattern of the hydrochloride crystal form C2 of the compound of Formula I-1 is shown in Figure 34.

The present invention also provides a method for preparing the hydrochloride crystal form C2 of the compound of Formula I-1, comprising adding the compound of Formula I-1 and hydrochloric acid to 2-methyl-tetrahydrofuran, and then performing recrystallization or slurrying to obtain it, wherein the ratio of the compound of Formula I-1 to hydrochloric acid is selected from 0.8 to 1.2, and the hydrochloride crystal form C2 of the compound of Formula I-1 is a 2-methyl tetrahydrofuran solvate and hydrate crystal form, with a 2-methyl tetrahydrofuran content of 0.21 equivalents and a water content of 2.8 equivalents.

The present invention also provides the hydrochloride crystal form D of the compound of Formula I-1, and its X-ray powder diffraction pattern has characteristic diffraction peaks at the following 2θ angles: 6.60±0.2°, 7.79±0.2°, 9.84±0.2°, 13.31 ± 0.2°, 15.23 ± 0.2°, 15.93 ± 0.2 °, 20.09 ± 0.2°, 20.41 ± 0.2°, 21.23 ± 0.2°, 21.80 ± 0.2 °.

In some embodiments of the present invention, the X-ray powder diffraction pattern of the hydrochloride crystal form D of the compound of Formula I-1 has characteristic diffraction peaks at the following 2θ angles: 6.60±0.2°, 7.26±0.2°, 7.79±0.2°, 8.48±0.2°, 9.84±0.2°, 11.18±0.2°, 13.31±0.2°, 13.89±0.2°, 14.64±0.2°, 14.98±0.2°, 15.23±0.2°, 15.93±0.2°, 16.27±0.2°, 17.04±0.2°, 18.21±0.2°, 19.33±0.2°, 20.09±0.2°, 20.41±0.2°, 21.23±0.2°, 21.80±0.2°, 23.49±0.2°, 24.79±0.2°, 28.43±0.2°.

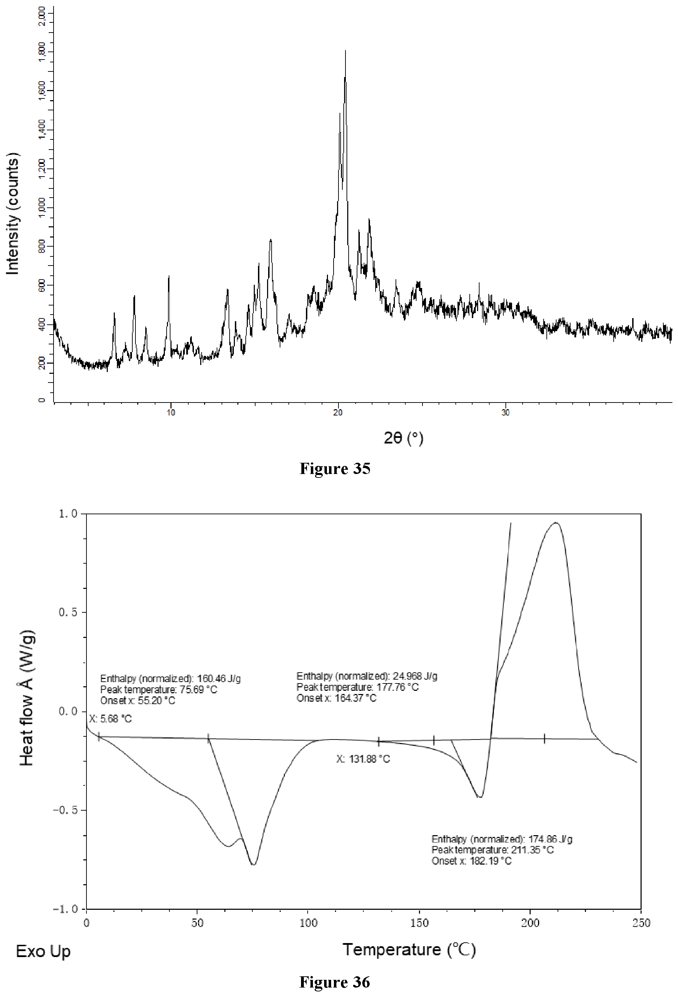

In some embodiments of the present invention, the XRPD pattern of the hydrochloride crystal form D of the compound of Formula I-1 is shown in Figure 35.

**Table 19 XRPD analysis data for the hydrochloride crystal form D of the compound of Formula I-1**

| Name | Angle | d Value | Net Intensity | Gross Intensity | Rel. Intensity | FWHM |
|---|---|---|---|---|---|---|
| Peak #1 | 6.601512 | 13.37856 | 164.5807 | 360.2378 | 0.1722114 | 0.100 |
| Peak # 2 | 7.256653 | 12.17213 | 58.27498 | 262.8102 | 0.06097686 | 0.111 |
| Peak # 3 | 7.786158 | 11.3455 | 216.3087 | 427.6613 | 0.2263378 | 0.100 |
| Peak # 4 | 8.479876 | 10.41884 | 94.11929 | 310.7975 | 0.09848309 | 0.100 |
| Peak # 5 | 9.837548 | 8.983783 | 292.5006 | 516.1531 | 0.3060622 | 0.100 |
| Peak # 6 | 11.17929 | 7.90838 | 73.00758 | 297.2161 | 0.07639254 | 0.277 |
| Peak # 7 | 13.30976 | 6.6469 | 222.7104 | 477.5637 | 0.2330362 | 0.166 |
| Peak # 8 | 13.88618 | 6.372257 | 72.43765 | 337.7217 | 0.07579618 | 0.133 |
| Peak # 9 | 14.63502 | 6.047843 | 144.9248 | 428.148 | 0.1516442 | 0.108 |
| Peak # 10 | 14.98003 | 5.90932 | 217.5238 | 511.2758 | 0.2276092 | 0.380 |
| Peak # 11 | 15.22908 | 5.813237 | 300.9823 | 601.7073 | 0.3149371 | 0.185 |
| Peak # 12 | 15.92681 | 5.560104 | 334.7283 | 652.176 | 0.3502478 | 0.198 |
| Peak # 13 | 16.26554 | 5.445069 | 139.724 | 463.7961 | 0.1462023 | 0.356 |
| Peak # 14 | 17.03894 | 5.199618 | 77.78125 | 421.2977 | 0.08138753 | 0.183 |
| Peak #15 | 18.21142 | 4.867415 | 117.7768 | 497.4332 | 0.1232375 | 0.100 |
| Peak # 16 | 19.32967 | 4.588276 | 229.9395 | 646.4288 | 0.2406005 | 0.100 |
| Peak # 17 | 20.08674 | 4.41702 | 708.8257 | 1144.215 | 0.74169 | 0.100 |
| Peak # 18 | 20.40698 | 4.348422 | 955.69 | 1397.604 | 1 | 0.156 |
| Peak # 19 | 21.2275 | 4.182158 | 313.6636 | 768.3183 | 0.3282064 | 0.236 |
| Peak # 20 | 21.80498 | 4.072686 | 320.5857 | 780.7736 | 0.3354495 | 0.287 |
| Peak # 21 | 23.48703 | 3.784683 | 59.54105 | 519.681 | 0.06230164 | 0.125 |
| Peak # 22 | 24.78517 | 3.589319 | 87.48044 | 546.9264 | 0.09153642 | 0.283 |
| Peak # 23 | 28.43383 | 3.136483 | 65.95297 | 507.909 | 0.06901085 | 0.100 |

In some embodiments of the present invention, the hydrochloride crystal form D of the compound of Formula I-1 has the onsets of the endothermic peaks at 55.20°C ± 2^{°}C and 164.37^{°}C ± 2^{°}C in its differential scanning calorimetry (DSC) curve, and decomposition of the sample begins above 211.35°C ± 2^{°}C.

In some embodiments of the present invention, the DSC pattern of the hydrochloride crystal form D of the compound of Formula I-1 is shown in Figure 36.

In some embodiments of the present invention, the hydrochloride crystal form D of the compound of Formula I-1 has two weight loss steps at 36.24°C ± 2^{°}C and 110.00^{°}C ± 2^{°}C in its thermogravimetric analysis (TGA) curve.

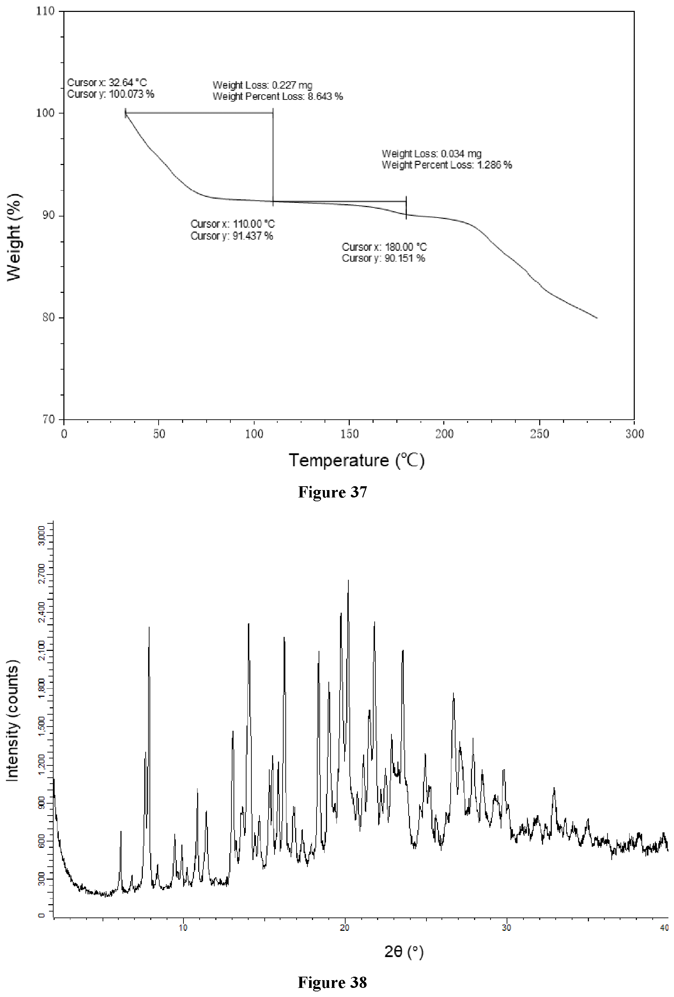

In some embodiments of the present invention, the TGA pattern of the hydrochloride crystal form D of the compound of Formula I-1 is shown in Figure 37.

The present invention also provides a method for preparing the hydrochloride crystal form D of the compound of Formula I-1, comprising adding the compound of Formula I-1 and hydrochloric acid to butanone, and then performing recrystallization or slurrying to obtain it, wherein the ratio of the compound of Formula I-1 to hydrochloric acid is selected from 0.8 to 1.2, and the hydrochloride crystal form D of the compound of Formula I-1 is an anhydrous crystal form.

The present invention also provides the hydrochloride crystal form E1 of the compound of Formula I-1, and its X-ray powder diffraction pattern has characteristic diffraction peaks at the following 2θ angles: 6.15±0.2^{°}, 7.66 ± 0.2 °, 7.90 ± 0.2 °, 13.06 ± 0.2 °, 14.06 ± 0.2 °, 16.25 ± 0.2 °, 18.37 ± 0.2 °, 19.01 ± 0.2^{°}, 19.75 ± 0.2 °, 20.19 ± 0.2 ° , 21.81 ± 0.2 ° , 23.56 ± 0.2 °, 24.95 ± 0.2 ° , 25.18 ± 0.2 °, 25.63 ± 0.2°, 26.71±0.2°, 27.10±0.2°, 27.91±0.2°, 29.81±0.2°, 32.93±0.2°.

In some embodiments of the present invention, the X-ray powder diffraction pattern of the hydrochloride crystal form E1 of the compound of Formula I-1 has characteristic diffraction peaks at the following 2θ angles: 6.15±0.2^{°}, 7.66 ± 0.2^{°}, 7.90 ± 0.2^{°}, 8.41 ± 0.2^{°}, 9.49 ± 0.2°, 9.91 ± 0.2°, 10.88 ± 0.2°, 11.42 ± 0.2°, 13.06±0.2°, 13.66±0.2°, 14.06±0.2°, 14.69±0.2°, 15.32±0.2°, 15.52±0.2°, 15.88±0.2°, 16.25±0.2°, 16.81±0.2°, 17.35±0.2°, 18.37±0.2°, 19.01±0.2°, 19.75±0.2°, 20.19±0.2°, 21.15±0.2°, 21.51±0.2°, 21.81±0.2°, 22.50±0.2°, 22.87±0.2°, 23.56±0.2°, 24.95±0.2°, 25.18±0.2°, 25.63±0.2°, 26.71±0.2°, 27.10±0.2°, 27.91±0.2°, 28.47±0.2°, 29.30±0.2°, 29.81±0.2°, 30.12±0.2°, 32.93±0.2°, 34.96±0.2°.

In some embodiments of the present invention, the X-ray diffraction (XRPD) pattern of the hydrochloride crystal form E1 of the compound of Formula I-1 is shown in Figure 38.

**Table 20 XRPD analysis data for the hydrochloride crystal form E1 of the compound of Formula I-1**

| Name | Angle | d Value | Net Intensity | Gross Intensity | Rel. Intensity | FWHM |
|---|---|---|---|---|---|---|
| Peak #1 | 6.145037 | 14.37131 | 302.5578 | 499.1663 | 0.2048961 | 0.100 |
| Peak # 2 | 7.657481 | 11.53586 | 704.3558 | 925.7552 | 0.476999 | 0.166 |
| Peak # 3 | 7.895144 | 11.18912 | 1359.082 | 1582.423 | 0.9203882 | 0.122 |
| Peak #4 | 8.406717 | 10.50935 | 126.4409 | 351.9904 | 0.08562741 | 0.100 |
| Peak # 5 | 9.487387 | 9.314556 | 269.7384 | 513.6247 | 0.1826704 | 0.100 |
| Peak # 6 | 9.912072 | 8.916405 | 216.5638 | 468.4002 | 0.1466598 | 0.100 |
| Peak # 7 | 10.87609 | 8.128153 | 501.5989 | 764.5867 | 0.3396893 | 0.100 |
| Peak # 8 | 11.42041 | 7.741942 | 388.9748 | 654.0305 | 0.2634188 | 0.103 |
| Peak # 9 | 13.0554 | 6.775825 | 780.8158 | 1077.419 | 0.5287787 | 0.100 |
| Peak # 10 | 13.66123 | 6.476674 | 386.5541 | 712.326 | 0.2617795 | 0.100 |
| Peak # 11 | 14.05527 | 6.295974 | 1337.484 | 1680.198 | 0.9057618 | 0.166 |
| Peak # 12 | 14.69153 | 6.024707 | 287.5207 | 654.2143 | 0.1947128 | 0.174 |
| Peak # 13 | 15.32495 | 5.777086 | 541.0035 | 927.4278 | 0.3663747 | 0.141 |
| Peak # 14 | 15.51518 | 5.706679 | 615.061 | 1006.604 | 0.4165273 | 0.127 |
| Peak # 15 | 15.88049 | 5.576216 | 593.2246 | 993.5515 | 0.4017395 | 0.100 |
| Peak # 16 | 16.24859 | 5.45071 | 1265.391 | 1673.182 | 0.8569396 | 0.100 |
| Peak # 17 | 16.80916 | 5.270176 | 302.3594 | 718.8266 | 0.2047618 | 0.124 |
| Peak # 18 | 17.3498 | 5.10715 | 190.8611 | 612.6349 | 0.1292536 | 0.104 |
| Peak # 19 | 18.37336 | 4.824878 | 1145.505 | 1605.355 | 0.7757506 | 0.100 |
| Peak # 20 | 19.00938 | 4.664856 | 933.6566 | 1421.984 | 0.6322845 | 0.121 |
| Peak # 21 | 19.75294 | 4.490902 | 1276.167 | 1792.503 | 0.8642372 | 0.143 |
| Peak # 22 | 20.1886 | 4.394964 | 1476.64 | 2006.738 | 1 | 0.108 |
| Peak # 23 | 21.1496 | 4.197384 | 506.0891 | 1059.635 | 0.3427301 | 0.132 |
| Peak # 24 | 21.51016 | 4.127835 | 703.582 | 1263.474 | 0.4764749 | 0.100 |
| Peak # 25 | 21.80766 | 4.07219 | 1196.181 | 1760.296 | 0.8100696 | 0.205 |
| Peak # 26 | 22.49543 | 3.949219 | 424.1585 | 994.5509 | 0.2872457 | 0.245 |
| Peak # 27 | 22.87291 | 3.884891 | 627.939 | 1199.706 | 0.4252485 | 0.100 |
| Peak # 28 | 23.56058 | 3.773034 | 1049.806 | 1620.304 | 0.7109421 | 0.109 |
| Peak # 29 | 24.94623 | 3.566509 | 489.7659 | 1058.782 | 0.3316759 | 0.205 |
| Peak # 30 | 25.18339 | 3.533457 | 337.6172 | 908.0272 | 0.2286388 | 0.210 |
| Peak # 31 | 25.63268 | 3.472533 | 183.0638 | 754.5255 | 0.1239732 | 0.135 |
| Peak # 32 | 26.70567 | 3.335397 | 778.4288 | 1371.446 | 0.5271622 | 0.163 |
| Peak # 33 | 27.10295 | 3.2874 | 496.8693 | 1098.879 | 0.3364864 | 0.226 |
| Peak # 34 | 27.90941 | 3.194215 | 564.1411 | 1179.394 | 0.3820437 | 0.137 |
| Peak # 35 | 28.46869 | 3.132721 | 384.4941 | 1004.999 | 0.2603845 | 0.167 |
| Peak # 36 | 29.30478 | 3.045216 | 189.4714 | 811.8213 | 0.1283125 | 0.284 |
| Peak # 37 | 29.81338 | 2.994414 | 368.8845 | 988.831 | 0.2498134 | 0.244 |
| Peak # 38 | 30.11576 | 2.965034 | 245.5084 | 862.7616 | 0.1662615 | 0.309 |
| Peak # 39 | 32.93307 | 2.717536 | 268.7507 | 882.8731 | 0.1820015 | 0.168 |
| Peak # 40 | 34.95511 | 2.564826 | 179.9061 | 751.8131 | 0.1218347 | 0.133 |

In some embodiments of the present invention, the hydrochloride crystal form E1 of the compound of Formula I-1 has the onsets of the endothermic peaks at 13.36°C ± 2^{°}C and 174.06^{°}C ± 2^{°}C in its differential scanning calorimetry (DSC) curve, and decomposition of the sample begins at 211.29°C ± 2^{°}C.

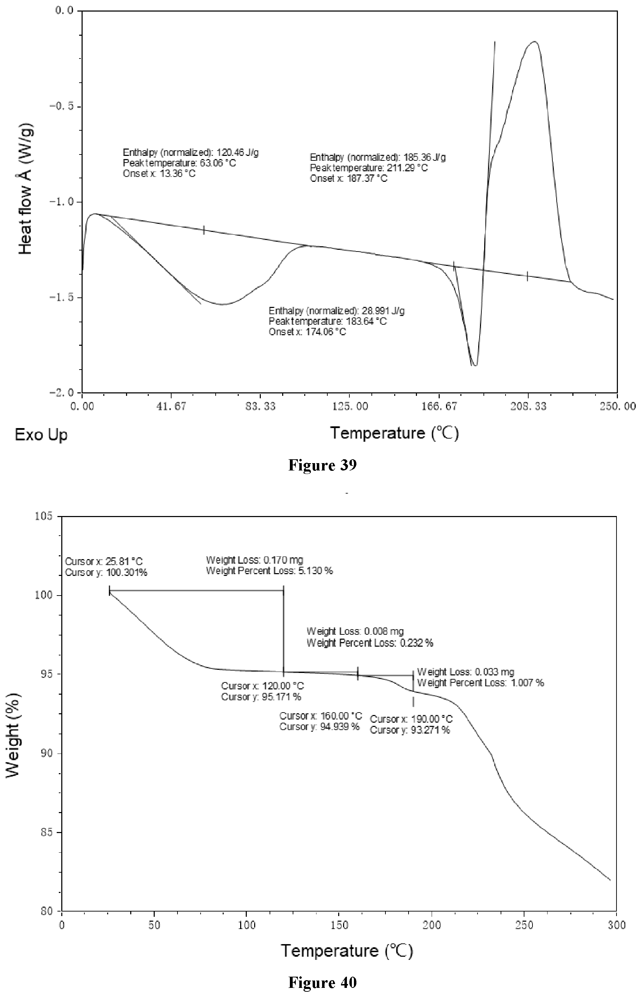

In some embodiments of the present invention, the DSC pattern of the hydrochloride crystal form E1 of the compound of Formula I-1 is shown in Figure 39.

In some embodiments of the present invention, the hydrochloride crystal form E1 of the compound of Formula I-1 has three weight loss steps at 25.81°C ± 2^{°}C, 120.00°C ± 2^{°}C, and 160.00^{°}C ± 2^{°}C in its thermogravimetric analysis (TGA) curve.

In some embodiments of the present invention, the TGA pattern of the hydrochloride crystal form E1 of the compound of Formula I-1 is shown in Figure 40.

The present invention also provides a method for preparing the hydrochloride crystal form E1 of the compound of Formula I-1, comprising adding the compound of Formula I-1 and hydrochloric acid to ethanol, and then performing recrystallization or slurrying to obtain it, wherein the ratio of the compound of Formula I-1 to hydrochloric acid is selected from 0.8 to 1.2, and the hydrochloride crystal form E1 of the compound of Formula I-1 is a hydrate crystal form with a water content of 1.7 equivalents.

The present invention also provides the hydrochloride crystal form E2 of the compound of Formula I-1, and its X-ray powder diffraction pattern has characteristic diffraction peaks at the following 2θ angles: 7.88±0.2°, 10.90±0.2°, 13.10±0.2°, 14.14±0.2°, 16.30±0.2°, 19.01±0.2°, 19.75±0.2°, 21.54±0.2°, 21.84±0.2°, 23.63±0.2°, 24.96±0.2°, 26.78±0.2°, 29.91±0.2^{°}.

In some embodiments of the present invention, the X-ray powder diffraction pattern of the hydrochloride crystal form E2 of the compound of Formula I-1 has characteristic diffraction peaks at the following 2θ angles: 7.88±0.2°, 9.51±0.2°, 10.90±0.2°, 11.49 ± 0.2°, 13.10 ± 0.2°, 13.64 ± 0.2 ° , 14.14 ± 0.2°, 14.76 ± 0.2°, 15.39 ± 0.2°, 15.75 ± 0.2°, 16.30±0.2°, 16.93±0.2°, 19.01±0.2°, 19.75±0.2°, 20.69±0.2°, 21.54±0.2°, 21.84±0.2°, 22.19±0.2°, 22.57±0.2°, 23.01±0.2°, 23.63±0.2°, 24.96±0.2°, 25.90±0.2°, 26.32±0.2°, 26.78±0.2°, 27.19±0.2°, 27.48±0.2°, 27.94±0.2°, 28.68±0.2°, 29.07±0.2°, 29.50±0.2°, 29.91±0.2°, 31.74±0.2°, 32.96±0.2°, 33.66±0.2°.

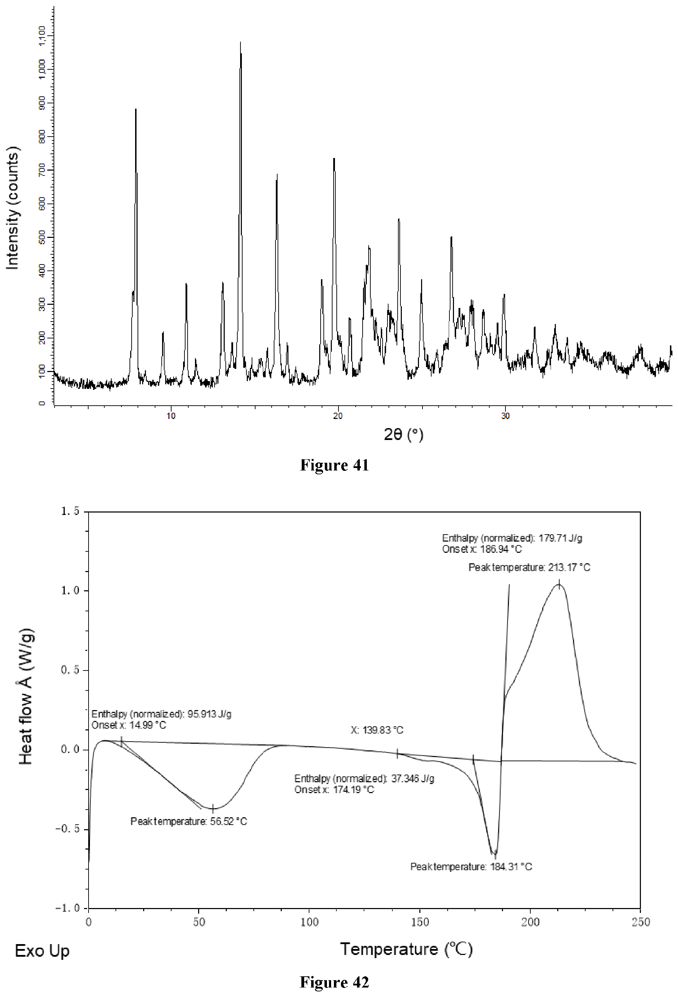

In some embodiments of the present invention, the X-ray diffraction (XRPD) pattern of the hydrochloride crystal form E2 of the compound of Formula I-1 is shown in Figure 41.

**Table 21 XRPD analysis data for the hydrochloride crystal form E2 of the compound of Formula I-1**

| Name | Angle | d Value | Net Intensity | Intensity Gross | Rel. Intensity | FWHM |
|---|---|---|---|---|---|---|
| Peak #1 | 7.884071 | 11.20481 | 540.4462 | 605.2851 | 0.7774932 | 0.100 |
| Peak # 2 | 9.512653 | 9.289872 | 103.279 | 168.8765 | 0.1485786 | 0.100 |
| Peak # 3 | 10.89717 | 8.112479 | 210.551 | 276.9264 | 0.3029015 | 0.100 |
| Peak # 4 | 11.48909 | 7.695814 | 43.11554 | 110.4364 | 0.0620266 | 0.100 |
| Peak # 5 | 13.10177 | 6.75195 | 192.5077 | 264.6825 | 0.2769442 | 0.100 |
| Peak # 6 | 13.63865 | 6.487342 | 77.58062 | 155.1348 | 0.1116085 | 0.100 |
| Peak # 7 | 14.13521 | 6.260545 | 695.1138 | 775.9518 | 1 | 0.100 |
| Peak # 8 | 14.76314 | 5.995642 | 28.98997 | 111.9226 | 0.04170537 | 0.133 |
| Peak # 9 | 15.39103 | 5.752432 | 33.00134 | 117.8404 | 0.04747616 | 0.121 |
| Peak # 10 | 15.74691 | 5.623217 | 62.01804 | 146.7878 | 0.08921998 | 0.100 |
| Peak # 11 | 16.30386 | 5.432357 | 432.7301 | 515.7111 | 0.6225314 | 0.100 |
| Peak # 12 | 16.92825 | 5.233366 | 61.63427 | 140.1845 | 0.08866789 | 0.100 |
| Peak # 13 | 19.01114 | 4.664429 | 210.1703 | 287.2018 | 0.3023539 | 0.115 |
| Peak # 14 | 19.75051 | 4.49145 | 445.9855 | 532.7929 | 0.6416007 | 0.109 |
| Peak # 15 | 20.69488 | 4.288574 | 111.2609 | 205.316 | 0.1600614 | 0.100 |
| Peak # 16 | 21.54478 | 4.12128 | 168.5169 | 267.2058 | 0.2424307 | 0.360 |
| Peak # 17 | 21.84486 | 4.06534 | 251.1638 | 351.1669 | 0.3613276 | 0.271 |
| Peak # 18 | 22.19098 | 4.002708 | 154.7814 | 255.5625 | 0.2226706 | 0.100 |
| Peak # 19 | 22.57326 | 3.935779 | 98.63 | 199.3557 | 0.1418904 | 0.148 |
| Peak # 20 | 23.01374 | 3.861435 | 105.6459 | 205.1103 | 0.1519836 | 0.100 |
| Peak # 21 | 23.62687 | 3.762597 | 325.2303 | 420.8155 | 0.4678806 | 0.106 |
| Peak # 22 | 24.95749 | 3.564925 | 196.3293 | 294.1708 | 0.282442 | 0.100 |
| Peak # 23 | 25.89573 | 3.437852 | 40.57758 | 148.8828 | 0.05837544 | 0.163 |
| Peak # 24 | 26.32397 | 3.382887 | 50.33425 | 163.4267 | 0.07241153 | 0.100 |
| Peak # 25 | 26.7799 | 3.326319 | 265.6382 | 383.6885 | 0.3821507 | 0.118 |
| Peak # 26 | 27.19104 | 3.276951 | 182.0309 | 303.3774 | 0.261872 | 0.349 |
| Peak # 27 | 27.47843 | 3.243327 | 83.98383 | 206.9736 | 0.1208203 | 0.100 |
| Peak # 28 | 27.9352 | 3.191324 | 112.1196 | 236.6009 | 0.1612967 | 0.196 |
| Peak # 29 | 28.68201 | 3.109905 | 100.0596 | 224.0822 | 0.1439471 | 0.191 |
| Peak # 30 | 29.06967 | 3.069308 | 59.76442 | 182.13 | 0.0859779 | 0.187 |
| Peak # 31 | 29.50209 | 3.025297 | 98.35264 | 217.703 | 0.1414914 | 0.100 |
| Peak # 32 | 29.90704 | 2.98525 | 144.627 | 260.0349 | 0.2080623 | 0.120 |
| Peak # 33 | 31.74403 | 2.816562 | 90.48898 | 199.877 | 0.1301787 | 0.130 |
| Peak # 34 | 32.95559 | 2.71573 | 84.64176 | 195.0735 | 0.1217668 | 0.157 |
| Peak # 35 | 33.65558 | 2.66083 | 72.07199 | 181.9605 | 0.1036837 | 0.165 |

In some embodiments of the present invention, the hydrochloride crystal form E2 of the compound of Formula I-1 has the onsets of the endothermic peaks at 14.99°C ± 2°C and 174.19^{°}C ± 2^{°}C in its differential scanning calorimetry (DSC) curve, and decomposition of the sample begins at 213.17°C ± 2^{°}C.

In some embodiments of the present invention, the DSC pattern of the hydrochloride crystal form E2 of the compound of Formula I-1 is shown in Figure 42.

In some embodiments of the present invention, the hydrochloride crystal form E2 of the compound of Formula I-1 has three weight loss steps at 33.18°C ± 2^{°}C, 90.00°C ± 2^{°}C, and 140.00^{°}C ± 2^{°}C in its thermogravimetric analysis (TGA) curve.

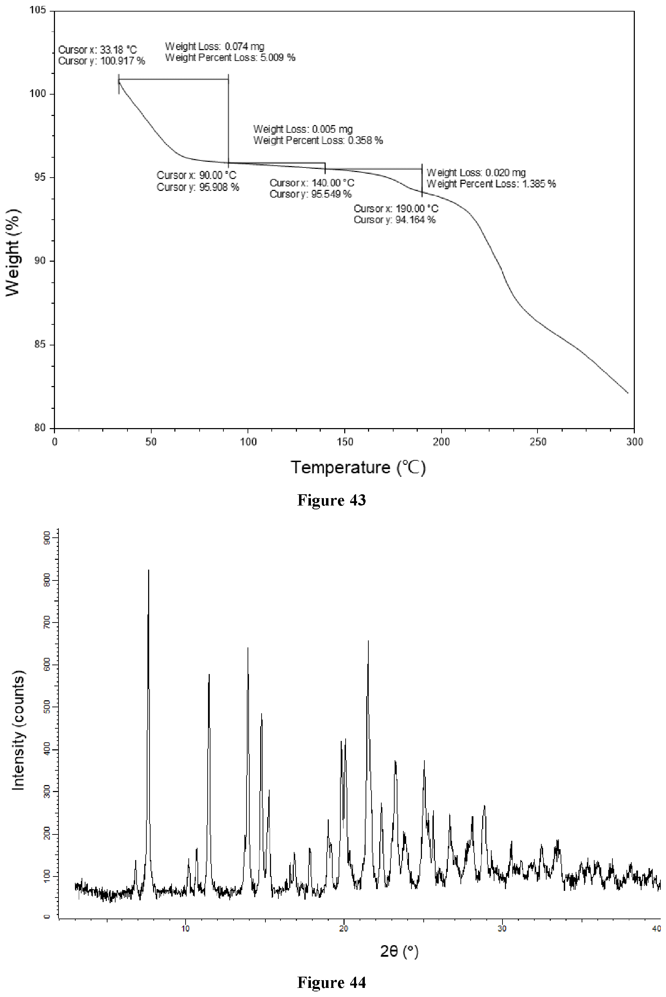

In some embodiments of the present invention, the TGA pattern of the hydrochloride crystal form E2 of the compound of Formula I-1 is shown in Figure 43.

The present invention also provides a method for preparing the hydrochloride crystal form E2 of the compound of Formula I-1, comprising drying the hydrochloride crystal form F of the compound of Formula I-1 under vacuum at 25^{°}C, 50^{°}C, or 100°C, wherein the ratio of the compound of Formula I-1 to hydrochloric acid is selected from 0.8 to 1.2, and the hydrochloride crystal form E2 of the compound of Formula I-1 is a hydrate crystal form with a water content of 1.8 equivalents.

The present invention also provides the hydrochloride crystal form F of the compound of Formula I-1, and its X-ray powder diffraction pattern has characteristic diffraction peaks at the following 2θ angles: 7.65 ± 0.2^{°}, 11.47 ± 0.2 °, 13.93 ± 0.2^{°}, 14.80 ± 0.2^{°}, 15.25 ± 0.2 °, 19.84 ± 0.2 °, 20.09 ± 0.2^{°}, 21.51 ± 0.2 °, 23.22 ± 0.2 °, 25.06 ± 0.2 °, 26.68 ± 0.2 ° , 28.87 ± 0.2 °, 32.45 ± 0.2^{°}, 33.59 ± 0.2^{°}.

In some embodiments of the present invention, the X-ray powder diffraction pattern of the hydrochloride crystal form F of the compound of Formula I-1 has characteristic diffraction peaks at the following 2θ angles: 6.85±0.2°, 7.65±0.2°, 10.21±0.2°, 10.70±0.2°, 11.47±0.2°, 13.93±0.2°, 14.80±0.2°, 15.25±0.2°, 16.60±0.2°, 16.88±0.2°, 17.84±0.2°, 19.00±0.2°, 19.16±0.2°, 19.84±0.2°, 20.09±0.2°, 20.34±0.2°, 21.51±0.2°, 22.35±0.2°, 23.22±0.2°, 23.86±0.2°, 25.06±0.2°, 25.31±0.2°, 25.63±0.2°, 26.68±0.2°, 28.10±0.2°, 28.87±0.2°, 30.53±0.2°, 32.45±0.2°, 33.59±0.2°.

In some embodiments of the present invention, the X-ray powder diffraction (XRPD) pattern of the hydrochloride crystal form F of the compound of Formula I-1 is shown in Figure 44.

**Table 22 XRPD analysis data for the hydrochloride crystal form F of the compound of Formula I-1**

| Name | Angle | d Value | Net Intensity | Gross Intensity | Rel. Intensity | FWHM |
|---|---|---|---|---|---|---|
| Peak #1 | 6.848274 | 12.89704 | 50.41388 | 108.1367 | 0.09768955 | 0.100 |
| Peak # 2 | 7.651177 | 11.54535 | 516.0621 | 576.032 | 1 | 0.100 |
| Peak # 3 | 10.20579 | 8.660445 | 49.75304 | 111.4053 | 0.096409 | 0.100 |
| Peak # 4 | 10.70466 | 8.257937 | 69.10229 | 130.8267 | 0.133903 | 0.100 |
| Peak #5 | 11.47234 | 7.707018 | 361.2969 | 420.3412 | 0.7001035 | 0.100 |
| Peak # 6 | 13.93093 | 6.351884 | 397.6063 | 461.8362 | 0.7704621 | 0.100 |
| Peak # 7 | 14.80051 | 5.98059 | 260.9636 | 327.9683 | 0.5056826 | 0.100 |
| Peak # 8 | 15.25096 | 5.804945 | 153.9308 | 220.6624 | 0.2982796 | 0.108 |
| Peak # 9 | 16.60372 | 5.334918 | 34.92417 | 97.03252 | 0.06767435 | 0.155 |
| Peak # 10 | 16.88418 | 5.246928 | 60.09497 | 120.1826 | 0.1164491 | 0.183 |
| Peak # 11 | 17.83583 | 4.969059 | 73.02078 | 128.1677 | 0.1414961 | 0.100 |
| Peak # 12 | 19.00091 | 4.666917 | 115.9687 | 180.328 | 0.2247185 | 0.128 |
| Peak # 13 | 19.16145 | 4.628178 | 78.67841 | 145.1145 | 0.1524592 | 0.128 |
| Peak # 14 | 19.83859 | 4.471706 | 220.5375 | 294.1044 | 0.4273469 | 0.172 |
| Peak # 15 | 20.08921 | 4.416483 | 227.1443 | 302.6826 | 0.440149 | 0.184 |
| Peak # 16 | 20.34485 | 4.361562 | 44.52192 | 121.6991 | 0.0862724 | 0.197 |
| Peak # 17 | 21.51468 | 4.126979 | 342.1507 | 422.0398 | 0.6630028 | 0.116 |
| Peak # 18 | 22.35365 | 3.973946 | 135.1561 | 215.3622 | 0.2618989 | 0.110 |
| Peak # 19 | 23.22226 | 3.827231 | 214.9216 | 298.199 | 0.4164645 | 0.154 |
| Peak # 20 | 23.85788 | 3.726686 | 87.52694 | 170.3065 | 0.1696054 | 0.210 |
| Peak # 21 | 25.06488 | 3.549895 | 214.0289 | 298.1458 | 0.4147348 | 0.168 |
| Peak # 22 | 25.30611 | 3.516599 | 90.23993 | 175.521 | 0.1748625 | 0.178 |
| Peak # 23 | 25.63199 | 3.472623 | 125.4472 | 211.7709 | 0.2430855 | 0.106 |
| Peak # 24 | 26.68101 | 3.338423 | 102.6051 | 191.2798 | 0.1988232 | 0.141 |
| Peak # 25 | 28.09525 | 3.173506 | 86.42886 | 182.8679 | 0.1674776 | 0.175 |
| Peak # 26 | 28.86665 | 3.090432 | 110.8668 | 209.6979 | 0.2148323 | 0.144 |
| Peak # 27 | 30.53301 | 2.925459 | 35.61448 | 133.7473 | 0.069012 | 0.100 |
| Peak # 28 | 32.44866 | 2.756991 | 82.21487 | 181.7001 | 0.159312 | 0.123 |
| Peak # 29 | 33.58943 | 2.665919 | 45.26086 | 135.2353 | 0.08770428 | 0.196 |

In some embodiments of the present invention, the hydrochloride crystal form F of the compound of Formula I-1 has the onsets of the endothermic peaks at 36.03°C ± 2^{°}C and 172.45^{°}C ± 2^{°}C in its differential scanning calorimetry (DSC) curve, and decomposition of the sample begins at 212.66°C ± 2^{°}C.

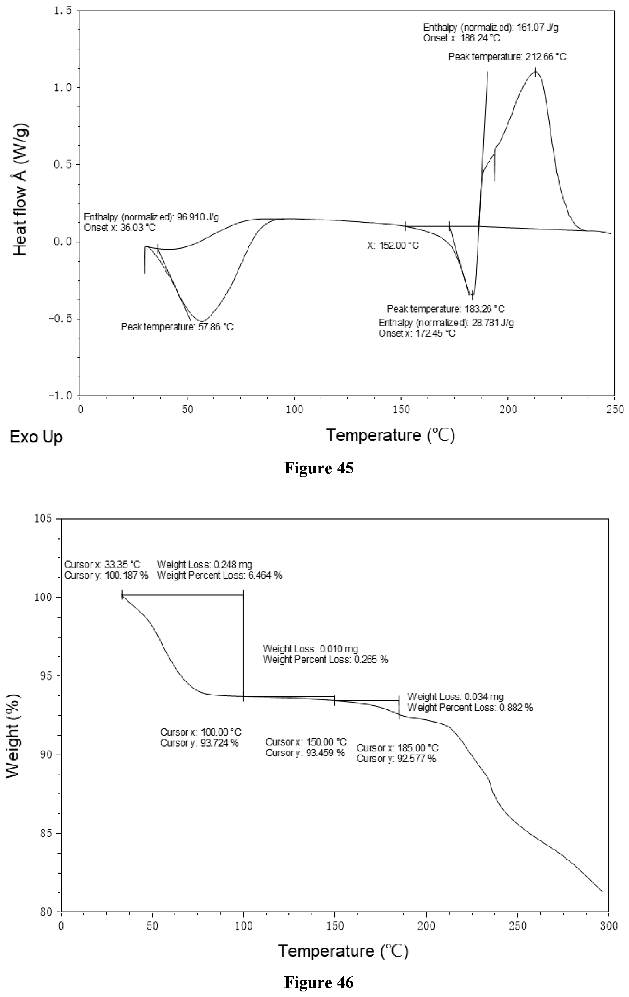

In some embodiments of the present invention, the DSC pattern of the hydrochloride crystal form F of the compound of Formula I-1 is shown in Figure 45.

In some embodiments of the present invention, the hydrochloride crystal form F of the compound of Formula I-1 has three weight loss steps at 33.35°C ± 2^{°}C, 100.00°C ± 2^{°}C, and 150.00^{°}C ± 2^{°}C in its thermogravimetric analysis (TGA) curve.

In some embodiments of the present invention, the TGA pattern of the hydrochloride crystal form F of the compound of Formula I-1 is shown in Figure 46.

The present invention also provides a method for preparing the hydrochloride crystal form F of the compound of Formula I-1, comprising adding the compound of Formula I-1 and hydrochloric acid to ethanol, acetone, ethyl acetate, acetonitrile, tetrahydrofuran, or a mixed solvent of methyl isobutyl ketone/trifluoroethanol, and then performing recrystallization at 50^{°}C or slurrying to obtain it, the ratio of the compound of Formula I-1 to hydrochloric acid is selected from 0.8 to 1.2, the hydrochloride crystal form F of the compound of Formula I-1 is a hydrate crystal form with a water content of 2.0 equivalents.

The present invention also provides the hydrochloride crystal form G of the compound of Formula I-1, and its X-ray powder diffraction pattern has characteristic diffraction peaks at the following 2θ angles: 5.83±0.2°, 9.97±0.2°, 13.26±0.2°, 14.17±0.2°, 15.52±0.2°, 16.77±0.2°, 18.37±0.2°, 20.27±0.2°, 20.48±0.2°, 21.35±0.2°, 21.82±0.2°, 22.65±0.2°, 23.04±0.2°, 24.43±0.2°, 26.24±0.2°, 26.54±0.2°, 28.94±0.2°, 30.18±0.2°.

In some embodiments of the present invention, the X-ray powder diffraction pattern of the hydrochloride crystal form G of the compound of Formula I-1 has characteristic diffraction peaks at the following 2θ angles: 5.83±0.2°, 7.18±0.2°, 9.97±0.2°, 11.63±0.2°, 12.00±0.2°, 12.35±0.2°, 13.26±0.2°, 13.58±0.2°, 14.17±0.2°, 14.60±0.2°, 15.52±0.2°, 16.77±0.2°, 17.64±0.2°, 18.37±0.2°, 18.81±0.2°, 19.14±0.2°, 19.80±0.2°, 20.27±0.2°, 20.48±0.2°, 21.35±0.2°, 21.82±0.2°, 22.65±0.2°, 23.04±0.2°, 23.35±0.2°, 24.43±0.2°, 25.47±0.2°, 26.24±0.2°, 26.54±0.2°, 26.84±0.2°, 27.92±0.2°, 28.94±0.2°, 29.31±0.2°, 30.18±0.2°.

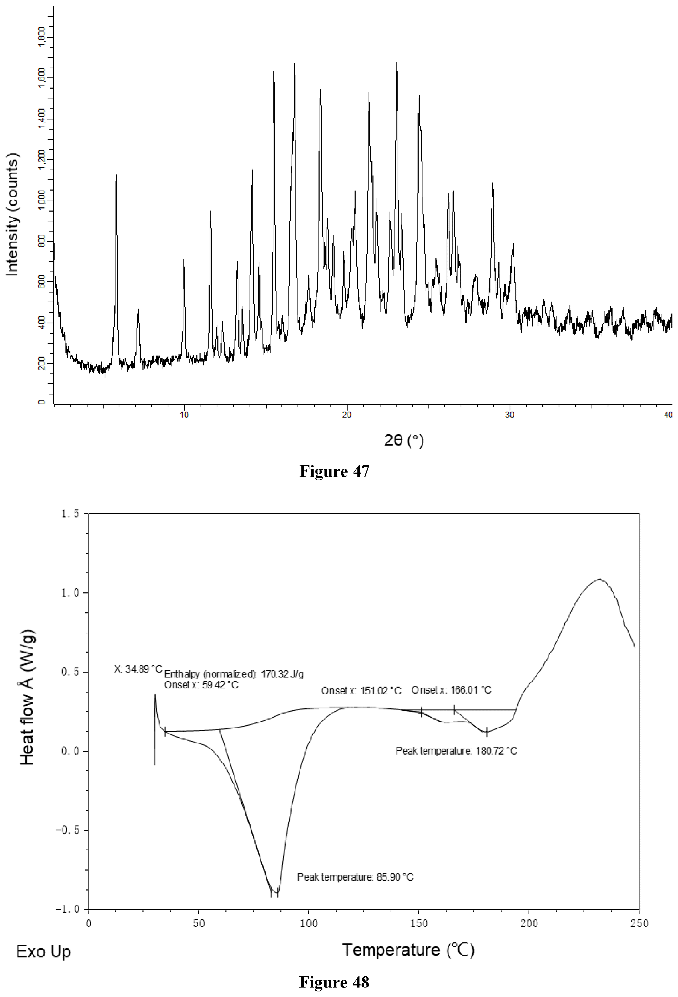

In some embodiments of the present invention, the X-ray powder diffraction (XRPD) pattern of the hydrochloride crystal form G of the compound of Formula I-1 is shown in Figure 47.

**Table 23 XRPD analysis data for the hydrochloride crystal form G of the compound of Formula I-1**

| Name | Angle | d Value | Net Intensity | Gross Intensity | Rel. Intensity | FWHM |
|---|---|---|---|---|---|---|
| Peak #1 | 5.828182 | 15.15189 | 579.1664 | 753.4305 | 0.6023378 | 0.100 |
| Peak # 2 | 7.17844 | 12.30457 | 172.8027 | 365.0822 | 0.1797163 | 0.100 |
| Peak # 3 | 9.974504 | 8.860736 | 346.1045 | 556.5733 | 0.3599515 | 0.100 |
| Peak # 4 | 11.62806 | 7.604156 | 506.9125 | 722.6564 | 0.5271932 | 0.100 |
| Peak # 5 | 11.99626 | 7.371572 | 122.0042 | 338.9887 | 0.1268854 | 0.100 |
| Peak # 6 | 12.34862 | 7.162008 | 128.8925 | 346.0245 | 0.1340493 | 0.171 |
| Peak # 7 | 13.25774 | 6.672863 | 324.0526 | 556.4399 | 0.3370174 | 0.153 |
| Peak # 8 | 13.58265 | 6.513966 | 171.4265 | 412.3184 | 0.178285 | 0.100 |
| Peak # 9 | 14.16958 | 6.245436 | 598.4322 | 852.499 | 0.6223745 | 0.111 |
| Peak # 10 | 14.59519 | 6.064257 | 283.763 | 545.6206 | 0.2951159 | 0.100 |
| Peak # 11 | 15.51762 | 5.705785 | 961.5308 | 1251.04 | 1 | 0.100 |
| Peak # 12 | 16.7697 | 5.282487 | 931.9397 | 1272.279 | 0.969225 | 0.205 |
| Peak # 13 | 17.63585 | 5.024953 | 178.8619 | 547.5662 | 0.1860179 | 0.140 |
| Peak # 14 | 18.37159 | 4.825339 | 771.5197 | 1159.492 | 0.8023869 | 0.116 |
| Peak # 15 | 18.80623 | 4.714787 | 372.962 | 770.2344 | 0.3878836 | 0.100 |
| Peak # 16 | 19.1386 | 4.633651 | 288.6279 | 691.9684 | 0.3001754 | 0.100 |
| Peak # 17 | 19.80226 | 4.479828 | 214.2406 | 626.9946 | 0.222812 | 0.100 |
| Peak # 18 | 20.26602 | 4.378349 | 301.9755 | 719.1638 | 0.314057 | 0.247 |
| Peak # 19 | 20.48124 | 4.332824 | 436.0151 | 854.6628 | 0.4534594 | 0.221 |
| Peak # 20 | 21.35452 | 4.157567 | 768.4391 | 1191.466 | 0.7991831 | 0.193 |
| Peak # 21 | 21.8184 | 4.07021 | 387.7249 | 811.9121 | 0.4032371 | 0.121 |
| Peak # 22 | 22.64517 | 3.923443 | 357.5557 | 779.4445 | 0.371861 | 0.137 |
| Peak # 23 | 23.04393 | 3.856443 | 885.9234 | 1304.702 | 0.9213678 | 0.100 |
| Peak # 24 | 23.34855 | 3.806815 | 376.3249 | 791.8521 | 0.391381 | 0.259 |
| Peak # 25 | 24.42592 | 3.641291 | 722.9464 | 1152.347 | 0.7518703 | 0.191 |
| Peak # 26 | 25.47185 | 3.494093 | 192.4108 | 637.1698 | 0.2001088 | 0.250 |
| Peak # 27 | 26.2382 | 3.393751 | 388.4084 | 838.7321 | 0.4039479 | 0.112 |
| Peak # 28 | 26.53974 | 3.355873 | 427.7524 | 878.9491 | 0.444866 | 0.256 |
| Peak #29 | 26.83974 | 3.319039 | 219.5827 | 670.9071 | 0.2283678 | 0.233 |
| Peak # 30 | 27.92213 | 3.192788 | 126.4753 | 572.1395 | 0.1315354 | 0.191 |
| Peak # 31 | 28.94164 | 3.082595 | 395.4236 | 826.9805 | 0.4112438 | 0.119 |
| Peak # 32 | 29.31307 | 3.044374 | 215.0207 | 639.3233 | 0.2236233 | 0.100 |
| Peak # 33 | 30.18123 | 2.958751 | 262.0633 | 665.0064 | 0.272548 | 0.174 |

In some embodiments of the present invention, the hydrochloride crystal form G of the compound of Formula I-1 has the onsets of the endothermic peaks at 59.42°C ± 2^{°}C, 151.02°C ± 2^{°}C, and 166.01°C ± 2^{°}C in its differential scanning calorimetry (DSC) curve.

In some embodiments of the present invention, the DSC pattern of the hydrochloride crystal form G of the compound of Formula I-1 is shown in Figure 48.

In some embodiments of the present invention, the hydrochloride crystal form G of the compound of Formula I-1 has three weight loss steps at 26.28°C ± 2^{°}C, 120.00°C ± 2^{°}C, and 150.00^{°}C ± 2^{°}C in its thermogravimetric analysis (TGA) curve.

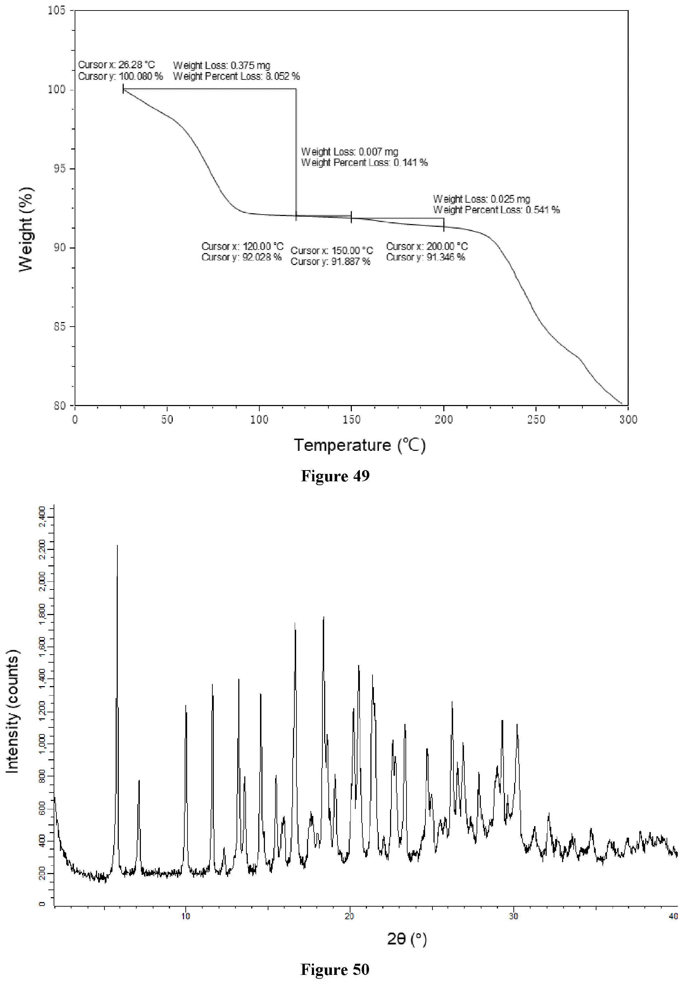

In some embodiments of the present invention, the TGA pattern of the hydrochloride crystal form G of the compound of Formula I-1 is shown in Figure 49.

The present invention also provides a method for preparing the hydrochloride crystal form G of the compound of Formula I-1, comprising adding the compound of Formula I-1 and hydrochloric acid to water, and then performing suspension recrystallization at 25^{°}C, 50^{°}C, or under heating-cooling cycles or slurrying to obtain it, wherein the ratio of the compound of Formula I-1 to hydrochloric acid is selected from 0.8 to 1.2, the hydrochloride crystal form G of the compound of Formula I-1 is a hydrate crystal form with a water content of 3.0 equivalents.

The present invention also provides the hydrochloride crystal form H of the compound of Formula I-1, and its X-ray powder diffraction pattern has characteristic diffraction peaks at the following 2θ angles: 5.83±0.2°, 7.16±0.2°, 10.02±0.2°, 11.64±0.2°, 13.23 ± 0.2 °, 14.58 ± 0.2°, 16.67 ± 0.2°, 18.40 ± 0.2°, 18.62 ± 0.2°, 20.23 ± 0.2°, 20.54 ± 0.2°, 21.40±0.2°, 22.62±0.2°, 22.78±0.2°, 23.36±0.2°, 24.73±0.2°, 26.23±0.2°, 26.57±0.2°, 26.91±0.2°, 29.30±0.2°, 30.21±0.2°.

In some embodiments of the present invention, the X-ray powder diffraction pattern of the hydrochloride crystal form H of the compound of Formula I-1 has characteristic diffraction peaks at the following 2θ angles: 5.83±0.2°, 7.16±0.2°, 10.02±0.2°, 11.64±0.2°, 12.35 ± 0.2°, 13.23 ± 0.2°, 13.59 ± 0.2°, 14.58 ± 0.2°, 15.51 ± 0.2°, 15.97 ± 0.2°, 16.67±0.2°, 17.63±0.2°, 18.40±0.2°, 18.62±0.2°, 19.11±0.2°, 20.23±0.2°, 20.54±0.2°, 21.40±0.2°, 22.07±0.2°, 22.62±0.2°, 22.78±0.2°, 23.36±0.2°, 24.73±0.2°, 24.98±0.2°, 26.23±0.2°, 26.57±0.2°, 26.91±0.2°, 27.88±0.2°, 29.01±0.2°, 29.30±0.2°, 29.61±0.2°, 30.21±0.2°, 31.21±0.2°, 32.12±0.2°, 34.66±0.2°.

In some embodiments of the present invention, the X-ray powder diffraction (XRPD) pattern of the hydrochloride crystal form H of the compound of Formula I-1 is shown in Figure 50.

**Table 24 XRPD analysis data for the hydrochloride crystal form H of the compound of Formula I-1**

| Name | Angle | d Value | Net Intensity | Gross Intensity | Rel. Intensity | FWHM |
|---|---|---|---|---|---|---|
| Peak #1 | 5.830629 | 15.14553 | 1285.098 | 1470.157 | 1 | 0.100 |
| Peak # 2 | 7.162398 | 12.3321 | 377.9528 | 573.1634 | 0.2941044 | 0.100 |
| Peak # 3 | 10.01798 | 8.822379 | 706.8278 | 901.1703 | 0.5500187 | 0.100 |
| Peak # 4 | 11.6369 | 7.598398 | 793.7754 | 997.5475 | 0.6176772 | 0.100 |
| Peak #5 | 12.34503 | 7.164086 | 105.4623 | 314.7852 | 0.0820656 | 0.100 |
| Peak # 6 | 13.23145 | 6.686064 | 823.9066 | 1041.888 | 0.6411238 | 0.100 |
| Peak # 7 | 13.59459 | 6.508269 | 401.2484 | 621.5343 | 0.3122319 | 0.100 |
| Peak # 8 | 14.57996 | 6.070556 | 742.7775 | 974.6781 | 0.5779932 | 0.100 |
| Peak # 9 | 15.50704 | 5.709655 | 380.8936 | 631.5181 | 0.2963928 | 0.100 |
| Peak # 10 | 15.97407 | 5.543761 | 171.5876 | 429.1406 | 0.133521 | 0.155 |
| Peak # 11 | 16.6685 | 5.31433 | 1043.568 | 1307.615 | 0.8120532 | 0.105 |
| Peak # 12 | 17.62552 | 5.027874 | 219.7633 | 491.7105 | 0.171009 | 0.216 |
| Peak # 13 | 18.40211 | 4.817406 | 1027.482 | 1307.842 | 0.7995363 | 0.141 |
| Peak # 14 | 18.61999 | 4.761522 | 548.1824 | 829.8808 | 0.4265687 | 0.141 |
| Peak # 15 | 19.11054 | 4.640391 | 363.7106 | 646.7841 | 0.2830218 | 0.183 |
| Peak # 16 | 20.23249 | 4.385529 | 650.7493 | 943.71 | 0.5063812 | 0.111 |
| Peak # 17 | 20.54409 | 4.31971 | 816.0809 | 1113.529 | 0.6350342 | 0.201 |
| Peak # 18 | 21.40318 | 4.148226 | 761.1138 | 1066.179 | 0.5922615 | 0.170 |
| Peak # 19 | 22.07037 | 4.024309 | 60.53448 | 366.7205 | 0.04710497 | 0.100 |
| Peak # 20 | 22.62083 | 3.927608 | 474.4857 | 778.4302 | 0.3692215 | 0.202 |
| Peak # 21 | 22.77518 | 3.90134 | 428.924 | 731.7242 | 0.3337676 | 0.202 |
| Peak # 22 | 23.35825 | 3.805256 | 580.0465 | 876.5105 | 0.4513638 | 0.100 |
| Peak # 23 | 24.73165 | 3.596966 | 435.214 | 762.8337 | 0.3386622 | 0.164 |
| Peak # 24 | 24.97894 | 3.561912 | 239.3857 | 574.4449 | 0.1862782 | 0.170 |
| Peak # 25 | 26.23346 | 3.394353 | 689.8587 | 1053.781 | 0.5368143 | 0.200 |
| Peak # 26 | 26.56802 | 3.352364 | 373.9151 | 743.0274 | 0.2909624 | 0.141 |
| Peak # 27 | 26.91138 | 3.310365 | 474.4957 | 847.8385 | 0.3692293 | 0.233 |
| Peak # 28 | 27.87534 | 3.198041 | 319.8667 | 699.1384 | 0.2489046 | 0.100 |
| Peak #29 | 29.00736 | 3.075759 | 319.4596 | 694.5037 | 0.2485878 | 0.100 |
| Peak # 30 | 29.30335 | 3.045362 | 580.9927 | 952.9413 | 0.4521 | 0.139 |
| Peak # 31 | 29.61104 | 3.014414 | 311.5282 | 679.3771 | 0.242416 | 0.100 |
| Peak # 32 | 30.20776 | 2.956213 | 486.7111 | 844.0698 | 0.3787347 | 0.169 |
| Peak # 33 | 31.21426 | 2.863146 | 132.2233 | 464.1539 | 0.1028897 | 0.149 |
| Peak # 34 | 32.11699 | 2.784701 | 156.8051 | 469.2422 | 0.122018 | 0.137 |
| Peak # 35 | 34.65822 | 2.586115 | 156.4141 | 451.3805 | 0.1217138 | 0.157 |

In some embodiments of the present invention, the hydrochloride crystal form H of the compound of Formula I-1 has the onsets of the endothermic peaks at 51.49°C ± 2°C and 153.61°C ± 2°C in its differential scanning calorimetry (DSC) curve, and decomposition of the sample begins at 211.73°C ± 2^{°}C.

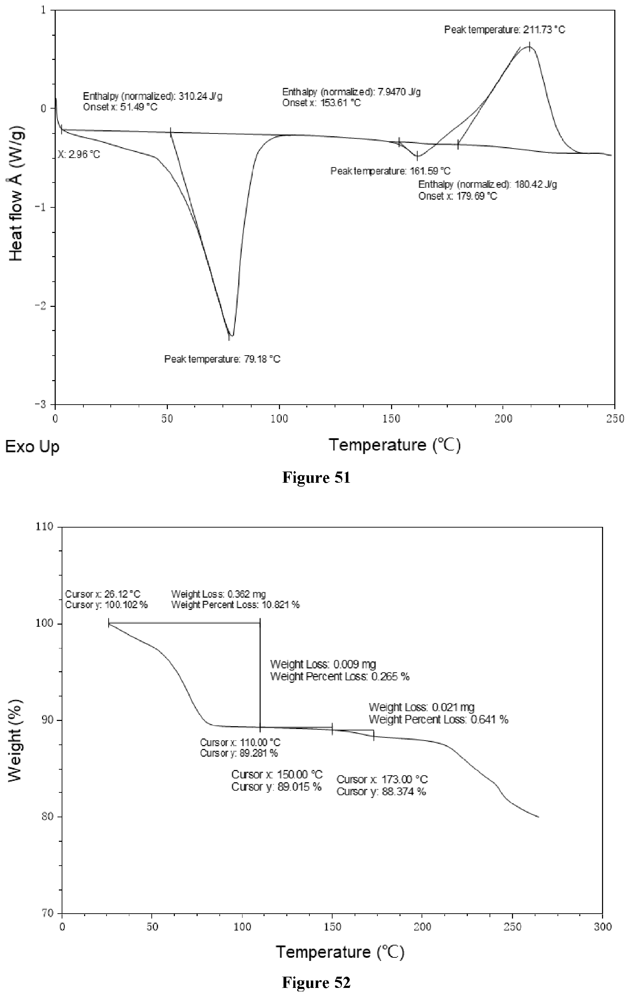

In some embodiments of the present invention, the DSC pattern of the hydrochloride crystal form H of the compound of Formula I-1 is shown in Figure 51.

In some embodiments of the present invention, the hydrochloride crystal form H of the compound of Formula I-1 has three weight loss steps at 26.12°C ± 2^{°}C, 110.00^{°}C ± 2^{°}C, and 150.00^{°}C ± 2^{°}C in its thermogravimetric analysis (TGA) curve.

In some embodiments of the present invention, the TGA pattern of the hydrochloride crystal form H of the compound of Formula I-1 is shown in Figure 52.

The present invention also provides a method for preparing the hydrochloride crystal form H of the compound of Formula I-1, comprising adding the compound of Formula I-1 and hydrochloric acid to a methanol/water mixed solvent, and then performing suspension recrystallization at 25^{°}C or 50^{°}C or slurrying to obtain it, wherein the ratio of the compound of Formula I-1 to hydrochloric acid is selected from 0.8 to 1.2, the hydrochloride crystal form H of the compound of Formula I-1 is a hydrate crystal form, with a water content of 3.9 equivalents.

The present invention also provides the hydrochloride crystal form I of the compound of Formula I-1, and its X-ray powder diffraction pattern has characteristic diffraction peaks at the following 2θ angles: 6.68±0.2°, 12.31±0.2°, 12.54 ± 0.2°, 13.34 ± 0.2°, 14.05 ± 0.2°, 14.96 ± 0.2°, 17.92 ± 0.2 °, 19.03 ± 0.2°, 19.74 ± 0.2°, 19.99 ± 0.2°, 20.96 ± 0.2°, 21.42 ± 0.2°, 21.62 ± 0.2°, 26.81±0.2°, 27.21±0.2°, 27.60±0.2°, 27.78±0.2°, 30.07±0.2°, 31.00±0.2°, 34.83±0.2°.

In some embodiments of the present invention, the X-ray powder diffraction pattern of the hydrochloride crystal form I of the compound of Formula I-1 has characteristic diffraction peaks at the following 2θ angles: 6.68±0.2°, 9.50±0.2°, 12.31±0.2°, 12.54 ± 0.2°, 13.34 ± 0.2°, 14.05 ± 0.2°, 14.96 ± 0.2°, 15.38 ± 0.2°, 17.18 ± 0.2°, 17.92±0.2°, 19.03±0.2°, 19.74±0.2°, 19.99±0.2°, 20.96±0.2°, 21.42±0.2°, 21.62±0.2°, 21.99±0.2°, 22.98±0.2°, 23.52±0.2°, 23.80±0.2°, 24.38±0.2°, 25.92±0.2°, 26.81±0.2°, 27.21±0.2°, 27.60±0.2°, 27.78±0.2°, 28.26±0.2°, 28.74±0.2°, 30.07±0.2°, 31.00±0.2°, 32.69±0.2°, 34.02±0.2°, 34.83±0.2°.

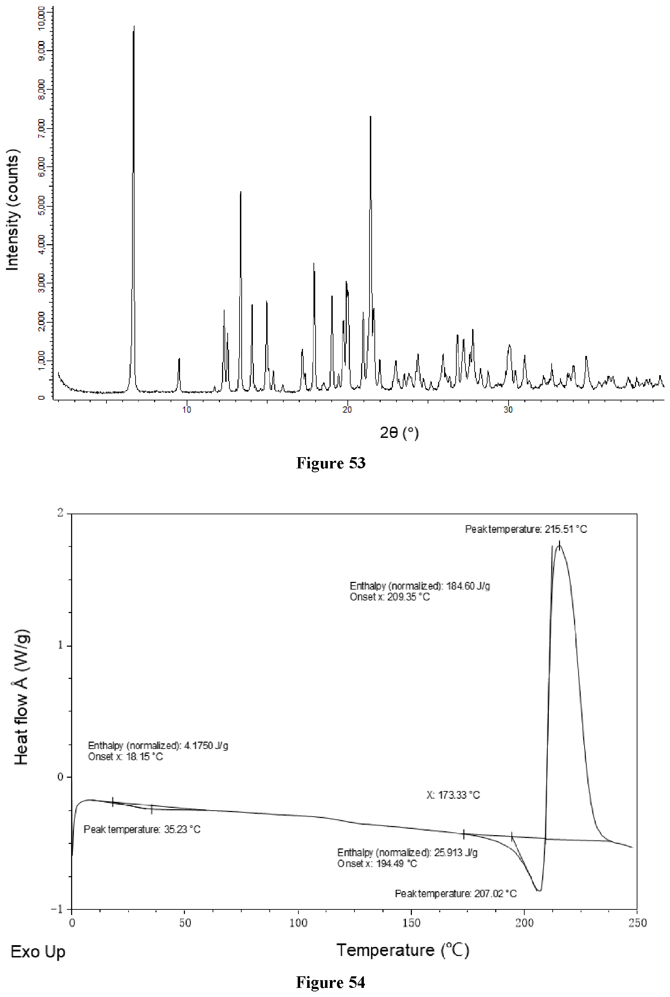

In some embodiments of the present invention, the X-ray diffraction (XRPD) pattern of the hydrochloride crystal form I of the compound of Formula I-1 is shown in Figure 53.

**Table 25 XRPD analysis data for the hydrochloride crystal form I of the compound of Formula I-1**

| Name | Angle | d Value | Net Intensity | Gross Intensity | Rel. Intensity | FWHM |
|---|---|---|---|---|---|---|
| Peak #1 | 6.683047 | 13.21552 | 6066.766 | 6257.472 | 1 | 0.100 |
| Peak # 2 | 9.502496 | 9.29978 | 589.4446 | 779.7198 | 0.09715961 | 0.100 |
| Peak # 3 | 12.30501 | 7.187298 | 1473.677 | 1683.435 | 0.2429098 | 0.123 |
| Peak # 4 | 12.54249 | 7.05174 | 1045.291 | 1257.185 | 0.1722979 | 0.127 |
| Peak # 5 | 13.34207 | 6.630879 | 3563.501 | 3783.915 | 0.5873806 | 0.100 |
| Peak # 6 | 14.04679 | 6.299753 | 1592.864 | 1818.627 | 0.2625557 | 0.100 |
| Peak # 7 | 14.95737 | 5.918223 | 1689.783 | 1912.131 | 0.2785311 | 0.100 |
| Peak # 8 | 15.37841 | 5.757124 | 366.1042 | 582.8315 | 0.06034585 | 0.100 |
| Peak # 9 | 17.17957 | 5.157372 | 679.5376 | 900.721 | 0.1120099 | 0.100 |
| Peak # 10 | 17.91742 | 4.946615 | 2463.704 | 2704.65 | 0.4060984 | 0.100 |
| Peak # 11 | 19.02736 | 4.66049 | 1726.037 | 1982.815 | 0.2845069 | 0.100 |
| Peak # 12 | 19.74237 | 4.493283 | 1203.647 | 1471.858 | 0.1984002 | 0.192 |
| Peak # 13 | 19.98544 | 4.43918 | 1433.109 | 1702.924 | 0.2362228 | 0.173 |
| Peak # 14 | 20.96464 | 4.233996 | 1457.9 | 1735.907 | 0.2403092 | 0.100 |
| Peak # 15 | 21.42234 | 4.144559 | 5250.236 | 5529.593 | 0.8654093 | 0.100 |
| Peak # 16 | 21.61991 | 4.107128 | 1554.836 | 1833.5 | 0.2562874 | 0.100 |
| Peak # 17 | 21.98783 | 4.039227 | 591.0263 | 866.3695 | 0.09742032 | 0.100 |
| Peak # 18 | 22.98335 | 3.866472 | 515.3694 | 786.2349 | 0.0849496 | 0.100 |
| Peak # 19 | 23.52402 | 3.778814 | 255.2394 | 529.0739 | 0.04207174 | 0.166 |
| Peak # 20 | 23.80309 | 3.735139 | 274.9154 | 548.0394 | 0.04531498 | 0.232 |
| Peak # 21 | 24.38212 | 3.647733 | 666.5188 | 933.2823 | 0.1098639 | 0.116 |
| Peak # 22 | 25.92394 | 3.434175 | 635.7052 | 928.6979 | 0.1047848 | 0.117 |
| Peak # 23 | 26.81065 | 3.322574 | 1095.841 | 1412.559 | 0.1806302 | 0.100 |
| Peak # 24 | 27.2072 | 3.275041 | 877.7557 | 1200.082 | 0.1446826 | 0.117 |
| Peak # 25 | 27.60325 | 3.228944 | 689.9982 | 1014.847 | 0.1137341 | 0.134 |
| Peak # 26 | 27.77863 | 3.208954 | 1194.642 | 1519.628 | 0.1969159 | 0.134 |
| Peak # 27 | 28.25596 | 3.155821 | 372.9469 | 695.2362 | 0.06147375 | 0.100 |
| Peak # 28 | 28.7368 | 3.1041 | 304.8406 | 620.1004 | 0.05024763 | 0.100 |
| Peak #29 | 30.06931 | 2.969508 | 807.1132 | 1116.959 | 0.1330384 | 0.171 |
| Peak # 30 | 30.99668 | 2.882746 | 682.3029 | 984.0446 | 0.1124657 | 0.100 |
| Peak # 31 | 32.68733 | 2.737403 | 496.4317 | 815.9324 | 0.08182806 | 0.100 |
| Peak # 32 | 34.01857 | 2.633264 | 468.1613 | 783.8876 | 0.07716818 | 0.178 |
| Peak # 33 | 34.83157 | 2.57364 | 645.0126 | 947.0595 | 0.106319 | 0.108 |

In some embodiments of the present invention, the hydrochloride crystal form I of the compound of Formula I-1 has the onsets of the endothermic peaks at 18.15°C ± 2°C and 194.49°C ± 2°C in its differential scanning calorimetry (DSC) curve, and decomposition of the sample begins at 215.51 °c ± 2°C

In some embodiments of the present invention, the DSC pattern of the hydrochloride crystal form I of the compound of Formula I-1 is shown in Figure 54.

In some embodiments of the present invention, the hydrochloride crystal form I of the compound of Formula I-1 has three weight loss steps at 28.31°C ± 2°C, 70.00°C ± 2°C, and 173.00°C ± 2°C in its thermogravimetric analysis (TGA) curve.

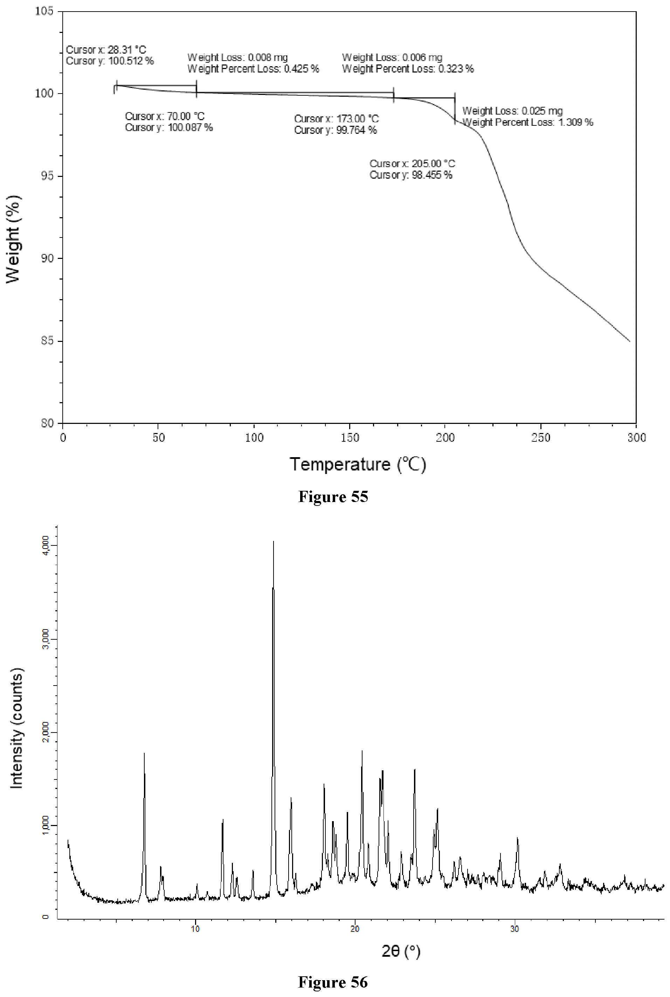

In some embodiments of the present invention, the TGA pattern of the hydrochloride crystal form I of the compound of Formula I-1 is shown in Figure 55.

The present invention also provides a method for preparing the hydrochloride crystal form I of the compound of Formula I-1, comprising adding the compound of Formula I-1 and hydrochloric acid to methanol or a methanol/tert-butyl methyl ether mixed solvent, and then performing suspension recrystallization in a tert-butyl methyl ether or slurrying to obtain it, the ratio of the compound of Formula I-1 to hydrochloric acid is selected from 0.8 to 1.2, and the hydrochloride crystal form I of the compound of Formula I-1 is an anhydrous crystal form.

The present invention also provides the hydrochloride crystal form J1 of the compound of Formula I-1, and its X-ray powder diffraction pattern has characteristic diffraction peaks at the following 2θ angles: 6.80 ± 0.2°, 11.69 ± 0.2°, 14.87 ± 0.2°, 15.98 ± 0.2°, 18.05±0.2°, 19.49±0.2°, 20.41±0.2°, 21.55±0.2°, 21.70±0.2°, 22.06±0.2°, 23.69±0.2°, 24.92±0.2°, 25.13±0.2°, 29.07±0.2°, 30.16±0.2°.

In some embodiments of the present invention, the X-ray powder diffraction pattern of the hydrochloride crystal form J1 of the compound of Formula I-1 has characteristic diffraction peaks at the following 2θ angles: 6.80±0.2°, 7.82±0.2°, 7.95±0.2°, 10.08±0.2°, 11.69 ± 0.2°, 12.30 ± 0.2°, 12.60 ± 0.2°, 13.60 ± 0.2°, 14.87 ± 0.2°, 15.98 ± 0.2°, 16.25 ± 0.2°, 18.05±0.2°, 18.60±0.2°, 18.79±0.2°, 19.49±0.2°, 20.41±0.2°, 20.82±0.2°, 21.55±0.2°, 21.70±0.2°, 22.06±0.2°, 22.88±0.2°, 23.69±0.2°, 24.92±0.2°, 25.13±0.2°, 26.19±0.2°, 26.57±0.2°, 29.07±0.2°, 30.16±0.2°, 31.85±0.2°, 32.80±0.2°.

In some embodiments of the present invention, the X-ray diffraction (XRPD) pattern of the hydrochloride crystal form J1 of the compound of Formula I-1 is shown in Figure 56.

**Table 26 XRPD analysis data for the hydrochloride crystal form J1 of the compound of Formula I-1**

| Name | Angle | d Value | Net Intensity | Gross Intensity | Rel. Intensity | FWHM |
|---|---|---|---|---|---|---|
| Peak #1 | 6.795494 | 12.99709 | 1045.542 | 1237.377 | 0.3823281 | 0.100 |
| Peak # 2 | 7.822898 | 11.2923 | 230.3243 | 429.0735 | 0.0842237 | 0.101 |
| Peak # 3 | 7.947547 | 11.11546 | 173.3303 | 372.1519 | 0.06338246 | 0.101 |
| Peak # 4 | 10.08289 | 8.765732 | 113.045 | 325.6056 | 0.04133766 | 0.100 |
| Peak # 5 | 11.69263 | 7.562305 | 581.4788 | 792.2735 | 0.212632 | 0.100 |
| Peak # 6 | 12.29569 | 7.192721 | 271.5138 | 485.7134 | 0.09928567 | 0.160 |
| Peak # 7 | 12.5974 | 7.021128 | 160.1172 | 374.2798 | 0.05855079 | 0.186 |
| Peak # 8 | 13.6026 | 6.504458 | 195.112 | 411.141 | 0.07134749 | 0.100 |
| Peak # 9 | 14.87382 | 5.951278 | 2734.673 | 2980.22 | 1 | 0.100 |
| Peak # 10 | 15.97916 | 5.542004 | 742.0891 | 1013.763 | 0.271363 | 0.100 |
| Peak # 11 | 16.25086 | 5.449955 | 156.1842 | 432.9994 | 0.05711259 | 0.129 |
| Peak # 12 | 18.05202 | 4.910034 | 797.7896 | 1127.659 | 0.2917313 | 0.100 |
| Peak # 13 | 18.59785 | 4.767141 | 489.3123 | 835.8884 | 0.178929 | 0.213 |
| Peak # 14 | 18.7898 | 4.718873 | 408.4137 | 759.966 | 0.1493465 | 0.213 |
| Peak # 15 | 19.4894 | 4.55103 | 554.901 | 920.6138 | 0.2029131 | 0.100 |
| Peak # 16 | 20.41075 | 4.347628 | 1084.362 | 1459.224 | 0.3965236 | 0.100 |
| Peak # 17 | 20.81767 | 4.263556 | 308.0087 | 683.4725 | 0.1126309 | 0.100 |
| Peak # 18 | 21.55439 | 4.119464 | 785.2869 | 1156.483 | 0.2871594 | 0.270 |
| Peak # 19 | 21.69789 | 4.092543 | 917.0377 | 1286.597 | 0.3353373 | 0.270 |
| Peak # 20 | 22.05619 | 4.026863 | 513.4854 | 877.8127 | 0.1877685 | 0.100 |
| Peak # 21 | 22.87765 | 3.884097 | 242.5795 | 597.0117 | 0.08870515 | 0.100 |
| Peak # 22 | 23.6933 | 3.752199 | 965.4345 | 1321.158 | 0.3530348 | 0.100 |
| Peak # 23 | 24.92196 | 3.569928 | 433.7296 | 789.9819 | 0.1586039 | 0.223 |
| Peak # 24 | 25.13496 | 3.540156 | 587.4705 | 941.8578 | 0.214823 | 0.149 |
| Peak # 25 | 26.18639 | 3.400348 | 228.3669 | 583.2324 | 0.08350793 | 0.100 |
| Peak # 26 | 26.56552 | 3.352674 | 207.2965 | 567.4351 | 0.07580306 | 0.127 |
| Peak # 27 | 29.07455 | 3.068804 | 275.8094 | 631.2031 | 0.1008565 | 0.100 |
| Peak # 28 | 30.16433 | 2.96037 | 409.7868 | 740.9128 | 0.1498486 | 0.100 |
| Peak #29 | 31.851 | 2.807346 | 160.3939 | 484.1836 | 0.05865197 | 0.206 |
| Peak # 30 | 32.79772 | 2.728441 | 190.6454 | 520.0348 | 0.06971417 | 0.182 |

In some embodiments of the present invention, the hydrochloride crystal form J1 of the compound of Formula I-1 has the onsets of the endothermic peaks at 70.82°C ± 2^{°}C and 165.48^{°}C ± 2^{°}C in its differential scanning calorimetry (DSC) curve, and decomposition of the sample begins at 212.96°C ± 2^{°}C.

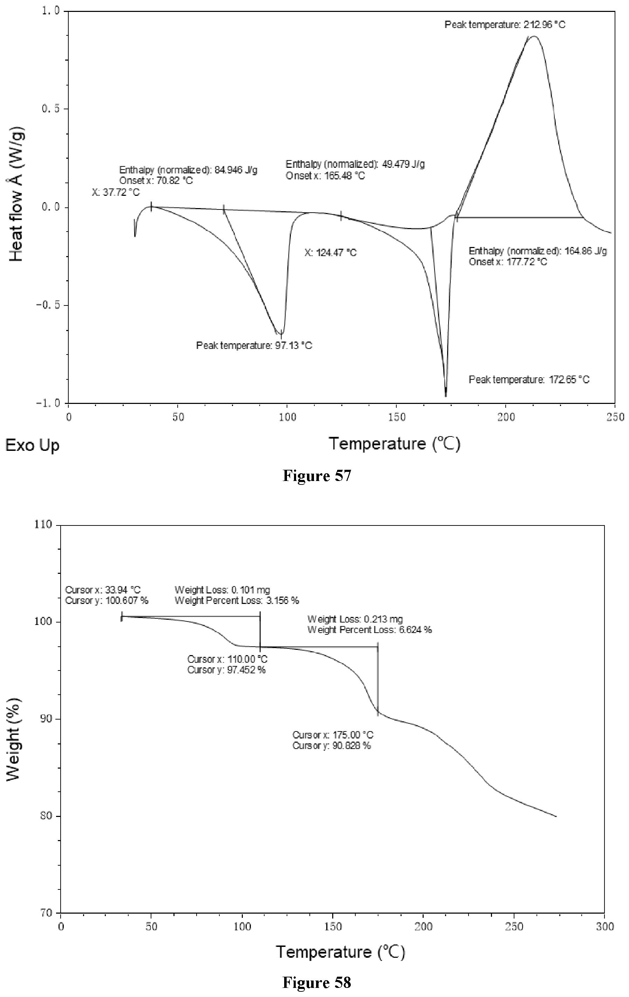

In some embodiments of the present invention, the DSC pattern of the hydrochloride crystal form J1 of the compound of Formula I-1 is shown in Figure 57.

In some embodiments of the present invention, the hydrochloride crystal form J1 of the compound of Formula I-1 has two weight loss steps at 33.94°C ± 2^{°}C and 110.00^{°}C ± 2^{°}C in its thermogravimetric analysis (TGA) curve.

In some embodiments of the present invention, the TGA pattern of the hydrochloride crystal form J1 of the compound of Formula I-1 is shown in Figure 58.

The present invention also provides a method for preparing the hydrochloride crystal form J1 of the compound of Formula I-1, comprising adding the compound of Formula I-1 and hydrochloric acid to 2-methyl-tetrahydrofuran, and then performing suspension recrystallization at 50^{°}C or under heating-cooling cycles or slurrying to obtain it, the ratio of the compound of Formula I-1 to hydrochloric acid is selected from 0.8 to 1.2, the hydrochloride crystal form J1 of the compound of Formula I-1 is a solvate of 2-methyl tetrahydrofuran and water, with a 2-methyl tetrahydrofuran content of 0.75 equivalents and a water content of 0.98 equivalents.

The present invention also provides the hydrochloride crystal form J2 of the compound of Formula I-1, and its X-ray powder diffraction pattern has characteristic diffraction peaks at the following 2θ angles: 6.78±0.2°, 11.59±0.2°, 12.24 ± 0.2°, 13.56 ± 0.2°, 14.69 ± 0.2°, 17.45 ± 0.2°, 18.12 ± 0.2°, 18.89 ± 0.2°, 19.51 ± 0.2°, 21.28±0.2°, 21.46±0.2°, 22.23±0.2°, 24.09±0.2°, 24.86±0.2°, 24.99±0.2°, 29.00±0.2°.

In some embodiments of the present invention, the X-ray powder diffraction pattern of the hydrochloride crystal form J2 of the compound of Formula I-1 has characteristic diffraction peaks at the following 2θ angles: 6.78±0.2°, 7.96±0.2°, 8.31±0.2°, 11.59±0.2°, 12.24 ± 0.2^{°}, 13.56 ± 0.2^{°}, 14.69 ± 0.2 °, 16.44 ± 0.2^{°}, 16.63 ± 0.2^{°}, 17.45 ± 0.2^{°}, 18.12 ± 0.2^{°}, 18.89±0.2°, 19.51±0.2°, 20.12±0.2°, 20.35±0.2°, 20.49±0.2°, 21.28±0.2°, 21.46±0.2°, 22.23±0.2°, 22.75±0.2°, 23.27±0.2°, 24.09±0.2°, 24.86±0.2°, 24.99±0.2°, 25.37±0.2°, 25.65±0.2°, 26.54±0.2°, 27.62±0.2°, 28.61±0.2°, 29.00±0.2°, 30.43±0.2°, 32.10±0.2°, 33.01±0.2°, 36.96±0.2°.

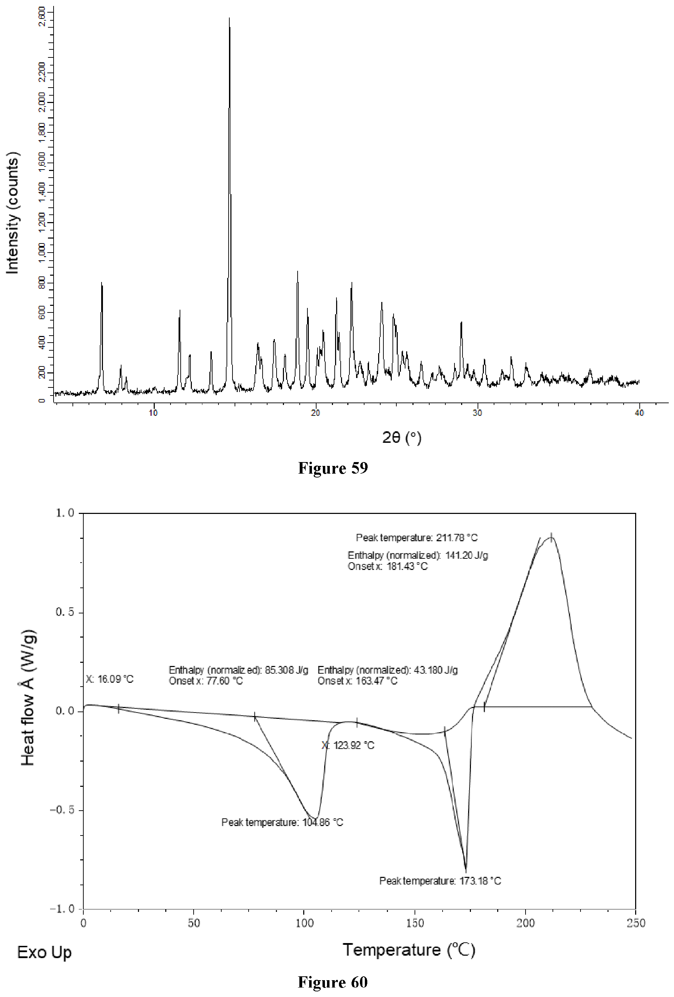

In some embodiments of the present invention, the X-ray diffraction (XRPD) pattern of the hydrochloride crystal form J2 of the compound of Formula I-1 is shown in Figure 59.

**Table 27 XRPD analysis data for the hydrochloride crystal form J2 of the compound of Formula I-1**

| Name | Angle | d Value | Net Intensity | Gross Intensity | Rel. Intensity | FWHM |
|---|---|---|---|---|---|---|
| Peak #1 | 6.780673 | 13.02546 | 486.214 | 549.5581 | 0.2816536 | 0.100 |
| Peak # 2 | 7.958976 | 11.09953 | 123.6075 | 188.0724 | 0.07160323 | 0.161 |
| Peak # 3 | 8.310797 | 10.63043 | 73.68546 | 139.4296 | 0.04268445 | 0.107 |
| Peak # 4 | 11.59174 | 7.627896 | 375.3433 | 450.2339 | 0.2174286 | 0.100 |
| Peak # 5 | 12.23828 | 7.226334 | 153.1708 | 226.7464 | 0.08872864 | 0.100 |
| Peak # 6 | 13.55637 | 6.526536 | 180.8496 | 254.4021 | 0.1047624 | 0.100 |
| Peak # 7 | 14.68543 | 6.027195 | 1726.283 | 1805.761 | 1 | 0.100 |
| Peak # 8 | 16.4355 | 5.389143 | 215.2298 | 299.1415 | 0.1246781 | 0.184 |
| Peak # 9 | 16.62825 | 5.327103 | 129.1316 | 213.7627 | 0.07480326 | 0.184 |
| Peak # 10 | 17.44625 | 5.079131 | 220.9248 | 310.6566 | 0.1279771 | 0.103 |
| Peak # 11 | 18.1169 | 4.892595 | 133.8183 | 226.216 | 0.07751816 | 0.100 |
| Peak # 12 | 18.88617 | 4.695011 | 516.7839 | 612.7637 | 0.2993622 | 0.100 |
| Peak # 13 | 19.5106 | 4.546134 | 397.7841 | 496.3569 | 0.2304281 | 0.100 |
| Peak # 14 | 20.11981 | 4.409835 | 184.1742 | 286.9377 | 0.1066883 | 0.320 |
| Peak # 15 | 20.34798 | 4.360898 | 241.6843 | 346.9122 | 0.1400027 | 0.238 |
| Peak # 16 | 20.48667 | 4.331687 | 265.6085 | 372.0766 | 0.1538615 | 0.238 |
| Peak # 17 | 21.28307 | 4.171363 | 432.1003 | 541.9163 | 0.2503067 | 0.121 |
| Peak # 18 | 21.45671 | 4.137996 | 276.4566 | 386.148 | 0.1601456 | 0.121 |
| Peak # 19 | 22.22805 | 3.996116 | 497.313 | 614.4642 | 0.2880831 | 0.100 |
| Peak # 20 | 22.75281 | 3.905124 | 142.7566 | 265.5026 | 0.08269595 | 0.156 |
| Peak # 21 | 23.27106 | 3.819316 | 113.8295 | 241.2689 | 0.06593904 | 0.171 |
| Peak # 22 | 24.09327 | 3.690806 | 385.4814 | 518.8451 | 0.2233013 | 0.130 |
| Peak # 23 | 24.85536 | 3.579343 | 212.2231 | 344.9595 | 0.1229364 | 0.175 |
| Peak # 24 | 24.98804 | 3.560637 | 267.8217 | 399.8474 | 0.1551436 | 0.175 |
| Peak # 25 | 25.3711 | 3.507738 | 162.8907 | 291.8631 | 0.09435921 | 0.226 |
| Peak # 26 | 25.64969 | 3.470267 | 130.3427 | 256.16 | 0.07550482 | 0.219 |
| Peak # 27 | 26.53912 | 3.355949 | 103.8878 | 220.8986 | 0.06018002 | 0.100 |
| Peak # 28 | 27.62185 | 3.226811 | 76.33185 | 194 | 0.04421745 | 0.256 |
| Peak #29 | 28.60708 | 3.11788 | 106.999 | 228.0308 | 0.06198227 | 0.100 |
| Peak # 30 | 29.00274 | 3.07624 | 319.7704 | 443.9528 | 0.1852363 | 0.100 |
| Peak # 31 | 30.42807 | 2.935309 | 124.64 | 247.0201 | 0.07220139 | 0.100 |
| Peak # 32 | 32.10201 | 2.785966 | 131.7526 | 254.2239 | 0.07632156 | 0.113 |
| Peak # 33 | 33.0099 | 2.711386 | 81.86591 | 203.6693 | 0.04742322 | 0.136 |
| Peak # 34 | 36.95634 | 2.430403 | 74.8323 | 193.9958 | 0.04334879 | 0.176 |

In some embodiments of the present invention, the hydrochloride crystal form J2 of the compound of Formula I-1 has the onsets of the endothermic peaks at 77.60°C ± 2°C and 163.47°C ± 2°C in its differential scanning calorimetry (DSC) curve, and decomposition of the sample begins at 211.78°C ± 2°C.

In some embodiments of the present invention, the DSC pattern of the hydrochloride crystal form J2 of the compound of Formula I-1 is shown in Figure 60.

In some embodiments of the present invention, the hydrochloride crystal form J2 of the compound of Formula I-1 has two weight loss steps at 28.73°C ± 2^{°}C and 120.00°C ± 2^{°}C in its thermogravimetric analysis (TGA) curve.

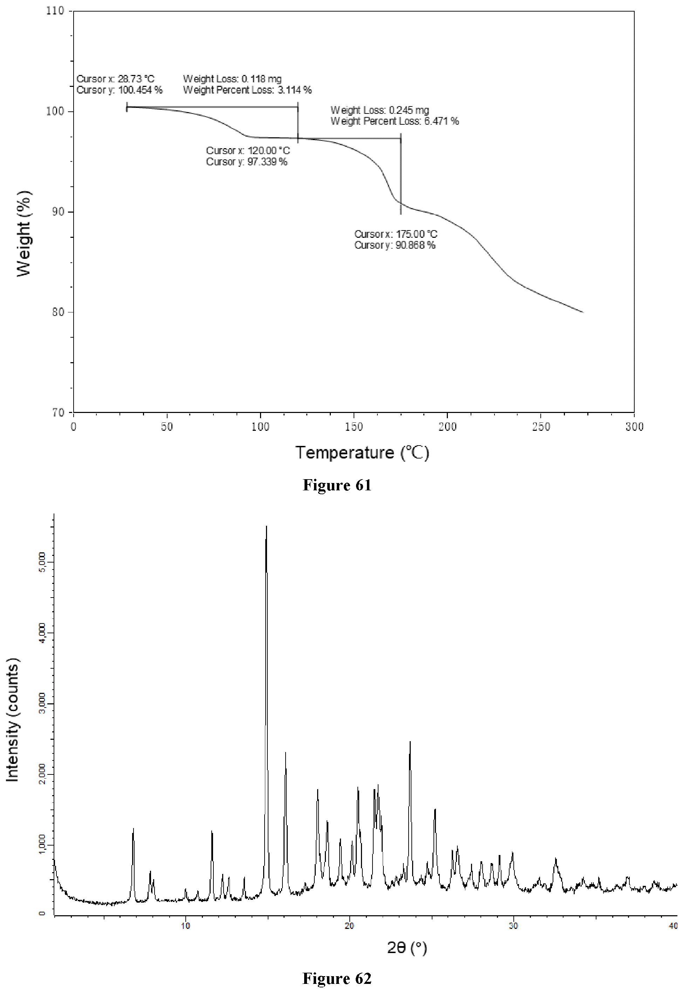

In some embodiments of the present invention, the TGA pattern of the hydrochloride crystal form J2 of the compound of Formula I-1 is shown in Figure 61.

The present invention also provides a method for preparing the hydrochloride crystal form J2 of the compound of Formula I-1, comprising heating the hydrochloride crystal form J1 of the compound of Formula I-1 to 110°C to obtain it, wherein the ratio of the compound of Formula I-1 to hydrochloric acid is selected from 0.8 to 1.2, the hydrochloride crystal form J2 of the compound of Formula I-1 is a solvate of 2-methyl-tetrahydrofuran and water, with a 2-methyl-tetrahydrofuran content of 0.39 equivalents and a water content of 0.94 equivalents.

The present invention also provides the hydrochloride crystal form K of the compound of Formula I-1, and its X-ray powder diffraction pattern has characteristic diffraction peaks at the following 2θ angles: 6.79 ± 0.2^{°}, 11.61 ± 0.2^{°}, 14.93 ± 0.2^{°}, 16.08 ± 0.2^{°}, 18.05 ± 0.2^{°}, 18.63 ± 0.2°, 20.49 ± 0.2°, 20.64 ± 0.2°, 21.52 ± 0.2°, 21.72 ± 0.2°, 21.93±0.2°, 23.68±0.2°, 25.19±0.2°, 26.25±0.2°, 26.57±0.2°, 29.91±0.2°, 32.56±0.2°.

In some embodiments of the present invention, the X-ray powder diffraction pattern of the hydrochloride crystal form K of the compound of Formula I-1 has characteristic diffraction peaks at the following 2θ angles: 6.79±0.2°, 7.83±0.2°, 8.04±0.2°, 10.00±0.2°, 10.74 ± 0.2°, 11.61 ± 0.2°, 12.25 ± 0.2 °, 12.64 ± 0.2°, 13.57 ± 0.2°, 14.93 ± 0.2°, 16.08 ± 0.2°, 18.05±0.2°, 18.63±0.2°, 19.43±0.2°, 20.15±0.2°, 20.49±0.2°, 20.64±0.2°, 21.52±0.2°, 21.72±0.2°, 21.93±0.2°, 23.27±0.2°, 23.68±0.2°, 24.73±0.2°, 25.19±0.2°, 26.25±0.2°, 26.57±0.2°, 27.42±0.2°, 28.02±0.2°, 28.67±0.2°, 29.13±0.2°, 29.91±0.2°, 32.56±0.2°.

In some embodiments of the present invention, the X-ray powder diffraction (XRPD) pattern of the hydrochloride crystal form K of the compound of Formula I-1 is shown in Figure 62.

**Table 28 XRPD analysis data for the hydrochloride crystal form K of compound of Formula I-1**

| Name | Angle | d Value | Net Intensity | Gross Intensity | Rel. Intensity | FWHM |
|---|---|---|---|---|---|---|
| Peak #1 | 6.794806 | 12.9984 | 691.3811 | 875.0555 | 0.192033 | 0.100 |
| Peak # 2 | 7.83426 | 11.27594 | 288.9213 | 486.3343 | 0.08024868 | 0.120 |
| Peak # 3 | 8.038795 | 10.9895 | 203.2409 | 403.895 | 0.05645072 | 0.120 |
| Peak # 4 | 9.997069 | 8.840785 | 117.7202 | 330.8371 | 0.03269712 | 0.100 |
| Peak # 5 | 10.74072 | 8.230294 | 107.3294 | 320.8759 | 0.02981103 | 0.100 |
| Peak # 6 | 11.60559 | 7.618825 | 687.957 | 909.0914 | 0.191082 | 0.100 |
| Peak # 7 | 12.25333 | 7.217494 | 245.9523 | 473.5915 | 0.06831394 | 0.100 |
| Peak # 8 | 12.63822 | 6.998539 | 204.09 | 432.644 | 0.05668657 | 0.100 |
| Peak # 9 | 13.57036 | 6.519837 | 198.4962 | 429.6321 | 0.05513286 | 0.100 |
| Peak # 10 | 14.92862 | 5.929554 | 3600.324 | 3866.778 | 1 | 0.100 |
| Peak # 11 | 16.0831 | 5.506421 | 1376.485 | 1669.815 | 0.3823225 | 0.100 |
| Peak # 12 | 18.04593 | 4.911676 | 1009.741 | 1363.736 | 0.2804584 | 0.100 |
| Peak # 13 | 18.63182 | 4.758526 | 671.5975 | 1046.877 | 0.1865381 | 0.100 |
| Peak # 14 | 19.42977 | 4.564864 | 496.6809 | 892.7581 | 0.1379545 | 0.100 |
| Peak # 15 | 20.14869 | 4.403579 | 441.9977 | 848.7093 | 0.1227661 | 0.100 |
| Peak # 16 | 20.48797 | 4.331416 | 1028.155 | 1437.222 | 0.2855728 | 0.128 |
| Peak # 17 | 20.64182 | 4.299478 | 583.2937 | 992.8682 | 0.1620114 | 0.128 |
| Peak # 18 | 21.52175 | 4.125638 | 978.1533 | 1383.857 | 0.2716848 | 0.100 |
| Peak # 19 | 21.72215 | 4.088027 | 1036.466 | 1439.683 | 0.2878813 | 0.308 |
| Peak # 20 | 21.92503 | 4.050655 | 739.6849 | 1139.781 | 0.2054495 | 0.308 |
| Peak # 21 | 23.27148 | 3.819247 | 248.8957 | 653.3616 | 0.06913146 | 0.102 |
| Peak # 22 | 23.67982 | 3.754304 | 1547.461 | 1952.999 | 0.4298115 | 0.100 |
| Peak # 23 | 24.72815 | 3.597466 | 280.2606 | 677.2075 | 0.07784315 | 0.158 |
| Peak # 24 | 25.18878 | 3.532713 | 791.9027 | 1179.913 | 0.2199532 | 0.104 |
| Peak # 25 | 26.25322 | 3.391844 | 441.882 | 818.9271 | 0.122734 | 0.100 |
| Peak # 26 | 26.57399 | 3.351624 | 425.2781 | 809.8372 | 0.1181222 | 0.210 |
| Peak # 27 | 27.41809 | 3.250327 | 260.4725 | 657.5086 | 0.07234696 | 0.100 |
| Peak # 28 | 28.02496 | 3.181306 | 278.7974 | 678.9731 | 0.07743675 | 0.112 |
| Peak #29 | 28.66725 | 3.111473 | 222.631 | 620.5598 | 0.06183639 | 0.100 |
| Peak # 30 | 29.12559 | 3.063542 | 364.7587 | 757.3303 | 0.1013127 | 0.100 |
| Peak # 31 | 29.90952 | 2.985008 | 394.9463 | 771.1329 | 0.1096974 | 0.179 |
| Peak # 32 | 32.55656 | 2.748099 | 310.4091 | 669.1288 | 0.08621699 | 0.227 |

In some embodiments of the present invention, the hydrochloride crystal form K of the compound of Formula I-1 has the onsets of the endothermic peaks at 80.73°C ± 2^{°}C and 171.74^{°}C ± 2^{°}C in its differential scanning calorimetry (DSC) curve, and decomposition of the sample begins at 211.42°C ± 2^{°}C.

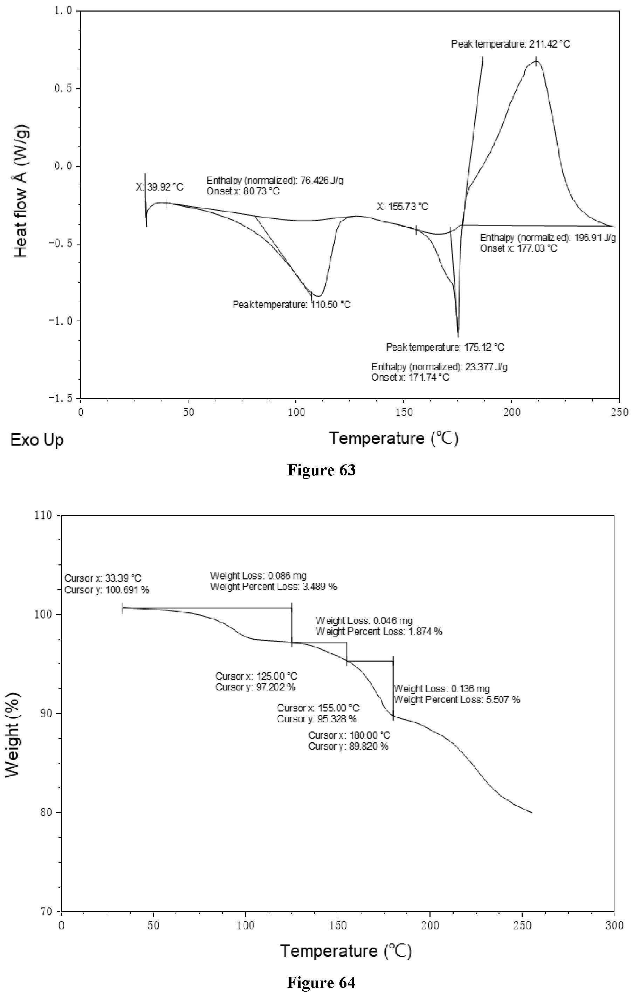

In some embodiments of the present invention, the DSC pattern of the hydrochloride crystal form K of the compound of Formula I-1 is shown in Figure 63.

In some embodiments of the present invention, the hydrochloride crystal form K of the compound of Formula I-1 has three weight loss steps at 33.39°C ± 2^{°}C, 125.00°C ± 2^{°}C, and 155.00^{°}C ± 2^{°}C in its thermogravimetric analysis (TGA) curve.

In some embodiments of the present invention, the TGA pattern of the hydrochloride crystal form K of the compound of Formula I-1 is shown in Figure 64.

The present invention also provides a method for preparing the hydrochloride crystal form K of the compound of Formula I-1, comprising adding the compound of Formula I-1 and hydrochloric acid to methyl tert-butyl ether, and then performing suspension recrystallization at 50^{°}C or under heating-cooling cycles or slurrying to obtain it, the ratio of the compound of Formula I-1 to hydrochloric acid is selected from 0.8 to 1.2, and the hydrochloride crystal form K of the compound of Formula I-1 is a solvate of methyl tert-butyl ether and water, with a methyl tert-butyl ether content of 0.5 equivalents and a water content of 0.93 equivalents.

The present invention also provides the hydrochloride crystal form L of the compound of Formula I-1, and its X-ray powder diffraction pattern has characteristic diffraction peaks at the following 2θ angles: 8.15±0.2°, 12.72±0.2°, 13.59±0.2°, 15.07±0.2°, 15.56 ± 0.2°, 16.60 ± 0.2°, 16.89 ± 0.2°, 17.56 ± 0.2°, 19.74 ± 0.2°, 20.52 ± 0.2°, 21.69±0.2°, 22.60±0.2°, 25.57±0.2°, 27.48±0.2°, 29.54±0.2°, 29.80±0.2°.

In some embodiments of the present invention, the X-ray powder diffraction pattern of the hydrochloride crystal form L of the compound of Formula I-1 has characteristic diffraction peaks at the following 2θ angles: 7.70±0.2°, 8.15±0.2°, 8.82±0.2°, 9.85±0.2°, 11.25±0.2°, 12.72±0.2°, 13.59±0.2°, 14.68±0.2°, 15.07±0.2°, 15.56±0.2°, 16.60±0.2°, 16.89±0.2°, 17.56±0.2°, 18.53±0.2°, 18.98±0.2°, 19.74±0.2°, 20.29±0.2°, 20.52±0.2°, 21.30±0.2°, 21.69±0.2°, 22.60±0.2°, 22.79±0.2°, 24.38±0.2°, 25.57±0.2°, 26.21±0.2°, 26.50±0.2°, 26.97±0.2°, 27.48±0.2°, 28.17±0.2°, 28.60±0.2°, 29.54±0.2°, 29.80±0.2°, 31.17±0.2°, 33.28±0.2°.

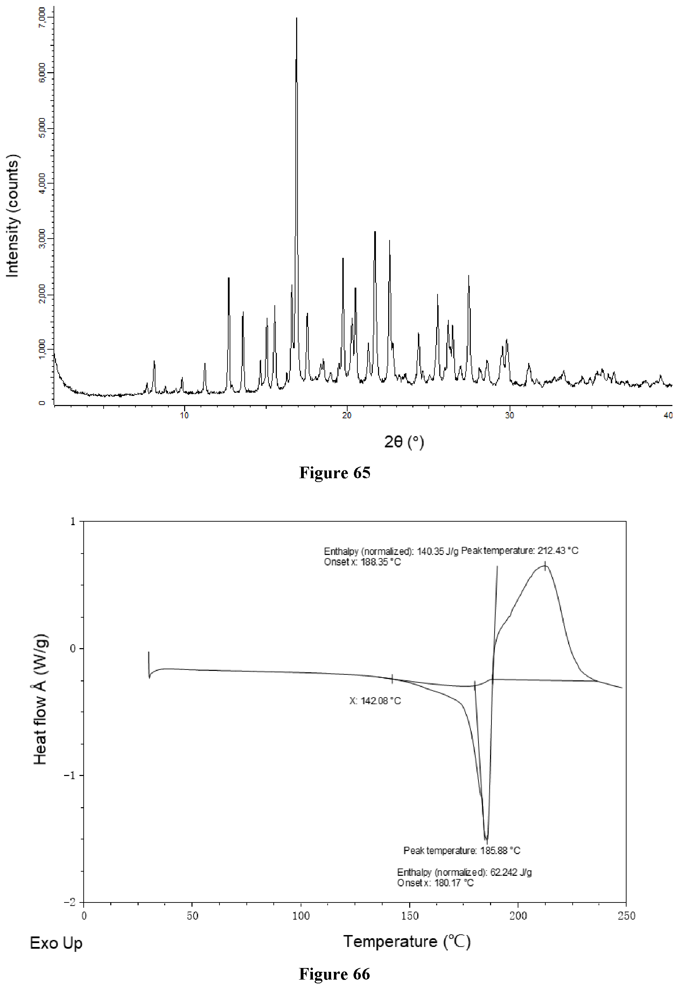

In some embodiments of the present invention, the hydrochloride crystal form L of the compound of Formula I-1 is shown in Figure 65.

**Table 29 XRPD analysis data for the hydrochloride crystal form L of the compound of Formula I-1**

| Name | Angle | d Value | Net Intensity | Gross Intensity | Rel. Intensity | FWHM |
|---|---|---|---|---|---|---|
| Peak #1 | 7.699858 | 11.47246 | 131.7752 | 336.0659 | 0.02713539 | 0.100 |
| Peak # 2 | 8.145549 | 10.84571 | 388.0066 | 598.1743 | 0.07989903 | 0.100 |
| Peak # 3 | 8.823183 | 10.0142 | 89.33794 | 302.069 | 0.01839663 | 0.100 |
| Peak # 4 | 9.853885 | 8.968925 | 197.7117 | 412.7841 | 0.04071316 | 0.100 |
| Peak # 5 | 11.24898 | 7.859537 | 369.8264 | 581.4573 | 0.07615533 | 0.100 |
| Peak # 6 | 12.71896 | 6.954291 | 1487.639 | 1717.048 | 0.3063374 | 0.100 |
| Peak # 7 | 13.58705 | 6.511866 | 1030.956 | 1271.22 | 0.2122963 | 0.100 |
| Peak # 8 | 14.6791 | 6.029779 | 391.6135 | 655.8375 | 0.08064177 | 0.100 |
| Peak # 9 | 15.07415 | 5.872634 | 902.8954 | 1182.24 | 0.1859259 | 0.100 |
| Peak # 10 | 15.55735 | 5.691307 | 1063.293 | 1357.591 | 0.2189553 | 0.100 |
| Peak # 11 | 16.60393 | 5.334851 | 1276.309 | 1618.283 | 0.2628199 | 0.175 |
| Peak # 12 | 16.89181 | 5.244576 | 4856.211 | 5212.304 | 1 | 0.100 |
| Peak # 13 | 17.55518 | 5.047862 | 946.9635 | 1330.313 | 0.1950005 | 0.100 |
| Peak # 14 | 18.52686 | 4.785248 | 321.5662 | 731.5641 | 0.0662175 | 0.147 |
| Peak # 15 | 18.98341 | 4.671179 | 102.7261 | 519.8016 | 0.02115355 | 0.100 |
| Peak # 16 | 19.74166 | 4.493443 | 1663.331 | 2084.444 | 0.3425161 | 0.100 |
| Peak # 17 | 20.29493 | 4.372176 | 825.2653 | 1243.265 | 0.1699401 | 0.169 |
| Peak # 18 | 20.5165 | 4.325457 | 1295.536 | 1710.853 | 0.2667791 | 0.134 |
| Peak # 19 | 21.29701 | 4.168664 | 519.0574 | 937.0649 | 0.1068853 | 0.100 |
| Peak # 20 | 21.68984 | 4.094043 | 2100.771 | 2521.513 | 0.4325947 | 0.100 |
| Peak # 21 | 22.59681 | 3.93173 | 1945.966 | 2363.155 | 0.400717 | 0.100 |
| Peak # 22 | 22.79003 | 3.898831 | 552.5369 | 967.1934 | 0.1137794 | 0.100 |
| Peak # 23 | 24.38056 | 3.647962 | 656.4279 | 1057.281 | 0.1351728 | 0.100 |
| Peak # 24 | 25.56504 | 3.481566 | 1270.748 | 1697.177 | 0.2616749 | 0.100 |
| Peak # 25 | 26.20858 | 3.397519 | 857.4071 | 1291.179 | 0.1765589 | 0.189 |
| Peak # 26 | 26.4961 | 3.3613 | 814.4917 | 1249.304 | 0.1677216 | 0.174 |
| Peak # 27 | 26.9705 | 3.303243 | 232.8028 | 666.3146 | 0.04793919 | 0.100 |
| Peak # 28 | 27.47811 | 3.243364 | 1526.117 | 1954.071 | 0.3142608 | 0.100 |
| Peak # 29 | 28.17182 | 3.165055 | 141.1872 | 554.5795 | 0.02907353 | 0.109 |
| Peak # 30 | 28.59997 | 3.118639 | 289.7929 | 690.1832 | 0.0596747 | 0.188 |
| Peak # 31 | 29.54222 | 3.02128 | 500.3598 | 880.1282 | 0.103035 | 0.203 |
| Peak # 32 | 29.80236 | 2.995497 | 633.8278 | 1010.739 | 0.130519 | 0.198 |
| Peak # 33 | 31.17271 | 2.866868 | 252.4929 | 605.6007 | 0.0519938 | 0.160 |
| Peak # 34 | 33.27863 | 2.690104 | 190.787 | 534.211 | 0.03928722 | 0.185 |

In some embodiments of the present invention, the hydrochloride crystal form L of the compound of Formula I-1 has the onsets of the endothermic peaks at 180.17°C ± 2^{°}C in its differential scanning calorimetry (DSC) curve, and decomposition of the sample begins at 212.43^{°}C ± 2^{°}C.

In some embodiments of the present invention, the DSC pattern of the hydrochloride crystal form L of the compound of Formula I-1 is shown in Figure 66.

In some embodiments of the present invention, the hydrochloride crystal form L of the compound of Formula I-1 has two weight loss steps at 33.53°C ± 2^{°}C and 140.00^{°}C ± 2^{°}C in its thermogravimetric analysis (TGA) curve.

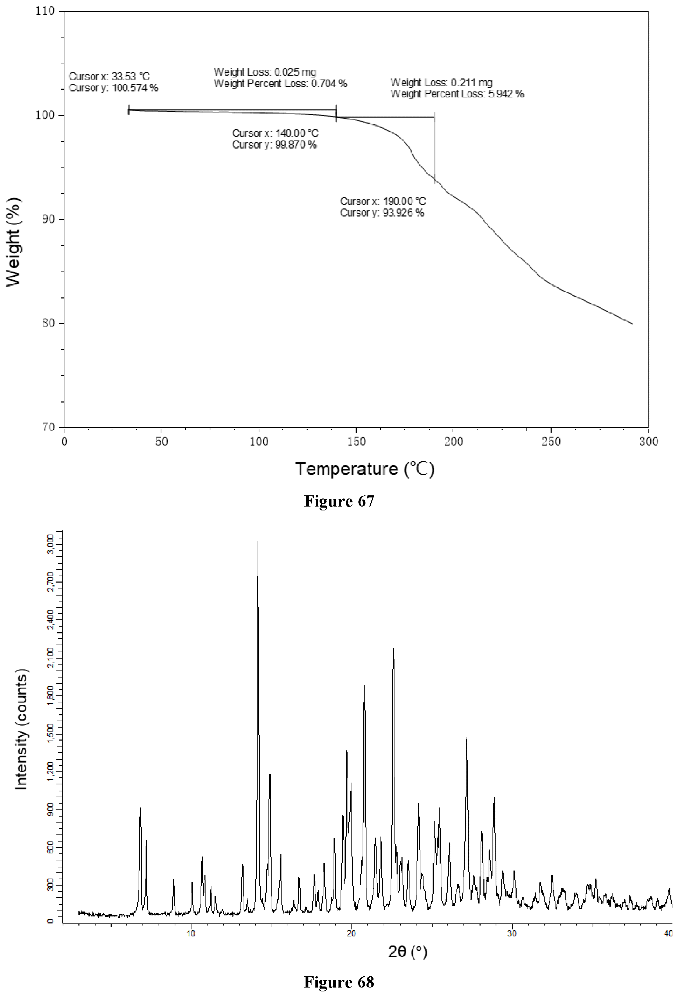

In some embodiments of the present invention, the TGA pattern of the hydrochloride crystal form L of the compound of Formula I-1 is shown in Figure 67.

The present invention also provides a method for preparing the hydrochloride crystal form L of the compound of Formula I-1, comprising adding the compound of Formula I-1 and hydrochloric acid to toluene, and then performing suspension recrystallization at 25^{°}C, 50^{°}C, or under heating-cooling cycles or slurrying to obtain it, wherein the ratio of the compound of Formula I-1 to hydrochloric acid is selected from 0.8 to 1.2, and the hydrochloride crystal form L of the compound of Formula I-1 is a solvate of toluene, with a toluene content of 0.46 equivalents.

The present invention also provides the hydrochloride crystal form M of the compound of Formula I-1, and its X-ray powder diffraction pattern has characteristic diffraction peaks at the following 2θ angles: 6.84 ± 0.2^{°}, 7.21 ± 0.2^{°}, 14.17 ± 0.2^{°}, 14.90 ± 0.2^{°}, 18.94 ± 0.2^{°}, 19.46 ± 0.2^{°}, 19.71 ± 0.2^{°}, 19.96 ± 0.2^{°}, 20.82 ± 0.2^{°}, 22.60±0.2^{°}, 24.17±0.2°, 25.17±0.2^{°}, 25.45±0.2°, 27.17±0.2°, 28.09±0.2°, 28.88±0.2°.

In some embodiments of the present invention, the X-ray powder diffraction pattern of the hydrochloride crystal form M of the compound of formula I-1 has characteristic diffraction peaks at the following 2θ angles: 6.84±0.2°, 7.21±0.2°, 8.92±0.2°, 10.07±0.2°, 10.69±0.2°, 10.89±0.2°, 11.24±0.2°, 13.23±0.2°, 14.17±0.2°, 14.90±0.2°, 15.59±0.2°, 16.74±0.2°, 17.67±0.2°, 18.29±0.2°, 18.94±0.2°, 19.46±0.2°, 19.71±0.2°, 19.96±0.2°, 20.82±0.2° , 21.49±0.2°, 21.83±0.2°, 22.60±0.2°, 23.14±0.2°, 23.54±0.2°, 24.17±0.2°, 25.17±0.2°, 25.45±0.2°, 26.10±0.2°, 27.17±0.2°, 27.61±0.2°, 28.09±0.2°, 28.88±0.2°, 29.42±0.2°, 30.12±0.2°, 32.48±0.2°, 35.22±0.2°.

In some embodiments of the present invention, the X-ray powder diffraction (XRPD) pattern of the hydrochloride crystal form M of the compound of Formula I-1 is shown in Figure 68.

**Table 30 XRPD analysis data for the hydrochloride crystal form M of the compound of Formula I-1**

| Name | Angle | d Value | Net Intensity | Gross Intensity | Rel. Intensity | FWHM |
|---|---|---|---|---|---|---|
| Peak #1 | 6.84472 | 12.90373 | 549.0949 | 615.6407 | 0.2778722 | 0.165 |
| Peak # 2 | 7.211685 | 12.24792 | 391.2005 | 460.1923 | 0.1979689 | 0.100 |
| Peak # 3 | 8.922095 | 9.903405 | 211.9704 | 281.5079 | 0.1072687 | 0.100 |
| Peak # 4 | 10.07171 | 8.775435 | 181.1558 | 251.1664 | 0.0916748 | 0.100 |
| Peak # 5 | 10.69185 | 8.267802 | 310.0935 | 381.4751 | 0.1569243 | 0.101 |
| Peak # 6 | 10.88628 | 8.120569 | 260.1903 | 332.5287 | 0.1316706 | 0.101 |
| Peak # 7 | 11.24487 | 7.862399 | 152.6891 | 225.697 | 0.07726907 | 0.133 |
| Peak # 8 | 13.22875 | 6.687422 | 265.7502 | 344.6351 | 0.1344842 | 0.100 |
| Peak # 9 | 14.16762 | 6.246297 | July 1976 | 2066.746 | 1 | 0.100 |
| Peak # 10 | 14.90028 | 5.940766 | 745.1891 | 838.3379 | 0.3771066 | 0.100 |
| Peak # 11 | 15.58676 | 5.680631 | 351.6713 | 441.7771 | 0.177965 | 0.100 |
| Peak # 12 | 16.74027 | 5.291707 | 195.157 | 284.6572 | 0.09876018 | 0.100 |
| Peak # 13 | 17.66522 | 5.016665 | 220.666 | 313.7185 | 0.1116691 | 0.126 |
| Peak # 14 | 18.29257 | 4.846004 | 266.1616 | 359.0701 | 0.1346924 | 0.100 |
| Peak # 15 | 18.94302 | 4.681048 | 442.7134 | 543.266 | 0.2240373 | 0.100 |
| Peak # 16 | 19.46487 | 4.556712 | 520.9281 | 631.4868 | 0.2636183 | 0.100 |
| Peak # 17 | 19.70504 | 4.501712 | 909.2918 | 1023.447 | 0.4601516 | 0.217 |
| Peak # 18 | 19.95502 | 4.445879 | 693.7634 | 810.9916 | 0.3510824 | 0.295 |
| Peak # 19 | 20.81669 | 4.263753 | 1294.718 | 1417.268 | 0.6551984 | 0.100 |
| Peak # 20 | 21.48724 | 4.132186 | 414.7583 | 540.3758 | 0.2098905 | 0.179 |
| Peak # 21 | 21.82966 | 4.068136 | 385.2101 | 512.3789 | 0.1949375 | 0.100 |
| Peak # 22 | 22.59636 | 3.931808 | 1560.034 | 1689.713 | 0.789463 | 0.100 |
| Peak # 23 | 23.14369 | 3.840045 | 310.0418 | 438.7188 | 0.1568982 | 0.160 |
| Peak # 24 | 23.5415 | 3.776049 | 285.9931 | 411.8756 | 0.1447282 | 0.100 |
| Peak # 25 | 24.17295 | 3.678821 | 614.5384 | 737.225 | 0.3109902 | 0.100 |
| Peak # 26 | 25.16647 | 3.535795 | 517.7031 | 646.9673 | 0.2619862 | 0.174 |
| Peak # 27 | 25.4543 | 3.496462 | 643.7325 | 778.7833 | 0.325764 | 0.188 |
| Peak # 28 | 26.09795 | 3.411669 | 351.5211 | 496.2128 | 0.177889 | 0.100 |
| Peak #29 | 27.16983 | 3.279461 | 944.2617 | 1104.001 | 0.4778483 | 0.101 |
| Peak # 30 | 27.61049 | 3.228113 | 145.7133 | 309.4552 | 0.07373893 | 0.135 |
| Peak # 31 | 28.09237 | 3.173826 | 448.1052 | 613.7818 | 0.2267659 | 0.100 |
| Peak # 32 | 28.87765 | 3.089279 | 616.2469 | 779.606 | 0.3118548 | 0.100 |
| Peak # 33 | 29.41578 | 3.033977 | 180.7685 | 338.6228 | 0.09147878 | 0.107 |
| Peak # 34 | 30.12402 | 2.964239 | 214.1834 | 359.9441 | 0.1083886 | 0.105 |
| Peak # 35 | 32.4794 | 2.754451 | 212.3529 | 339.9138 | 0.1074622 | 0.100 |
| Peak # 36 | 35.21609 | 2.546415 | 164.973 | 299.9358 | 0.0834854 | 0.157 |

In some embodiments of the present invention, the hydrochloride crystal form M of the compound of Formula I-1 has the onsets of the endothermic peaks at 31.74°C ± 2°C and 150.05°C ± 2°C in its differential scanning calorimetry (DSC) curve, and decomposition of the sample begins at 215.09°C ± 2°C.

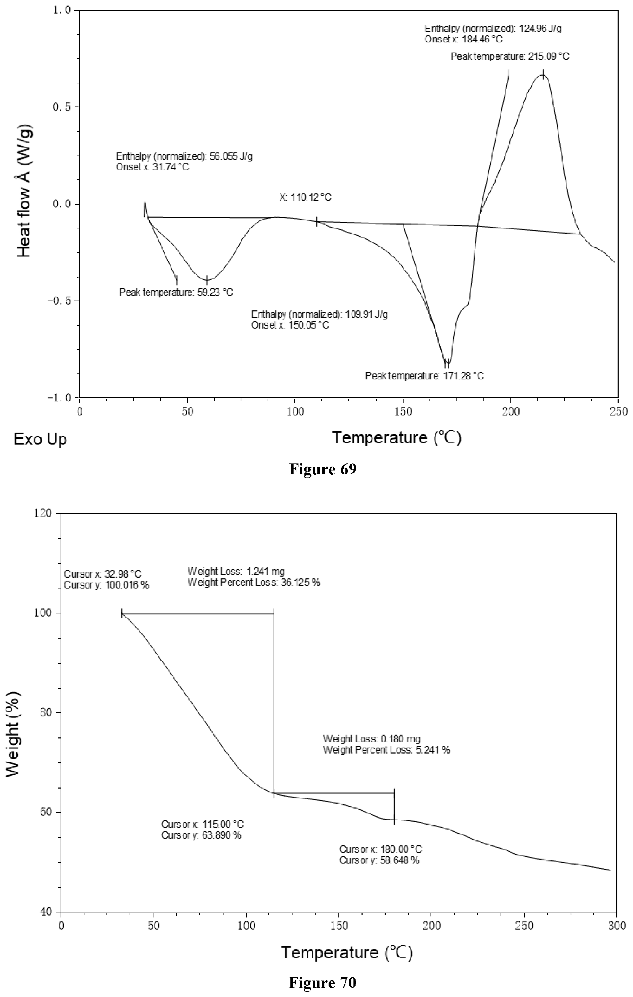

In some embodiments of the present invention, the DSC pattern of the hydrochloride crystal form M of the compound of Formula I-1 is shown in Figure 69.

In some embodiments of the present invention, the hydrochloride crystal form M of the compound of Formula I-1 has two weight loss steps at 32.98°C ± 2^{°}C and 115.00°C ± 2^{°}C in its thermogravimetric analysis (TGA) curve.

In some embodiments of the present invention, the TGA pattern of the hydrochloride crystal form M of the compound of Formula I-1 is shown in Figure 70.

The present invention also provides a method for preparing the hydrochloride crystal form M of the compound of Formula I-1, comprising adding the compound of Formula I-1 and hydrochloric acid to isopropanol/dimethyl sulfoxide, isopropyl acetate/dimethyl sulfoxide, or a mixed solvent of water/dimethyl sulfoxide, and then performing suspension recrystallization at 25^{°}C, 50^{°}C, or under heating-cooling cycles or slurrying to obtain it, the ratio of the compound of Formula I-1 to hydrochloric acid is selected from 0.8 to 1.2, the hydrochloride crystal form M of the compound of Formula I-1 is a solvate of dimethyl sulfoxide and water, with a dimethyl sulfoxide content of 3.4 equivalents and a water content of 13.7 equivalents.

The present invention also provides the hydrochloride crystal form N of the compound of Formula I-1, and its X-ray powder diffraction pattern has characteristic diffraction peaks at the following 2θ angles: 6.61 ± 0.2^{°}, 7.80 ± 0.2^{°}, 13.17 ± 0.2^{°}, 15.24 ± 0.2^{°}, 16.02 ± 0.2^{°}, 16.19 ± 0.2^{°}, 20.10 ± 0.2^{°}, 21.57 ± 0.2^{°}, 21.95 ± 0.2^{°}, 28.42 ± 0.2^{°}.

In some embodiments of the present invention, the X-ray powder diffraction pattern of the hydrochloride crystal form N of the compound of Formula I-1 has characteristic diffraction peaks at the following 2θ angles: 6.61±0.2°, 7.80±0.2°, 9.75±0.2°, 11.19±0.2°, 12.53 ± 0.2°, 13.17 ± 0.2°, 14.61 ± 0.2°, 15.24 ± 0.2°, 16.02 ± 0.2°, 16.19 ± 0.2°, 18.51±0.2°, 19.36±0.2°, 20.10±0.2°, 21.57±0.2°, 21.95±0.2°, 23.47±0.2°, 25.60±0.2°, 27.32±0.2°, 28.42±0.2°, 29.12±0.2°.

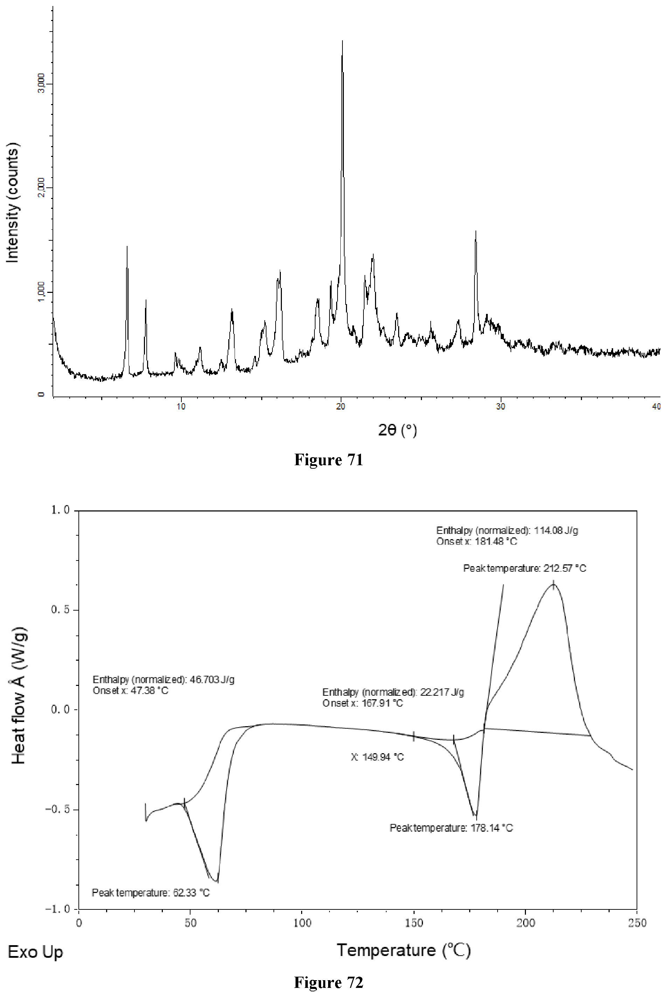

In some embodiments of the present invention, the XRPD pattern of the hydrochloride crystal form N of the compound of Formula I-1 is shown in Figure 71.

**Table 31 XRPD analysis data for the hydrochloride crystal form N of the compound of Formula I-1**

| Name | Angle | d Value | Net Intensity | Gross Intensity | Rel. Intensity | FWHM |
|---|---|---|---|---|---|---|
| Peak #1 | 6.609798 | 13.36181 | 794.7955 | 980.8873 | 0.3862052 | 0.100 |
| Peak # 2 | 7.795767 | 11.33153 | 487.4999 | 690.8655 | 0.2368848 | 0.100 |
| Peak # 3 | 9.745835 | 9.068117 | 34.00436 | 256.8145 | 0.01652332 | 0.133 |
| Peak # 4 | 11.18676 | 7.903115 | 155.3221 | 382.2601 | 0.07547374 | 0.141 |
| Peak # 5 | 12.52697 | 7.060439 | 86.51295 | 323.2003 | 0.04203818 | 0.159 |
| Peak # 6 | 13.16559 | 6.719363 | 421.9255 | 664.773 | 0.2050211 | 0.190 |
| Peak # 7 | 14.60967 | 6.058279 | 86.97061 | 345.4379 | 0.04226056 | 0.133 |
| Peak # 8 | 15.23705 | 5.810213 | 304.1442 | 589.247 | 0.147789 | 0.271 |
| Peak # 9 | 16.0249 | 5.52629 | 820.8987 | 1132.93 | 0.3988892 | 0.225 |
| Peak # 10 | 16.18629 | 5.471551 | 893.4421 | 1210.095 | 0.4341393 | 0.225 |
| Peak # 11 | 18.5144 | 4.788438 | 351.1131 | 770.6374 | 0.1706121 | 0.174 |
| Peak # 12 | 19.35669 | 4.581933 | 455.0124 | 908.4713 | 0.2210986 | 0.105 |
| Peak # 13 | 20.09929 | 4.414289 | 2057.962 | 2534.464 | 1 | 0.100 |
| Peak # 14 | 21.57023 | 4.116475 | 320.2355 | 823.3632 | 0.1556081 | 0.100 |
| Peak # 15 | 21.95477 | 4.045236 | 557.7156 | 1063.636 | 0.2710039 | 0.291 |
| Peak # 16 | 23.47203 | 3.787067 | 201.8493 | 701.9348 | 0.09808215 | 0.109 |
| Peak #17 | 25.59957 | 3.476949 | 170.8813 | 642.3811 | 0.08303423 | 0.126 |
| Peak # 18 | 27.31775 | 3.262038 | 172.0372 | 641.0505 | 0.08359591 | 0.164 |
| Peak # 19 | 28.42086 | 3.137885 | 858.4133 | 1341.339 | 0.4171182 | 0.100 |
| Peak # 20 | 29.12304 | 3.063805 | 213.3153 | 697.6971 | 0.1036537 | 0.238 |

In some embodiments of the present invention, the hydrochloride crystal form N of the compound of Formula I-1 has the onsets of the endothermic peaks at 47.38°C ± 2^{°}C and 167.91^{°}C ± 2^{°}C in its differential scanning calorimetry (DSC) curve, and decomposition of the sample begins at 212.57°C ± 2^{°}C.

In some embodiments of the present invention, the DSC pattern of the hydrochloride crystal form N of the compound of Formula I-1 is shown in Figure 72.

In some embodiments of the present invention, the hydrochloride crystal form N of the compound of Formula I-1 has three weight loss steps at 33.22°C ± 2^{°}C, 75.00°C ± 2^{°}C, and 150.00^{°}C ± 2^{°}C in its thermogravimetric analysis (TGA) curve.

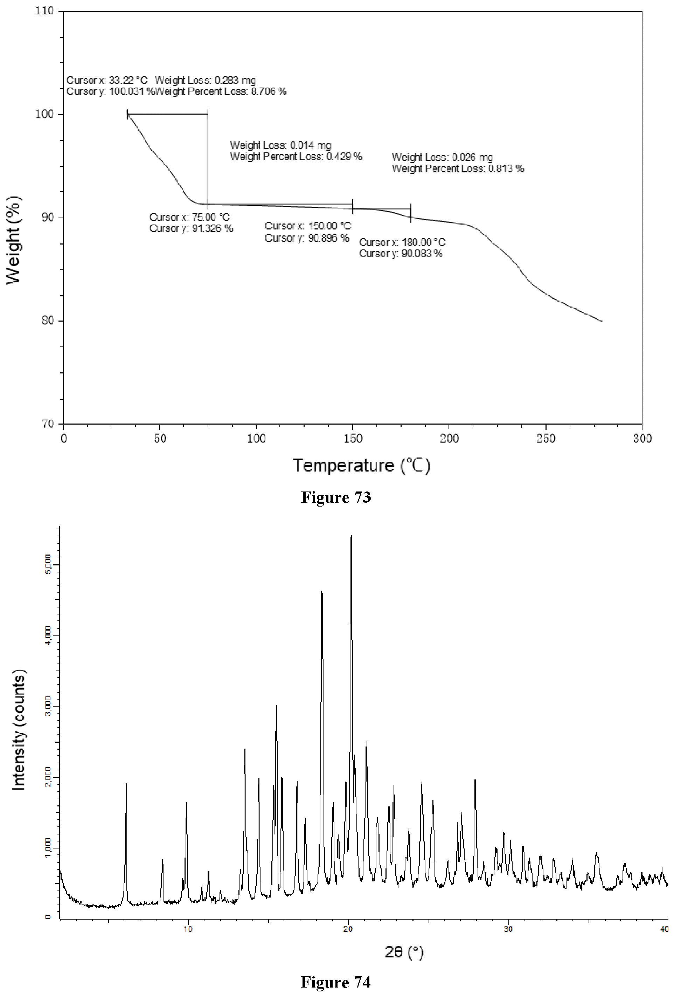

In some embodiments of the present invention, the TGA pattern of the hydrochloride crystal form N of the compound of Formula I-1 is shown in Figure 73.

The present invention also provides a method for preparing the hydrochloride crystal form N of the compound of Formula I-1, comprising adding the compound of Formula I-1 and hydrochloric acid to methanol, and then performing suspension recrystallization at 25^{°}C or slurrying to obtain it, the ratio of the compound of Formula I-1 to hydrochloric acid is selected from 0.8 to 1.2, and the hydrochloride crystal form N of the compound of Formula I-1 is a hydrate crystal form, with a water content of 3.0 equivalents.

The present invention also provides the hydrochloride crystal form O of the compound of Formula I-1, and its X-ray powder diffraction pattern has characteristic diffraction peaks at the following 2θ angles: 6.14±0.2°, 9.90±0.2°, 13.54 ± 0.2°, 14.40 ± 0.2°, 15.33 ± 0.2°, 15.51 ± 0.2°, 15.87 ± 0.2°, 16.79 ± 0.2°, 18.36±0.2°, 20.17±0.2°, 21.14±0.2°, 22.51±0.2°, 22.84±0.2°, 24.59±0.2°, 25.28±0.2°, 26.82±0.2°, 27.07±0.2°, 27.93±0.2°.

In some embodiments of the present invention, the X-ray powder diffraction pattern of the hydrochloride crystal form O of the compound of Formula I-1 has characteristic diffraction peaks at the following 2θ angles: 6.14±0.2°, 8.39±0.2°, 9.90±0.2°, 11.28±0.2°, 13.19 ± 0.2°, 13.54 ± 0.2°, 14.40 ± 0.2°, 15.33 ± 0.2°, 15.51 ± 0.2°, 15.87 ± 0.2°, 16.79 ± 0.2°, 17.32±0.2°, 18.36±0.2°, 19.03±0.2°, 19.38±0.2°, 19.84±0.2°, 20.17±0.2°, 20.39±0.2°, 21.14±0.2°, 21.82±0.2°, 22.51±0.2°, 22.84±0.2°, 23.77±0.2°, 24.59±0.2°, 25.28±0.2°, 26.21±0.2°, 26.82±0.2°, 27.07±0.2°, 27.93±0.2°, 28.45±0.2°, 29.24±0.2°, 29.70±0.2°, 30.12±0.2°, 30.91±0.2°, 31.32±0.2°, 32.02±0.2°, 32.81±0.2°, 33.99±0.2°, 35.51±0.2°, 37.27±0.2°.

In some embodiments of the present invention, the XRPD pattern of the hydrochloride crystal form O of the compound of Formula I-1 is shown in Figure 74.

**Table 32 XRPD analysis data for the hydrochloride crystal form O of the compound of Formula I-1**

| Name | Angle | d Value | Net Intensity | Intensity Gross | Rel. Intensity | FWHM |
|---|---|---|---|---|---|---|
| Peak #1 | 6.140894 | 14.38099 | 1107.957 | 1293.828 | 0.3147685 | 0.100 |
| Peak # 2 | 8.388501 | 10.53213 | 408.1169 | 632.1091 | 0.1159452 | 0.100 |
| Peak # 3 | 9.903625 | 8.923991 | 940.8444 | 1184.968 | 0.2672921 | 0.100 |
| Peak # 4 | 11.27951 | 7.838338 | 283.761 | 536.4181 | 0.08061594 | 0.100 |
| Peak # 5 | 13.19286 | 6.705534 | 181.3849 | 438.5068 | 0.0515311 | 0.100 |
| Peak # 6 | 13.54282 | 6.533034 | 1454.357 | 1725.367 | 0.4131799 | 0.100 |
| Peak # 7 | 14.39867 | 6.146578 | 1165.407 | 1462.794 | 0.3310898 | 0.100 |
| Peak # 8 | 15.33 | 5.775193 | 1091.177 | 1411.573 | 0.3100013 | 0.209 |
| Peak # 9 | 15.50935 | 5.708809 | 1968.812 | 2294.23 | 0.5593358 | 0.209 |
| Peak # 10 | 15.87208 | 5.579152 | 1217.473 | 1551.602 | 0.3458818 | 0.100 |
| Peak # 11 | 16.79091 | 5.275862 | 1137.58 | 1502.341 | 0.3231842 | 0.100 |
| Peak # 12 | 17.31762 | 5.116568 | 762.1778 | 1150.802 | 0.2165332 | 0.100 |
| Peak # 13 | 18.35663 | 4.829239 | 3,033.994 | 3474.369 | 0.8619518 | 0.100 |
| Peak # 14 | 19.03416 | 4.658839 | 835.817 | 1306.477 | 0.237454 | 0.169 |
| Peak # 15 | 19.38 | 4.576475 | 502.326 | 985.8411 | 0.1427098 | 0.108 |
| Peak # 16 | 19.84427 | 4.470439 | 1056.817 | 1554.823 | 0.3002395 | 0.100 |
| Peak # 17 | 20.17144 | 4.398664 | 3519.912 | 4026.221 | 1 | 0.100 |
| Peak # 18 | 20.38873 | 4.352273 | 1279.111 | 1790.063 | 0.363393 | 0.100 |
| Peak # 19 | 21.14244 | 4.198789 | 1425.925 | 1947.604 | 0.4051024 | 0.105 |
| Peak # 20 | 21.8204 | 4.069841 | 566.3649 | 1090.542 | 0.1609031 | 0.125 |
| Peak # 21 | 22.50812 | 3.947022 | 756.1846 | 1275.989 | 0.2148305 | 0.191 |
| Peak # 22 | 22.83675 | 3.89096 | 979.7973 | 1495.056 | 0.2783585 | 0.178 |
| Peak # 23 | 23.77264 | 3.739855 | 546.3175 | 1050.328 | 0.1552077 | 0.105 |
| Peak # 24 | 24.58534 | 3.618039 | 980.3226 | 1470.129 | 0.2785077 | 0.122 |
| Peak # 25 | 25.27836 | 3.520397 | 896.8149 | 1367.909 | 0.2547834 | 0.136 |
| Peak # 26 | 26.21456 | 3.396758 | 248.3358 | 716.5672 | 0.0705517 | 0.100 |
| Peak # 27 | 26.82125 | 3.321285 | 737.2324 | 1215.387 | 0.2094463 | 0.100 |
| Peak # 28 | 27.06781 | 3.291589 | 750.5667 | 1231.206 | 0.2132345 | 0.191 |
| Peak # 29 | 27.92502 | 3.192464 | 1087.508 | 1569.829 | 0.3089589 | 0.100 |
| Peak # 30 | 28.45316 | 3.134396 | 279.407 | 757.3731 | 0.07937898 | 0.101 |
| Peak # 31 | 29.24147 | 3.051666 | 309.071 | 791.8425 | 0.08780645 | 0.161 |
| Peak # 32 | 29.70277 | 3.005313 | 501.2726 | 988.7209 | 0.1424106 | 0.121 |
| Peak # 33 | 30.11823 | 2.964796 | 490.5232 | 979.5004 | 0.1393567 | 0.105 |
| Peak # 34 | 30.91374 | 2.890291 | 465.6483 | 950.4669 | 0.1322898 | 0.100 |
| Peak # 35 | 31.32369 | 2.853393 | 169.9911 | 649.0291 | 0.04829413 | 0.129 |
| Peak # 36 | 32.01775 | 2.793105 | 265.0306 | 734.7343 | 0.07529467 | 0.163 |
| Peak # 37 | 32.81191 | 2.727294 | 268.0586 | 727.3953 | 0.07615492 | 0.130 |
| Peak # 38 | 33.98699 | 2.635639 | 302.6802 | 746.7783 | 0.08599085 | 0.131 |
| Peak # 39 | 35.51149 | 2.525907 | 319.0914 | 750.0336 | 0.09065324 | 0.193 |
| Peak # 40 | 37.27088 | 2.410611 | 289.6581 | 733.1247 | 0.08229129 | 0.248 |

In some embodiments of the present invention, the hydrochloride crystal form O of the compound of Formula I-1 has the onsets of the endothermic peaks starting at 12.36°C ± 2^{°}C and 174.87°C ± 2^{°}C in its differential scanning calorimetry (DSC) curve, with decomposition of the sample begins at 212.00°C ± 2^{°}C.

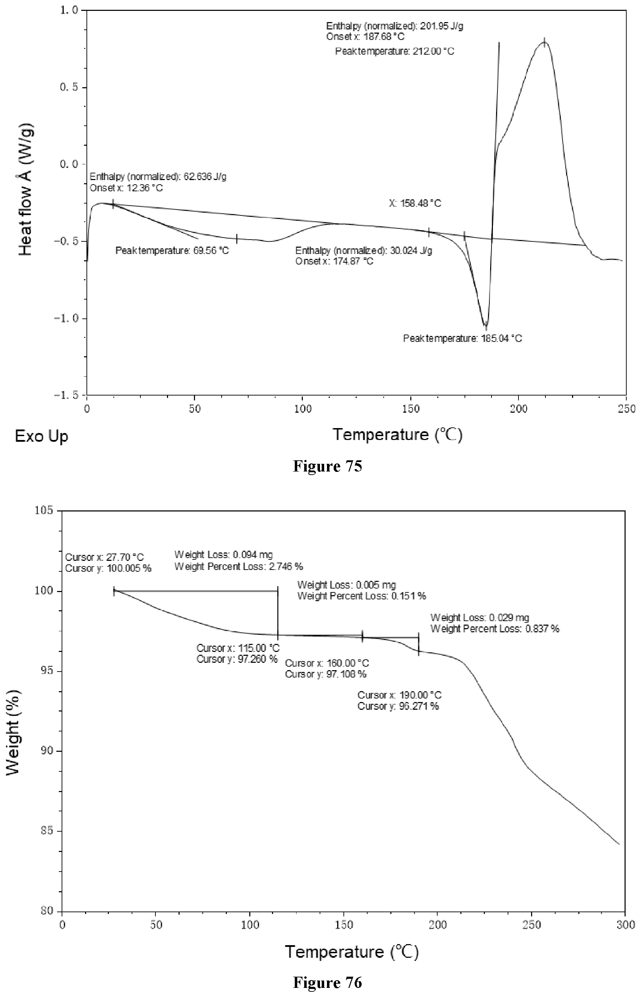

In some embodiments of the present invention, the DSC pattern of the hydrochloride crystal form O of the compound of Formula I-1 is shown in Figure 75.

In some embodiments of the present invention, the hydrochloride crystal form O of the compound of Formula I-1 has three weight loss steps at 27.70°C ± 2^{°}C, 115.00°C ± 2^{°}C, and 160.00^{°}C ± 2^{°}C in its thermogravimetric analysis (TGA) curve.

In some embodiments of the present invention, the TGA pattern of the hydrochloride crystal form O of the compound of Formula I-1 is shown in Figure 76.

The present invention also provides a method for preparing the hydrochloride crystal form O of the compound of Formula I-1, comprising adding the compound of Formula I-1 and hydrochloric acid to dichloromethane, and then performing suspension recrystallization at 25^{°}C or slurrying to obtain it, the ratio of the compound of Formula I-1 to hydrochloric acid is selected from 0.8 to 1.2, and the hydrochloride crystal form O of the compound of Formula I-1 is a hydrate crystal form, with a water content of 0.9 equivalents.

In another aspect, the present invention provides a method for preparing a crystal form of a pharmaceutically acceptable salt of the compound of formula (I), characterized by comprising the following steps:
a. dissolving or dispersing the compound of formula (I) and an acid in a molar ratio of 1: (0.8-3) in 1-20 times of solvent A;
b. performing suspension, recrystallization, or slurrying treatment;

wherein the acid is not selected from hydrochloric acid (preferably, the acid is selected from the group consisting of: maleic acid, fumaric acid, glycolic acid, L-malic acid, succinic acid, sulfuric acid, L-tartaric acid, hippuric acid, glutaric acid, p-toluenesulfonic acid, or methanesulfonic acid);
wherein the solvent A is selected from the group consisting of: acetonitrile, dichloromethane, tetrahydrofuran, or a combination thereof;
or the acid is hydrochloric acid or hydrogen chloride in dioxane; and the solvent A is selected from the group consisting of: ethyl acetate, 2-methyl-tetrahydrofuran, butanone, ethanol, acetone, ethyl acetate, acetonitrile, tetrahydrofuran, methyl isobutyl ketone, trifluoroethanol, water, methanol, tert-butyl methyl ether, toluene, isopropanol, dimethyl sulfoxide, isopropyl acetate, dichloromethane, or a combination thereof.

In another preferred embodiment, the solvent A is selected from the group consisting of: methyl isobutyl ketone/trifluoroethanol, methanol/water, methanol/tert-butyl methyl ether, isopropanol/dimethyl sulfoxide, isopropyl acetate/dimethyl sulfoxide, water/dimethyl sulfoxide.

In another preferred embodiment, the suspension comprises the following steps:
a. suspending the compound of formula (I), acid, and solvent at 45-65°C for 1-3 h;
b. allowing the mixture to cool naturally to 20-30°C and continuing performing the suspension for at least 48 h;
c. centrifuging the resulting suspension through a 0.4-0.5 µm filter membrane at a speed of 12,000-16,000 rpm;
d. vacuum-drying the resulting solid at 45-65°C.

In another aspect, the present invention provides a method for preparing a crystal form of a pharmaceutically acceptable salt of the compound of formula (I), characterized in that the method is a crystal form transformation by converting one crystal form of a pharmaceutically acceptable salt of the compound of formula (I) into another crystal form thereof;
wherein the method of the crystal form transformation comprises the following steps:
   a. dissolving or dispersing a crystal form of a pharmaceutically acceptable salt of the compound of formula (I) in 1 to 20 times of solvent A;
   b. performing suspension treatment;
   c. centrifuging the resulting suspension through a 0.4 to 0.5 µm filter membrane at a speed of 12,000 to 16,000 rpm;
wherein the solvent A is selected from the group consisting of: ethanol, acetone, ethyl acetate, acetonitrile, tetrahydrofuran, methyl isobutyl ketone, trifluoroethanol, methanol, 2-methyl-tetrahydrofuran, methyl tert-butyl ether, toluene, isopropanol, dimethyl sulfoxide, isopropyl acetate, dimethyl sulfoxide, methanol, dichloromethane, water, or combinations thereof.

In another preferred embodiment, the solvent A is selected from the group consisting of: methyl isobutyl ketone/trifluoroethanol, methanol/water, isopropanol/dimethyl sulfoxide, isopropyl acetate/dimethyl sulfoxide, dimethyl sulfoxide/water.

In another preferred embodiment, the suspension treatment comprises the step of stirring at a rate of 300-400 rpm at 20-30^{°}C.

In another preferred embodiment, the suspension treatment comprises the step of stirring at a rate of 300-400 rpm at 40-60^{°}C.

In another preferred embodiment, the suspension treatment comprises the step of performing 8 to 12 heating-cooling cycles at a rate of 0.05 to 0.2^{°}C/min between 5 and 50^{°}C while stirring at a rate of 300 to 400 rpm, with the final temperature of the suspension being 10 to 15^{°}C.

In another preferred embodiment, the suspension treatment comprises the step of filtering through a 0.4-0.5 µm filter membrane to obtain a clear solution, followed by performing suspension treatment after adding methyl tert-butyl ether at a ratio of 1: (8-12).

In another aspect, the present invention also provides a pharmaceutical composition comprising the compound of formula (I), the crystal form A, or a crystal form of the compound of formula (I);
and
one or more pharmaceutically acceptable carriers, excipients, adjuvants, ingredients, and/or diluents.

In another preferred embodiment, the pharmaceutical composition further comprises other therapeutic agents.

In another preferred embodiment, the other therapeutic agents are selected from the group consisting of: chemotherapeutic agents, kinase inhibitors, targeted epigenetic modulators, antibody drugs, immune checkpoint inhibitors, or combinations thereof. In another preferred embodiment, the chemotherapeutic agent is selected from the group consisting of: cisplatin, doxorubicin, paclitaxel, etoposide, irinotecan, cyclophosphamide, gemcitabine, ifosfamide, tamoxifen, toremifene, fulvestrant, anastrozole, exemestane, goserelin, leprorelin, melphalan, chlorambucil, busulfan, floxuridine, cytarabine, oxaliplatin, leucovorin, pentostatin, and diethylstilbestrol.

In another preferred embodiment, the kinase inhibitor is selected from the group consisting of: Akt, TGF-βR, Pim, PKA, PKG, PKC, CaM kinase, CDK2, CDK4, CDK4/6, MEK, ERK, MAPK, mTOR, EGFR, HER2, HER3, HER4, PDGFαR, PDGFβR, CSFIR, KIT, c-Met, TRKA, TRKB, TRKC, FLT3, VEGFR, BTK, FAK, SYK, FRK, JAK, HPK1, AXL, ALK, and B-Raf inhibitors. In another preferred embodiment, the targeted epigenetic modulator is selected from the group consisting of: bromodomain inhibitors, histone lysine methyltransferases, histone arginine methyltransferases, histone demethylases, histone deacetylases, histone acetylases, and DNA methyltransferases. In another preferred embodiment, the antibody drug is selected from the group consisting of: anti-HER2 antibodies, anti-VEGFR antibodies, anti-EGFR antibodies, anti-c-MET antibodies, and anti-CD20 antibodies.

In another preferred embodiment, the immune checkpoint inhibitor is selected from the group consisting of: CD27, CD28, CD40, CD122, CD96, CD73, CD47, OX40, GITR, A2AR, B7-H3, B7-H4, BTLA, CTLA-4, LAG3, TIM3, VISTA, PD-1, PD-L1, and PD-L2.

In another aspect, the present invention also provides a use of the compound of the formula (I), the crystal form A, the crystal form of the compound of formula (I), and the pharmaceutical composition in preparing a medicament for the prevention and/or treatment of diseases associated with increased activity or expression of FGFR2.

In another preferred embodiment, the increased activity or expression of FGFR2 is selected from the group consisting of: FGFR2 amplification, FGFR2 gene mutation, FGFR2 gene fusion/rearrangement, FGFR2 gene translocation, and FGFR2 gene activation.

In another preferred embodiment, the diseases associated with increased activity or expression of FGFR2 are selected from the group consisting of: cholangiocarcinoma, liver cancer, breast cancer, prostate cancer, lung cancer, thyroid cancer, gastric cancer, ovarian cancer, esophageal cancer, pancreatic cancer, cervical cancer, colorectal cancer, salivary gland cancer, endometrial cancer, and urothelial cancer, and the like.

In another preferred embodiment, the cholangiocarcinoma is intrahepatic cholangiocarcinoma.

In another preferred embodiment, the liver cancer is hepatocellular carcinoma.

In another preferred embodiment, the lung cancer is lung squamous cell carcinoma or non-small cell lung cancer.

### Description of the Drawings

Figure 1 shows the XRPD pattern of crystal form A of the compound of Formula I-1.
Figure 2 shows the DSC pattern of crystal form A of the compound of Formula I-1.
Figure 3 shows the TGA pattern of crystal form A of the compound of Formula I-1.
Figure 4 shows the XRPD pattern of maleate crystal form A of the compound of Formula I-1.
Figure 5 shows the XRPD pattern of monomaleate crystal form B of the compound of Formula I-1.
Figure 6 shows the DSC pattern of monomaleate crystal form B of the compound of Formula I-1.
Figure 7 shows the TGA spectrum of monomaleate crystal form B of the compound of Formula I-1.
Figure 8 shows the XRPD pattern of fumarate crystal form A of the compound of Formula I-1.
Figure 9 shows the XRPD pattern of hemifumarate crystal form B of the compound of Formula I-1.
Figure 10 shows the DSC pattern of hemifumarate crystal form B of the compound of Formula I-1.
Figure 11 shows the TGA pattern of hemifumarate crystal form B of the compound of Formula I-1.
Figure 12 shows the XRPD pattern of monoglycolate crystal form A of the compound of Formula I-1.
Figure 13 shows the DSC pattern of monoglycolate crystal form A of the compound of Formula I-1.
Figure 14 shows the TGA pattern of monoglycolate, crystal form A of the compound of Formula I-1.
Figure 15 shows the XRPD pattern of glycolate crystal form B of the compound of Formula I-1, .
Figure 16 shows the XRPD pattern of the mono-L-malate crystal form A of the compound of Formula I-1.
Figure 17 shows the DSC pattern of mono-L-malate crystal form A of the compound of Formula I-1.
Figure 18 shows the TGA spectrum of mono-L-malate crystal form A of the compound of Formula I-1.
Figure 19 shows the XRPD pattern of monosuccinate crystal form A of the compound of Formula I-1.
Figure 20 shows the DSC pattern of monosuccinate crystal form A of the compound of Formula I-1.
Figure 21 shows the TGA pattern of monosuccinate crystal form A of the compound of Formula I-1.
Figure 22 shows the XRPD pattern of sulfate crystal form A of the compound of Formula I-1.
Figure 23 shows the XRPD pattern of L-tartrate crystal form A of the compound of Formula I-1.
Figure 24 shows the XRPD pattern of hippurate crystal form A of the compound of Formula I-1.
Figure 25 shows the XRPD pattern of glutarate crystal form A of the compound of Formula I-1.
Figure 26 shows the XRPD pattern of p-toluenesulfonate crystal form A of the compound of Formula I-1.
Figure 27 shows the XRPD pattern of p-toluenesulfonate crystal form B of the compound of Formula I-1.
Figure 28 shows the XRPD pattern of methanesulfonate crystal form A of the compound of Formula I-1.
Figure 29 shows the XRPD pattern of hydrochloride crystal form C1 of the compound of Formula I-1.
Figure 30 shows the DSC pattern of hydrochloride crystal form C1 of the compound of Formula I-1.
Figure 31 shows the TGA pattern of hydrochloride crystal form C1 of the compound of Formula I-1.
Figure 32 shows the XRPD pattern of hydrochloride crystal form C2 of the compound of Formula I-1.
Figure 33 shows the DSC pattern of hydrochloride crystal form C2 of the compound of Formula I-1.
Figure 34 shows the TGA pattern of hydrochloride crystal form C2 of the compound of Formula I-1.
Figure 35 shows the XRPD pattern of hydrochloride crystal form D of the compound of Formula I-1.
Figure 36 shows the DSC pattern of hydrochloride crystal form D of the compound of Formula I-1.
Figure 37 shows the TGA pattern of hydrochloride crystal form D of the compound of Formula I-1.
Figure 38 shows the XRPD pattern of hydrochloride crystal form E1 of the compound of Formula I-1.
Figure 39 shows the DSC pattern of hydrochloride crystal form E1 of the compound of Formula I-1.
Figure 40 shows the TGA pattern of hydrochloride crystal form E1 of the compound of Formula I-1.
Figure 41 shows the XRPD pattern of hydrochloride crystal form E2 of the compound of Formula I-1.
Figure 42 shows the DSC pattern of hydrochloride crystal form E2 of the compound of Formula I-1.
Figure 43 shows the TGA pattern of hydrochloride crystal form E2 of the compound of Formula I-1.
Figure 44 shows the XRPD pattern of hydrochloride crystal form F of the compound of Formula I-1.
Figure 45 shows the DSC pattern of hydrochloride crystal form F of the compound of Formula I-1.
Figure 46 shows the TGA pattern of hydrochloride crystal form F of the compound of Formula I-1.
Figure 47 shows the XRPD pattern of hydrochloride, crystal form G of the compound of Formula I-1.
Figure 48 shows the DSC pattern of hydrochloride crystal form G of the compound of Formula I-1.
Figure 49 shows the TGA pattern of hydrochloride crystal form G of the compound of Formula I-1.
Figure 50 shows the XRPD pattern of hydrochloride crystal form H of the compound of Formula I-1.
Figure 51 shows the DSC pattern of hydrochloride crystal form H of the compound of Formula I-1.
Figure 52 shows the TGA pattern of hydrochloride crystal form H of the compound of Formula I-1.
Figure 53 shows the XRPD pattern of hydrochloride crystal form I of the compound of Formula I-1.
Figure 54 shows the DSC pattern of hydrochloride crystal form I of the compound of Formula I-1.
Figure 55 shows the TGA pattern of hydrochloride crystal form I of the compound of Formula I-1.
Figure 56 shows the XRPD pattern of hydrochloride crystal form J1 of the compound of Formula I-1.
Figure 57 shows the DSC pattern of hydrochloride crystal form J1 of the compound of Formula I-1.
Figure 58 shows the TGA pattern of hydrochloride crystal form J1 of the compound of Formula I-1.
Figure 59 shows the XRPD pattern of hydrochloride crystal form J2 of the compound of Formula I-1.
Figure 60 shows the DSC pattern of hydrochloride crystal form J2 of the compound of Formula I-1.
Figure 61 shows the TGA pattern of hydrochloride crystal form J2 of the compound of Formula I-1.
Figure 62 shows the XRPD pattern of hydrochloride crystal form K of the compound of Formula I-1.
Figure 63 shows the DSC pattern of hydrochloride crystal form K of the compound of Formula I-1.
Figure 64 shows the TGA pattern of hydrochloride crystal form K of the compound of Formula I-1.
Figure 65 shows the XRPD pattern of hydrochloride crystal form L of the compound of Formula I-1.
Figure 66 shows the DSC pattern of hydrochloride crystal form L of the compound of Formula I-1.
Figure 67 shows the TGA pattern of hydrochloride crystal form L of the compound of Formula I-1.
Figure 68 shows the XRPD pattern of hydrochloride crystal form M of the compound of Formula I-1.
Figure 69 shows the DSC pattern of hydrochloride crystal form M of the compound of Formula I-1.
Figure 70 shows the TGA pattern of hydrochloride crystal form M of the compound of Formula I-1.
Figure 71 shows the XRPD pattern of hydrochloride crystal form N of the compound of Formula I-1.
Figure 72 shows the DSC pattern of hydrochloride crystal form N of the compound of Formula I-1.
Figure 73 shows the TGA pattern of hydrochloride crystal form N of the compound of Formula I-1.
Figure 74 shows the XRPD pattern of hydrochloride crystal form O of the compound of Formula I-1.
Figure 75 shows the DSC pattern of hydrochloride crystal form O of the compound of Formula I-1.
Figure 76 shows the TGA pattern of hydrochloride crystal form O of the compound of Formula I-1.

### Detailed Description of the Invention

To address the issues in the prior art, the inventors conducted in-depth research on the various forms of the compound of formula I and developed multiple crystal forms and acid salt forms of the compound of formula I, particularly the hydrochloride, which significantly improved the physicochemical properties of the compound of formula I, such as solubility, hygroscopicity, and chemical stability. The crystalline acid salt compound raw materials comply with industrial production standards and meet the requirements for pharmaceutical formulation development. With important clinical application potential, these compounds are expected to accelerate the development of a new generation of FGFR2 inhibitors. Based on this, the present invention was completed.

### Definitions

Unless otherwise specified, the following terms and phrases used herein are intended to have the meanings set forth below. A particular phrase or term should not be considered ambiguous or unclear in the absence of a specific definition, but should be understood in its ordinary sense. When trade names appear herein, they are intended to refer to the corresponding products or their active ingredients.

As used herein, the term "n or more 2θ values selected from the group consisting of" refers to any positive integer greater than or equal to n (e.g., n, n+1, ...), where the upper limit Nup is the total number of 2θ peaks in that set. For example, "1 or more" includes not only 1, 2, 3, 4, 5, 6, 7, 8, 9, 10, 11, 12, 13, 14, 15, 16, 17, 18, 19, 20, 21, ...positive integers up to the upper limit Nup, but also includes ranges such as "2 or more", "3 or more", "4 or more", "5 or more", "6 or more", "7 or more", "8 or more", "9 or more" and "10 or more". For example, "3 or more" includes not only 3, 4, 5, 6, 7, 8, 9, 10, 11, 12, 13, 14, 15, 16, 17, 18, 19, 20, 21,···up to the upper limit Nup, but also includes ranges such as "4 or more", "5 or more", "6 or more", "7 or more", "8 or more", "9 or more" and "10 or more".

As used herein, "dissolving or dispersing the compound of formula (I) in 1 to 5 times of solvent" means dissolving or dispersing 1 gram of the compound of formula (I) in 1 to 5 milliliters of solvent, wherein the solvent includes, but is not limited to, acetonitrile, alcoholic solvents, ester solvents, ether solvents, or mixtures of alcoholic solvents and water.

The intermediate compounds of the present invention may be prepared by various synthetic methods known to those skilled in the art, including the specific embodiments listed below, embodiments formed by combining them with other chemical synthesis methods, and equivalent substitutions known to those skilled in the art; preferred embodiments include, but are not limited to, the examples of the present invention.

The chemical reactions of the specific embodiments of the present invention are carried out in suitable solvents, which must be appropriate for the chemical transformations of the present invention and the reagents and materials required therein. To obtain the compounds of the present invention, it may sometimes be necessary for those skilled in the art to modify or select synthetic steps or reaction procedures based on existing embodiments.

The present invention will be described in detail below through examples, these examples do not intended to exert any limitation on the scope of the invention.

All solvents used in the present invention are commercially available and can be used without further purification.

**The following abbreviations are used in the present invention:**

| Abbreviation | Full Name |
|---|---|
| EtOH | Ethanol |
| MeOH | Methanol |
| IPA | Isopropyl alcohol |
| MTBE | Methyl tert-butyl ether |
| MIBK | Methyl isobutyl ketone |
| EA | Ethyl acetate |
| IPAc | Isopropyl acetate |
| ACN | Acetonitrile |
| THF | Tetrahydrofuran |
| 2-MeTHF | 2-Methyl-1,4-dihydrofuran |
| DCM | Dichloromethane |
| DMSO | Dimethyl sulfoxide |
| FaSSGF | Fasted-state simulated gastric fluid |
| FaSSIF-v1 | Fasted-state simulated intestinal fluid - Version 1 |
| FeSSIF-v1 | Fed-State simulated intestinal fluid - Version 1 |
| XRPD | X-ray powder diffraction |
| DSC | Differential Scanning Calorimetry |
| TGA | Thermogravimetric Analysis |
| DVS | Dynamic Vapor Sorption |
| PLM | Polarizing Microscope |
| ¹H-NMR | Hydrogen Nuclear Magnetic Resonance Spectroscopy |
| KF | Karl Fischer (titrator) |
| HPLC | High-Performance Liquid Chromatography |
| IC | Ion Chromatography |
| LOQ | Limit of quantification |

**The test items and methods used in this invention are as follows:**

| **X-ray Powder Diffractometer (XRPD)** | |
|---|---|
| Instrument | Bruker D8 Advance |
| **Method 1** | |
| X-ray Path | Reflection Mode |
| Detector | LYNXEYE_XE_T (1D mode) |
| Opening Angle | 2.9^{°}(max) |
| Radioactive Source | Cu/K-Alpha1 (λ=1.5406 Å) |
| X-ray source power | 40 kV, 40 mA |
| Primary beam slit | Dual motorized slits: 20.0 mm; Primary Soller slit: 2.5^{°} |
| Secondary beam slit | Secondary Soller slit: 2.5^{°} |
| Scanning mode | Continuous scan |
| Scan type | Dual-beam mode |
| Step size | 0.02^{°} |
| Time per step | 0.4 s/step |
| Scan range | 2^{°}to 40^{°} |
| Sample rotation speed | 15 rpm |
| Sample plate | Monocrystalline silicon wafer, flat wafer |

| **Method 2** | |
|---|---|
| X-ray path | Reflection mode |
| Detector | LYNXEYE_XE_T (1D mode) |
| Opening Angle | 2.9^{°}(max) |
| Radioactive Source | Cu/K-Alpha1 (λ=1.5406 Å) |
| X-ray source power | 40 kV, 40 mA |
| Primary beam slit | Dual main motorized slits: 10.0 mm; Primary Soller slit: 2.5^{°} |
| Secondary beam slit | Secondary Soller slit: 2.5^{°} |
| Scanning mode | Continuous scan |
| Scan type | Dual-beam mode |
| Step size | 0.02^{°} |
| Time per step | 0.12 s/step |
| Scan Range | 3^{°}to 40^{°} |
| Sample rotation speed | 15 rpm |
| Sample plate | Monocrystalline silicon wafer, flat plate |

| **Differential Scanning Calorimeter (DSC)** | | | |
|---|---|---|---|
| Instrument | TA Instruments Discovery 2500 | | |
| Sample tray | Tzero tray and Tzero hermetic lid with apertures of approximately 0.7 mm in diameter | | |
| Temperature Range | ~30 to 250^{°}C | | |
| Heating rate | 10^{°}C/min | | |
| Nitrogen flow rate | 50 mL/min | | |
| Sample mass | ~0.5-5 mg | | |

| **Thermogravimetric Analyzer (TGA)** | | | |
|---|---|---|---|
| Instrument | TA Instruments Discovery 5500 | | |
| Sample tray | Aluminum dish, open-top | | |
| Starting Temperature | Ambient temperature (below 35^{°}C) | | |
| Final temperature | 300^{°}C | | |
| Heating rate | 10^{°}C/min | | |
| Nitrogen flow rate | Equilibrium: 10 mL/min; Sample chamber: 25 mL/min | | |
| Sample volume | ~1-10 mg | | |

| **High-Performance Liquid Chromatography (HPLC)** | | | |
|---|---|---|---|
| Instrument | Agilent 1260 Infinity II Binary Pump | | |
| Method 1 | For purity and solubility determination | | |
| | Wavelength: 220 nm | | |
| | Column: Waters Sunfire C18 150 × 460 5µm | | |
| | Detector: DAD | | |
| | Column temperature: 40^{°}C | | |
| | Flow rate: 1.2 mL/min | | |
| | Mobile phase A: 0.1% trifluoroacetic acid | | |
| HPLC Method | Mobile phase B: Acetonitrile | | |
| | Diluent: Acetonitrile | | |
| | Injection volume: 5 µL | | |
| | Gradient: | | |
| | Time (min) | Mobile Phase A (%) | Mobile Phase B (%) |
| | 0.00 | 95 | 5 |
| | 9.00 | 5 | 95 |
| | 13.00 | 5 | 95 |
| | 13.10 | 95 | 5 |
| | 17.00 | 95 | 5 |
| Method 2 | For HPLC-IC hyphenated analysis | | |
| | Wavelength: 220 nm | | |
| | Column: Waters Sunfire C18 150 × 460 µm, 5 µm | | |
| | Detector: DAD | | |
| | Column temperature: 40^{°}C | | |
| | Flow rate: 1.2 mL/min | | |
| HPLC Method | Mobile phase A: 0.1% trifluoroacetic acid | | |
| | Mobile phase B: Acetonitrile | | |
| | Diluent: Acetonitrile/water (1:1, v/v) | | |
| | Injection volume: 8 µL | | |
| | Gradient: | | |
| | Time (min) | Mobile Phase A (%) | Mobile Phase B (%) |
| | 0.00 | 95 | 5 |
| | 9.00 | 5 | 95 |
| | 13.00 | 5 | 95 |
| | 13.10 | 95 | 5 |
| | 17.00 | 95 | 5 |

To better understand the content of the present invention, further explanation is provided below with reference to specific examples; however, the specific examples are not intended to limit the scope of the present invention.

### Example 1: Preparation of Compound (I-1)

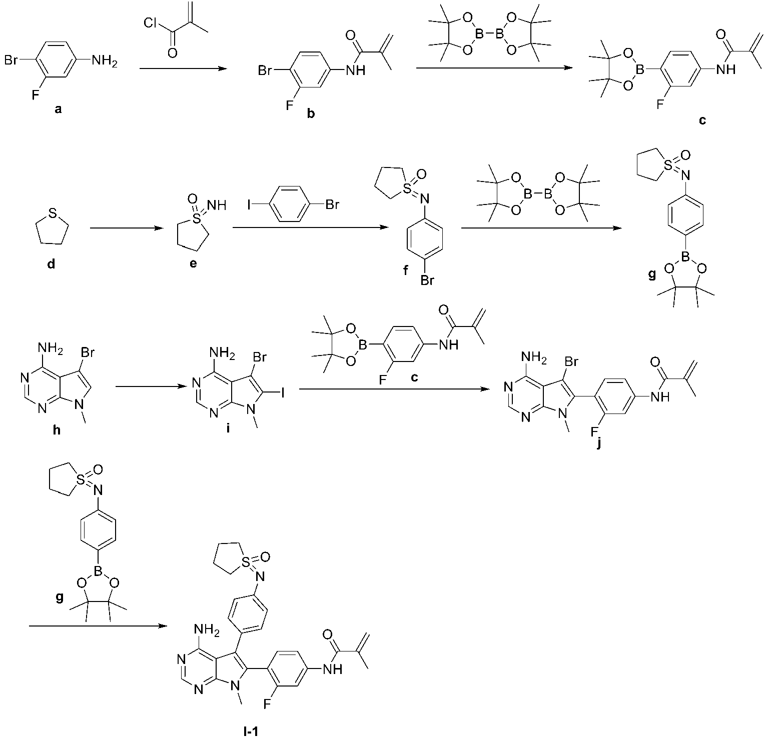

### Step 1:

Compound **a** (5.0 g, 26.3 mmol) and triethylamine (8.0 g, 11 ml) were dissolved in DCM (50 ml). At 0 °C, 2-methacryloyl chloride (3.03 g, 2.8 ml) was added dropwise to the reaction solution and stirred for 1 h. The reaction solution was diluted with water (100 mL) and extracted three times with DCM (100 mL). The organic phase was washed with saturated saline solution, dried over anhydrous Na₂SO₄, and concentrated under reduced pressure. The obtained crude product was purified by silica gel column chromatography (EA/PE = 1/4) to obtain compound **b** (4.9 g, 72%), as a pale yellowish-white solid.

MS (ESI) m/z 258, 260 [M + H]⁺.

### Step 2:

Compound **b** (2.0 g, 7.75 mmol), bis(pinacolato)diboron (3.94 g, 15.5 mmol), Pd(dppf)Cl₂(567 mg, 0.77 mmol), AcOK (2.28 g, 23.25 mmol), and 1,4-dioxane (20 mL) were placed in a sealed tube, which was purged with argon three times. The mixture was reacted at 90^{°}C for 5 h. The reaction was quenched with water and extracted with DCM. The organic phase was washed with saturated saline, dried over anhydrous Na₂SO₄, and concentrated under reduced pressure. The obtained crude product was purified by silica gel column chromatography (EA/PE = 1/8 to 1/4) to obtain compound **c** (1.52 g, 64%), as a yellow solid.

MS (ESI) m/z 306 [M + H]⁺ .

¹H NMR (500 MHz, CDCl₃) δ 7.70-7.65 (m, 2H), 7.53 (dd, J = 11.5, 1.9 Hz, 1H), 7.20 (dd, J = 8.2, 1.9 Hz, 1H), 5.78 (s, 1H), 5.48 (d, J = 1.7 Hz, 1H), 2.04 (s, 3H), 1.34 (s, 12H).

### Step 3:

Compound **d** (2.00 g, 19.6 mmol) and ammonium carbamate (2.29 g, 29.4 mmol) were added sequentially to a flask, followed by the addition of MeOH (40 mL) and iodobenzene diacetate (13.24 g, 41.1 mmol). The reaction solution was stirred uncovered at room temperature for 30 min, then concentrated under reduced pressure to remove the solvent to obtain the crude product. The crude product was purified by column chromatography (MeOH/DCM = 1/80) to obtain compound **e** (1.62 g), as a white solid.

MS (ESI) m/z 120 [M + H]⁺ .

### Step 4:

Compound **e** (282 mg, 2.35 mmol), cesium carbonate (892 mg, 2.74 mmol), 1-bromo-4-iodobenzene (552 mg, 1.96 mmol), Pd₂(dba)₃(40 mg, 0.05 mmol), and Xantphos (77 mg, 0.13 mmol) were added to 1,4-dioxane (10 mL), and the reaction solution was heated to 105°C and reacted for 13 h. The reaction solution was filtered through diatomaceous earth; the filtrate was washed with ethyl acetate (50 mL) and saturated saline solution (50 mL), and the organic phase was dried over anhydrous sodium sulfate and concentrated under reduced pressure to obtain the crude product. The crude product was purified by column chromatography (PE/EtOAc = 4/1 to 1/1) to obtain compound **f** (410 mg), as a yellow oily liquid.

MS (ESI) m/z 274 [M + H]⁺ .

### Step 5:

To a 100 mL round-bottom flask were added sequentially compound **f** (1.02 g, 3.10 mmol), bis(pinacolato)diboron (0.973 g, 3.83 mmol), Pd(dppf)Cl₂(0.461 mg, 0.63 mmol), potassium acetate (0.943 g, 9.61 mmol), and 1,4-dioxane (20 mL). The mixture was heated to 100^{°}C and reacted for 4 h. After monitoring the reaction to completion, the mixture was cooled to room temperature, filtered through diatomaceous earth, and extracted with ethyl acetate (50 mL × 3), and the organic phases was combined, washed with saturated saline solution (50 mL), and concentrated under reduced pressure to obtain the crude product. The crude product was purified by column chromatography (PE/EtOAc = 1/1) to obtain compound **g** (0.80 g), as a yellow solid.

¹H NMR (400 MHz, CDCl₃) δ 7.62 (d, J = 8.3 Hz, 2H), 6.98 (d, J = 8.3 Hz, 2H), 3.37-3.30 (m, 1H), 3.15-3.08 (m, 1H), 2.20 (dd, J = 29.3, 6.9 Hz, 2H), 1.26 (s, 6H).

### Step 6:

Compound **h** (10.6 g, 0.0466 mol) was dissolved in dichloromethane (100 mL) and trifluoroacetic acid (26.7 g, 0.2334 mol), and stirred at 10^{°}C. N-iodobutyric imide (10.5 g, 0.0466 mol) was added portionwise, and the mixture was reacted for 4 h. Upon completion of the reaction, 150 mL of saturated sodium bicarbonate solution was slowly added dropwise at 10^{°}C, resulting in the precipitation of solids, which was filtrated and dried to obtain compound **i** (13.4 g), as a purple solid.

¹H NMR (400 MHz, CDCl₃) δ 8.22 (s, 1H), 5.61 (s, 2H), 3.80 (s, 3H).

### Step 7:

Compound **i** (2 g, 6.5 mmol), compound **c** (2.3 g, 6.5 mmol), tetrakis(triphenylphosphine)palladium (0.65 g, 0.56 mmol), and potassium phosphate (3.6 g, 0.017 mmol) were added to a reaction flask, which was purged with nitrogen three times. Then DMF (35 mL) and water (5 mL) were added, and stirred at 50^{°}C for 16 h. Upon completion of the reaction, water (150 mL) and dichloromethane (200 mL) were added for extraction and liquid-liquid separation. The organic phase was washed with saturated saline solution and concentrated to obtain the crude product. The crude product was purified by column chromatography to obtain compound **j** (1.1 g), as a pale yellow solid.

ESI-MS m/z 404.2 [M+H]⁺
¹H NMR (400 MHz, CDCl₃) δ 8.35 (s, 1H), 7.82 (dd, J = 11.7, 2.1 Hz, 1H), 7.69 (s, 1H), 7.44-7.33 (m, 2H), 5.87 (s, 1H), 5.66 (s, 2H), 5.58 (q, J = 1.5 Hz, 1H), 3.66 (d, J = 1.1 Hz, 3H), 2.12 (dd, J = 1.6, 0.9 Hz, 3H).

### Step 8:

To a 25 mL round-bottom flask were added sequentially compound **g** (45 mg, 0.14 mmol), compound **j** (50 mg, 0.12 mmol), Pd(dppf)Cl₂(20 mg, 0.03 mmol), potassium phosphate (80 mg, 0.38 mmol), 1,4-dioxane (5 mL), and water (1 mL). The reaction was heated to 80^{°}C and reacted for 0.5 h. After monitoring the reaction to completion, the reaction was cooled to room temperature, filtered through diatomaceous earth, and extracted with ethyl acetate (20 mL × 3), and the organic layers were combined, washed with saturated saline solution (20 mL), and concentrated under reduced pressure to obtain the crude product. The crude product was purified by column chromatography (DCM/MeOH = 6/1) to obtain the compound of Formula I-1 (22 mg), as a white solid.

MS (ESI) m/z 519.4 [M + H]⁺
¹H NMR (400 MHz, CDCl₃) δ 8.28 (s, 1H), 7.65-7.61 (m, 1H), 7.48 (s, 1H), 7.05 (d, J = 7.7 Hz, 2H), 7.00 (t, J = 4.1 Hz, 2H), 6.91 (d, J = 8.5 Hz, 2H), 5.73 (s, 1H), 5.45 (d, J = 1.3 Hz, 1H), 4.98 (s, 2H), 3.59 (d, J = 0.9 Hz, 3H), 3.36 (dd, J = 12.6, 6.0 Hz, 2H), 3.13 (dd, J = 12.8, 6.6 Hz, 2H), 2.29-2.19 (m, 3H), 1.99 (d, J = 10.1 Hz, 3H).

### Example 2: Preparation of crystal form A of the compound of Formula I-1

The compound of formula I-1 (61 g) was added to 100 mL of ethyl acetate, stirred at room temperature overnight, filtered, and dried to obtain a sample of crystal form A of the compound of formula I-1 (58.2 g), as a white solid.

### Example 3: Preparation of the maleate crystal form A of the compound of Formula I-1

3.1: Approximately 20 mg of the compound of Formula I-1 and 1.0 equivalent of maleic acid were weighed into a 2-mL glass vial, and 0.15 mL of acetonitrile was added to perform a screening experiment using the suspension method. The resulting sample was suspened at 50^{°}C for 2 h, then allowed to cool naturally to 25^{°}C, and suspened at 25^{°}C for at least 48 h. The resulting suspension was centrifuged at 14,000 rpm through a 0.45 µm nylon filter membrane, and the resulting solid was vacuum-dried at 50^{°}C for 2 h to yield a sample of the maleate crystal form A of the compound of Formula I-1.

3.2: Approximately 20 mg of the compound of Formula I-1 and 1.0 equivalent of counterion were weighed into a 2-mL glass vial, and 0.15 mL of dichloromethane was added to perform a screening experiment using the suspension method. The resulting sample was suspended at 50^{°}C for 2 hours, then allowed to cool naturally to 25^{°}C, and suspended at 25^{°}C for at least 48 hours. The resulting suspension was centrifuged at 14,000 rpm through a 0.45 µm nylon filter membrane, and the resulting solid was vacuum-dried at 50^{°}C for 2 hours to yield a sample of the maleate crystal form A of the compound of Formula I-1.

### Example 4: Preparation of the monomaleate crystal form B of the compound of Formula I-1

Approximately 20 mg of compound of Formula I-1 and 1.0 equivalent of maleic acid were weighed into a 2-mL glass vial, 0.15 mL of tetrahydrofuran was added to perform a screening experiment using the suspension method. The resulting sample was suspended at 50°C for 2 h, then allowed to cool naturally to 25°C, and suspended at 25°C for at least 48 h. The resulting suspension was centrifuged at 14,000 rpm through a 0.45 µm nylon filter membrane, and the resulting solid was vacuum-dried at 50°C for 2 h to obtain a sample of the monomaleate form B of the compound of Formula I-1.

### Example 5: Preparation of the fumarate crystal form A of the Compound of Formula I-1

5.1: Approximately 20 mg of the compound of Formula I-1 and 1.0 equivalent of fumaric acid were weighed into a 2 mL glass vial, and 0.15 mL of acetonitrile was added to perform a screening experiment using the suspension method. The resulting sample was suspended at 50^{°}C for 2 h, then allowed to cool naturally to 25^{°}C, and suspended at 25^{°}C for at least 48 h. The resulting suspension was centrifuged at 14,000 rpm through a 0.45 µm nylon filter membrane, and the resulting solid was vacuum-dried at 50^{°}C for 2 h to yield a sample of the fumarate crystal form A of the compound of Formula I-1.

5.2: Approximately 20 mg of the compound of Formula I-1 and 1.0 equivalent of fumaric acid were weighed into a 2-mL glass vial, and 0.15 mL of dichloromethanewas added to perform a screening experiment using the suspension method. The resulting sample was suspended at 50^{°}C for 2 hours, then allowed to cool naturally to 25^{°}C, and suspended at 25^{°}C for at least 48 hours. The resulting suspension was centrifuged at 14,000 rpm through a 0.45 µm nylon filter membrane, and the resulting solid was vacuum-dried at 50^{°}C for 2 hours to obtain a sample of the fumarate crystal form A of the compound of Formula I-1.

### Example 6: Preparation of the hemifumarate crystal form B of the compound of Formula I-1

Approximately 20 mg of the compound of Formula I-1 and 1.0 equivalent of fumaric acid were weighed into a 2-mL glass vial, and 0.15 mL of tetrahydrofuran was added to perform a screening experiment using the suspension method. The resulting sample was suspended at 50^{°}C for 2 h, then allowed to cool naturally to 25^{°}C, and suspended at 25^{°}C for at least 48 h. The resulting suspension was centrifuged at 14,000 rpm through a 0.45 µm nylon filter membrane, and the resulting solid was vacuum-dried at 50^{°}C for 2 h to yield a sample of the hemifumarate crystal form B of the compound of Formula I-1.

### Example 7: Preparation of the monoglycolate crystal form A of the compound of Formula I-1

Approximately 20 mg of the compound of Formula I-1 and 1.0 equivalent of glycolic acid were weighed into a 2 mL glass vial, and 0.15 mL of acetonitrile was added to perform a screening experiment using the suspension method. The resulting sample was suspended at 50^{°}C for 2 h, then allowed to cool naturally to 25^{°}C, and suspended at 25^{°}C for at least 48 h. The resulting suspension was centrifuged at 14,000 rpm through a 0.45 µm nylon filter membrane, and the resulting solid was vacuum-dried at 50^{°}C for 2 h to yield a sample of the monoglycolate crystal form A of the compound of Formula I-1.

### Example 8: Preparation of the glycolate crystal form B of the Compound of Formula I-1

8.1: Approximately 20 mg of the compound of Formula I-1 and 1.0 equivalent of glycolic acid were weighed into a 2 mL glass vial, and 0.15 mL of tetrahydrofuran was added to perform a screening experiment using the suspension method. The resulting mixture was suspended at 50^{°}C for 2 h, then allowed to cool naturally to 25^{°}C, and suspended at 25^{°}C for at least 48 h. The resulting suspension was centrifuged at 14,000 rpm through a 0.45 µm nylon filter membrane, and the resulting solid was vacuum-dried at 50^{°}C for 2 h to yield a sample of the glycolate crystal form B of the compound of Formula I-1.

8.2: Approximately 20 mg of the compound of Formula I-1 and 1.0 equivalent of glycolic acid were weighed into a 2-mL glass vial, and 0.15 mL of dichloromethane was added to perform a screening experiment using the suspension method. The resulting sample was suspended at 50^{°}C for 2 h, then allowed to cool naturally to 25^{°}C, and suspended at 25^{°}C for at least 48 h. The resulting suspension was centrifuged at 14,000 rpm through a 0.45 µm nylon filter membrane, and the resulting solid was vacuum-dried at 50^{°}C for 2 h to obtain a sample of the glycolate form B of the compound of Formula I-1.

### Example 9: Preparation of the mono L-malate crystal form A of the compound of Formula I-1

9.1: Approximately 20 mg of the compound of Formula I-1 and 1.0 equivalent of L-malic acid were weighed into a 2-mL glass vial, and 0.15 mL of tetrahydrofuran was added to perform a screening experiment using the suspension method. The resulting mixture was suspended at 50^{°}C for 2 h, then allowed to cool naturally to 25^{°}C, and suspended at 25^{°}C for at least 48 h. The resulting suspension was centrifuged at 14,000 rpm through a 0.45 µm nylon filter membrane. The resulting solid was vacuum-dried at 50^{°}C for 2 h to yield a sample of the mono L-malate crystal form A of the compound of Formula I-1.

9.2: Approximately 20 mg of the compound of Formula I-1 and 1.0 equivalent of glycolic acid were weighed into a 2-mL glass vial, and 0.15 mL of dichloromethane was added to perform a screening experiment using the suspension method. The resulting sample was suspended at 50^{°}C for 2 h, then allowed to cool naturally to 25^{°}C, and suspended at 25^{°}C for at least 48 h. The resulting suspension was centrifuged at 14,000 rpm through a 0.45 µm nylon filter membrane. The resulting solid was vacuum-dried at 50^{°}C for 2 h to yield a sample of the mono L-malate crystal form A of the compound of Formula I-1.

9.3: Approximately 20 mg of the compound of Formula I-1 and 1.0 equivalent of glycolic acid were weighed into a 2-mL glass vial, and 0.15 mL of acetonitrile was added to perform a screening experiment using the suspension method. The resulting sample was suspended at 50^{°}C for 2 hours, then allowed to cool naturally to 25^{°}C, and suspended at 25^{°}C for at least 48 hours. The resulting suspension was centrifuged at 14,000 rpm through a 0.45 µm nylon filter membrane. The resulting solid was vacuum-dried at 50^{°}C for 2 hours to yield a sample of the mono L-malate crystal form A of compound of Formula I-1.

### Example 10: Preparation of the monosuccinate crystal form A of the compound of Formula I-1

10.1: Approximately 20 mg of the compound of Formula I-1 and 1.0 equivalent of succinic acid were weighed into a 2 mL glass vial, and 0.15 mL of acetonitrile was added to perform a screening experiment using the suspension method. The resulting mixture was suspended at 50^{°}C for 2 h, then allowed to cool naturally to 25^{°}C, and suspended at 25^{°}C for at least 48 h. The resulting suspension was centrifuged at 14,000 rpm through a 0.45 µm nylon filter membrane. The resulting solid was vacuum-dried at 50^{°}C for 2 h to obtain a sample of the monosuccinate crystal form A of compound of Formula I-1.

10.2: Approximately 20 mg of the compound of Formula I-1 and 1.0 equivalent of succinic acid were weighed into a 2-mL glass vial, and 0.15 mL of dichloromethane was added to perform a screening experiment using the suspension method. The resulting sample was suspended at 50^{°}C for 2 h, then allowed to cool naturally to 25^{°}C, and suspended at 25^{°}C for at least 48 h. The resulting suspension was centrifuged at 14,000 rpm through a 0.45 µm nylon filter membrane. The resulting solid was vacuum-dried at 50^{°}C for 2 h to obtain a sample of the monosuccinate crystal form A of the compound of Formula I-1.

### Example 11: Preparation of the sulfate crystal form A of the compound of Formula I-1

Approximately 20 mg of the compound of Formula I-1 and 1.0 equivalent of sulfuric acid were weighed into a 2 mL glass vial, and 0.15 mL of acetonitrile was added to perform a screening experiment using the suspension method. The resulting sample was suspended at 50^{°}C for 2 h, then allowed to cool naturally to 25^{°}C, and suspended at 25^{°}C for at least 48 h. The resulting suspension was centrifuged at 14,000 rpm through a 0.45 µm nylon filter membrane. The resulting solid was vacuum-dried at 50^{°}C for 2 h to yield a sample of the sulfate crystal form A of the compound of Formula I-1.

### Example 12: Preparation of the L-tartrate crystal form A of the compound of Formula I-1

Approximately 20 mg of the compound of Formula I-1 and 1.0 equivalent of L-tartaric acid were weighed into a 2-mL glass vial, and 0.15 mL of acetonitrile was added to perform a screening experiment using the suspension method. The resulting sample was suspended at 50^{°}C for 2 h, then allowed to cool naturally to 25^{°}C, and suspended at 25^{°}C for at least 48 h. The resulting suspension was centrifuged at 14,000 rpm through a 0.45 µm nylon filter membrane. The resulting solid was vacuum-dried at 50^{°}C for 2 h to obtain a sample of the L-tartarate crystal form A of the compound of Formula I-1.

### Example 13: Preparation of the hippurate crystal form A of the compound of Formula I-1

Approximately 20 mg of the compound of Formula I-1 and 1.0 equivalent of hippuric acid were weighed into a 2 mL glass vial, and 0.15 mL of tetrahydrofuran was added to perform a screening experiment using the suspension method. The resulting sample was suspended at 50^{°}C for 2 h, then allowed to cool naturally to 25^{°}C, and suspended at 25^{°}C for at least 48 h. The resulting suspension was centrifuged at 14,000 rpm through a 0.45 µm nylon filter membrane. The resulting solid was vacuum-dried at 50^{°}C for 2 h to yield a sample of the hippurate crystal form A of the compound of Formula I-1.

### Example 14: Preparation of the glutarate crystal form A of the compound of Formula I-1

14.1: Approximately 20 mg of the compound of Formula I-1 and 1.0 equivalent of glutaric acid were weighed into a 2-mL glass vial, and 0.15 mL of acetonitrile was added to perform a screening experiment using the suspension method. The resulting mixture was suspended at 50^{°}C for 2 h, then allowed to cool naturally to 25^{°}C, and suspended at 25^{°}C for at least 48 h. The resulting suspension was centrifuged at 14,000 rpm through a 0.45 µm nylon filter membrane. The resulting solid was vacuum-dried at 50^{°}C for 2 h to yield a sample of the glutarate crystal form A of the compound of Formula I-1.

14.2: Approximately 20 mg of the compound of Formula I-1 and 1.0 equivalent of glutaric acid were weighed into a 2-mL glass vial, and 0.15 mL of dichloromethane was added to perform a screening experiment using the suspension method. The resulting sample was suspended at 50^{°}C for 2 h, then allowed to cool naturally to 25^{°}C, and suspended at 25^{°}C for at least 48 h. The resulting suspension was centrifuged at 14,000 rpm through a 0.45 µm nylon filter membrane. The resulting solid was vacuum-dried at 50^{°}C for 2 h to yield a sample of the glutarate crystal form A of the compound of Formula I-1.

### Example 15: Preparation of the p-toluenesulfonate crystal form A of the compound of Formula I-1

Approximately 20 mg of the compound of Formula I-1 and 1.0 equivalent of p-toluenesulfonic acid were weighed into a 2 mL glass vial, and 0.15 mL of acetonitrile was added to perform a screening experiment using the suspension method. The resulting mixture was suspended at 50^{°}C for 2 h, then allowed to cool naturally to 25^{°}C, and suspended at 25^{°}C for at least 48 h. The resulting suspension was centrifuged at 14,000 rpm through a 0.45 µm nylon filter membrane. The resulting solid was vacuum-dried at 50^{°}C for 2 h to obtain a sample of the p-toluenesulfonate crystal form A of the compound of Formula I-1.

### Example 16: Preparation of the p-toluenesulfonate crystal form B of the compound of Formula I-1

Approximately 20 mg of the compound of Formula I-1 and 1.0 equivalent of p-toluenesulfonic acid were weighed into a 2 mL glass vial, and 0.15 mL of tetrahydrofuran was added to perform a screening experiment using the suspension method. The resulting sample was suspended at 50^{°}C for 2 h, then allowed to cool naturally to 25^{°}C, and suspended at 25^{°}C for at least 48 h. The resulting suspension was centrifuged at 14,000 rpm through a 0.45 µm nylon filter membrane. The resulting solid was vacuum-dried at 50^{°}C for 2 h to obtain a sample of the p-toluenesulfonate crystal form B of the compound of Formula I-1.

### Example 17: Preparation of the methanesulfonate crystal form A of the compound of Formula I-1

Approximately 20 mg of the compound of Formula I-1 and 1.0 equivalent of methanesulfonic acid were weighed into a 2 mL glass vial, and 0.15 mL of tetrahydrofuran was added to perform a screening experiment using the suspension method. The resulting mixture was suspended at 50^{°}C for 2 h, then allowed to cool naturally to 25^{°}C, and suspended at 25^{°}C for at least 48 h. The resulting suspension was centrifuged at 14,000 rpm through a 0.45 µm nylon filter membrane. The resulting solid was vacuum-dried at 50^{°}C for 2 h to obtain a sample of the methanesulfonate crystal form A of the compound of Formula I-1.

### Example 18: Preparation of the hydrochloride crystal form C1 of the compound of Formula I-1

Compound of Formula I-1 (16.5 g) was added to ethyl acetate (165 mL), cooled to approximately 0^{°}C, and 1.0 equivalent of a 4 M solution of hydrochloric acid in dioxane was added dropwise. After the addition was complete, the mixture was allowed to warm to room temperature and stirred overnight, then filtered and dried to obatin a sample of the hydrochloride crystal form C1 of the compound of Formula I-1 (18.3 g), as a white solid.

### Example 19: Preparation of the hydrochloride crystal form C2 of the compound of Formula I-1

Approximately 40 mg of the hydrochloride crystal form E1 of the compound of Formula I-1 was weighed, 0.2-0.5 mL of 2-methyl-tetrahydrofuran was added, and the mixture was suspended under magnetic stirring at 25^{°}C at a speed of 300-400 rpm. The resulting suspension was subjected to centrifugal filtration at 14,000 rpm using a 0.45 µm nylon-membrane centrifuge tube. The resulting solid was the sample of the hydrochloride crystal form C2 of the compound of Formula I-1.

### Example 20: Preparation of the hydrochloride crystal form D of the compound of Formula I-1

Approximately 40 mg of the compound of Formula I-1 and 1.05 equivalents of hydrochloric acid (dilute hydrochloric acid diluted 10-fold with acetone) were weighed into a 2 mL glass vial, and the screening solvent was added to perform the screening experiment using the suspension method. The resulting sample was suspended at 50^{°}C (35^{°}C, dichloromethane) for 2 h, then allowed to cool naturally to 25^{°}C, and suspended at 25^{°}C for at least 48 h. The resulting suspension was centrifuged at 14,000 rpm through a 0.45 µm nylon filter membrane. The resulting solid was vacuum-dried at 50^{°}C for 2 h to yield a sample of the hydrochloride crystal form D of the compound of Formula I-1.

### Example 21: Preparation of the hydrochloride crystal form E1 of the compound of Formula I-1

3.0 g of the compound of Formula I-1 was weighed to a 40 mL glass bottle. 4.6 mL of ethanol and approximately 1.2 equivalents of HCl (a 12 N HCl aqueous solution was diluted with ethanol to 1.2 N) were added, and stirred for 10 minutes at 50^{°}C. Approximately 5 mg of the seed crystal of hydrochloride crystal form E2 of the compound of Formula I-1 was added to the system, and stirred at 50^{°}C for 2 h, then the solution was cooled naturally to 25^{°}C and stirred at 25^{°}C for 3 days. ~0.1 equivalents of HCl (12N HCl aqueous solution was diluted with ethanol to 1.2N) was added to the system and stirred at 25^{°}C for another 1 day. The solid fraction was collected by vacuum filtration, and the resulting solid was dried under vacuum at 25^{°}C for approximately 17 hours. The obtained solid was then dried further under vacuum at 50^{°}C for 2 hours to yield a sample of the hydrochloride crystal form E1 of the compound of Formula I-1 (2.8 g), as a nearly white solid.

### Example 22: Preparation of the hydrochloride crystal form E2 of the compound of Formula I-1

Approximately 40 mg of the compound of Formula I-1 and 1.05 equivalents of hydrochloric acid (dilute hydrochloric acid diluted 10-fold with ethanol) were weighed into a 2 mL glass vial, and the screening solvent was added to perform the screening experiment using the suspension method. The resulting sample was suspended at 50^{°}C (35^{°}C, dichloromethane) for 2 h, then allowed to cool naturally to 25°C, and suspended at 25°C for at least 48 h. The resulting suspension was centrifuged at 14,000 rpm through a 0.45 µm nylon filter membrane. The resulting solid was a sample of hydrochloride crystal form F of the compound of Formula I-1, and it was vacuum-dried at 50°C for 2 h to obtain a sample of the hydrochloride crystal form E2 of the compound of Formula I-1.

### Example 23: Preparation of the hydrochloride crystal form F of the compound of Formula I-1

23.1: Approximately 40 mg of the hydrochloride crystal form E1 of the compound of Formula I-1 was weighed, and 0.2-0.5 mL of ethanol was added, and the mixture was suspended under magnetic stirring at 25^{°}C at a speed of 300-400 rpm. The resulting suspension was subjected to centrifugal filtration at 14,000 rpm using a 0.45 µm nylon-membrane centrifuge tube. The resulting solid was a sample of the hydrochloride crystal form F of the compound of Formula I-1.

23.2: Approximately 40 mg of the hydrochloride crystal form E1 of the compound of Formula I-1 was weighed, and 0.2-0.5 mL of acetone was added, and the mixture was suspended under magnetic stirring at 25^{°}C at a speed of 300-400 rpm. The resulting suspension was subjected to centrifugal filtration at 14,000 rpm using a 0.45 µm nylon-membrane centrifuge tube. The resulting solid was a sample of the hydrochloride crystal form F of the compound of Formula I-1.

23.3: Approximately 40 mg of the hydrochloride crystal form E1 of the compound of formula I-1 were weighed, and 0.2-0.5 mL of acetonitrile was added, and the mixture was suspended under magnetic stirring at 25^{°}C at a speed of 300-400 rpm. The resulting suspension was subjected to centrifugal filtration at 14,000 rpm using a 0.45 µm nylon-membrane centrifuge tube. The resulting solid was a sample of the hydrochloride crystal form F of the compound of Formula I-1.

23.4: Approximately 40 mg of the hydrochloride crystal form E1 of the compound of formula I-1 were weighed, and 0.2-0.5 mL of tetrahydrofuran was added, and the mixture was suspended under magnetic stirring at 25^{°}C at a speed of 300-400 rpm. The resulting suspension was subjected to centrifugal filtration at 14,000 rpm using a 0.45 µm nylon-membrane centrifuge tube. The resulting solid was a sample of the hydrochloride crystal form F of the compound of Formula I-1.

23.5: Approximately 40 mg of the hydrochloride crystal form E1 of the compound of Formula I-1 was weighed, and 0.2-0.5 mL of methyl isobutyl ketone/trifluoroethanol (9:1, v/v) was added, and the mixture was suspended under magnetic stirring at 25^{°}C at a speed of 300-400 rpm. The resulting suspension was subjected to centrifugal filtration at 14,000 rpm using a 0.45 µm nylon-membrane centrifuge tube. The resulting solid was the hydrochloride crystal form F of compound of Formula I-1.

23.6: Approximately 40 mg of the hydrochloride crystal form E1 of the compound of Formula I-1 was weighed, and 0.1-0.5 mL of ethanol was added, and the mixture was suspended under magnetic stirring at a speed of 300-400 rpm at 50^{°}C. The resulting suspension was subjected to centrifugal filtration at 14,000 rpm using a 0.45 µm nylon-membrane centrifuge tube. The resulting solid was the hydrochloride crystal form F of compound of Formula I-1.

23.7: Approximately 40 mg of the hydrochloride crystal form E1 of the compound of Formula I-1 was weighed, and 0.1-0.5 mL of acetone was added, and the mixture was suspended under magnetic stirring at a speed of 300-400 rpm at 50^{°}C. The resulting suspension was subjected to centrifugal filtration at 14,000 rpm using a 0.45 µm nylon-membrane centrifuge tube. The resulting solid was the hydrochloride crystal form F of the compound of Formula I-1.

23.8: Approximately 40 mg of the hydrochloride crystal form E1 of the compound of Formula I-1 was weighed, and 0.1-0.5 mL of ethyl acetate was added, and the mixture was suspended under magnetic stirring at a speed of 300-400 rpm at 50^{°}C. The resulting suspension was subjected to centrifugal filtration at 14,000 rpm using a 0.45 µm nylon-membrane centrifuge tube. The resulting solid was the hydrochloride crystal form F of the compound of Formula I-1.

23.9: Approximately 40 mg of the hydrochloride crystal form E1 of the compound of Formula I-1 was weighed, and 0.1-0.5 mL of acetonitrile was added, and the mixture was suspended under magnetic stirring at a speed of 300-400 rpm at 50^{°}C. The resulting suspension was subjected to centrifugal filtration at 14,000 rpm using a 0.45 µm nylon-membrane centrifuge tube. The resulting solid was the hydrochloride crystal form F of the compound of Formula I-1.

23.10: Approximately 40 mg of the hydrochloride crystal form E1 of the compound of Formula I-1 was weighed, and 0.1-0.5 mL of tetrahydrofuran was added, and the mixture was suspended under magnetic stirring at 50^{°}C at a speed of 300-400 rpm. The resulting suspension was subjected to centrifugal filtration at 14,000 rpm using a 0.45 µm nylon-membrane centrifuge tube. The resulting solid was the hydrochloride crystal form F of the compound of Formula I-1.

23.11: Approximately 40 mg of the hydrochloride crystal form E1 of the compound of Formula I-1 was weighed, and 0.1-0.5 mL of methyl isobutyl ketone/trifluoroethanol (9/1, v/v) was added, and the mixture was suspended under magnetic stirring at a speed of 300-400 rpm at 50^{°}C. The resulting suspension was subjected to centrifugal filtration at 14,000 rpm using a 0.45 µm nylon-membrane centrifuge tube. The resulting solid was the hydrochloride crystal form F of the compound of Formula I-1.

23.12: Approximately 40 mg of the hydrochloride crystal form E1 of the compound of Formula I-1 was weighed, and 0.1-0.5 mL of ethanol was added. The mixture was subjected to 10 heating-cooling cycles between 5^{°}C and 50^{°}C at a rate of 0.1^{°}C/min, while suspended under magnetic stirring at a speed of 300-400 rpm, and the temperature at sampling was 10^{°}C. The resulting suspension was subjected to centrifugal filtration at 14,000 rpm using a 0.45 µm nylon-membrane centrifuge tube. The resulting solid was the hydrochloride crystal form F of the compound of Formula I-1.

23.13: Approximately 40 mg of the hydrochloride crystal form E1 of the compound of Formula I-1 was weighed, and 0.1-0.5 mL of acetone was added. The mixture was subjected to 10 heating-cooling cycles between 5^{°}C and 50^{°}C at a rate of 0.1^{°}C/min, while suspended under magnetic stirring at a speed of 300-400 rpm, and the temperature at sampling was 10^{°}C. The resulting suspension was subjected to centrifugal filtration at 14,000 rpm using a 0.45 µm nylon-membrane centrifuge tube. The resulting solid was the hydrochloride crystal form F of the compound of Formula I-1.

23.14: Approximately 40 mg of the hydrochloride crystal form E1 of the compound of Formula I-1 was weighed, and 0.1-0.5 mL of ethyl acetate was added. The mixture was subjected to 10 heating-cooling cycles between 5^{°}C and 50^{°}C at a rate of 0.1^{°}C/min, while suspended under magnetic stirring at a speed of 300-400 rpm, and the temperature at sampling was 10^{°}C. The resulting suspension was subjected to centrifugal filtration at 14,000 rpm using a 0.45 µm nylon-membrane centrifuge tube. The resulting solid was the hydrochloride crystal form F of the compound of Formula I-1.

23.15: Approximately 40 mg of the hydrochloride crystal form E1 of the compound of Formula I-1 was weighed, and 0.1-0.5 mL of acetonitrile was added. The mixture was subjected to 10 heating-cooling cycles between 5^{°}C and 50^{°}C at a rate of 0.1^{°}C/min, while suspended under magnetic stirring at a speed of 300-400 rpm, and the temperature at sampling was 10^{°}C. The resulting suspension was subjected to centrifugal filtration at 14,000 rpm using a 0.45 µm nylon-membrane centrifuge tube. The resulting solid was the hydrochloride crystal form F of the compound of Formula I-1.

23.16: Approximately 40 mg of the hydrochloride crystal form E1 of the compound of Formula I-1 was weighed, and 0.1-0.5 mL of tetrahydrofuran was added. The mixture was subjected to 10 heating-cooling cycles between 5^{°}C and 50^{°}C at a rate of 0.1^{°}C/min, while suspended under magnetic stirring at a speed of 300-400 rpm, and the temperature at sampling was 10^{°}C. The resulting suspension was subjected to centrifugal filtration at 14,000 rpm using a 0.45 µm nylon-membrane centrifuge tube. The resulting solid was the hydrochloride crystal form F of the compound of Formula I-1.

23.17: Approximately 40 mg of the hydrochloride crystal form E1 of the compound of Formula I-1 was weighed, and 0.1-0.5 mL of methyl isobutyl ketone/trifluoroethanol (9:1, v/v) was added. The mixture was subjected to 10 heating-cooling cycles between 5^{°}C and 50^{°}C at a rate of 0.1^{°}C/min, while suspended under magnetic stirring at a speed of 300-400 rpm, and the temperature at sampling was 10^{°}C. The resulting suspension was subjected to centrifugal filtration at 14,000 rpm using a 0.45 µm nylon-membrane centrifuge tube. The resulting solid was the hydrochloride crystal form F of the compound of Formula I-1.

### Example 24: Preparation of the hydrochloride crystal form G of the compound of Formula I-1

24.1: Approximately 40 mg of the hydrochloride crystal form E1 of the compound of Formula I-1 was weighed, and 0.2-0.5 mL of water was added, and the mixture was suspended under magnetic stirring at 25^{°}C at a speed of 300-400 rpm. The resulting suspension was subjected to centrifugal filtration at 14,000 rpm using a 0.45 µm nylon-membrane centrifuge tube. The resulting solid was the hydrochloride crystal form G of the compound of Formula I-1.

24.2: Approximately 40 mg of the hydrochloride crystal form E1 of the compound of Formula I-1 was weighed, and 0.1-0.5 mL of methanol was added, and the mixture was suspended under magnetic stirring at a speed of 300-400 rpm at 50^{°}C. The resulting suspension was subjected to centrifugal filtration at 14,000 rpm using a 0.45 µm nylon-membrane centrifuge tube. The resulting solid was the hydrochloride crystal form G of the compound of Formula I-1.

24.3: Approximately 40 mg of the hydrochloride crystal form E1 of the compound of Formula I-1 was weighed, and 0.1-0.5 mL of methyl isobutyl ketone/trifluoroethanol (9:1, v/v) was added. The mixture was subjected to 10 heating-cooling cycles between 5^{°}C and 50^{°}C at a rate of 0.1^{°}C/min, while suspended under magnetic stirring at a speed of 300-400 rpm, and the temperature at sampling was 10^{°}C. The resulting suspension was subjected to centrifugal filtration at 14,000 rpm using a 0.45 µm nylon-membrane centrifuge tube. The resulting solid was the hydrochloride crystal form G of the compound of Formula I-1.

### Example 25: Preparation of the hydrochloride crystal form H of the compound of Formula I-1

25.1: Approximately 40 mg of the hydrochloride crystal form E1 of the compound of Formula I-1 was weighed, and 0.2-0.5 mL of methanol/water (1:1, v/v) was added, and the mixture was suspended under magnetic stirring at a speed of 300-400 rpm at 25^{°}C. The resulting suspension was subjected to centrifugal filtration at 14,000 rpm using a 0.45 µm nylon-membrane centrifuge tube. The resulting solid was the hydrochloride crystal form H of the compound of Formula I-1.

25.2: Approximately 40 mg of the hydrochloride crystal form E1 of the compound of Formula I-1 was weighed, and 0.1-0.5 mL of methanol/water (1:1, v/v) was added, and the mixture was suspended under magnetic stirring at a speed of 300-400 rpm at 50^{°}C. The resulting suspension was subjected to centrifugal filtration at 14,000 rpm using a 0.45 µm nylon-membrane centrifuge tube. The resulting solid was the hydrochloride crystal form H of the compound of Formula I-1.

### Example 26: Preparation of the hydrochloride crystal form I of the compound of Formula I-1

26.1: Approximately 40 mg of the hydrochloride crystal form E1 of the compound of Formula I-1 was weighed, and 0.1-0.5 mL of methanol was added, and the mixture was suspended under magnetic stirring at a speed of 300-400 rpm at 50^{°}C. The resulting suspension was subjected to centrifugal filtration at 14,000 rpm using a 0.45 µm nylon-membrane centrifuge tube. The resulting solid was the hydrochloride crystal form I of the compound of Formula I-1.

26.2: Approximately 40 mg of the hydrochloride crystal form E1 of the compound of Formula I-1 was weighed, and the minimum volume of methanol was added to dissolve it thoroughly at 25^{°}C. The resulting dilute suspension was filtered through a 0.45 µm nylon filter cartridge to obtain a clear solution. 0.8 mL of each clear solution was taken, and 8 mL of methyl tert-butyl ether was added slowly to each. The resulting suspension was subjected to centrifugal filtration at 14,000 rpm using a 0.45 µm nylon-membrane centrifuge tube. The resulting solid was the hydrochloride crystal form I of the compound of Formula I-1.

### Example 27: Preparation of the hydrochloride crystal form J1 of the compound of Formula I-1

27.1: Approximately 40 mg of the hydrochloride crystal form E1 of the compound of Formula I-1 was weighed, and 0.1-0.5 mL of 2-methyl-tetrahydrofuran was added, and the mixture was suspended under magnetic stirring at a speed of 300-400 rpm at 50^{°}C. The resulting suspension was subjected to centrifugal filtration at 14,000 rpm using a 0.45 µm nylon-membrane centrifuge tube. The resulting solid was the hydrochloride crystal form J1 of the compound of Formula I-1.

27.2: Approximately 40 mg of the hydrochloride crystal form E1 of the compound of Formula I-1 was weighed, and 0.1-0.5 mL of 2-methyl tetrahydrofuran was added. The mixture was subjected to 10 heating-cooling cycles between 5^{°}C and 50^{°}C at a rate of 0.1^{°}C/min, while suspended under magnetic stirring at a speed of 300-400 rpm, and the temperature at sampling was 10^{°}C. The resulting suspension was subjected to centrifugal filtration at 14,000 rpm using a 0.45 µm nylon-membrane centrifuge tube. The resulting solid was the hydrochloride crystal form J1 of the compound of Formula I-1.

### Example 28: Preparation of the hydrochloride crystal form J2 of the compound of Formula I-1

The sample of the hydrochloride crystal form J1 of the compound of Formula I-1 was heated to 110^{°}C to obtain a solid sample of the hydrochloride crystal form J2 of the compound of Formula I-1.

### Example 29: Preparation of the hydrochloride crystal form K of the compound of Formula I-1

29.1: Approximately 40 mg of the hydrochloride crystal form E1 of the compound of Formula I-1 was weighed, and 0.1-0.5 mL of methyl tert-butyl ether was added, and the mixture was suspended under magnetic stirring at 50^{°}C at a speed of 300-400 rpm. The resulting suspension was subjected to centrifugal filtration at 14,000 rpm using a 0.45 µm nylon-membrane centrifuge tube. The resulting solid was the hydrochloride crystal form K of the compound of Formula I-1.

29.2: Approximately 40 mg of the hydrochloride crystal form E1 of the compound of Formula I-1 was weighed, and 0.1-0.5 mL of methyl tert-butyl ether was added. The mixture was subjected to 10 heating-cooling cycles between 5^{°}C and 50^{°}C at a rate of 0.1^{°}C/min, while suspended under magnetic stirring at a speed of 300-400 rpm, and the temperature at sampling was 10^{°}C. The resulting suspension was subjected to centrifugal filtration at 14,000 rpm using a 0.45 µm nylon-membrane centrifuge tube. The resulting solid was the hydrochloride crystal form K of the compound of Formula I-1.

29.3: Approximately 40 mg of the hydrochloride crystal form E1 of the compound of Formula I-1 was weighed, and 0.1-0.5 mL of toluene was added. The mixture was subjected to 10 heating-cooling cycles between 5^{°}C and 50^{°}C at a rate of 0.1^{°}C/min, while suspended under magnetic stirring at a speed of 300-400 rpm, and the temperature at sampling was 10^{°}C. The resulting suspension was subjected to centrifugal filtration at 14,000 rpm using a 0.45 µm nylon-membrane centrifuge tube. The resulting solid was the hydrochloride crystal form K of the compound of Formula I-1.

### Example 30: Preparation of the hydrochloride crystal form L of the compound of Formula I-1

30.1: Approximately 40 mg of the hydrochloride crystal form E1 of the compound of Formula I-1 was weighed, and 0.1-0.5 mL of toluene was added. The mixture was suspended under magnetic stirring at 25^{°}C at a speed of 300-400 rpm. The resulting suspension was subjected to centrifugal filtration at 14,000 rpm using a 0.45 µm nylon-membrane centrifuge tube. The resulting solid was the hydrochloride crystal form L of the compound of Formula I-1.

30.2: Approximately 40 mg of the hydrochloride crystal form E1 of the compound of Formula I-1 was weighed, and 0.1-0.5 mL of toluene was added. The mixture was suspended under magnetic stirring at 50^{°}C at a speed of 300-400 rpm. The resulting suspension was

subjected to centrifugal filtration at 14,000 rpm using a 0.45 µm nylon-membrane centrifuge tube. The resulting solid was the hydrochloride crystal form L of the compound of Formula I-1.

### Example 31: Preparation of the hydrochloride crystal form M of the compound of Formula I-1

31.1: Approximately 40 mg of the hydrochloride crystal form E1 of the compound of Formula I-1 was weighed, and 0.1-0.5 mL of isopropanol/dimethyl sulfoxide (9:1, v/v) was added. The mixture was suspended under magnetic stirring at a speed of 300-400 rpm at 25^{°}C. The resulting suspension was subjected to centrifugal filtration at 14,000 rpm using a 0.45 µm nylon-membrane centrifuge tube. The resulting solid was the hydrochloride crystal form M of the compound of Formula I-1.

31.2: Approximately 40 mg of the hydrochloride crystal form E1 of the compound of Formula I-1 was weighed, and 0.1-0.5 mL of isopropyl acetate/dimethyl sulfoxide (9:1, v/v) was added. The mixture was suspended under magnetic stirring at a speed of 300-400 rpm at 25^{°}C. The resulting suspension was subjected to centrifugal filtration at 14,000 rpm using a 0.45 µm nylon-membrane centrifuge tube. The resulting solid was the hydrochloride crystal form M of the compound of Formula I-1.

31.3: Approximately 40 mg of the hydrochloride crystal form E1 of the compound of Formula I-1 was weighed, and 0.1-0.5 mL of dimethyl sulfoxide/water (1/1, v/v) was added. The mixture was suspended under magnetic stirring at a speed of 300-400 rpm at 25^{°}C. The resulting suspension was subjected to centrifugal filtration at 14,000 rpm using a 0.45 µm nylon-membrane centrifuge tube. The resulting solid was the hydrochloride crystal form M of the compound of Formula I-1.

31.4: Approximately 40 mg of the hydrochloride crystal form E1 of the compound of Formula I-1 was weighed, and 0.1-0.5 mL of isopropanol/dimethyl sulfoxide (9:1, v/v) was added. The mixture was suspended under magnetic stirring at a speed of 300-400 rpm at 50^{°}C. The resulting suspension was subjected to centrifugal filtration at 14,000 rpm using a 0.45 µm nylon-membrane centrifuge tube. The resulting solid was the hydrochloride crystal form M of the compound of Formula I-1.

31.5: Approximately 40 mg of the hydrochloride crystal form E1 of the compound of Formula I-1 was weighed, and 0.1-0.5 mL of isopropyl acetate/dimethyl sulfoxide (9:1, v/v) was added. The mixture was suspended under magnetic stirring at a speed of 300-400 rpm at 50^{°}C. The resulting suspension was subjected to centrifugal filtration at 14,000 rpm using a 0.45 µm nylon-membrane centrifuge tube. The resulting solid was the hydrochloride crystal form M of the compound of Formula I-1.

31.6: Approximately 40 mg of the hydrochloride crystal form E1 of the compound of Formula I-1 was weighed, and 0.1-0.5 mL of dimethyl sulfoxide/water (1/1, v/v) was added. The mixture was suspended under magnetic stirring at a speed of 300-400 rpm at 50^{°}C. The resulting suspension was subjected to centrifugal filtration at 14,000 rpm using a 0.45 µm nylon-membrane centrifuge tube. The resulting solid was the hydrochloride crystal form M of the compound of Formula I-1.

31.7: Approximately 40 mg of the hydrochloride crystal form E1 of the compound of Formula I-1 was weighed, and 0.1-0.5 mL of isopropanol/dimethyl sulfoxide (9:1, v/v) was added. The mixture was subjected to 10 heating-cooling cycles between 5^{°}C and 50^{°}C at a rate of 0.1^{°}C/min, while suspended under magnetic stirring at a speed of 300-400 rpm, and the temperature at sampling was 10^{°}C. The resulting suspension was subjected to centrifugal filtration at 14,000 rpm using a 0.45 µm nylon-membrane centrifuge tube. The resulting solid was the hydrochloride crystal form M of the compound of Formula I-1.

31.8: Approximately 40 mg of the hydrochloride crystal form E1 of the compound of Formula I-1 was weighed, and 0.1-0.5 mL of isopropyl acetate/dimethyl sulfoxide (9:1, v/v) was added. The mixture was subjected to 10 heating-cooling cycles between 5^{°}C and 50^{°}C at a rate of 0.1^{°}C/min, while suspended under magnetic stirring at a speed of 300-400 rpm, and the temperature at sampling was 10^{°}C. The resulting suspension was subjected to centrifugal filtration at 14,000 rpm using a 0.45 µm nylon-membrane centrifuge tube. The resulting solid was the hydrochloride crystal form M of the compound of Formula I-1.

31.9: Approximately 40 mg of the hydrochloride crystal form E1 of the compound of Formula I-1 was weighed, and 0.1-0.5 mL of dimethyl sulfoxide/water (1:1, v/v) was added. The mixture was subjected to 10 heating-cooling cycles between 5^{°}C and 50^{°}C at a rate of 0.1^{°}C/min, while suspended under magnetic stirring at a speed of 300-400 rpm, and the temperature at sampling was 10^{°}C. The resulting suspension was subjected to centrifugal filtration at 14,000 rpm using a 0.45 µm nylon-membrane centrifuge tube. The resulting solid was the hydrochloride crystal form M of the compound of Formula I-1.

### Example 32: Preparation of the hydrochloride crystal form N of the compound of Formula I-1

32.1: Approximately 40 mg of the hydrochloride crystal form E1 of the compound of Formula I-1 was weighed, and 0.2-0.5 mL of methanol was added. The mixture was suspended under magnetic stirring at a speed of 300-400 rpm at 25^{°}C. The resulting suspension was subjected to centrifugal filtration at 14,000 rpm using a 0.45 µm nylon-membrane centrifuge tube. The resulting solid was the hydrochloride crystal form N of the compound of Formula I-1.

32.2: Approximately 40 mg of the hydrochloride crystal form E1 of the compound of Formula I-1 was weighed, and 0.1-0.5 mL of methanol was added. The mixture was subjected to 10 heating-cooling cycles between 5^{°}C and 50^{°}C at a rate of 0.1^{°}C/min, while suspended under magnetic stirring at a speed of 300-400 rpm, and the temperature at sampling was 10^{°}C. The resulting suspension was subjected to centrifugal filtration at 14,000 rpm using a 0.45 µm nylon-membrane centrifuge tube. The resulting solid was the hydrochloride crystal form N of the compound of Formula I-1.

### Example 33: Preparation of the hydrochloride crystal form O of the compound of Formula I-1

Approximately 40 mg of the hydrochloride crystal form E1 of the compound of Formula I-1 was weighed, and 0.2-0.5 mL of dichloromethane was added. The mixture was suspended under magnetic stirring at a speed of 300-400 rpm at 25^{°}C. The resulting suspension was subjected to centrifugal filtration at 14,000 rpm using a 0.45 µm nylon-membrane centrifuge tube. The resulting solid was the hydrochloride crystal form O of the compound of Formula I-1.

### Experimental Example 1: Solid Stability and Solubility of the Hydrochloride Crystal Form I of the Compound of Formula I-1

### 1.1 Solid Stability of the Hydrochloride Crystal Form I of the Compound of Formula I-1

An open container containing the hydrochloride crystal form I of the compound of formula I-1 was placed at 25^{°}C/60% RH for one week. A sealed container containing the hydrochloride crystal form I of the compound of formula I-1 was placed at 60^{°}C for one week. Stability samples under these conditions were subjected to XRPD, HPLC, and stoichiometric ratio testing, and visual observation was performed for color changes of the samples.

**Table 33 Solid Stability of Hydrochloride Crystal Form I of the Compound of Formula I-1**

| Experiment No. 1 | Initial Purity | 99.6% | |
|---|---|---|---|
| | Initial stoichiometric ratio (IC-HPLC) | Compound of formula (I): HCl = 1:0.97 | |
| | | Purity | Color |
| | Solid, 25^{°}C/60% RH, open container, 1 week | | |
| | Solid (HPLC) | 99.5% | No color change |
| | Stoichiometric ratio (IC-HPLC) | Compound of formula (I): HCl = 1:0.99 | |
| | Solid (XRPD) | Hydrochloride crystal form I (no change) | |

| 2 | Solid, 60^{°}C, sealed container, 1 week | | |
|---|---|---|---|
| | Solid (HPLC) | 99.6% | No color change |
| | Stoichiometric ratio (IC-HPLC) | Compound of formula (I): HCl = 1:0.98 | |
| | Solid (XRPD) | Hydrochloride crystal form I (no change) | |

### 1.2 Solubility of the Free-base Crystal Form A of the Compound of Formula I-1

10 mg of the free-base crystal form A of the compound of formula I-1 or 10.8 mg (equivalent to 10 mg of the free-base anhydrous form) of the hydrochloride crystal form I of the compound of formula I-1 was weighed and placed in an 8 mL glass vial. 5 mL of dissolution medium was added. The resulting suspension/clear solution was stirred at 37^{°}C and 400 rpm for 2 h, 8 h, and 24 h, then the resulting suspension/clear solution was centrifuged at 37^{°}C and 14,000 rpm for 5 min. The solubility of the supernatant was determined by HPLC, and the pH of the supernatant was measured by a pH meter, and the residual solid was detected by XRPD.

**Table 34 Solubility testing of the free-base crystal form A of compound of formula I-1**

| Method | Solubility at 37^{°}C, target concentration of 2 mg/mL (calculated as free-base form), equilibrated for 24 h, LOQ: 0.0003 mg/mL | | | |
|---|---|---|---|---|
| Experiment No. | Dissolution Medium | Solubility (mg/mL) | | Residual solid XRPD |
| | | 2 h | 24 h (pH) | |
| 1 | Water | 0.0012 | 0.0013 (7.72) | Free-base form A |
| 2 | FaSSGF, pH 1.6 | 0.38 | 0.42 (1.55) | Free-base form A |
| 3 | FaSSIF-v1, pH 6.5 | 0.0004 | 0.0011 (6.50) | Free-base form A |
| 4 | FeSSIF-v1, pH 5.0 | 0.0045 | 0.0066 (4.97) | Free-base form A |

**Table 35 Solubility testing of the hydrochloride crystal form I of the compound of Formula I-1**

| Method | Solubility at 37^{°}C, target concentration of 2 mg/mL (calculated as free-base form), equilibrated for 24 h, LOQ: 0.0002 mg/mL | | | | |
|---|---|---|---|---|---|
| Experiment No. | Dissolution Medium | Solubility (mg/mL) | | | Residual solid XRPD |
| | | 2 h | 8h | 24 h (pH) | |
| 1 | Water | 0.12 | 0.10 | 0.10 (2.55) | Free-base form A |
| 2 | FaSSGF, pH 1.6 | ≥2.0 | ≥2.0 | 1.7 (1.56) | - |
| 3 | FaSSIF-v1, pH 6.5 | 0.001 | 0.001 | 0.002 (6.18) | Free-base form A |
| 4 | FeSSIF-v1, pH 5.0 | 0.015 | 0.015 | 0.015 (4.90) | Free-base form A |

Conclusion: The hydrochloride crystal form I of the compound of Formula I-1 exhibited no change in total impurities or crystal form, showing favorable physical stability. Moreover, compared with the free-base crystal form of the compound of formula I-1, hydrochloride crystal form I of the compound of Formula I-1 displayed significantly improved solubility in all four media tested.

Experiment Example 2: FGFR2 receptor activity inhibition assay for the hydrochloride crystal form I

### 2.3 Assessment of the activity of hydrochloride crystal form I of the compound of Formula I-1 on FGFR2 background cells

This experiment investigates the compound's ability to inhibit cell proliferation by assessing its effect on in vitro cell viability in seven tumor cell lines (KATO III, NCI-H716, SNU-16, AN3CA, MFE-296, SUM52PE, MFM223).

The cell lines were cultured in an incubator at 37^{°}C and 5% CO₂ under standard conditions, passaged periodically, and cells in the logarithmic growth phase were used for plating. The test compounds were prepared as 10 mM solutions using DMSO.

Preparation of the compound storage plate (tube): A 4-fold serial dilution with DMSO was set up from the highest to the lowest concentration to generate a total of 9 concentrations, and the internal reference compound was likewise subjected to 4-fold serial dilution to generate another 9 concentrations. Next, the compound working solutions were prepared as follows: 98 µL of cell culture medium was added to each well of a flat-bottomed 96-well transparent plate, and 2 µL of compound was pipetted from the compound storage plate to the cell culture medium in the 96-well plate. 2 µL of DMSO was added to the solvent control well. After the compound or DMSO was added, it was mixed thoroughly by pipetting.

Cell seeding and drug administration: 1) Cells were stained with trypan blue and live cells were counted, cell viability must be ≥90%; 2) The cell concentration was adjusted to an appropriate level; 3) 95 µL of cell suspension was added to each well of the compound assay plate (6,000-10,000 cells/well), and cell-free medium (containing 0.1% DMSO) was added to the Min control wells; 4) Compound administration in the compound assay plate: 5 µL of a 20× compound stock solution was added to the cell culture plate as shown in Table 1. 5 µL of a DMSO-cell culture medium mixture was added to the Max control well. The final DMSO concentration was 0.1%; 5) The culture plate was incubated in a 37^{°}C, 5% CO₂ incubator for 72-96 hours.

The assay was performed according to the instructions for the Promega CellTiter-Glo Luminescent Cell Viability Assay Kit (Promega-G7573): 1) CellTiter-Glo buffer was thawed and equilibrated to room temperature; 2) CellTiter-Glo substrate was equilibrated to room temperature; 3) CellTiter-Glo buffer was added to one bottle of CellTiter-Glo substrate to dissolve the substrate, thereby the CellTiter-Glo working solution was prepared; 4) Slow vortex was performed to ensure complete dissolution; 5) The cell culture plate was taken and placed for 10 minutes to be equilibrated to room temperature; 6) 50 µL of CellTiter-Glo working solution (equivalent to half the volume of the cell culture medium in each well) was added to each well; 7) The plate was shaken on a shaking incubator for 2 minutes to induce cell lysis; 8) The plate was placed at room temperature for 10 minutes to stabilize the luminescence signal; 9) The luminescence signal was measured using a SpectraMax Paradigm reader.

The cell proliferation inhibition rate data is calculated using the following formula: $Inhibition Rate \left(Inh%\right) = 100 - \left({RLU}_{Drug}-{RLU}_{Min}\right) / \left({RLU}_{Max}-{RLU}_{Min}\right) \times 100 % .$

The inhibition rates corresponding to different compound concentrations was calculated in Excel, then GraphPad Prism software was used to plot inhibition rate curves and the relevant parameters were calculated, including the maximum and minimum inhibition rates, IC₅₀ value.

**Table 36 Results of the inhibitory effects of test compounds on the proliferation of FGFR2 background cell lines**

| Cell Line | Hydrochloride crystal form I of compound of Formula I-1 |
|---|---|
| KATO III | 0.8 |
| NCI-H716 | 2.4 |
| SNU-16 | 3.0 |
| AN3CA | 9.9 |
| MFE-296 | 14.9 |
| SUM52PE | 6.2 |
| MFM223 | 1.0 |

As shown in Table 36, the compounds of the present invention exhibit good inhibitory effects on cell proliferation of FGFR2 background cells.

All documents mentioned in the present invention are cited herein as references, as if each document were individually cited as a reference. Furthermore, it should be understood that, after reading the description of the present invention, those skilled in the art may make various changes or modifications to the present invention, and such equivalent forms also fall within the scope defined by the appended claims.

## Claims

1. A compound of formula (I), 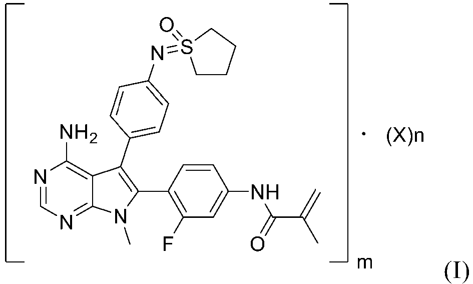
in an amorphous form or a crystal form or a solvate thereof;
wherein m is 1, 2, 3, 4, 5, 6, 7, 8, or 9;
n is 0, 0.5, 1, 1.5, 2, 2.5, or 3; and X is selected from the group consisting of: hydrochloric acid, hydrobromic acid, hydrofluoric acid, sulfuric acid, nitric acid, or phosphoric acid, formic acid, acetic acid, trifluoroacetic acid, propionic acid, oxalic acid, malonic acid, succinic acid, fumaric acid, maleic acid, lactic acid, malic acid, tartaric acid, citric acid, picric acid, benzoic acid, methanesulfonic acid, ethanesulfonic acid, p-toluenesulfonic acid, benzenesulfonic acid, naphthalenesulfonic acid, hippuric acid, glycolic acid, or glutaric acid.

2. A crystal form A of the compound of formula (I) according to claim 1, wherein, the compound of formula (I) is I-1, n = 0, and the X-ray powder diffraction pattern of the crystal form A has 2θ angles selected from the group consisting of: 14.16 ± 0.2°, 16.74 ± 0.2°, and 23.01 ± 0.2^{°}.
In another preferred embodiment, the crystal form A further comprises one or more of the following characteristics:
a. the X-ray powder diffraction pattern of the crystal form A further has one or more (e.g., 1, 2, 3, 4, 5, 6, 7, 8, 9, or 10) 2θ angles selected from the group consisting of: 8.44±0.2^{°}, 11.59±0.2^{°}, 12.00±0.2^{°}, 12.34 ± 0.2^{°}, 15.50 ± 0.2^{°}, 16.50 ± 0.2^{°}, 18.38 ± 0.2^{°}, 18.81 ± 0.2^{°}, 19.79 ± 0.2 ^{°}, 20.44 ± 0.2^{°}, 21.32±0.2^{°}, 21.53±0.2^{°}, 21.80±0.2^{°}, 22.24±0.2^{°}, 24.37±0.2^{°}, 25.43±0.2^{°}, 26.50±0.2^{°}, 27.13±0.2^{°}, 27.74±0.2^{°}, 28.90±0.2^{°}, 29.72±0.2^{°}, 29.93±0.2^{°}, 30.83±0.2^{°}, 31.56 ± 0.2 ^{°}, 32.47 ± 0.2^{°}, 33.64 ± 0.2^{°}, 34.12 ± 0.2 °, 35.03 ± 0.2^{°}, 36.17 ± 0.2^{°};
b. the differential scanning calorimetry curve of the crystal form A has the onsets of the endothermic peaks at 26.10^{°}C ± 2^{°}C and 223.34^{°}C ± 2^{°}C;
c. the thermogravimetric analysis curve of the crystal form A has three small weight loss steps at 24.00^{°}C ± 2^{°}C, 110.00^{°}C ± 2^{°}C, and 200.00^{°}C ± 2^{°}C, and decomposition begins above 250.00^{°}C ± 2^{°}C.
In another preferred embodiment, the crystal form A has an X-ray powder diffraction pattern substantially as shown in Figure 1.
In another preferred embodiment, the crystal form A has a differential scanning calorimetry pattern substantially as shown in Figure 2.
In another preferred embodiment, the crystal form A has a thermogravimetric analysis pattern substantially as shown in Figure 3.

3. A method for preparing the crystal form A according to claim 2, wherein, the method comprises the following steps:
a. dissolving or dispersing compound I-1 in acetonitrile, an alcoholic solvent, an ester solvent, an ether solvent, or a mixed solvent of the alcoholic solvent and water at a volume ratio of 1-5 times;
b. performing recrystallization or slurrying;
wherein the alcoholic solvent is selected from the group consisting of: methanol, ethanol, isopropanol, or a combination thereof;
the ester solvent is selected from the group consisting of: ethyl acetate, isopropyl acetate, methyl formate, ethyl formate, isopropyl formate, or a combination thereof;
the ether solvent is selected from the group consisting of: methyl tert-butyl ether, tetrahydrofuran, ethylene glycol dimethyl ether, or a combination thereof;
the mixed solvent of the alcoholic solvent and water is selected from the group consisting of: a mixture of methanol and water, a mixture of ethanol and water, or a mixture of isopropanol and water; wherein the volume ratio of the alcoholic solvent to water is 1: (0.1-1.5).

4. A crystal form of the compound of formula (I) according to claim 1, wherein, the compound of formula (I) is a salt formed by compound I-1 and an acid; wherein the acid is an inorganic acid or an organic acid;
preferably, the inorganic acid is selected from the group consisting of: hydrochloric acid, hydrobromic acid, hydrofluoric acid, sulfuric acid, nitric acid, or phosphoric acid;
preferably, the organic acid is selected from the group consisting of: formic acid, acetic acid, trifluoroacetic acid, propionic acid, oxalic acid, malonic acid, succinic acid, fumaric acid, maleic acid, lactic acid, malic acid, tartaric acid, citric acid, picric acid, benzoic acid, methanesulfonic acid, ethanesulfonic acid, p-toluenesulfonic acid, benzenesulfonic acid, naphthalenesulfonic acid, hippuric acid, glycolic acid, or glutaric acid;
preferably, the molar ratio of compound I-1 to the acid is 1: (0.8-3); more preferably, 1: (0.8-2).

5. The crystal form according to claim 4, wherein, the crystal form is the maleate crystal form A of the compound of formula I-1, the X-ray powder diffraction pattern of the maleate crystal form A has 2θ angles selected from the group consisting of: 15.17 ± 0.2^{°}, 23.02 ± 0.2 ^{°}, 24.42 ± 0.2^{°}.
In another preferred embodiment, the X-ray powder diffraction pattern of the maleate crystal form A further comprises one or more (e.g., 1, 2, 3, 4, 5, 6, 7, 8, 9, or 10) 2θ angles selected from the group consisting of: 5.82±0.2^{°}, 9.86±0.2^{°}, 11.06 ± 0.2 ^{°}, 11.29 ± 0.2 ^{°}, 11.64 ± 0.2 ^{°}, 11.90 ± 0.2 ^{°}, 12.72 ± 0.2 ^{°}, 13.04 ± 0.2 ^{°}, 15.66 ± 0.2^{°}, 16.36±0.2^{°}, 17.70±0.2^{°}, 17.88±0.2^{°}, 18.11±0.2^{°}, 18.35±0.2^{°}, 18.61±0.2^{°}, 19.41±0.2^{°}, 20.16±0.2^{°}, 20.38±0.2^{°}, 20.69±0.2^{°}, 21.32±0.2^{°}, 21.62±0.2^{°}, 22.28±0.2^{°}, 22.71±0.2^{°}, 25.00±0.2^{°}, 25.34±0.2^{°}, 26.24±0.2^{°}, 26.77±0.2^{°}, 29.85±0.2^{°}, 34.09±0.2^{°}.
In another preferred embodiment, the maleate crystal form A has an X-ray powder diffraction pattern substantially as shown in Figure 4.

6. The crystal form according to claim 4, wherein, the crystal form is the monomaleate crystal form B of the compound of Formula I-1, the X-ray powder diffraction pattern of the monomaleate crystal form B has 2θ angles selected from the group consisting of: 16.57±0.2^{°}, 17.38 ± 0.2^{°}, and 20.39 ± 0.2^{°}.
In another preferred embodiment, the monomaleate crystal form B further comprises one or more of the following characteristics:
a. the X-ray powder diffraction pattern of the monomaleate crystal form B further has one or more (e.g., 1, 2, 3, 4, 5, 6, 7, 8, 9, or 10) 2θ angles selected from the group consisting of: 7.98±0.2^{°}, 8.29±0.2^{°}, 10.27±0.2^{°}, 11.61 ± 0.2 ^{°}, 13.21 ± 0.2 ^{°}, 13.99 ± 0.2 ^{°}, 14.52 ± 0.2 ^{°}, 15.25 ± 0.2 ^{°}, 15.95 ± 0.2 ^{°}, 16.89 ± 0.2^{°}, 18.06±0.2^{°}, 20.61±0.2^{°}, 21.25±0.2^{°}, 21.61±0.2^{°}, 22.17±0.2^{°}, 23.31±0.2^{°}, 23.54±0.2^{°}, 23.98±0.2^{°}, 24.70±0.2^{°}, 24.93±0.2^{°}, 25.32±0.2^{°}, 25.91±0.2^{°}, 26.55±0.2^{°}, 27.24±0.2^{°}, 28.23±0.2^{°}, 28.54±0.2^{°}, 29.00±0.2^{°}, 29.47±0.2^{°}, 29.71±0.2^{°}, 32.64±0.2^{°}, 35.12±0.2^{°};
b. the differential scanning calorimetry curve of the monomaleate crystal form B has the onset of the endothermic peak at 213.92^{°}C ± 2^{°}C;
c. the thermogravimetric analysis curve of the monomaleate crystal form B has two weight loss steps at 24.00^{°}C ± 2^{°}C and 180.00^{°}C ± 2^{°}C, and decomposition begins above 250.00^{°}C ± 2^{°}C.
In another preferred embodiment, the maleate crystal form B has an X-ray powder diffraction pattern substantially as shown in Figure 5.
In another preferred embodiment, the maleate crystal form B has a differential scanning calorimetry pattern substantially as shown in Figure 6.
In another preferred embodiment, the maleate crystal form B has a thermogravimetric analysis pattern substantially as shown in Figure 7.

7. The crystal form according to claim 4, wherein, the crystal form is the fumarate crystal form A of the compound of formula I-1, the X-ray powder diffraction pattern of the fumarate crystal form A has 2θ angles selected from the group consisting of: 5.11±0.2^{°}, 14.23±0.2^{°}, 28.85 ± 0.2^{°}.
In another preferred embodiment, the X-ray powder diffraction pattern of the fumarate crystal form A further has one or more (e.g., 1, 2, 3, 4, 5, 6, 7, 8, 9, or 10) 2θ angles selected from the group consisting of: 7.26±0.2^{°}, 10.20±0.2^{°}, 10.51 ± 0.2 ^{°}, 14.47 ± 0.2 ^{°}, 14.93 ± 0.2^{°}, 16.17 ± 0.2^{°}, 17.03 ± 0.2^{°}, 17.90 ± 0.2^{°}, 18.79 ± 0.2^{°}, 19.44±0.2^{°}, 20.19±0.2^{°}, 20.80±0.2^{°}, 22.48±0.2^{°}, 23.07±0.2^{°}, 23.74±0.2^{°}, 24.07±0.2^{°}, 27.06±0.2^{°}, 27.85±0.2^{°}, 28.30±0.2^{°}, 29.46±0.2^{°}, 38.24±0.2^{°}.
In another preferred embodiment, the fumarate crystal form A has an X-ray powder diffraction pattern substantially as shown in Figure 8.

8. The crystal form according to claim 4, wherein, the crystal form is the hemifumarate crystal form B of the compound of formula I-1, the X-ray powder diffraction pattern of the hemifumarate crystal form B has 2θ angles selected from the group consisting of: 5.12±0.2^{°}, 14.24±0.2^{°}, 10.20±0.2^{°}.
In another preferred embodiment, the hemifumarate crystal form B further comprises one or more of the following characteristics:
a. the X-ray powder diffraction pattern of the hemifumarate crystal form B further comprises one or more (e.g., 1, 2, 3, 4, 5, 6, 7, 8, 9, or 10) 2θ angles selected from the group consisting of: 7.27±0.2^{°}, 7.48±0.2^{°}, 8.77 ± 0.2^{°}, 9.18 ± 0.2^{°}, 9.74 ± 0·2^{°}, 10.53 ± 0.2^{°}, 14.96 ± 0.2^{°}, 16.09 ± 0.2^{°}, 16.99 ± 0.2^{°}, 17.61±0.2^{°}, 17.95±0.2^{°}, 18.81±0.2^{°}, 19.45±0.2^{°}, 20.22±0.2^{°}, 20.43±0.2^{°}, 20.84±0.2^{°}, 22.51±0.2^{°}, 23.11±0.2^{°}, 23.76±0.2^{°}, 24.19±0.2^{°}, 25.24±0.2^{°}, 27.04±0.2^{°}, 27.90±0.2^{°}, 28.25±0.2^{°}, 28.75±0.2^{°}, 29.73±0.2^{°}, 30.14±0.2^{°}, 30.77±0.2^{°}, 31.28±0.2^{°}, 33.95±0.2^{°}, 34.52±0.2^{°}, 37.07±0.2^{°}, 38.26±0.2^{°};
b. the differential scanning calorimetry curve of the hemifumarate crystal form B has the onsets of the endothermic peaks at 21.34^{°}C ± 2^{°}C, 172.21^{°}C ± 2^{°}C, and 234.34^{°}C ± 2^{°}C;
c. the thermogravimetric analysis curve of the hemifumarate crystal form B has three weight loss steps at 31.90^{°}C ± 2^{°}C, 90.00^{°}C ± 2^{°}C, and 190.00^{°}C ± 2^{°}C, and decomposition begins above 270.00^{°}C ± 2^{°}C.
In another preferred embodiment, the hemifumarate crystal form B has an X-ray powder diffraction pattern substantially as shown in Figure 9.
In another preferred embodiment, the hemifumarate crystal form B has a differential scanning calorimetry pattern substantially as shown in Figure 10.
In another preferred embodiment, the hemifumarate crystal form B has a thermogravimetric analysis pattern substantially as shown in Figure 11.

9. The crystal form according to claim 4, wherein, the crystal form is the monoglycolate crystal form A of the compound of formula I-1, the X-ray powder diffraction pattern of the monoglycolate crystal form A has 2θ angles selected from the group consisting of: 18.22±0.2^{°}, 22.83±0.2^{°}, 26.41±0.2^{°}.
In another preferred embodiment, the monoglycolate crystal form A further comprises one or more of the following characteristics:
a. the X-ray powder diffraction pattern of the monoglycolate crystal form A also has one or more (e.g., 1, 2, 3, 4, 5, 6, 7, 8, 9, or 10) 2θ angles selected from the group consisting of: 6.37±0.2^{°}, 10.37±0.2^{°}, 11.09 ± 0.2^{°}, 11.70 ± 0.2^{°}, 12.69 ± 0.2^{°}, 13.55 ± 0.2^{°}, 14.06 ± 0.2^{°}, 15.97 ± 0.2^{°}, 17.34 ± 0.2^{°}, 18.47±0.2^{°}, 19.09±0.2^{°}, 20.41±0.2^{°}, 20.71±0.2^{°}, 21.25±0.2^{°}, 22.06±0.2^{°}, 22.43±0.2^{°}, 23.49±0.2^{°}, 24.09±0.2^{°}, 24.79±0.2^{°}, 25.21±0.2^{°}, 27.23±0.2^{°}, 28.00±0.2^{°}, 29.59±0.2^{°}, 30.44±0.2^{°}, 30.96±0.2^{°}, 38.67±0.2^{°};
b. the differential scanning calorimetry curve of the monoglycolate crystal form A has the onsets of the endothermic peaks at 109.66^{°}C ± 2^{°}C and 198.83^{°}C ± 2^{°}C;
c. the thermogravimetric analysis curve of the monoglycolate crystal form A has three weight loss steps at 33.00^{°}C ± 2^{°}C, 100.00^{°}C ± 2^{°}C, and 165.00^{°}C ± 2^{°}C, and decomposition begins above 200.00^{°}C ± 2^{°}C.
In another preferred embodiment, the monoglycolate crystal form A has an X-ray powder diffraction pattern substantially as shown in Figure 12.
In another preferred embodiment, the monoglycolate crystal form A has a differential scanning calorimetry pattern substantially as shown in Figure 13.
In another preferred embodiment, the monoglycolate crystal form A has a thermogravimetric analysis pattern substantially as shown in Figure 14.

10. The crystal form according to claim 4, wherein, the crystal form is the glycolate crystal form B of the compound of formula I-1, the X-ray powder diffraction pattern of the glycolate crystal form B has 2θ angles selected from the group consisting of: 13.87±0.2^{°}, 17.30±0.2^{°}, 19.49±0.2^{°}, 23.50±0.2^{°}.
In another preferred embodiment, the X-ray powder diffraction pattern of the glycolate crystal form B further has one or more (e.g., 1, 2, 3, 4, 5, 6, 7, 8, 9, or 10) 2θ angles selected from the group consisting of: 5.95±0.2^{°}, 11.06±0.2^{°}, 12.20 ± 0.2^{°}, 15.01 ± 0.2^{°}, 15.79 ± 0.2^{°}, 15.97 ± 0.2^{°}, 16.44 ± 0.2^{°}, 16.63 ± 0.2^{°}, 16.79±0.2^{°}, 17.90±0.2^{°}, 18.33±0.2^{°}, 18.91±0.2^{°}, 21.59±0.2^{°}, 22.18±0.2^{°}, 22.80±0.2^{°}, 23.16±0.2^{°}, 23.93±0.2^{°}, 24.49±0.2^{°}, 25.13±0.2^{°}, 26.68±0.2^{°}, 27.95±0.2^{°}, 30.49±0.2^{°}, 31.62±0.2^{°}, 32.27±0.2^{°}.
In another preferred embodiment, the glycolate crystal form B has an X-ray powder diffraction pattern substantially as shown in Figure 15.

11. The crystal form according to claim 4, wherein, the crystal form is of the compound of formula I-1, the X-ray powder diffraction pattern of the mono-L-malate crystal form A has 2θ angles selected from the group consisting of: 17.99±0.2^{°}, 22.58±0.2^{°}, and 24.00±0.2^{°}.
In another preferred embodiment, the mono-L-malate crystal form A further comprises one or more of the following characteristics:
a. the X-ray powder diffraction pattern of the mono-L-malate crystal form A also has one or more (e.g., 1, 2, 3, 4, 5, 6, 7, 8, 9, or 10) 2θ angles selected from the group consisting of: 6.01±0.2^{°}, 10.06±0.2^{°}, 11.04 ± 0.2^{°}, 11.98 ± 0.2^{°}, 12.66 ± 0.2^{°}, 12.86 ± 0.2^{°}, 13.39 ± 0.2^{°}, 15.15 ± 0.2^{°}, 15.62 ± 0.2^{°}, 16.46±0.2^{°}, 17.27±0.2^{°}, 18.58±0.2^{°}, 19.53±0.2^{°}, 19.77±0.2^{°}, 20.25±0.2^{°}, 21.01±0.2^{°}, 21.57±0.2^{°}, 22.04±0.2^{°}, 23.67±0.2^{°}, 24.50±0.2^{°}, 25.00±0.2^{°}, 27.34±0.2^{°}, 28.54±0.2^{°}, 28.91 ± 0.2 ^{°} , 29.40 ± 0.2 ^{°}, 29.89 ± 0.2 ^{°}, 30.53 ± 0.2^{°}, 31.33 ± 0.2^{°}, 33.67 ± 0.2^{°}, 36.16 ± 0.2^{°};
b. the differential scanning calorimetry curve of the mono-L-malate crystal form A has the onset of the endothermic peak at 204.74^{°}C ± 2^{°}C;
c. the thermogravimetric analysis curve of the mono-L-malate crystal form A has two weight loss steps at 33.00^{°}C ± 2^{°}C and 185.00^{°}C ± 2^{°}C, and decomposition begins at 250.00^{°}C ± 2^{°}C.
In another preferred embodiment, the mono-L-malate crystal form A has an X-ray powder diffraction pattern substantially as shown in Figure 16.
In another preferred embodiment, the mono-L-malate crystal form A has a differential scanning calorimetry pattern substantially as shown in Figure 17.
In another preferred embodiment, the mono-L-malate crystal form A has a thermogravimetric analysis pattern substantially as shown in Figure 18.

12. The crystal form according to claim 4, wherein, the crystal form is the monosuccinate crystal form A of the compound of formula I-1, the X-ray powder diffraction pattern of the monosuccinate crystal form A has 2θ angles selected from the group consisting of: 18.40±0.2^{°}, 24.09 ± 0.2^{°}, and 24.62 ± 0.2^{°}.
In another preferred embodiment, the monosuccinate crystal form A further comprises one or more of the following characteristics:
a. the X-ray powder diffraction pattern of the monosuccinate crystal form A also has one or more (e.g., 1, 2, 3, 4, 5, 6, 7, 8, 9, or 10) 2θ angles selected from the group consisting of: 6.15±0.2^{°}, 9.64±0.2^{°}, 9.95 ± 0.2^{°}, 11.19 ± 0.2^{°}, 12.26 ± 0.2^{°}, 12.70 ± 0.2^{°}, 13.30 ± 0.2^{°}, 14.95 ± 0.2^{°}, 15.25 ± 0.2^{°}, 16.18±0.2^{°}, 17.59±0.2^{°}, 18.89±0.2^{°}, 19.11±0.2^{°}, 19.73±0.2^{°}, 20.28±0.2^{°}, 20.90±0.2^{°}, 21.27±0.2^{°}, 21.64±0.2^{°}, 22.12±0.2^{°}, 23.12±0.2^{°}, 23.83±0.2^{°}, 24.35±0.2^{°}, 24.93±0.2^{°}, 25.17±0.2^{°}, 26.05±0.2^{°}, 27.00±0.2^{°}, 28.27±0.2^{°}, 28.72±0.2^{°}, 30.12±0.2^{°}, 31.20±0.2^{°}, 33.57±0.2^{°}, 34.13±0.2^{°}, 34.58±0.2^{°}, 35.60±0.2^{°}, 38.23±0.2^{°};
b. the differential scanning calorimetry curve of the monosuccinate crystal form A has the onsets of the endothermic peaks at 72.75^{°}C ± 2^{°}C, 146.41^{°}C ± 2^{°}C, and 175.72^{°}C ± 2^{°}C;
c. the thermogravimetric analysis curve of the monosuccinate crystal form A has three weight loss steps at 33.00^{°}C ± 2^{°}C, 90.00^{°}C ± 2^{°}C, and 160.00^{°}C ± 2^{°}C, and decomposition begins above 260.00^{°}C ± 2^{°}C.
In another preferred embodiment, the monosuccinate crystal form A has an X-ray powder diffraction pattern substantially as shown in Figure 19.
In another preferred embodiment, the monosuccinate crystal form A has a differential scanning calorimetry pattern substantially as shown in Figure 20.
In another preferred embodiment, the monosuccinate crystal form A has a thermogravimetric analysis pattern substantially as shown in Figure 21.

13. The crystal form according to claim 4, wherein, the crystal form is sulfate crystal form A of the compound of formula I-1, the X-ray powder diffraction pattern of sulfate crystal form A has 2θ angles selected from the group consisting of: 14.97±0.2^{°}, 20.21±0.2^{°}, 22.58 ± 0.2^{°}.
In another preferred embodiment, the X-ray powder diffraction pattern of the sulfate crystal form A further comprises one or more (e.g., 1, 2, 3, 4, 5, 6, 7, 8, 9, or 10) 2θ angles selected from the group consisting of: 6.74±0.2^{°}, 9.56±0.2^{°}, 12.89 ± 0.2^{°}, 13.78 ± 0.2^{°}, 14.68 ± 0.2^{°}, 15.49 ± 0.2^{°}, 15.70 ± 0.2^{°}, 16.41 ± 0.2^{°}, 16.90 ± 0.2^{°}, 18.47±0.2^{°}, 18.68±0.2^{°}, 18.86±0.2^{°}, 19.33±0.2^{°}, 19.87±0.2^{°}, 20.70±0.2^{°}, 21.54±0.2^{°}, 21.76±0.2^{°}, 22.25±0.2^{°}, 23.75±0.2^{°}, 24.11±0.2^{°}, 24.50±0.2^{°}, 25.25±0.2^{°}, 25.86±0.2^{°}, 26.10±0.2^{°}, 26.71±0.2^{°}, 26.96±0.2^{°}, 27.73±0.2^{°}, 28.41±0.2^{°}, 28.90±0.2^{°}, 29.70±0.2^{°}, 30.21±0.2^{°}, 30.99±0.2^{°}.
In another preferred embodiment, the sulfate crystal form A has an X-ray powder diffraction pattern substantially as shown in Figure 22.

14. The crystal form according to claim 4, wherein, the crystal form is the L-tartrate crystal form A of the compound of Formula I-1, the X-ray powder diffraction pattern of the L-tartrate crystal form A has 2θ angles selected from the group consisting of: 5.53±0.2^{°}, 16.95±0.2^{°}, 20.79±0.2^{°}.
In another preferred embodiment, the X-ray powder diffraction pattern of L-tartarate crystal form A further has one or more (e.g., 1, 2, 3, 4, 5, 6, 7, 8, 9, or 10) 2θ angles selected from the group consisting of: 8.90±0.2^{°}, 9.35±0.2^{°}, 11.05±0.2^{°}, 12.54±0.2^{°}, 12.90±0.2^{°}, 14.35±0.2^{°}, 14.64±0.2^{°}, 18.70±0.2^{°}, 19.45±0.2^{°}, 19.74±0.2^{°}, 20.38±0.2^{°}, 21.45±0.2^{°}, 21.94±0.2^{°}, 22.88±0.2^{°}, 23.80±0.2^{°}, 25.01±0.2^{°}, 25.77±0.2^{°}, 27.33±0.2^{°}, 27.80±0.2^{°}, 28.65±0.2^{°}, 29.41±0.2^{°}, 30.00±0.2^{°}, 31.09±0.2^{°}.
In another preferred embodiment, the L-tartarate crystal form A has an X-ray powder diffraction pattern substantially as shown in Figure 23.

15. The crystal form according to claim 4, wherein, the crystal form is the hippurate crystal form A of the compound of Formula I-1, the X-ray powder diffraction pattern of the hippurate crystal form A has 2θ angles selected from the group consisting of: 6.17±0.2^{°}, 12.30 ± 0.2^{°}, and 18.78 ± 0.2^{°}.
In another preferred embodiment, the X-ray powder diffraction pattern of the hippurate crystal form A further has one or more (e.g., 1, 2, 3, 4, 5, 6, 7, 8, 9, or 10) 2θ angles selected from the group consisting of: 6.52±0.2^{°}, 8.77±0.2^{°}, 10.74±0.2^{°}, 11.16±0.2^{°}, 14.18±0.2^{°}, 14.70±0.2^{°}, 15.62±0.2^{°}, 16.06±0.2^{°}, 16.78±0.2^{°}, 17.64±0.2^{°}, 20.42±0.2^{°}, 21.15±0.2^{°}, 21.29±0.2^{°}, 21.79±0.2^{°}, 23.03±0.2^{°}, 24.05±0.2^{°}, 24.42±0.2^{°}, 25.32±0.2^{°}, 26.45±0.2^{°}, 26.87±0.2^{°}, 28.85±0.2^{°}.
In another preferred embodiment, the hippurate crystal form A has an X-ray powder diffraction pattern substantially as shown in Figure 24.

16. The crystal form according to claim 4, wherein, the crystal form is the glutarate crystal form A of the compound of Formula I-1, the X-ray powder diffraction pattern of the glutarate crystal form A has 2θ angles selected from the group consisting of: 17.82±0.2^{°}, 23.20 ± 0.2^{°}, and 24.47 ± 0.2^{°}.
In another preferred embodiment, the X-ray powder diffraction pattern of the glutarate crystal form A further has one or more (e.g., 1, 2, 3, 4, 5, 6, 7, 8, 9, or 10) 2θ angles selected from the group consisting of: 9.86±0.2^{°}, 10.97±0.2^{°}, 11.82 ± 0.2^{°}, 12.29 ± 0.2^{°}, 12.75 ± 0.2^{°}, 13.19 ± 0.2^{°}, 14.87 ± 0.2^{°}, 15.10 ± 0.2^{°}, 15.37 ± 0.2^{°}, 16.33±0.2^{°}, 17.10±0.2^{°}, 18.35±0.2^{°}, 19.17±0.2^{°}, 19.75±0.2^{°}, 20.14±0.2^{°}, 20.70±0.2^{°}, 21.14±0.2^{°}, 22.23±0.2^{°}, 24.26±0.2^{°}, 24.86±0.2^{°}, 25.28±0.2^{°}, 29.92±0.2^{°}.
In another preferred embodiment, the glutarate crystal form A has an X-ray powder diffraction pattern substantially as shown in Figure 25.

17. The crystal form according to claim 4, wherein, the crystal form is the p-toluenesulfonate crystal form A of the compound of Formula I-1, the X-ray powder diffraction pattern of the p-toluenesulfonate crystal form A has 2θ angles selected from the group consisting of: 19.12±0.2^{°}, 20.11 ± 0.2^{°}, and 20.53 ± 0.2^{°}.
In another preferred embodiment, the X-ray powder diffraction pattern of the p-toluenesulfonate crystal form A further has one or more (e.g., 1, 2, 3, 4, 5, 6, 7, 8, 9, or 10) 2θ angles selected from the group consisting of: 7.08±0.2^{°}, 8.00±0.2^{°}, 9.39 ± 0.2^{°}, 10.23 ± 0.2^{°}, 13.36 ± 0.2 ^{°}, 14.16 ± 0.2^{°}, 14.81 ± 0.2^{°}, 15.77 ± 0.2^{°}, 16.21±0.2^{°}, 17.77±0.2^{°}, 18.10±0.2^{°}, 18.90±0.2^{°}, 20.91±0.2^{°}, 21.29±0.2^{°}, 22.49±0.2^{°}, 23.11±0.2^{°}, 24.41±0.2^{°}, 25.51±0.2^{°}, 26.77±0.2^{°}, 28.28±0.2^{°}, 28.81±0.2^{°}, 29.22±0.2^{°}, 29.95±0.2^{°}, 30.98±0.2^{°}, 35.22±0.2^{°}.
In another preferred embodiment, the p-toluenesulfonate crystal form A has an X-ray powder diffraction pattern substantially as shown in Figure 26.

18. The crystal form according to claim 4, wherein, the crystal form is the p-toluenesulfonate crystal form B of the compound of formula I-1, the X-ray powder diffraction pattern of p-toluenesulfonate crystal form B has 2θ angles selected from the group consisting of: 13.95±0.2^{°}, 19.52 ± 0.2^{°}, and 25.40 ± 0.2^{°}.
In another preferred embodiment, the X-ray powder diffraction pattern of the p-toluenesulfonate crystal form B further has one or more (e.g., 1, 2, 3, 4, 5, 6, 7, 8, 9, or 10) 2θ angles selected from the group consisting of: 8.27 ± 0.2^{°}, 12.59 ± 0.2^{°}, 13.69 ± 0.2^{°}, 14.63 ± 0.2^{°}, 16.15 ± 0.2^{°}, 17.72 ± 0.2^{°}, 18.68 ± 0.2^{°}, 20.75 ± 0.2^{°}, 21.05±0.2^{°}, 21.92±0.2^{°}, 22.45±0.2^{°}, 23.23±0.2^{°}, 23.91±0.2^{°}, 27.33±0.2^{°}, 29.25±0.2^{°}.
In another preferred embodiment, the p-toluenesulfonate crystal form B has an X-ray powder diffraction pattern substantially as shown in Figure 27.

19. The crystal form according to claim 4, wherein, the crystal form is the methanesulfonate crystal form A of the compound of formula I-1, the X-ray powder diffraction pattern of the methanesulfonate crystal form A has 2θ angles selected from the group consisting of: 19.57±0.2^{°}, 14.27±0.2^{°}, 24.99±0.2^{°}.
In another preferred embodiment, the X-ray powder diffraction pattern of the methanesulfonate crystal form A further comprises one or more (e.g., 1, 2, 3, 4, 5, 6, 7, 8, 9, or 10) 2θ angles selected from the group consisting of: 6.70±0.2^{°}, 9.10±0.2^{°}, 9.81 ± 0.2^{°}, 10.16 ± 0.2^{°}, 13.34 ± 0.2^{°}, 13.87 ± 0.2^{°}, 15.37 ± 0.2^{°}, 15.79 ± 0.2^{°}, 17.21 ± 0.2^{°}, 18.75±0.2^{°}, 21.16±0.2^{°}, 22.09±0.2^{°}, 23.31±0.2^{°}, 23.86±0.2^{°}, 24.30±0.2^{°}, 27.18±0.2^{°}, 28.04±0.2^{°}, 28.83±0.2^{°}.
In another preferred embodiment, the methanesulfonate crystal form A has an X-ray powder diffraction pattern substantially as shown in Figure 28.

20. The crystal form according to claim 4, wherein, the crystal form is the hydrochloride crystal form C1 of the compound of Formula I-1, the X-ray powder diffraction pattern of the hydrochloride crystal form C1 has 2θ angles selected from the group consisting of: 7.73±0.2^{°}, 19.57±0.2^{°}, 19.78 ± 0.2^{°}.
In another preferred embodiment, the hydrochloride crystal form C1 further comprises one or more of the following characteristics:
a. the X-ray powder diffraction pattern of the hydrochloride crystal form C1 also has one or more (e.g., 1, 2, 3, 4, 5, 6, 7, 8, 9, or 10) 2θ angles selected from the group consisting of: 7.47±0.2^{°}, 9.63±0.2^{°}, 11.46±0.2^{°}, 12.25 ± 0.2^{°}, 13.18 ± 0.2^{°}, 13.51 ± 0.2^{°}, 14.96 ± 0.2^{°}, 15.46 ± 0.2^{°}, 16.79 ± 0.2^{°}, 17.04 ± 0.2^{°}, 17.31±0.2^{°}, 17.72±0.2^{°}, 18.56±0.2^{°}, 19.34±0.2^{°}, 20.01±0.2^{°}, 20.41±0.2^{°}, 20.76±0.2^{°}, 22.60±0.2^{°}, 23.09±0.2^{°}, 23.80±0.2^{°}, 24.36±0.2^{°}, 25.63±0.2^{°}, 26.47 ± 0.2^{°}, 27.51 ± 0.2^{°}, 29.09 ± 0.2^{°}, 30.76 ± 0.2^{°}, 31.99 ± 0.2^{°}, 36.24 ± 0.2^{°};
b. the differential scanning calorimetry curve of the hydrochloride crystal form C1 has the onsets of the endothermic peaks at 39.76^{°}C ± 2^{°}C and 153.80^{°}C ± 2^{°}C;
c. the thermogravimetric analysis curve of the hydrochloride crystal form C1 has three weight loss steps at 26.03^{°}C ± 2^{°}C, 100.00^{°}C ± 2^{°}C, and 150.00^{°}C ± 2^{°}C, and decomposition begins at 214.75^{°}C ± 2^{°}C.
In another preferred embodiment, the hydrochloride crystal form C1 has an X-ray powder diffraction pattern substantially as shown in Figure 29.
In another preferred embodiment, the hydrochloride crystal form C1 has a differential scanning calorimetry pattern substantially as shown in Figure 30.
In another preferred embodiment, the hydrochloride crystal form C1 has a thermogravimetric analysis pattern substantially as shown in Figure 31.
In another preferred embodiment, the hydrochloride crystal form C1 is an ethyl acetate solvate and hydrate crystal form, wherein the ethyl acetate content is 0.1 to 1 equivalent and the water content is 1 to 5 equivalents.

21. The crystal form according to claim 4, wherein, the crystal form is the hydrochloride crystal form C2 of the compound of formula I-1, the X-ray powder diffraction pattern of the hydrochloride crystal form C2 has 2θ angles selected from the group consisting of: 7.79±0.2^{°}, 19.66±0.2^{°}, 19.82 ± 0.2^{°}.
In another preferred embodiment, the hydrochloride crystal form C2 further comprises one or more of the following characteristics:
a. the X-ray powder diffraction pattern of the hydrochloride crystal form C2 further has one or more (e.g., 1, 2, 3, 4, 5, 6, 7, 8, 9, or 10) 2θ angles selected from the group consisting of: 7.56±0.2^{°}, 9.65±0.2^{°}, 11.48±0.2^{°}, 12.32 ± 0.2^{°}, 13.21 ± 0.2^{°}, 13.63 ± 0.2^{°}, 15.10 ± 0.2^{°}, 15.56 ± 0.2^{°}, 16.86 ± 0.2^{°}, 17.16 ± 0.2^{°}, 17.41±0.2^{°}, 17.77±0.2^{°}, 18.58±0.2^{°}, 19.36±0.2^{°}, 20.32±0.2^{°}, 20.91±0.2^{°}, 22.71±0.2^{°}, 23.21±0.2^{°}, 23.80±0.2^{°}, 24.35±0.2^{°}, 25.71±0.2^{°}, 26.56±0.2^{°}, 27.53±0.2^{°}, 27.80±0.2^{°}, 28.20±0.2^{°}, 29.15±0.2^{°}, 30.74±0.2^{°}, 32.06±0.2^{°}, 36.30±0.2^{°}, 38.22±0.2^{°};
b. the differential scanning calorimetry curve of the hydrochloride crystal form C2 has the onsets of the endothermic peaks at 33.27^{°}C ± 2^{°}C and 155.72^{°}C ± 2^{°}C;
c. the thermogravimetric analysis curve of the hydrochloride crystal form C2 has three weight loss steps at 28.19^{°}C ± 2^{°}C, 115.00^{°}C ± 2^{°}C, and 150.00^{°}C ± 2^{°}C, and decomposition begins at 212.45^{°}C ± 2^{°}C.
In another preferred embodiment, the hydrochloride crystal form C2 has an X-ray powder diffraction pattern substantially as shown in Figure 32.
In another preferred embodiment, the hydrochloride crystal form C2 has a differential scanning calorimetry pattern substantially as shown in Figure 33.
In another preferred embodiment, the hydrochloride crystal form C2 has a thermogravimetric analysis pattern substantially as shown in Figure 34.
In another preferred embodiment, the hydrochloride crystal form C2 is a 2-methyl-tetrahydrofuran solvate and hydrate crystal form, wherein the 2-methyl-tetrahydrofuran content is 0.05 to 0.5 equivalents and the water content is 1 to 5 equivalents.

22. The crystal form according to claim 4, wherein, the crystal form is the hydrochloride crystal form D of the compound of formula I-1, the X-ray powder diffraction pattern of the hydrochloride crystal form D has 2θ angles selected from the group consisting of: 15.93±0.2 ^{°}, 20.09±0.2^{°}, 20.41 ± 0.2^{°}.
In another preferred embodiment, the hydrochloride crystal form D further comprises one or more of the following characteristics:
a. the X-ray powder diffraction pattern of the hydrochloride crystal form D also has one or more (e.g., 1, 2, 3, 4, 5, 6, 7, 8, 9, or 10) 2θ angles selected from the group consisting of: 6.60±0.2^{°}, 7.26 ± 0.2 ^{°}, 7.79 ± 0.2 ^{°}, 8.48 ± 0·2 ^{°}, 9.84 ± 0.2 ^{°}, 11.18 ± 0.2 ^{°}, 13.31 ± 0.2^{°}, 13.89±0.2^{°}, 14.64±0.2^{°}, 14.98±0.2^{°}, 15.23±0.2^{°}, 16.27±0.2^{°}, 17.04±0.2^{°}, 18.21±0.2^{°}, 19.33±0.2^{°}, 21.23±0.2^{°}, 21.80±0.2^{°}, 23.49±0.2^{°}, 24.79±0.2^{°}, 28.43±0.2^{°};
b. the differential scanning calorimetry curve of the hydrochloride crystal form D has the onsets of the endothermic peaks at 55.20^{°}C ± 2^{°}C and 164.37^{°}C ± 2^{°}C,
c. the thermogravimetric analysis curve of the hydrochloride crystal form D has two weight loss steps at 36.24^{°}C ± 2^{°}C and 110.00^{°}C ± 2^{°}C, and decomposition begins at 211.35^{°}C ± 2^{°}C.
In another preferred embodiment, the hydrochloride crystal form D has an X-ray powder diffraction pattern substantially as shown in Figure 35.
In another preferred embodiment, the hydrochloride crystal form D has a differential scanning calorimetry pattern substantially as shown in Figure 36.
In another preferred embodiment, the hydrochloride crystal form D has a thermogravimetric analysis pattern substantially as shown in Figure 37.

23. The crystal form according to claim 4, wherein, the crystal form is the hydrochloride crystal form E1 of the compound of formula I-1, the X-ray powder diffraction pattern of the hydrochloride crystal form E1 has 2θ angles selected from the group consisting of: 7.90±0.2^{°}, 14.06 ± 0.2 ^{°}, 20.19 ± 0.2 ^{°}.
In another preferred embodiment, the hydrochloride crystal form E1 further comprises one or more of the following characteristics:
a. the X-ray powder diffraction pattern of the hydrochloride crystal form E1 further has one or more (e.g., 1, 2, 3, 4, 5, 6, 7, 8, 9, or 10) 2θ angles selected from the group consisting of: 6.15±0.2^{°}, 7.66±0.2^{°}, 8.41 ± 0.2^{°}, 9.49 ± 0.2^{°}, 9.91 ± 0·2^{°}, 10.88 ± 0.2^{°}, 11.42 ± 0.2^{°}, 13.06 ± 0.2^{°}, 13.66 ± 0.2^{°}, 14.69 ± 0.2 ^{°}, 15.32 ± 0.2 ^{°}, 15.52 ± 0.2 ^{°}, 15.88 ± 0·2 ^{°}, 16.25 ± 0.2 ^{°}, 16.81 ± 0.2 ^{°}, 17.35 ± 0.2^{°}, 18.37±0.2^{°}, 19.01±0.2^{°}, 19.75±0.2^{°}, 21.15±0.2^{°}, 21.51±0.2^{°}, 21.81±0.2^{°}, 22.50±0.2^{°}, 22.87±0.2^{°}, 23.56±0.2^{°}, 24.95±0.2^{°}, 25.18±0.2^{°}, 25.63±0.2^{°}, 26.71±0.2^{°}, 27.10±0.2^{°}, 27.91±0.2^{°}, 28.47±0.2^{°}, 29.30±0.2^{°}, 29.81±0.2^{°}, 30.12±0.2^{°}, 32.93±0.2^{°}, 34.96±0.2^{°};
b. the differential scanning calorimetry curve of the hydrochloride crystal form E1 has the onsets of the endothermic peaks at 13.36^{°}C ± 2^{°}C and 174.06^{°}C ± 2^{°}C;
c. The thermogravimetric analysis curve of the hydrochloride crystal form E1 has three weight loss steps at 25.81^{°}C ± 2^{°}C, 120.00^{°}C ± 2^{°}C, and 160.00^{°}C ± 2^{°}C, and decomposition begins at 211.29^{°}C ± 2^{°}C.
In another preferred embodiment, the hydrochloride crystal form E1 has an X-ray powder diffraction pattern substantially as shown in Figure 38.
In another preferred embodiment, the hydrochloride crystal form E1 has a differential scanning calorimetry pattern substantially as shown in Figure 39.
In another preferred embodiment, the hydrochloride crystal form E1 has a thermogravimetric analysis pattern substantially as shown in Figure 40.
In another preferred embodiment, the hydrochloride crystal form E1 of the compound of formula (I) is a hydrate crystal form, wherein the water content is 0.5 to 4 equivalents.

24. The crystal form according to claim 4, wherein, the crystal form is the hydrochloride crystal form E2 of the compound of formula I-1, the X-ray powder diffraction pattern of the hydrochloride crystal form E2 has 2θ angles selected from the group consisting of: 7.88±0.2^{°}, 14.14±0.2^{°}, 19.75 ± 0.2^{°}.
In another preferred embodiment, the hydrochloride crystal form E2 further comprises one or more of the following characteristics:
a. the X-ray powder diffraction pattern of the hydrochloride crystal form E2 also has one or more (e.g., 1, 2, 3, 4, 5, 6, 7, 8, 9, or 10) 2θ angles selected from the group consisting of: 9.51±0.2^{°}, 10.90±0.2^{°}, 11.49±0.2^{°}, 13.10±0.2^{°}, 13.64±0.2^{°}, 14.76±0.2^{°}, 15.39±0.2^{°}, 15.75±0.2^{°}, 16.30±0.2^{°}, 16.93±0.2^{°}, 19.01±0.2^{°}, 20.69±0.2^{°}, 21.54±0.2^{°}, 21.84±0.2^{°}, 22.19±0.2^{°}, 22.57±0.2^{°}, 23.01±0.2^{°}, 23.63±0.2^{°}, 24.96±0.2^{°}, 25.90±0.2^{°}, 26.32±0.2^{°}, 26.78±0.2^{°}, 27.19±0.2^{°}, 27.48±0.2^{°}, 27.94±0.2^{°}, 28.68±0.2^{°}, 29.07±0.2^{°}, 29.50±0.2^{°}, 29.91±0.2^{°}, 31.74±0.2^{°}, 32.96±0.2^{°}, 33.66±0.2^{°};
b. the differential scanning calorimetry curve of the hydrochloride crystal form E2 has the onsets of the endothermic peaks at 14.99^{°}C ± 2^{°}C and 174.19^{°}C ± 2^{°}C;
c. the thermogravimetric analysis curve of the hydrochloride crystal form E2 has three weight loss steps at 33.18^{°}C ± 2^{°}C, 90.00^{°}C ± 2^{°}C, and 140.00^{°}C ± 2^{°}C, and decomposition begins at 213.17^{°}C ± 2^{°}C.
In another preferred embodiment, the hydrochloride crystal form E2 has an X-ray powder diffraction pattern substantially as shown in Figure 41.
In another preferred embodiment, the hydrochloride crystal form E2 has a differential scanning calorimetry pattern substantially as shown in Figure 42.
In another preferred embodiment, the hydrochloride crystal form E2 has a thermogravimetric analysis pattern substantially as shown in Figure 43.
In another preferred embodiment, the hydrochloride crystal form E2 of the compound of formula (I) is prepared by subjecting the hydrochloride crystal form F of the compound of formula (I) to vacuum at 10^{°}C to 70^{°}C.
In another preferred embodiment, the hydrochloride crystal form E2 of the compound of formula (I) is prepared by drying the hydrochloride crystal form F of the compound of formula (I) at 80^{°}C to 120^{°}C.
In another preferred embodiment, the hydrochloride crystal form E2 of the compound of formula (I) is a hydrate crystal form, wherein the water content is 0.5 to 4 equivalents.

25. The crystal form according to claim 4, wherein, the crystal form is the hydrochloride crystal form F of the compound of Formula I-1, wherein the X-ray powder diffraction pattern of the hydrochloride crystal form F has 2θ angles selected from the group consisting of: 7.65±0.2^{°}, 11.47 ± 0.2^{°}, and 13.93 ± 0.2^{°}.
In another preferred embodiment, the hydrochloride crystal form F further comprises one or more of the following characteristics:
a. the X-ray powder diffraction pattern of the hydrochloride crystal form F also has one or more (e.g., 1, 2, 3, 4, 5, 6, 7, 8, 9, or 10) 2θ angles selected from the group consisting of: 6.85±0.2^{°}, 10.21±0.2^{°}, 10.70±0.2^{°}, 14.80 ± 0.2^{°}, 15.25 ± 0.2^{°}, 16.60 ± 0.2^{°}, 16.88 ± 0.2 ^{°}, 17.84 ± 0.2 ^{°}, 19.00 ± 0.2 ^{°}, 19.16 ± 0.2 ^{°}, 19.84 ± 0.2^{°} , 20.09 ± 0.2^{°}, 20.34 ± 0.2^{°}, 21.51±0.2^{°}, 22.35±0.2^{°}, 23.22±0.2^{°}, 23.86±0.2^{°}, 25.06±0.2^{°}, 25.31±0.2^{°}, 25.63±0.2^{°}, 26.68 ± 0.2 ^{°}, 28.10 ± 0.2 ^{°}, 28.87 ± 0.2 ^{°}, 30.53 ± 0.2 ^{°}, 32.45 ± 0.2 ^{°}, 33.59 ± 0.2^{°};
b. the differential scanning calorimetry curve of the hydrochloride crystal form F has the onsets of the endothermic peaks at 36.03^{°}C ± 2^{°}C and 172.45^{°}C ± 2^{°}C;
c. the thermogravimetric analysis curve of the hydrochloride crystal form F has three weight loss steps at 33.35^{°}C ± 2^{°}C, 100.00^{°}C ± 2^{°}C, and 150.00^{°}C ± 2^{°}C, and decomposition begins at 212.66^{°}C ± 2^{°}C.
In another preferred embodiment, the hydrochloride crystal form F has an X-ray powder diffraction pattern substantially as shown in Figure 44.
In another preferred embodiment, the hydrochloride crystal form F has a differential scanning calorimetry pattern substantially as shown in Figure 45.
In another preferred embodiment, the hydrochloride crystal form F has a thermogravimetric analysis pattern substantially as shown in Figure 46.
In another preferred embodiment, the hydrochloride crystal form F of the compound of formula (I) is a hydrate crystal form, wherein the water content is 1 to 4 equivalents.

26. The crystal form according to claim 4, wherein, the crystal form is the hydrochloride crystal form G of the compound of formula I-1, the X-ray powder diffraction pattern of the hydrochloride crystal form G has 2θ angles selected from the group consisting of: 15.52±0.2^{°}, 16.77 ± 0.2^{°}, and 23.04 ± 0.2^{°}.
In another preferred embodiment, the hydrochloride crystal form G further comprises one or more of the following characteristics:
a. the X-ray powder diffraction pattern of the hydrochloride crystal form G also has one or more (e.g., 1, 2, 3, 4, 5, 6, 7, 8, 9, or 10) 2θ angles selected from the group consisting of: 5.83±0.2^{°}, 7.18±0.2^{°}, 9.97±0.2^{°}, 11.63 ± 0.2^{°}, 12.00 ± 0.2^{°}, 12.35 ± 0.2^{°}, 13.26 ± 0.2^{°}, 13.58 ± 0.2^{°}, 14.17 ± 0.2^{°} , 14.60 ± 0.2^{°}, 17.64±0.2^{°}, 18.37±0.2^{°}, 18.81±0.2^{°}, 19.14±0.2^{°}, 19.80±0.2^{°}, 20.27±0.2^{°}, 20.48±0.2^{°}, 21.35±0.2^{°}, 21.82±0.2^{°}, 22.65±0.2^{°}, 23.35±0.2^{°}, 24.43±0.2^{°}, 25.47±0.2^{°}, 26.24±0.2^{°}, 26.54±0.2^{°}, 26.84±0.2^{°}, 27.92±0.2^{°}, 28.94±0.2^{°}, 29.31±0.2^{°}, 30.18±0.2^{°};
b. the differential scanning calorimetry curve of the hydrochloride crystal form G has the onsets of the endothermic peaks at 59.42^{°}C ± 2^{°}C, 151.02^{°}C ± 2^{°}C, and 166.01^{°}C ± 2^{°}C;
c. the thermogravimetric analysis curve of the hydrochloride crystal form G has three weight loss steps at 26.28^{°}C ± 2^{°}C, 120.00^{°}C ± 2^{°}C, and 150.00^{°}C ± 2^{°}C.
In another preferred embodiment, the hydrochloride crystal form G has an X-ray powder diffraction pattern substantially as shown in Figure 47.
In another preferred embodiment, the hydrochloride crystal form G has a differential scanning calorimetry pattern substantially as shown in Figure 48.
In another preferred embodiment, the hydrochloride crystal form G has a thermogravimetric analysis pattern substantially as shown in Figure 49.
In another preferred embodiment, the hydrochloride crystal form G of the compound of formula (I) is a hydrate crystal form, wherein the water content is 1.0 to 6 equivalents.

27. The crystal form according to claim 4, wherein, the crystal form is the hydrochloride crystal form H of the compound of formula I-1, the X-ray powder diffraction pattern of the hydrochloride crystal form H has 2θ angles selected from the group consisting of: 5.83±0.2^{°}, 16.67±0.2^{°}, 18.40 ± 0.2^{°}.
In another preferred embodiment, the hydrochloride crystal form H further comprises one or more of the following characteristics:
a. the X-ray powder diffraction pattern of the hydrochloride crystal form H further has one or more (e.g., 1, 2, 3, 4, 5, 6, 7, 8, 9, or 10) 2θ angles selected from the group consisting of: 7.16±0.2^{°}, 10.02±0.2^{°}, 11.64±0.2^{°}, 12.35 ± 0.2 ^{°}, 13.23 ± 0.2 ^{°}, 13.59 ± 0.2 ^{°}, 14.58 ± 0.2 ^{°}, 15.51 ± 0.2 ^{°}, 15.97 ± 0.2 ^{°}, 17.63 ± 0.2^{°}, 18.62±0.2^{°}, 19.11±0.2^{°}, 20.23±0.2^{°}, 20.54±0.2^{°}, 21.40±0.2^{°}, 22.07±0.2^{°}, 22.62±0.2^{°}, 22.78±0.2^{°}, 23.36±0.2^{°}, 24.73±0.2^{°}, 24.98±0.2^{°}, 26.23±0.2^{°}, 26.57±0.2^{°}, 26.91±0.2^{°}, 27.88±0.2^{°}, 29.01±0.2^{°}, 29.30±0.2^{°}, 29.61±0.2^{°}, 30.21±0.2^{°}, 31.21±0.2^{°}, 32.12±0.2^{°}, 34.66±0.2^{°};
b. the differential scanning calorimetry curve of the hydrochloride crystal form H has the onsets of the endothermic peaks at 51.49^{°}C ± 2^{°}C and 153.61^{°}C ± 2^{°}C;
c. the thermogravimetric analysis curve of the hydrochloride crystal form H has three weight loss steps at 26.12^{°}C ± 2^{°}C, 110.00^{°}C ± 2^{°}C, and 150.00^{°}C ± 2^{°}C, and decomposition begins at 211.73^{°}C ± 2^{°}C.
In another preferred embodiment, the hydrochloride crystal form H has an X-ray powder diffraction pattern substantially as shown in Figure 50.
In another preferred embodiment, the hydrochloride crystal form H has a differential scanning calorimetry pattern substantially as shown in Figure 51.
In another preferred embodiment, the hydrochloride crystal form H has a thermogravimetric analysis pattern substantially as shown in Figure 52.
In another preferred embodiment, the hydrochloride crystal form H of the compound of formula (I) is a hydrate crystal form, wherein the water content is 2 to 8 equivalents.

28. The crystal form according to claim 4, wherein, the crystal form is hydrochloride crystal form I, the X-ray powder diffraction pattern of the hydrochloride crystal form I has 2θ angles selected from the group consisting of: 6.68 ± 0.2^{°}, 13.34 ± 0.2^{°}, and 21.42 ± 0.2^{°}.
In another preferred embodiment, the hydrochloride crystal form I further comprises one or more of the following characteristics:
a. the X-ray powder diffraction pattern of the hydrochloride crystal form I also has one or more (e.g., 1, 2, 3, 4, 5, 6, 7, 8, 9, or 10) 2θ angles selected from the group consisting of: 9.50±0.2^{°}, 12.31±0.2^{°}, 12.54 ± 0.2^{°}, 14.05 ± 0.2^{°}, 14.96 ± 0.2^{°}, 15.38 ± 0.2^{°} , 17.18 ± 0.2^{°}, 17.92 ± 0.2^{°}, 19.03 ± 0.2^{°}, 19.74±0.2^{°}, 19.99±0.2^{°}, 20.96±0.2^{°}, 21.62±0.2^{°}, 21.99±0.2^{°}, 22.98±0.2^{°}, 23.52±0.2^{°}, 23.80±0.2^{°}, 24.38±0.2^{°}, 25.92±0.2^{°}, 26.81±0.2^{°}, 27.21±0.2^{°}, 27.60±0.2^{°}, 27.78±0.2^{°}, 28.26±0.2^{°}, 28.74±0.2^{°}, 30.07±0.2^{°}, 31.00±0.2^{°}, 32.69±0.2^{°}, 34.02±0.2^{°}, 34.83±0.2^{°};
b. the differential scanning calorimetry curve of the hydrochloride crystal form I has the onsets of the endothermic peaks at 18.15^{°}C ± 2^{°}C and 194.49^{°}C ± 2^{°}C;
c. the thermogravimetric analysis curve of the hydrochloride crystal form I has three weight loss steps at 28.31^{°}C ± 2^{°}C, 70.00^{°}C ± 2^{°}C, and 173.00^{°}C ± 2^{°}C, and decomposition begins at 215.51^{°}C ± 2^{°}C.
In another preferred embodiment, the hydrochloride crystal form I has an X-ray powder diffraction pattern substantially as shown in Figure 53.
In another preferred embodiment, the hydrochloride crystal form I has a differential scanning calorimetry pattern substantially as shown in Figure 54.
In another preferred embodiment, the hydrochloride crystal form I has a thermogravimetric analysis pattern substantially as shown in Figure 55.
In another preferred embodiment, the hydrochloride crystal form I of the compound of formula (I) is an anhydrous crystal form.

29. The crystal form according to claim 4, wherein, the crystal form is hydrochloride crystal form J1, the X-ray powder diffraction pattern of hydrochloride crystal form J1 comprises 2θ angles selected from the group consisting of: 6.80±0.2^{°}, 14.87±0.2^{°}, and 20.41±0.2^{°}.
In another preferred embodiment, the hydrochloride crystal form J1 further comprises one or more of the following characteristics:
a. the X-ray powder diffraction pattern of the hydrochloride crystal form J1 also has one or more (e.g., 1, 2, 3, 4, 5, 6, 7, 8, 9, or 10) 2θ angles selected from the group consisting of: 7.82±0.2^{°}, 7.95±0.2^{°}, 10.08±0.2^{°}, 11.69 ± 0.2^{°}, 12.30 ± 0.2^{°}, 12.60 ± 0.2^{°}, 13.60 ± 0.2^{°}, 15.98 ± 0.2^{°}, 16.25 ± 0.2^{°}, 18.05 ± 0.2^{°}, 18.60±0.2^{°}, 18.79±0.2^{°}, 19.49±0.2^{°}, 20.82±0.2^{°}, 21.55±0.2^{°}, 21.70±0.2^{°}, 22.06±0.2^{°}, 22.88±0.2^{°}, 23.69±0.2^{°}, 24.92±0.2^{°}, 25.13±0.2^{°}, 26.19±0.2^{°}, 26.57 ± 0.2 ^{°}, 29.07 ± 0.2 ^{°}, 30.16 ± 0.2 ^{°}, 31.85 ± 0.2 ^{°}, 32.80 ± 0.2 ^{°};
b. the differential scanning calorimetry curve of the hydrochloride crystal form J1 has the onsets of the endothermic peaks at 70.82^{°}C ± 2^{°}C and 165.48^{°}C ± 2^{°}C;
c. the thermogravimetric analysis curve of the hydrochloride crystal form J1 has two weight loss steps at 33.94^{°}C ± 2^{°}C and 110.00^{°}C ± 2^{°}C, and decomposition begins at 212.96^{°}C ± 2^{°}C.
In another preferred embodiment, the hydrochloride crystal form J1 has an X-ray powder diffraction pattern substantially as shown in Figure 56.
In another preferred embodiment, the hydrochloride crystal form J1 has a differential scanning calorimetry pattern substantially as shown in Figure 57.
In another preferred embodiment, the hydrochloride crystal form J1 has a thermogravimetric analysis pattern substantially as shown in Figure 58.
In another preferred embodiment, the hydrochloride crystal form J1 of the compound of formula (I) is a solvate of 2-methyl-tetrahydrofuran and water, wherein the content of 2-methyl-tetrahydrofuran is 0.1 to 2 equivalents and the content of water is 0.1 to 2 equivalents.

30. The crystal form according to claim 4, wherein, the crystal form is hydrochloride crystal form J2, the X-ray powder diffraction pattern of hydrochloride crystal form J2 has 2θ angles selected from the group consisting of: 14.69 ± 0.2^{°}, 18.89 ± 0.2^{°}, and 22.23 ± 0.2^{°}.
In another preferred embodiment, the hydrochloride crystal form J2 further comprises one or more of the following characteristics:
a. the X-ray powder diffraction pattern of the hydrochloride crystal form J2 also has one or more (e.g., 1, 2, 3, 4, 5, 6, 7, 8, 9, or 10) 2θ angles selected from the group consisting of: 6.78±0.2^{°}, 7.96±0.2^{°}, 8.31±0.2^{°}, 11.59 ± 0.2^{°}, 12.24 ± 0.2^{°}, 13.56 ± 0.2^{°}, 16.44 ± 0.2^{°}, 16.63 ± 0.2^{°}, 17.45 ± 0.2^{°} , 18.12 ± 0.2^{°}, 19.51±0.2^{°}, 20.12±0.2^{°}, 20.35±0.2^{°}, 20.49±0.2^{°}, 21.28±0.2^{°}, 21.46±0.2^{°}, 22.75±0.2^{°}, 23.27±0.2^{°}, 24.09±0.2^{°}, 24.86±0.2^{°}, 24.99±0.2^{°}, 25.37±0.2^{°}, 25.65±0.2^{°}, 26.54±0.2^{°}, 27.62±0.2^{°}, 28.61±0.2^{°}, 29.00±0.2^{°}, 30.43±0.2^{°}, 32.10±0.2^{°}, 33.01±0.2^{°}, 36.96±0.2^{°};
b. the differential scanning calorimetry curve of hydrochloride crystal form J2 has the onsets of the endothermic peaks at 77.60^{°}C ± 2^{°}C and 163.47^{°}C ± 2^{°}C;
c. the thermogravimetric analysis curve of the hydrochloride crystal form J2 has two weight loss steps at 28.73^{°}C ± 2^{°}C and 120.00^{°}C ± 2^{°}C, and decomposition begins at 211.78^{°}C ± 2^{°}C.
In another preferred embodiment, the hydrochloride crystal form J2 has an X-ray powder diffraction pattern substantially as shown in Figure 59.
In another preferred embodiment, the hydrochloride crystal form J2 has a differential scanning calorimetry pattern substantially as shown in Figure 60.
In another preferred embodiment, the hydrochloride crystal form J2 has a thermogravimetric analysis pattern substantially as shown in Figure 61.
In another preferred embodiment, the hydrochloride crystal form J2 of the compound of formula (I) is prepared by heating the hydrochloride crystal form J1 of the compound of formula (I) to 90^{°}C to 130^{°}C.
In another preferred embodiment, the hydrochloride crystal form J2 of the compound of formula (I) is a solvate of 2-methyl-tetrahydrofuran and water, wherein the content of 2-methyl-tetrahydrofuran is 0.2 to 0.8 equivalents and the content of water is 0.5 to 2 equivalents.

31. The crystal form according to claim 4, wherein, the crystal form is hydrochloride crystal form K, the X-ray powder diffraction pattern of the hydrochloride crystal form K has 2θ angles selected from the group consisting of: 14.93 ± 0.2^{°}, 16.08 ± 0.2^{°}, and 23.68 ± 0.2^{°}.
In another preferred embodiment, the hydrochloride crystal form K further comprises one or more of the following characteristics:
a. the X-ray powder diffraction pattern of the hydrochloride crystal form K also has one or more (e.g., 1, 2, 3, 4, 5, 6, 7, 8, 9, or 10) 2θ angles selected from the group consisting of: 6.79±0.2^{°}, 7.83 ± 0.2^{°}, 8.04 ± 0.2 ^{°}, 10.00 ± 0.2 ^{°}, 10.74 ± 0.2 ^{°}, 11.61 ± 0.2 ^{°}, 12.25 ± 0.2 ^{°}, 12.64 ± 0.2^{°}, 13.57±0.2^{°}, 18.05±0.2^{°}, 18.63±0.2^{°}, 19.43±0.2^{°}, 20.15±0.2^{°}, 20.49±0.2^{°}, 20.64±0.2^{°}, 21.52±0.2^{°}, 21.72±0.2^{°}, 21.93±0.2^{°}, 23.27±0.2^{°}, 24.73±0.2^{°}, 25.19±0.2^{°}, 26.25±0.2^{°}, 26.57±0.2^{°}, 27.42±0.2^{°}, 28.02±0.2^{°}, 28.67±0.2^{°}, 29.13±0.2^{°}, 29.91±0.2^{°}, 32.56±0.2^{°};
b. the differential scanning calorimetry curve of the hydrochloride crystal form K has the onsets of the endothermic peaks at 80.73^{°}C ± 2^{°}C and 171.74^{°}C ± 2^{°}C;
c. the thermogravimetric analysis curve of the hydrochloride crystal form K has three weight loss steps at 33.39^{°}C ± 2^{°}C, 125.00^{°}C ± 2^{°}C, and 155.00^{°}C ± 2^{°}C, and decomposition begins at 211.42^{°}C ± 2^{°}C.
In another preferred embodiment, the hydrochloride crystal form K has an X-ray powder diffraction pattern substantially as shown in Figure 62.
In another preferred embodiment, the hydrochloride crystal form K has a differential scanning calorimetry pattern substantially as shown in Figure 63.
In another preferred embodiment, the hydrochloride crystal form K has a thermogravimetric analysis pattern substantially as shown in Figure 64.
In another preferred embodiment, the hydrochloride crystal form K of the compound of formula (I) is a solvate of methyl tert-butyl ether and water, wherein the content of methyl tert-butyl ether is 0.1 to 1 equivalent and the content of water is 0.5 to 2 equivalents.

32. The crystal form according to claim 4, wherein, the crystal form is hydrochloride crystal form L, the X-ray powder diffraction pattern of hydrochloride crystal form L has 2θ angles selected from the group consisting of: 16.89 ± 0.2^{°}, 21.69 ± 0.2^{°}, and 22.60 ± 0.2^{°}.
In another preferred embodiment, the hydrochloride crystal form L further comprises one or more of the following characteristics:
a. the X-ray powder diffraction pattern of the hydrochloride crystal form L further has one or more (e.g., 1, 2, 3, 4, 5, 6, 7, 8, 9, or 10) 2θ angles selected from the group consisting of: 7.70±0.2^{°}, 8.15±0.2^{°}, 8.82±0.2^{°}, 9.85 ± 0.2 ^{°}, 11.25 ± 0.2 ^{°}, 12.72 ± 0.2 ^{°}, 13.59 ± 0.2 ^{°}, 14.68 ± 0.2 ^{°}, 15.07 ± 0.2 ^{°}, 15.56 ± 0.2^{°}, 16.60±0.2^{°}, 17.56±0.2^{°}, 18.53±0.2^{°}, 18.98±0.2^{°}, 19.74±0.2^{°}, 20.29±0.2^{°}, 20.52±0.2^{°}, 21.30±0.2^{°}, 22.79±0.2^{°}, 24.38±0.2^{°}, 25.57±0.2^{°}, 26.21±0.2^{°}, 26.50±0.2^{°}, 26.97±0.2^{°}, 27.48±0.2^{°}, 28.17±0.2^{°}, 28.60±0.2^{°}, 29.54±0.2^{°}, 29.80±0.2^{°}, 31.17±0.2^{°}, 33.28±0.2^{°};
b. the differential scanning calorimetry curve of the hydrochloride crystal form L has an onset of an endothermic peak at 180.17^{°}C ± 2^{°}C;
c. the thermogravimetric analysis curve of the hydrochloride crystal form L has two weight loss steps at 33.53^{°}C ± 2^{°}C and 140.00^{°}C ± 2^{°}C, and decomposition begins at 212.43^{°}C ± 2^{°}C.
In another preferred embodiment, the hydrochloride crystal form L has an X-ray powder diffraction pattern substantially as shown in Figure 65.
In another preferred embodiment, the hydrochloride crystal form L has a differential scanning calorimetry pattern substantially as shown in Figure 66.
In another preferred embodiment, the hydrochloride crystal form L has a thermogravimetric analysis pattern substantially as shown in Figure 67.
In another preferred embodiment, the hydrochloride crystal form L of the compound of formula (I) is a toluene solvate, wherein the toluene content is 0.2 to 1 equivalent.

33. The crystal form according to claim 4, wherein, the crystal form is hydrochloride crystal form M, the X-ray powder diffraction pattern of hydrochloride crystal form M has 2θ angles selected from the group consisting of: 14.17 ± 0.2^{°}, 20.82 ± 0.2^{°}, and 22.60 ± 0.2^{°}.
In another preferred embodiment, the hydrochloride crystal form M further comprises one or more of the following characteristics:
a. the X-ray powder diffraction pattern of the hydrochloride crystal form M also has one or more (e.g., 1, 2, 3, 4, 5, 6, 7, 8, 9, or 10) 2θ angles selected from the group consisting of: 6.84±0.2^{°}, 7.21 ± 0.2 ^{°}, 8.92 ± 0.2 ^{°}, 10.07 ± 0.2 ^{°}, 10.69 ± 0.2 ^{°}, 10.89 ± 0.2^{°}, 11.24 ± 0.2 ^{°}, 13.23 ± 0.2 ^{°}, 14.90 ± 0.2 ^{°}, 15.59 ± 0.2 ^{°}, 16.74 ± 0.2 ^{°}, 17.67 ± 0.2 ^{°}, 18.29 ± 0.2^{°}, 18.94±0.2^{°}, 19.46±0.2^{°}, 19.71±0.2^{°}, 19.96±0.2^{°}, 21.49±0.2^{°}, 21.83±0.2^{°}, 23.14±0.2^{°}, 23.54±0.2^{°}, 24.17±0.2^{°}, 25.17±0.2^{°}, 25.45±0.2^{°}, 26.10±0.2^{°}, 27.17±0.2^{°}, 27.61±0.2^{°}, 28.09±0.2^{°}, 28.88±0.2^{°}, 29.42±0.2^{°}, 30.12±0.2^{°}, 32.48±0.2^{°}, 35.22±0.2^{°};
b. the differential scanning calorimetry curve of the hydrochloride crystal form M has the onsets of the endothermic peaks at 31.74^{°}C ± 2^{°}C and 150.05^{°}C ± 2^{°}C;
c. the thermogravimetric analysis curve of the hydrochloride crystal form M has two weight loss steps at 32.98^{°}C ± 2^{°}C and 115.00^{°}C ± 2^{°}C, and decomposition begins at 215.09^{°}C ± 2^{°}C.
In another preferred embodiment, the hydrochloride crystal form M has an X-ray powder diffraction pattern substantially as shown in Figure 68.
In another preferred embodiment, the hydrochloride crystal form M has a differential scanning calorimetry pattern substantially as shown in Figure 69.
In another preferred embodiment, the hydrochloride crystal form M has a thermogravimetric analysis pattern substantially as shown in Figure 70.
In another preferred embodiment, the hydrochloride crystal form M of the compound of formula (I) is a solvate of dimethyl sulfoxide and water, wherein the content of dimethyl sulfoxide is 1 to 7 equivalents and the content of water is 5 to 25 equivalents.

34. The crystal form according to claim 4, wherein, the crystal form is hydrochloride crystal form N, the X-ray powder diffraction pattern of hydrochloride crystal form N has 2θ angles selected from the group consisting of: 16.19 ± 0.2^{°}, 20.10 ± 0.2^{°}, and 28.42 ± 0.2^{°}.
In another preferred embodiment, the hydrochloride crystal form N further comprises one or more of the following characteristics:
a. the X-ray powder diffraction pattern of the hydrochloride crystal form N also has one or more (e.g., 1, 2, 3, 4, 5, 6, 7, 8, 9, or 10) 2θ angles selected from the group consisting of: 6.61±0.2^{°}, 7.80±0.2^{°}, 9.75±0.2^{°}, 11.19 ± 0.2^{°}, 12.53 ± 0.2^{°}, 13.17 ± 0.2^{°}, 14.61 ± 0.2^{°}, 15.24 ± 0.2^{°}, 16.02 ± 0.2^{°}, 18.51 ± 0.2^{°}, 19.36 ± 0.2^{°}, 21.57 ± 0.2^{°}, 21.95 ± 0.2^{°}, 23.47 ± 0.2^{°}, 25.60 ± 0.2^{°}, 27.32 ± 0.2^{°}, 29.12 ± 0.2^{°};
b. the differential scanning calorimetry curve of the hydrochloride crystal form N has the onsets of the endothermic peaks at 47.38^{°}C ± 2^{°}C and 167.91^{°}C ± 2^{°}C;
c. the thermogravimetric analysis curve of the hydrochloride crystal form N has three weight loss steps at 33.22^{°}C ± 2^{°}C, 75.00^{°}C ± 2^{°}C, and 150.00^{°}C ± 2^{°}C, and decomposition begins at 212.57^{°}C ± 2^{°}C.
In another preferred embodiment, the hydrochloride crystal form N has an X-ray powder diffraction pattern substantially as shown in Figure 71.
In another preferred embodiment, the hydrochloride crystal form N has a differential scanning calorimetry pattern substantially as shown in Figure 72.
In another preferred embodiment, the hydrochloride crystal form N has a thermogravimetric analysis pattern substantially as shown in Figure 73.
In another preferred embodiment, the hydrochloride crystal form N of the compound of formula (I) is a hydrate crystal form, wherein the water content is 1 to 6 equivalents.

35. The crystal form according to claim 4, wherein, the crystal form is hydrochloride crystal form O, the X-ray powder diffraction pattern of hydrochloride crystal form O has 2θ angles selected from the group consisting of: 15.51 ± 0.2^{°}, 18.36 ± 0.2^{°}, and 20.17 ± 0.2^{°}.
In another preferred embodiment, the hydrochloride crystal form O further comprises one or more of the following characteristics:
a. the X-ray powder diffraction pattern of the hydrochloride crystal form O also has one or more (e.g., 1, 2, 3, 4, 5, 6, 7, 8, 9, or 10) 2θ angles selected from the group consisting of: 6.14±0.2^{°}, 8.39±0.2^{°}, 9.90±0.2^{°}, 11.28 ± 0.2 ^{°}, 13.19 ± 0.2 ^{°}, 13.54 ± 0.2 ^{°}, 14.40 ± 0.2 ^{°}, 15.33 ± 0.2 ^{°}, 15.87 ± 0.2 ^{°}, 16.79 ± 0.2^{°}, 17.32±0.2^{°}, 19.03±0.2^{°}, 19.38±0.2^{°}, 19.84±0.2^{°}, 20.39±0.2^{°}, 21.14±0.2^{°}, 21.82±0.2^{°}, 22.51±0.2^{°}, 22.84±0.2^{°}, 23.77±0.2^{°}, 24.59±0.2^{°}, 25.28±0.2^{°}, 26.21±0.2^{°}, 26.82±0.2^{°}, 27.07±0.2^{°}, 27.93±0.2^{°}, 28.45±0.2^{°}, 29.24±0.2^{°}, 29.70±0.2^{°}, 30.12±0.2^{°}, 30.91±0.2^{°}, 31.32±0.2^{°}, 32.02±0.2^{°}, 32.81±0.2^{°}, 33.99±0.2^{°}, 35.51±0.2^{°}, 37.27±0.2^{°};
b. the differential scanning calorimetry curve of the hydrochloride crystal form O has the onsets of the endothermic peaks at 12.36^{°}C ± 2^{°}C and 174.87^{°}C ± 2^{°}C;
c. the thermogravimetric analysis curve of the hydrochloride crystal form O has three weight loss steps at 27.70^{°}C ± 2^{°}C, 115.00^{°}C ± 2^{°}C, and 160.00^{°}C ± 2^{°}C, and decomposition begins at 212.00^{°}C ± 2^{°}C.
In another preferred embodiment, the hydrochloride crystal form O has an X-ray powder diffraction pattern substantially as shown in Figure 74.
In another preferred embodiment, the hydrochloride crystal form O has a differential scanning calorimetry pattern substantially as shown in Figure 75.
In another preferred embodiment, the hydrochloride crystal form O has a thermogravimetric analysis pattern substantially as shown in Figure 76.
In another preferred embodiment, the hydrochloride crystal form O of the compound of formula (I) is a hydrate crystal form, wherein the water content is 0.4 to 2 equivalents.

36. A method for preparing a crystal form of a pharmaceutically acceptable salt of the compound of formula (I) according to any one of claims 5-35, wherein, the method comprises the following steps:
a. dissolving or dispersing the compound of formula (I) and an acid at a molar ratio of 1: (0.8-3) into 1-20 times of solvent A;
b. performing suspension, recrystallization, or slurrying treatment;
wherein the acid is not selected from hydrochloric acid (preferably, the acid is selected from the group consisting of: maleic acid, fumaric acid, glycolic acid, L-malic acid, succinic acid, sulfuric acid, L-tartaric acid, hippuric acid, glutaric acid, p-toluenesulfonic acid, or methanesulfonic acid);
wherein solvent A is selected from the group consisting of: acetonitrile, dichloromethane, tetrahydrofuran, or a combination thereof;
or the acid is hydrochloric acid or hydrogen chloride in dioxane; and the solvent A is selected from the group consisting of: ethyl acetate, 2-methyl-4-hydroxyfuran, butanone, ethanol, acetone, ethyl acetate, acetonitrile, tetrahydrofuran, methyl isobutyl ketone, trifluoroethanol, water, methanol, tert-butyl methyl ether, toluene, isopropanol, dimethyl sulfoxide, isopropyl acetate, dichloromethane, or combinations thereof.
In another preferred embodiment, the solvent A is selected from the group consisting of: methyl isobutyl ketone/trifluoroethanol, methanol/water, methanol/tert-butyl methyl ether, isopropanol/dimethyl sulfoxide, isopropyl acetate/dimethyl sulfoxide, and water/dimethyl sulfoxide.
In another preferred embodiment, the suspension treatment comprises the following steps:
a. suspending the compound of formula (I), acid, and solvent at 45-65^{°}C for 1-3 h;
b. allowing the mixture to cool naturally to 20-30^{°}C and continuing performing the suspension for at least 48 h;
c. centrifuging the resulting suspension through a 0.4-0.5 µm filter membrane at a speed of 12,000-16,000 rpm;
d. vacuum-drying the resulting solid at 45-65^{°}C.

37. A method for preparing a crystal form of a pharmaceutically acceptable salt of the compound of formula (I) according to any one of claims 5-36, wherein, the method is a crystal form transformation by converting one crystal form of a pharmaceutically acceptable salt of the compound of formula (I) into another crystal form thereof;
wherein the method of crystal form transformation comprises the following steps:
a. dissolving or dispersing a crystal form of a pharmaceutically acceptable salt of the compound of formula (I) in 1-20 times of solvent A;
b. performing suspension treatment;
c. centrifuging the resulting suspension through a 0.4 to 0.5 µm filter membrane at a speed of 12,000 to 16,000 rpm;
wherein the solvent A is selected from the group consisting of: ethanol, acetone, ethyl acetate, acetonitrile, tetrahydrofuran, methyl isobutyl ketone, trifluoroethanol, methanol, 2-methyl-tetrahydrofuran, methyl tert-butyl ether, toluene, isopropanol, dimethyl sulfoxide, isopropyl acetate, dimethyl sulfoxide, methanol, dichloromethane, water, or a combination thereof.
In another preferred embodiment, the solvent A is selected from the group consisting of: methyl isobutyl ketone/trifluoroethanol, methanol/water, isopropanol/dimethyl sulfoxide, isopropyl acetate/dimethyl sulfoxide, and dimethyl sulfoxide/water.
In another preferred embodiment, the suspension treatment comprises the step of stirring at a rate of 300-400 rpm at 20 - 30^{°}C.
In another preferred embodiment, the suspension treatment comprises the step of stirring at a rate of 300-400 rpm at 40 - 60^{°}C.
In another preferred embodiment, the suspension treatment comprises the step of performing 8 to 12 heating-cooling cycles at a rate of 0.05 to 0.2^{°}C/min between 5 and 50^{°}C while stirring at a rate of 300 to 400 rpm, with the final temperature of the suspension being 10 to 15^{°}C.
In another preferred embodiment, the suspension treatment comprises the step of filtering through a 0.4-0.5 µm filter membrane to obtain a clear solution, followed by performing suspension treatment after adding methyl tert-butyl ether at a ratio of 1: (8-12).

38. A pharmaceutical composition comprising the compound of formula (I) according to any one of claims 1-2, the crystal form A according to any one of claims 3-4, or the crystal form of the compound of formula (I) according to any one of claims 5-36;
and
one or more pharmaceutically acceptable carriers, excipients, adjuvants, ingredients, and/or diluents.
In another preferred embodiment, the pharmaceutical composition further comprises other therapeutic agents.
In another preferred embodiment, the other therapeutic agents are selected from the group consisting of: chemotherapeutic agents, kinase inhibitors, targeted epigenetic modulators, antibody drugs, immune checkpoint inhibitors, or combinations thereof. In another preferred embodiment, the chemotherapeutic agent is selected from the group consisting of: cisplatin, doxorubicin, paclitaxel, etoposide, irinotecan, cyclophosphamide, gemcitabine, ifosfamide, tamoxifen, toremifene, fulvestrant, anastrozole, exemestane, goserelin, leuprorelin, melphalan, chlorambucil, busulfan, floxuridine, cytarabine, oxaliplatin, leucovorin, pentostatin, and diethylstilbestrol.
In another preferred embodiment, the kinase inhibitor is selected from the group consisting of: Akt, TGF-βR, Pim, PKA, PKG, PKC, CaM kinase, CDK2, CDK4, CDK4/6, MEK, ERK, MAPK, mTOR, EGFR, HER2, HER3, HER4, PDGFαR, PDGFβR, CSFIR, KIT, c-Met, TRKA, TRKB, TRKC, FLT3, VEGFR, BTK, FAK, SYK, FRK, JAK, HPK1, AXL, ALK, and B-Raf inhibitors. In another preferred embodiment, the targeted epigenetic modulator is selected from the group consisting of: bromodomain inhibitors, histone lysine methyltransferases, histone arginine methyltransferases, histone demethylases, histone deacetylases, histone acetylases, and DNA methyltransferases. In another preferred embodiment, the antibody drug is selected from the group consisting of: anti-HER2 antibodies, anti-VEGFR antibodies, anti-EGFR antibodies, anti-c-MET antibodies, and anti-CD20 antibodies.
In another preferred embodiment, the immune checkpoint inhibitor is selected from the group consisting of: CD27, CD28, CD40, CD122, CD96, CD73, CD47, OX40, GITR, A2AR, B7-H3, B7-H4, BTLA, CTLA-4, LAG3, TIM3, VISTA, PD-1, PD-L1, and PD-L2.

39. A use of the compound of formula (I) according to any one of claims 1-2, the crystal form A according to any one of claims 3-4, the crystal forms of the compound of formula (I) according to any one of claims 5-36, and the pharmaceutical composition according to claim 38 in preparing a medicament for the prevention and/or treatment of diseases associated with increased activity or expression of FGFR2.
In another preferred embodiment, the increased activity or expression of FGFR2 is selected from the group consisting of: FGFR2 amplification, FGFR2 gene mutation, FGFR2 gene fusion/rearrangement, FGFR2 gene translocation, and FGFR2 gene activation.
In another preferred embodiment, the diseases associated with increased FGFR2 activity or expression are selected from the group consisting of: cholangiocarcinoma, liver cancer, breast cancer, prostate cancer, lung cancer, thyroid cancer, gastric cancer, ovarian cancer, esophageal cancer, pancreatic cancer, cervical cancer, colorectal cancer, salivary gland cancer, endometrial cancer, and urothelial cancer, and the like.
In another preferred embodiment, the cholangiocarcinoma is intrahepatic cholangiocarcinoma.
In another preferred embodiment, the liver cancer is hepatocellular carcinoma.
In another preferred embodiment, the lung cancer is lung squamous cell carcinoma or non-small cell lung cancer.
